# EUROPEAN PATENT APPLICATION

(11) **EP 4 265 613 A1**
(43) Date of publication of application: **25.10.2023**
(21) Application number: 21905845.0
(22) Date of filing: 17.12.2021
(51) Int. Cl.: C07D 471/04, C07D 213/70, C07D 401/04, C07C 237/40, C07D 277/42, C07D 233/60, C07D 401/06, C07D 239/42, C07D 213/75, A61P 35/00, A61P 35/02, C12Q 1/6886, C12Q 1/02

(54) **BENZENE RING COMPOUND AND USE THEREOF**

(30) Priority: 18.12.2020 CN 202011511391
(71) Applicant: Nanjing Shijiang Medicine Technology Co., Ltd, Nanjing, Jiangsu 200120 (CN)
(72) Inventor: SHI, Yufeng, Nanjing, Jiangsu 200120 (CN); MA, Wenjiang, Nanjing, Jiangsu 200120 (CN)
(74) Representative: reuteler & cie SA
(86) International application number: PCT/CN2021/139355
(87) International publication number: WO 2022/127923

(57) **Abstract**

The present invention relates to a benzene ring compound and a use thereof. In particular, provided is a compound as shown in formula I, or an optical isomer thereof or a racemate thereof, or a solvate thereof, or a pharmaceutically acceptable salt thereof. The present compound has good therapeutic effects on tumors

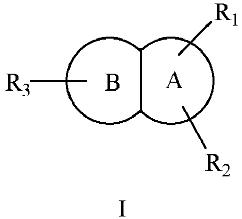

## Description

### FIELD OF THE INVENTION

The present invention relates to the field of medicine. Specifically, the present invention relates to benzene ring compound and use thereof.

### BACKGROUND TECHNOLOGY

Tumor is a common disease that seriously endangers human health, and the mortality rate of malignant tumors is rising. Due to the heterogeneity of tumor and individual difference in patient, simply using the same treatment method or medicine based on source or pathological characteristic of tumor can easily cause improper treatment, which can delay valuable treatment time and opportunities for patient. Therefore, it is very necessary to take personalized treatment according to the different situations of patient. With the development of biological technology, the treatment of tumor has entered the era of precision treatment, and more and more changes in tumor-related gene expression have been discovered. Changes in related genes play an important role in the development of malignant tumor. The discovery and application of biomarkers can provide precise guidance for the application of related drug and make personalized treatment on tumor possible, thereby achieving targeted administration of drug and significantly improving treatment effect.

Therefore, there is an urgent need in the art to develop a drug that can have precise treatment on tumor.

### SUMMARY OF THE INVENTION

It is an object of the present invention to provide a compound that can safely and effectively inhibit cancer and use thereof.

It is another object of the present invention to provide a marker for determining whether the compound of present invention is suitable for use in the prevention and/or treatment of patient tumor, the marker is mitochondrial oxidative phosphorylation pathway, mitochondria permeability transition pore, NNMT gene, and/or the methylation level of DNA CpG site of NNMT gene. The compound of present invention has more significant treatment effect on tumor with up-regulation of mitochondrial oxidative phosphorylation pathway, low activity of mitochondria permeability transition pore, low or no expression of NNMT gene, and/or high methylation level of DNA CpG site of NNMT gene.

In the first aspect of the present invention, it provides a compound of formula I, or an optical isomer thereof, or a racemate thereof, or a solvate thereof, or a pharmaceutically acceptable salt thereof 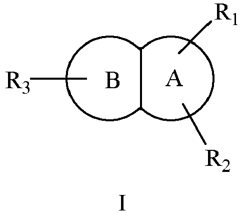 wherein,
ring A is substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring;
ring B is absent, substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring;
R₁ is 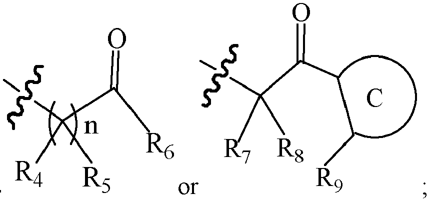
R₂ is hydrogen, 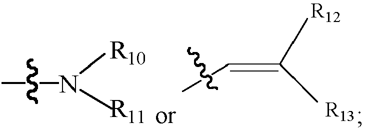
R₃ is none, hydrogen, halogen, -CN, hydroxyl, sulfhydryl, nitro, amino, F₃C-, F₃C-O-, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, substituted or unsubstituted 5-12 membered heteroaryl, substituted or unsubstituted C6-C16 aryl-substituted or unsubstituted C1-C10 alkyl-, substituted or unsubstituted 5-12 membered heteroaryl-substituted or unsubstituted C1-C10 alkyl;
R₄ and R₅ are each independently hydrogen, halogen, -CN, hydroxyl, sulfhydryl, nitro, amino, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, or substituted or unsubstituted 5-12 membered heteroaryl;
R₆ is substituted or unsubstituted C6-C16 aryl, substituted or unsubstituted 5-12 membered heteroaryl, substituted or unsubstituted 3-16 membered cycloalkyl, substituted or unsubstituted 3-16 membered heterocycloalkyl;
n is 0, 1, 2, 3, 4, 5, or 6;
ring C is absent, substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring;
R₇ is hydrogen, halogen, -CN, hydroxyl, sulfhydryl, nitro, amino, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, or substituted or unsubstituted 5-12 membered heteroaryl;
R₈ and R₉ are connected to form substituted or unsubstituted 3-16 membered cycloalkane ring, substituted or unsubstituted 3-16 membered heterocycloalkane ring;
R₁₀ and R₁₁ are each independently hydrogen, halogen, -CN, hydroxyl, sulfhydryl, nitro, amino, F₃C-O-, F₃C-, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, substituted or unsubstituted 5-12 membered heteroaryl, substituted or unsubstituted C6-C16 aryl-substituted or unsubstituted C1-C10 alkyl-, substituted or unsubstituted 5-12 membered heteroaryl-substituted or unsubstituted C1-C10 alkyl-, substituted or unsubstituted C1-C6 alkyl-C(O)-; or R₁₀ and R₁₁ are connected to form substituted or unsubstituted 3-16 membered heterocycloalkane ring, substituted or unsubstituted 3-16 membered heteroaromatic ring;
R₁₂ and R₁₃ are independently hydrogen, halogen, -CN, hydroxyl, sulfhydryl, nitro, amino, F₃C-, F₃C-O-, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, substituted or unsubstituted 5-12 membered heteroaryl, substituted or unsubstituted C6-C16 aryl-substituted or unsubstituted C1-C10 alkyl-, substituted or unsubstituted 5-12 membered heteroaryl-substituted or unsubstituted C1-C10 alkyl-;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl;
the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

In another preferred embodiment, each "substituted" means that one or more (preferably 1, 2, 3, 4, 5, 6, 7, or 8) hydrogen atoms on the ring or group are substituted by a substituent selected from the group consisting of C1-C6 alkyl, C3-C8 cycloalkyl, C1-C6 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C6 alkoxyl, C1-C6 alkylthio, C1-C6 haloalkoxyl, C1-C6 haloalkylthio, C6-C12 aryl, 5-10 membered heteroaryl, 5-10 membered heterocycloalkyl.

In another preferred embodiment, each "substituted" means that one or more (preferably 1, 2, 3, 4, 5, 6, 7, or 8) hydrogen atoms on the ring or group are substituted by a substituent selected from the group consisting of C1-C4 alkyl, C3-C8 cycloalkyl, C1-C4 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C4 alkoxyl, C1-C4 alkylthio, C1-C4 haloalkoxyl, C1-C4 haloalkylthio, C6-C12 aryl, 5-10 membered heteroaryl, 5-10 membered heterocycloalkyl.

In another preferred embodiment, the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

In another preferred embodiment, the halogen is F, Cl, Br, or I.

In another preferred embodiment, the cycloalkane ring has 1, 2, or 3 C=C ring double bonds.

In another preferred embodiment, the heterocycloalkane ring has 1, 2, or 3 C=C ring double bonds.

In another preferred embodiment, the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

In another preferred embodiment, ring A is substituted or unsubstituted C6-C12 aromatic ring, substituted or unsubstituted C3-C12 cycloalkane ring, substituted or unsubstituted 3-12 membered heterocycloalkane ring, or substituted or unsubstituted 3-12 membered heteroaromatic ring.

In another preferred embodiment, ring A is substituted or unsubstituted C6-C10 aromatic ring, substituted or unsubstituted C3-C10 cycloalkane ring, substituted or unsubstituted 3-10 membered heterocycloalkane ring, or substituted or unsubstituted 3-10 membered heteroaromatic ring.

In another preferred embodiment, ring A is substituted or unsubstituted C6-C8 aromatic ring, substituted or unsubstituted C3-C8 cycloalkane ring, substituted or unsubstituted 3-8 membered heterocycloalkane ring, or substituted or unsubstituted 5-8 membered heteroaromatic ring.

In another preferred embodiment, ring A is absent, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring.

In another preferred embodiment, ring A is substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring.

In another preferred embodiment, ring A is substituted or unsubstituted pyridine ring, substituted or unsubstituted pyrimidine ring, substituted or unsubstituted benzene ring, substituted or unsubstituted naphthalene ring, substituted or unsubstituted thiazole ring, substituted or unsubstituted imidazole ring, substituted or unsubstituted pyrrole ring.

In another preferred embodiment, ring A is pyridine ring, pyrimidine ring, benzene ring, naphthalene ring, thiazole ring, imidazole ring.

In another preferred embodiment, R₁ is connected to the heteroatom on the ring of ring A.

In another preferred embodiment, R₁ is connected to the N, O, or S atom on the ring of ring A.

In another preferred embodiment, R₁ is connected to the N atom on the ring of ring A.

In another preferred embodiment, R₂ is connected to the carbon atom on the ring of ring A.

In another preferred embodiment, ring B is absent, substituted or unsubstituted C6-C12 aromatic ring, substituted or unsubstituted C3-C12 cycloalkane ring, substituted or unsubstituted 3-12 membered heterocycloalkane ring, or substituted or unsubstituted 3-12 membered heteroaromatic ring.

In another preferred embodiment, ring B is absent, substituted or unsubstituted C6-C10 aromatic ring, substituted or unsubstituted C3-C10 cycloalkane ring, substituted or unsubstituted 3-10 membered heterocycloalkane ring, or substituted or unsubstituted 3-10 membered heteroaromatic ring.

In another preferred embodiment, ring B is absent, substituted or unsubstituted C6-C8 aromatic ring, substituted or unsubstituted C5-C8 cycloalkane ring, substituted or unsubstituted 5-8 membered heterocycloalkane ring, or substituted or unsubstituted 5-8 membered heteroaromatic ring.

In another preferred embodiment, ring B is absent, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring.

In another preferred embodiment, the ring of heterocycloalkane ring has 1, 2, or 3 C=C ring double bonds.

In another preferred embodiment, ring B is absent, substituted or unsubstituted tetrahydropyridine ring, substituted or unsubstituted pyrrole ring, substituted or unsubstituted dihydropyrrole ring, substituted or unsubstituted imidazole ring, or substituted or unsubstituted pyrazole ring.

In another preferred embodiment, ring B is absent, tetrahydropyridine ring, piperidine ring, pyrrole ring, dihydropyrrole ring, tetrahydropyrrole ring, imidazole ring, pyrazole ring, pyridine ring, 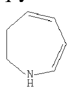 ring, 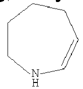 ring.

In another preferred embodiment, R₃ is connected to the carbon atom on the ring of ring B.

In another preferred embodiment, R₃ is connected to the heteroatom atom on the ring of ring B.

In another preferred embodiment, R₃ is connected to the N, O, or S atom on the ring of ring B.

In another preferred embodiment, R₃ is connected to the N atom on the ring of ring B.

In another preferred embodiment, the connected mode of 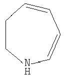 to ring A is

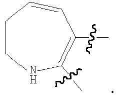

In another preferred embodiment, the connected mode of 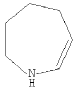 to ring A is

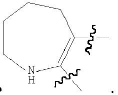

In another preferred embodiment, the tetrahydropyridine ring is 1,2,3,4-tetrahydropyridine ring.

In another preferred embodiment, the pyrrole ring is 1-hydropyrrole ring.

In another preferred embodiment, the dihydropyrrole ring is 2,3-dihydropyrrole ring.

In another preferred embodiment, ring B is absent.

In the present invention, it should be understood that when the ring B is absent, the compound of formula I has the following structure of formula 1-1:

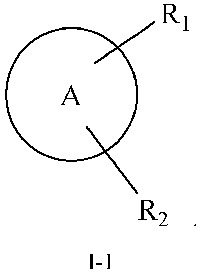

In another preferred embodiment, the compound of formula I has the following structure of formula I-1:

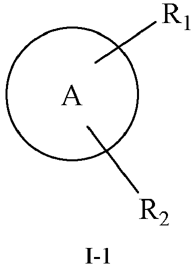

In another preferred embodiment, ring B is absent, R₂ is

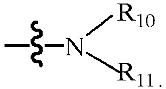

In another preferred embodiment, ring B is substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring, R₂ is hydrogen.

In another preferred embodiment, R₁ is

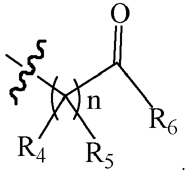

In another preferred embodiment, R₁ is

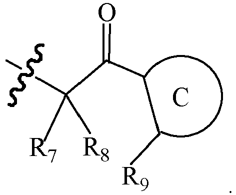

In another preferred embodiment, R₂ is hydrogen,

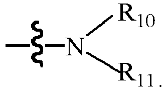

In another preferred embodiment, R₂ is hydrogen,

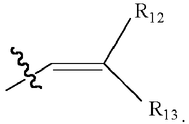

In another preferred embodiment, R₃ is hydrogen, halogen, -CN, hydroxyl, sulfhydryl, nitro, amino, F₃C-O-, F₃C-, substituted or unsubstituted C1-C8 alkyl, substituted or unsubstituted C3-C10 cycloalkyl, substituted or unsubstituted C1-C8 alkoxyl, substituted or unsubstituted C1-C8 alkylthio, substituted or unsubstituted C6-C12 aryl, substituted or unsubstituted 5-10 membered heteroaryl, substituted or unsubstituted C6-C12 aryl-substituted or unsubstituted C1-C8 alkyl-, substituted or unsubstituted 5-10 membered heteroaryl-substituted or unsubstituted C1-C8 alkyl-.

In another preferred embodiment, R₃ is hydrogen, halogen, -CN, hydroxyl, sulfhydryl, nitro, amino, F₃C-O-, F₃C-, substituted or unsubstituted C1-C6 alkyl, substituted or unsubstituted C3-C8 cycloalkyl, substituted or unsubstituted C1-C6 alkoxyl, substituted or unsubstituted C1-C6 alkylthio, substituted or unsubstituted C6-C10 aryl, substituted or unsubstituted 5-10 membered heteroaryl, substituted or unsubstituted C6-C10 aryl-substituted or unsubstituted C1-C4 alkyl-, substituted or unsubstituted 5-10 membered heteroaryl-substituted or unsubstituted C1-C4 alkyl-.

In another preferred embodiment, R₃ is hydrogen, halogen, -CN, hydroxyl, sulfhydryl, nitro, amino, F₃C-O-, F₃C-, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted C6-C8 aryl, substituted or unsubstituted 5-8 membered heteroaryl, substituted or unsubstituted C6-C8 aryl-substituted or unsubstituted C1-C2 alkyl-, substituted or unsubstituted 5-8 membered heteroaryl-substituted or unsubstituted C1-C2 alkyl-.

In another preferred embodiment, R₃ is hydrogen, substituted or unsubstituted C1-C6 alkyl, substituted or unsubstituted C3-C6 cycloalkyl, substituted or unsubstituted phenyl, substituted or unsubstituted phenyl-methyl-, substituted or unsubstituted pyridyl, substituted or unsubstituted pyridyl-methyl-, substituted or unsubstituted phenyl-ethyl-, substituted or unsubstituted pyridinyl-ethyl-.

In another preferred embodiment, R₃ is hydrogen, substituted or unsubstituted C1-C4 alkyl, substituted or unsubstituted C3-C6 cycloalkyl, substituted or unsubstituted phenyl, substituted or unsubstituted phenyl-methyl-, substituted or unsubstituted pyridyl, substituted or unsubstituted pyridyl-methyl-, substituted or unsubstituted phenyl-ethyl-, substituted or unsubstituted pyridinyl-ethyl-.

In another preferred embodiment, R₃ is hydrogen, substituted or unsubstituted methyl, substituted or unsubstituted ethyl, substituted or unsubstituted propyl, substituted or unsubstituted cyclopropyl, substituted or unsubstituted phenyl, substituted or unsubstituted phenyl-methyl-, substituted or unsubstituted pyridyl, substituted or unsubstituted pyridyl-methyl-, substituted or unsubstituted phenyl-ethyl-, substituted or unsubstituted pyridinyl-ethyl-.

In another preferred embodiment, in the substituted or unsubstituted phenyl, the substituted or unsubstituted phenyl is monosubstituted or unsubstituted phenyl.

In another preferred embodiment, in the substituted or unsubstituted phenyl, the substituted or unsubstituted phenyl is monosubstituted or unsubstituted phenyl, and the substitution is ortho, para or meta substitution on the phenyl.

In another preferred embodiment, in the substituted or unsubstituted phenyl, the substituted or unsubstituted phenyl is monosubstituted or unsubstituted phenyl, and the substitution is ortho, para or meta substitution on the phenyl, the substituent is halogen (e.g., Cl ), nitro, amino, C1-C4 alkyl (e.g., methyl), C1-C4 alkoxyl, C1-C4 alkylthio, F₃C -, F₃C-O -.

In another preferred embodiment, in the substituted or unsubstituted phenyl-methyl-, the substituted or unsubstituted phenyl is monosubstituted or unsubstituted phenyl.

In another preferred embodiment, in the substituted or unsubstituted phenyl-methyl-, the substituted or unsubstituted phenyl is monosubstituted or unsubstituted phenyl, and the substitution is ortho, para or meta substitution on the phenyl.

In another preferred embodiment, in the substituted or unsubstituted phenyl-methyl-, the substituted or unsubstituted phenyl is monosubstituted or unsubstituted phenyl, and the substitution is ortho, para or meta substitution on the phenyl, the substituent is halogen (e.g., Cl), nitro, amino, C1-C4 alkyl (e.g., methyl), C1-C4 alkoxyl, C1-C4 alkylthio, F₃C -, F₃C-O -.

In another preferred embodiment, the substituted phenyl is monosubstituted phenyl.

In another preferred embodiment, R₄ and R₅ are each independently hydrogen, halogen, -CN, hydroxyl, sulfhydryl, nitro, amino, substituted or unsubstituted C1-C8 alkyl, substituted or unsubstituted C3-C10 cycloalkyl, substituted or unsubstituted C1-C8 alkoxyl, substituted or unsubstituted C1-C8 alkylthio, substituted or unsubstituted C6-C12 aryl, or substituted or unsubstituted 5-10 membered heteroaryl.

In another preferred embodiment, R₄ and R₅ are each independently hydrogen, halogen, -CN, hydroxyl, sulfhydryl, nitro, amino, substituted or unsubstituted C1-C6 alkyl, substituted or unsubstituted C3-C10 cycloalkyl, substituted or unsubstituted C1-C8 alkoxyl, substituted or unsubstituted C1-C8 alkylthio, substituted or unsubstituted C6-C12 aryl, or substituted or unsubstituted 5-10 membered heteroaryl.

In another preferred embodiment, R₄ and R₅ are each independently hydrogen, halogen, -CN, hydroxyl, sulfhydryl, nitro, amino, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted C6-C8 aryl, or substituted or unsubstituted 5-8 membered heteroaryl.

In another preferred embodiment, R₄ and R₅ are each independently hydrogen, methyl, ethyl, propyl, or butyl.

In another preferred embodiment, n is 0 1 2, 3 4, 5, or 6

In the present invention, it should be understood that when n is 0, 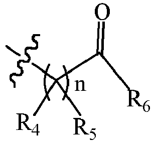 is

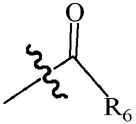

In the present invention, R₆ is substituted or unsubstituted C6-C12 aryl, substituted or unsubstituted 5-12 membered heteroaryl, substituted or unsubstituted 3-12 membered cycloalkyl, substituted or unsubstituted 3-12 membered heterocycloalkyl.

In the present invention, R₆ is substituted or unsubstituted C6-C10 aryl, substituted or unsubstituted 5-10 membered heteroaryl, substituted or unsubstituted 3-8 membered cycloalkyl, substituted or unsubstituted 3-8 membered heterocycloalkyl.

In the present invention, R₆ is substituted or unsubstituted C6-C8 aryl, substituted or unsubstituted 5-8 membered heteroaryl, substituted or unsubstituted 3-8 membered cycloalkyl, substituted or unsubstituted 3-8 membered heterocycloalkyl.

In another preferred embodiment, R₆ is substituted or unsubstituted phenyl, substituted or unsubstituted pyridyl, or substituted or unsubstituted pyrazinyl.

In another preferred embodiment, the substituted or unsubstituted phenyl is monosubstituted or unsubstituted phenyl.

In another preferred embodiment, the substituted or unsubstituted phenyl is monosubstituted or unsubstituted phenyl, and the substitution is ortho, para or meta substitution on the phenyl.

In another preferred embodiment, the substituted or unsubstituted phenyl is monosubstituted or unsubstituted phenyl, the substitution is ortho, para or meta substitution on the phenyl, the substituent is halogen, nitro, amino, C1-C4 alkyl, C1-C4 alkoxyl, C1-C4 alkylthio, F₃C -, F₃C-O -, C6-C12 aryl, 5-10 membered heteroaryl.

In another preferred embodiment, the substituted or unsubstituted phenyl is monosubstituted or unsubstituted phenyl, the substitution is ortho, para or meta substitution on the phenyl, the substituent is halogen, nitro, amino, C1-C4 alkyl, C1-C4 alkoxyl, C1-C4 alkylthio, F₃C -, F₃C-O -, phenyl.

In another preferred embodiment, the substituted phenyl is monosubstituted phenyl.

In another preferred embodiment, R₆ is m-nitrophenyl, o-nitrophenyl, p-nitrophenyl, phenyl, p-methylphenyl, o-methylphenyl, m-methylphenyl, m-aminophenyl, p-aminophenyl, o-aminophenyl, m-methoxyphenyl, p-methoxyphenyl, o-methoxyphenyl, m-trifluoromethoxyphenyl, p-trifluoromethoxyphenyl, o-trifluoromethoxyphenyl, p-halophenyl, o-halophenyl, m-halophenyl, p-trifluoromethylphenyl, o-trifluoromethylphenyl, m-trifluoromethylphenyl, p-phenylphenyl, o-phenylphenyl, m-phenylphenyl, pyridyl, 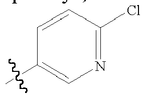 , pyrazinyl, pyrazinyl monosubstituted with methyl.

In another preferred embodiment, R₇ is hydrogen, halogen, -CN, hydroxyl, sulfhydryl, nitro, amino, substituted or unsubstituted C1-C8 alkyl, substituted or unsubstituted C3-C8 cycloalkyl, substituted or unsubstituted C1-C8 alkoxyl, substituted or unsubstituted C1-C8 alkylthio, substituted or unsubstituted C6-C12 aryl, or substituted or unsubstituted 5-10 membered heteroaryl.

In another preferred embodiment, R₇ is hydrogen, halogen, -CN, hydroxyl, sulfhydryl, nitro, amino, substituted or unsubstituted C1-C4 alkyl, substituted or unsubstituted C3-C6 cycloalkyl, substituted or unsubstituted C1-C6 alkoxyl, substituted or unsubstituted C1-C4 alkylthio, substituted or unsubstituted C6-C8 aryl, or substituted or unsubstituted 5-8 membered heteroaryl.

In another preferred embodiment, R₇ is hydrogen, halogen, -CN, hydroxyl, sulfhydryl, nitro, amino, substituted or unsubstituted C1-C4 alkyl, substituted or unsubstituted C3-C6 cycloalkyl, substituted or unsubstituted C1-C6 alkoxyl, substituted or unsubstituted C1-C4 alkylthio, substituted or unsubstituted C6-C8 aryl, or substituted or unsubstituted 5-8 membered heteroaryl.

In another preferred embodiment, R₇ is hydrogen.

In another preferred embodiment, R₈ and R₉ are connected to form substituted or unsubstituted 3-12 membered cycloalkane ring, substituted or unsubstituted 3-12 membered heterocycloalkane ring.

In another preferred embodiment, R₈ and R₉ are connected to form substituted or unsubstituted 3-10 membered cycloalkane ring, substituted or unsubstituted 3-10 membered heterocycloalkane ring.

In another preferred embodiment, R₈ and R₉ are connected to form substituted or unsubstituted 3-8 membered cycloalkane ring, substituted or unsubstituted 3-8 membered heterocycloalkane ring.

In another preferred embodiment, R₈ and R₉ are connected to form substituted or unsubstituted 5-8 membered cycloalkane ring, substituted or unsubstituted 5-8 membered heterocycloalkane ring.

In another preferred embodiment, R₈ and R₉ are connected to form substituted or unsubstituted 5-6 membered cycloalkane ring, substituted or unsubstituted 5-6 membered heterocycloalkane ring.

In another preferred embodiment, R₈ and R₉ are connected to form substituted or unsubstituted 5 membered cycloalkane ring or substituted or unsubstituted 6 membered cycloalkane ring.

In another preferred embodiment, the ring of cycloalkane ring has 1, 2, or 3 C=C double bonds.

In another preferred embodiment, R₈ and R₉ are connected to form substituted or unsubstituted cyclopentene ring or cyclohexene ring.

In another preferred embodiment, ring C is absent, substituted or unsubstituted C6-C12 aromatic ring, substituted or unsubstituted C3-C12 cycloalkane ring, substituted or unsubstituted 3-12 membered heterocycloalkane ring, or substituted or unsubstituted 3-12 membered heteroaromatic ring.

In another preferred embodiment, ring C is absent, substituted or unsubstituted C6-C10 aromatic ring, substituted or unsubstituted C3-C10 cycloalkane ring, substituted or unsubstituted 3-10 membered heterocycloalkane ring, or substituted or unsubstituted 3-10 membered heteroaromatic ring.

In another preferred embodiment, ring C is absent, substituted or unsubstituted C6-C8 aromatic ring, substituted or unsubstituted C5-C10 cycloalkane ring, substituted or unsubstituted 5-10 membered heterocycloalkane ring, or substituted or unsubstituted 5-8 membered heteroaromatic ring.

In another preferred embodiment, ring C is substituted or unsubstituted benzene ring, substituted or unsubstituted pyridine ring.

In another preferred embodiment, ring C is substituted or unsubstituted benzene ring, substituted or unsubstituted pyridine ring, each "substituted" means that 1, 2 or 3 hydrogen atoms on the group are substituted by a substituent selected from the group consisting of methyl, halogen, nitro, -CN, hydroxyl, sulfhydryl, amino, F₃C-O -, F₃C-, methoxyl-.

In another preferred embodiment, R₁₀ and R₁₁ are each independently hydrogen, halogen, -CN, hydroxyl, sulfhydryl, nitro, amino, F₃C-O-, F₃C-, substituted or unsubstituted C1-C10 alkyl, substituted or unsubstituted C3-C10 cycloalkyl, substituted or unsubstituted C1-C8 alkoxyl, substituted or unsubstituted C1-C8 alkylthio, substituted or unsubstituted C6-C12 aryl, substituted or unsubstituted 5-12 membered heteroaryl, substituted or unsubstituted C6-C12 aryl-substituted or unsubstituted C1-C8 alkyl-, substituted or unsubstituted 5-12 membered heteroaryl-substituted or unsubstituted C1-C8 alkyl-, substituted or unsubstituted C1-C4 alkyl-C(O)-; or R₁₀ and R₁₁ are connected to form substituted or unsubstituted 3-12 membered heterocycloalkane ring, substituted or unsubstituted 3-12 membered heteroaromatic ring.

In another preferred embodiment, R₁₀ and R₁₁ are each independently hydrogen, halogen, -CN, hydroxyl, sulfhydryl, nitro, amino, F₃C-O-, F₃C-, substituted or unsubstituted C1-C6 alkyl, substituted or unsubstituted C3-C8 cycloalkyl, substituted or unsubstituted C1-C6 alkoxyl, substituted or unsubstituted C1-C6 alkylthio, substituted or unsubstituted C6-C8 aryl, substituted or unsubstituted 5-10 membered heteroaryl, substituted or unsubstituted C6-C10 aryl-substituted or unsubstituted C1-C4 alkyl-, substituted or unsubstituted 5-10 membered heteroaryl-substituted or unsubstituted C1-C4 alkyl-, substituted or unsubstituted C1-C4 alkyl-C(O)-; or R₁₀ and R₁₁ are connected to form substituted or unsubstituted 5-12 membered heterocycloalkane ring, substituted or unsubstituted 5-12 membered heteroaromatic ring.

In another preferred embodiment, R₁₀ and R₁₁ are each independently hydrogen, halogen, -CN, hydroxyl, sulfhydryl, nitro, amino, F₃C-O-, F₃C-, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted C6-C8 aryl, substituted or unsubstituted 5-8 membered heteroaryl, substituted or unsubstituted C6-C8 aryl-substituted or unsubstituted C1-C2 alkyl-, substituted or unsubstituted 5-8 membered heteroaryl-substituted or unsubstituted C1-C2 alkyl-, substituted or unsubstituted C1-C4 alkyl-C(O)-; or R₁₀ and R₁₁ are connected to form substituted or unsubstituted 5-12 membered heterocycloalkane ring, substituted or unsubstituted 5-12 membered heteroaromatic ring.

In another preferred embodiment, the substituted or unsubstituted 3-12 membered heterocycloalkane ring is substituted or unsubstituted C6-C8 aromatic ring and substituted or unsubstituted monocyclic C5-C8 heterocycloalkane ring

In another preferred embodiment, the substituted or unsubstituted 5-12 membered heterocycloalkane ring is substituted or unsubstituted C6-C8 aromatic ring and substituted or unsubstituted monocyclic C5-C8 heterocycloalkane ring.

In another preferred embodiment, R₁₀ and R₁₁ are each independently hydrogen, methyl, substituted or unsubstituted phenyl, substituted or unsubstituted phenyl-C1-C2 alkyl-, substituted or unsubstituted pyridyl, substituted or unsubstituted pyridyl-C1-C2 alkyl-, substituted or unsubstituted C1-C3 alkyl-C(O)-; or R₁₀ and R₁₁ are connected to form substituted or unsubstituted isoquinoline ring, substituted or unsubstituted quinoline ring, substituted or unsubstituted tetrahydroisoquinoline ring, substituted or unsubstituted tetrahydroquinoline ring, substituted or unsubstituted dihydroisoquinoline-1-one ring.

In another preferred embodiment, the tetrahydroquinoline ring is 1,2,3,4-tetrahydroquinoline ring.

In another preferred embodiment, the dihydroisoquinolin-1-one ring is 3,4-dihydroisoquinolin-1-one ring.

In another preferred embodiment, the substituted or unsubstituted phenyl-C1-C2 alkyl- is substituted or unsubstituted phenyl-methyl- or substituted or unsubstituted phenyl-ethyl-.

In another preferred embodiment, in the substituted or unsubstituted phenyl-C1-C2 alkyl-, the substituted or unsubstituted phenyl is monosubstituted or unsubstituted phenyl.

In another preferred embodiment, in the substituted or unsubstituted phenyl-C1-C2 alkyl-, the substituted or unsubstituted phenyl is monosubstituted or unsubstituted phenyl, and the substitution is ortho, para or meta substitution on the phenyl.

In another preferred embodiment, in the substituted or unsubstituted phenyl-C1-C2 alkyl-, the substituted or unsubstituted phenyl is monosubstituted or unsubstituted phenyl, and the substitution is ortho, para or meta substitution on the phenyl, the substituent is halogen, nitro, amino, C1-C4 alkyl, C1-C4 alkoxyl, C1-C4 alkylthio, F₃C -, F₃C-O -.

In another preferred embodiment, the substituted phenyl is monosubstituted phenyl.

In another preferred embodiment, the substituted or unsubstituted phenyl-C1-C2 alky- is substituted or unsubstituted phenyl-methyl- or substituted or unsubstituted phenylethyl-ethyl-.

In another preferred embodiment, R₁₂ and R₁₃ are independently hydrogen, halogen, -CN, hydroxyl, sulfhydryl, nitro, amino, F₃C-, F₃C-O-, substituted or unsubstituted C1-C8 alkyl, substituted or unsubstituted C3-C8 cycloalkyl, substituted or unsubstituted C1-C6 alkoxyl, substituted or unsubstituted C1-C6 alkylthio, substituted or unsubstituted C6-C12 aryl, substituted or unsubstituted 5-12 membered heteroaryl, substituted or unsubstituted C6-C12 aryl-substituted or unsubstituted C1-C6 alkyl-, substituted or unsubstituted 5-12 membered heteroaryl-substituted or unsubstituted C1-C6 alkyl-.

In another preferred embodiment, R₁₂ and R₁₃ are independently hydrogen, halogen, -CN, hydroxyl, sulfhydryl, nitro, amino, F₃C-, F₃C-O-, substituted or unsubstituted C1-C4 alkyl, substituted or unsubstituted C3-C6 cycloalkyl, substituted or unsubstituted C1-C4 alkoxyl, substituted or unsubstituted C1-C4 alkylthio, substituted or unsubstituted C6-C8 aryl, substituted or unsubstituted 5-10 membered heteroaryl, substituted or unsubstituted C6-C8 aryl-substituted or unsubstituted C1-C2 alkyl-, substituted or unsubstituted 5-8 membered heteroaryl-substituted or unsubstituted C1-C2 alkyl-.

In another preferred embodiment, R₁₂ and R₁₃ are each independently hydrogen, halogen, phenyl.

In another preferred embodiment, the compound of formula I has the following structure of formula 1-2: 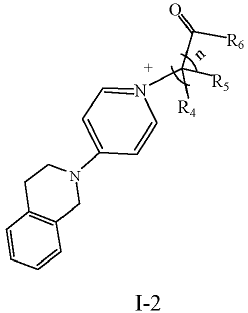 wherein, R₄, R₅, R₆ and n are as defined above.

In another preferred embodiment, the compound of formula I has the following structure of formula I-3: 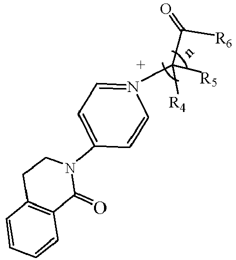 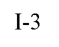 wherein, R₄, R₅, R₆ and n are as defined above.

In another preferred embodiment, the compound of formula I has the following structure of formula I-4: 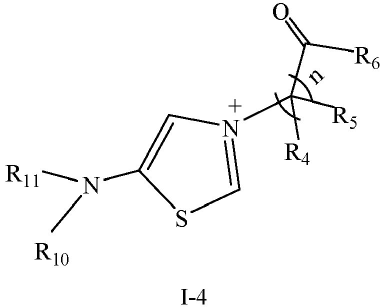 wherein, R₄, R₅, R₆, R₁₀, R₁₁ and n are as defined above.

In another preferred embodiment, the compound of formula I has the following structure of formula I-5: 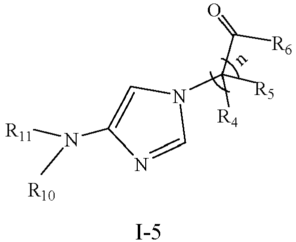 wherein, R₄, R₅, R₆, R₁₀, R₁₁ and n are as defined above.

In another preferred embodiment, the compound of formula I has the following structure of formula 1-6: 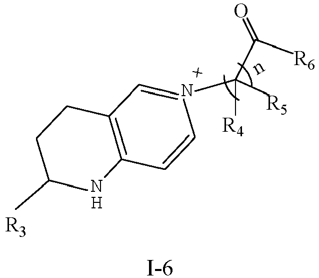 wherein, R₃, R₄, R₅, R₆ and n are as defined above.

In another preferred embodiment, the compound of formula I has the following structure of formula I-7: 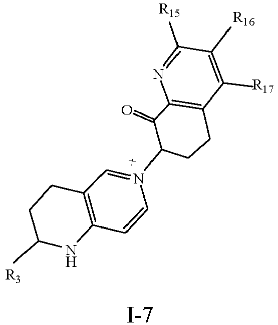
wherein, R₃ is as defined above;
R₁₅, R₁₆ and R₁₇ are each independently hydrogen, halogen (e.g., Cl, F), methyl, nitro, phenyl, -OCF₃, -CF₃.

In another preferred embodiment, the compound of formula I has the following structure of formula I-8: 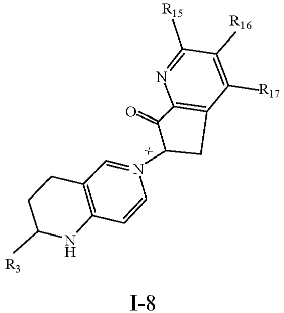
wherein, R₃ is as defined above;
R₁₅, R₁₆ and R₁₇ are each independently hydrogen, halogen (e.g., Cl ), methyl, nitro, phenyl.

In another preferred embodiment, the compound of formula I has the following structure of formula I-9: 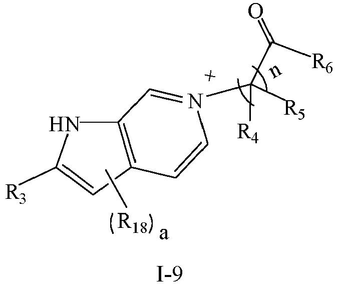
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula 1-10: 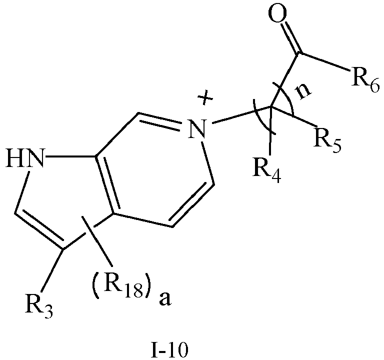
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-11: 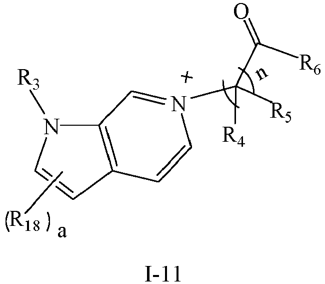
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-12: 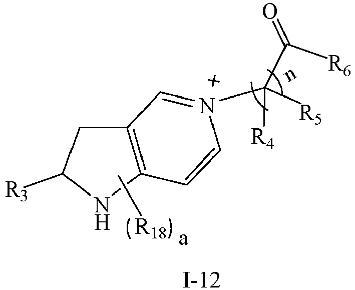
wherein, R₃, R₄, R₅, R₆ and n are as defined above;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-13: 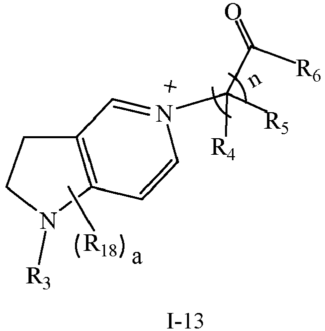
wherein, R₃, R₄, R₅, R₆ and n are as defined above;
a is 0, 1, 2, 3 or 4;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-14: 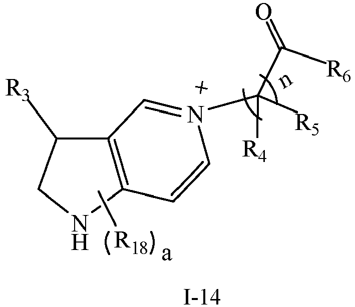
wherein, R₃, R₄, R₅, R₆ and n are as defined above;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-15: 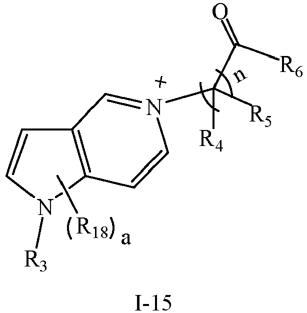
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-16: 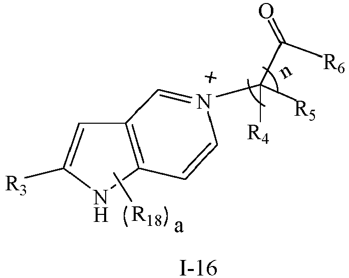
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula 1-17: 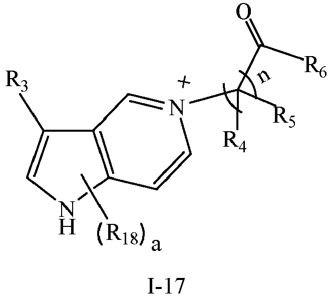
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-18: 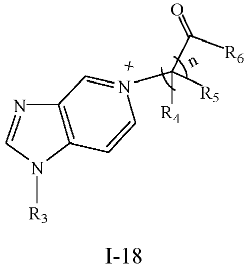 wherein, R₃, R₄, R₅, R₆ and n are as defined above.

In another preferred embodiment, the compound of formula I has the following structure of formula 1-19: 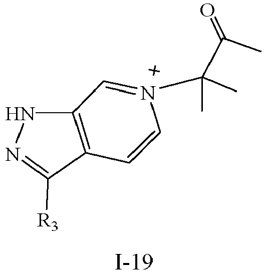 wherein, R₃ is as defined above.

In another preferred embodiment, the compound of formula I has the following structure of formula I-20: 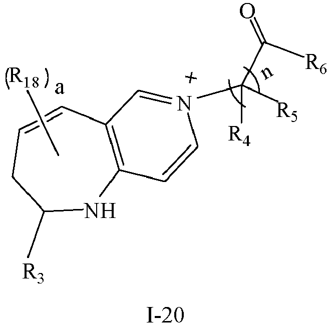
wherein, R₃, R₄, R₅, R₆ and n are as defined above;
a is 0, 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula 1-21: 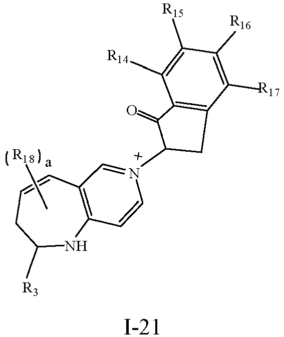
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-22: 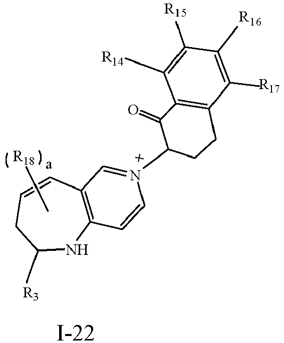
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-23: 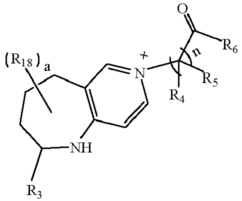 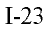
wherein, R₃, R₄, R₅, R₆ and n are as defined above;
a is 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-24: 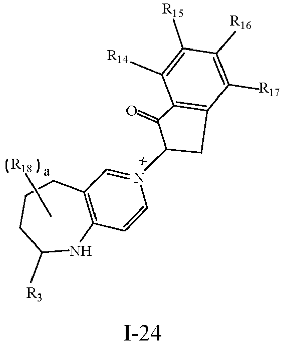
wherein, R₃ is as defined above;
a is 0, 1, 2, 3, 4, or 5;
R₁₄, R₁₅, R₁₆ and R₁₇ are each independently hydrogen, halogen (e.g., Cl, F), methyl, nitro, phenyl, -OCF₃, -CF₃;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-25: 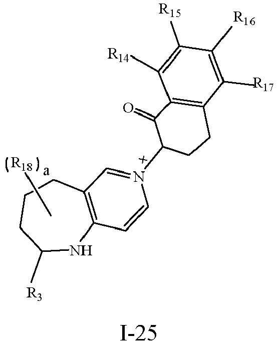
wherein, R₃ is as defined above;
a is 0, 1, 2, 3, 4, or 5;
R₁₄, R₁₅, R₁₆ and R₁₇ are each independently hydrogen, halogen (e.g., Cl, F), methyl, nitro, phenyl, -OCF₃, -CF₃;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-26: 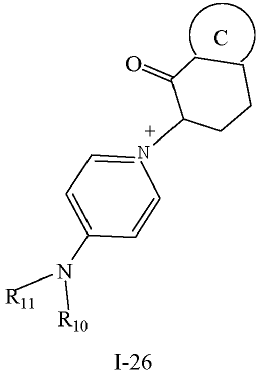 wherein, R₁₀, R₁₁ and ring C are as defined above.

In another preferred embodiment, the compound of formula I has the following structure of formula I-27: 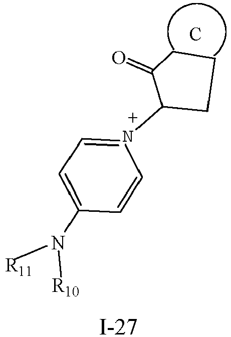 wherein, R₁₀, R₁₁ and ring C are as defined above.

In another preferred embodiment, the compound of formula I has the following structure of formula I-28: 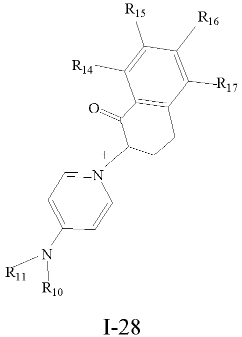
wherein, R₁₀ and R₁₁ are as defined above;
R₁₄, R₁₅, R₁₆ and R₁₇ are each independently hydrogen, halogen (e.g., Cl, F), methyl, nitro, phenyl, -OCF₃, -CF₃.

In another preferred embodiment, the compound of formula I has the following structure of formula I-29: 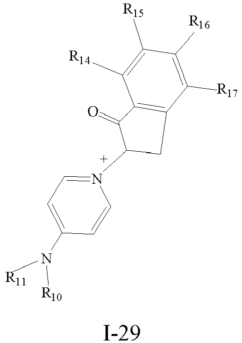
wherein, R₁₀ and R₁₁ are as defined above;
R₁₄, R₁₅, R₁₆ and R₁₇ are each independently hydrogen, halogen (e.g., Cl, F), methyl, nitro, phenyl, -OCF₃, -CF₃.

In another preferred embodiment, the compound of formula I has the following structure of formula I-30: 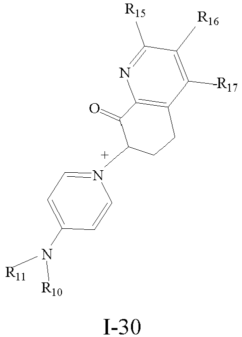
wherein, R₁₀ and R₁₁ are as defined above;
R₁₅, R₁₆ and R₁₇ are each independently hydrogen, halogen (e.g., Cl, F), methyl, nitro, phenyl, -OCF₃, -CF₃.

In another preferred embodiment, the compound of formula I has the following structure of formula 1-31: 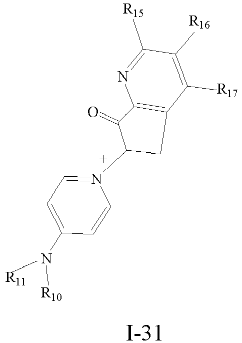
wherein, R₁₀ and R₁₁ are as defined above;
R₁₅, R₁₆ and R₁₇ are each independently hydrogen, halogen (e.g., Cl, F), methyl, nitro, phenyl, -OCF₃, -CF₃.

In another preferred embodiment, the compound of formula I has the following structure of formula I-32: 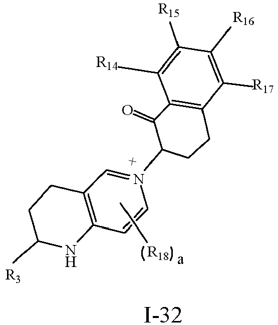
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-33: 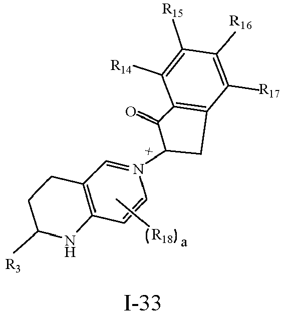
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-34: 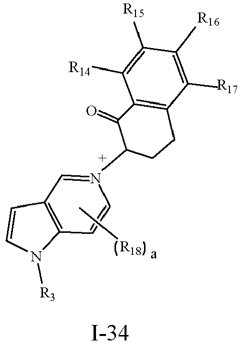
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula 1-35: 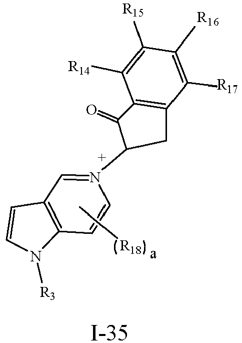
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-36: 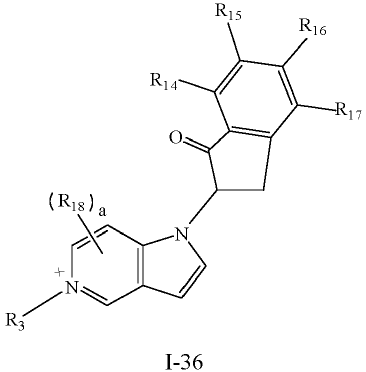
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-37: 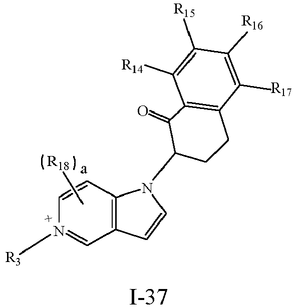
wherein, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-38: 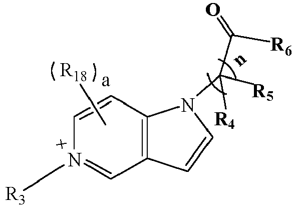 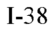
wherein, R₃, R₄, R₅, R₆ and n are as defined above;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl.

In another preferred embodiment, the compound of formula I has the following structure of formula I-39: 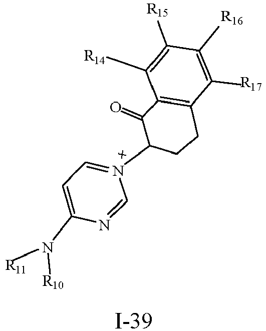
wherein, R₁₀ and R₁₁ are as defined above;
R₁₄, R₁₅, R₁₆ and R₁₇ are each independently hydrogen, halogen (e.g., Cl, F), methyl, nitro, phenyl, -OCF₃, -CF₃;

In another preferred embodiment, the compound of formula I has the following structure of formula I-40: 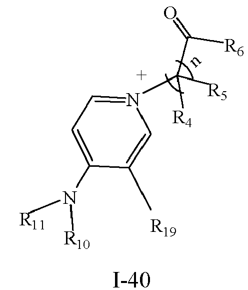
wherein, R₄, R₅, R₆, R₁₀, R₁₁ and n are as defined above;
R₁₉ is hydrogen, substituted or unsubstituted C1-C6 alkyl.

In another preferred embodiment, R₁₉ is hydrogen, substituted or unsubstituted C1-C4 alkyl (e.g., methyl).

In another preferred embodiment, the compound of formula I has the following structure of formula 1-41: 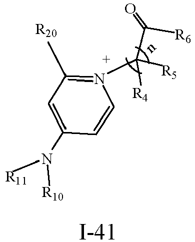
wherein, R₄, R₅, R₆, R₁₀, R₁₁ and n are as defined above;
R₂₀ is hydrogen, halogen.

In another preferred embodiment, R₁₉ is hydrogen, substituted or unsubstituted C1-C4 alkyl (e.g., methyl).

In another preferred embodiment, the compound of formula I has the following structure of formula I-42: 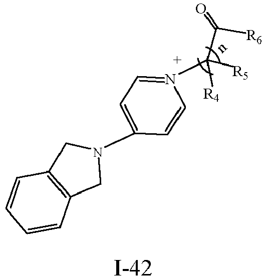 wherein, R₄, R₅, R₆ and n are as defined above.

In another preferred embodiment, the compound of formula I has the following structure of formula I-43: 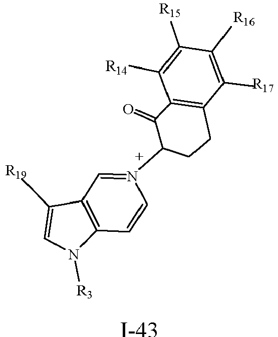
wherein, R₃ is as defined above;
R₁₄, R₁₅, R₁₆ and R₁₇ are each independently hydrogen, -CF₃, halogen (e.g., Cl, F), methyl, nitro, phenyl, -OCF₃;
R₁₉ is hydrogen, substituted or unsubstituted C1-C6 alkyl.

In another preferred embodiment, R₁₉ is hydrogen, substituted or unsubstituted C1-C4 alkyl (e.g., methyl).

In another preferred embodiment, the compound of formula I has the following structure of formula I-44: 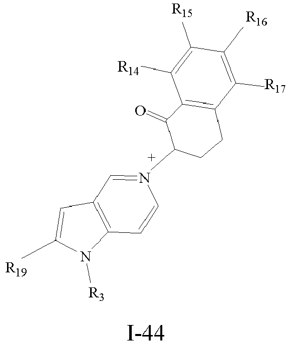
wherein, R₃ is as defined above;
R₁₄, R₁₅, R₁₆ and R₁₇ are each independently hydrogen, -CF₃, halogen (e.g., Cl, F), methyl, nitro, phenyl, -OCF₃;
R₁₉ is hydrogen, substituted or unsubstituted C1-C6 alkyl.

In another preferred embodiment, R₁₉ is hydrogen, substituted or unsubstituted C1-C4 alkyl (e.g., methyl).

In another preferred embodiment, the compound of formula I has the following structure of formula I-45: 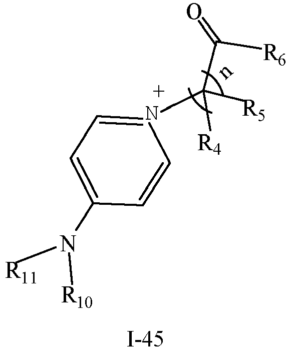 wherein, R₄, R₅, R₆, R₁₀, R₁₁ and n are as defined above.

In another preferred embodiment, the compound of formula I has the following structure of formula I-46: 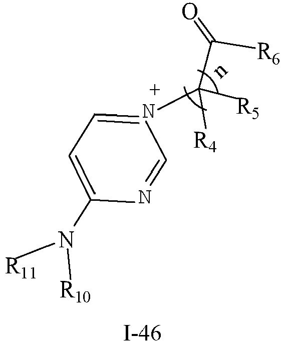 wherein, R₄, R₅, R₆, R₁₀, R₁₁ and n are as defined above.

In another preferred embodiment, the pharmaceutically acceptable salt of the compound of formula I is the salt formed by the compound of formula I and the acid selected from the group consisting of hydrochloric acid, mucic acid, D-glucuronic acid, hydrobromic acid, hydrofluoric acid, sulfuric acid, nitric acid, phosphoric acid, formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid, glutamic acid, and combinations thereof.

In another preferred embodiment, the compound of formula I is selected from the following group:

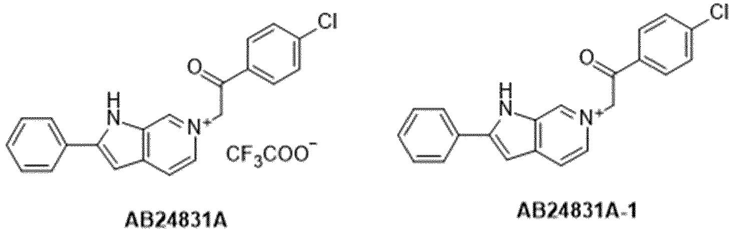

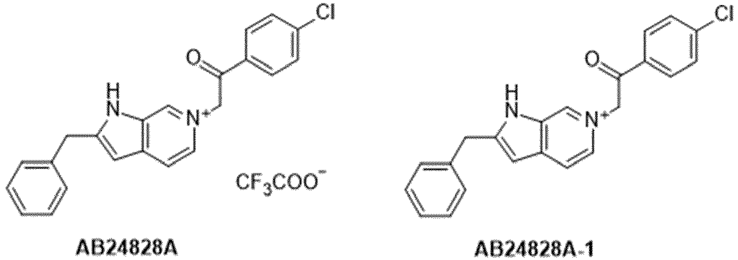

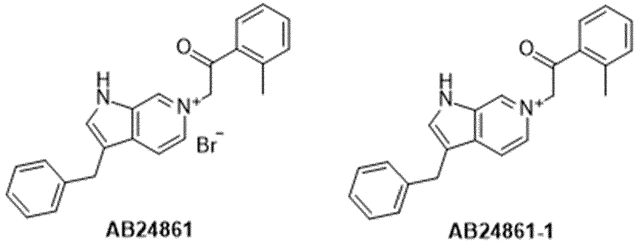

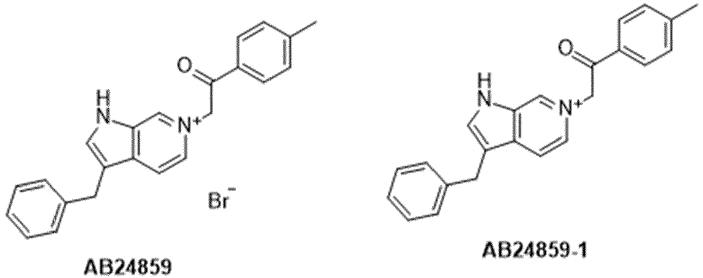

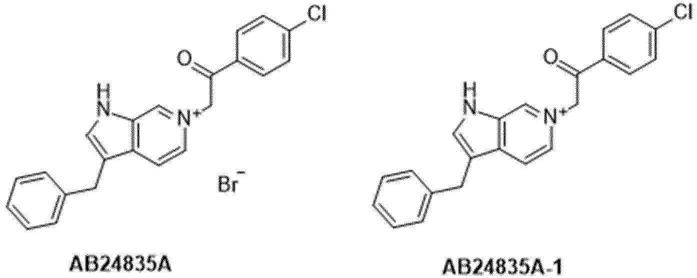

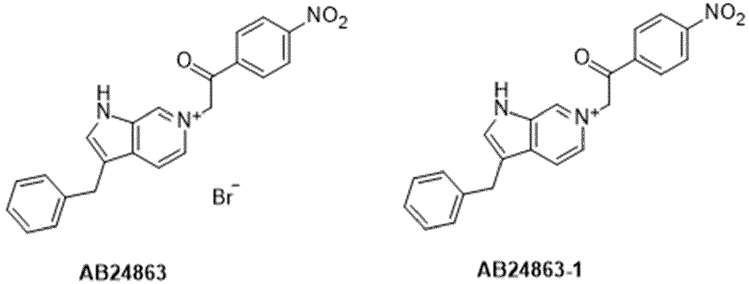

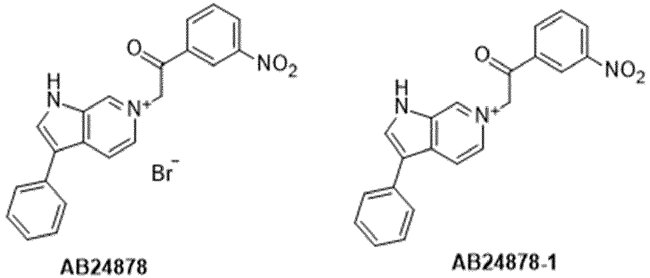

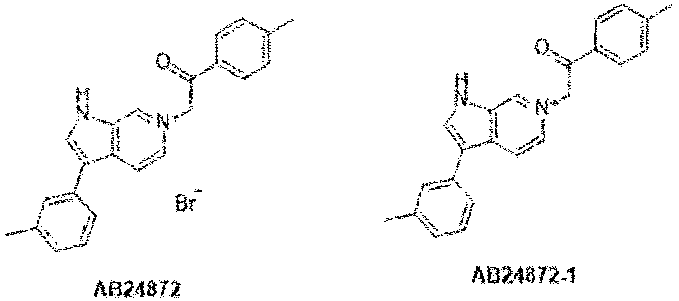

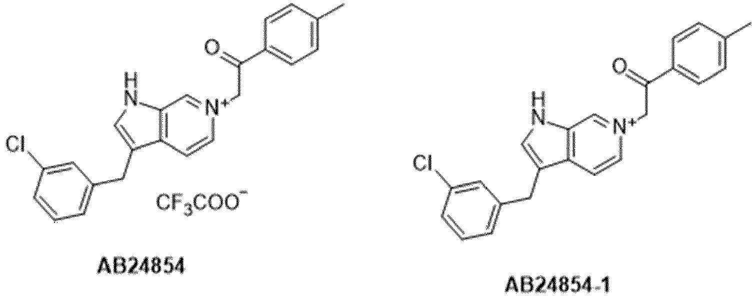

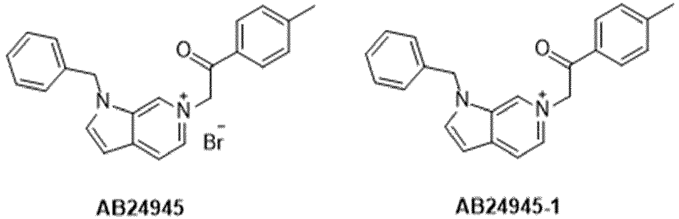

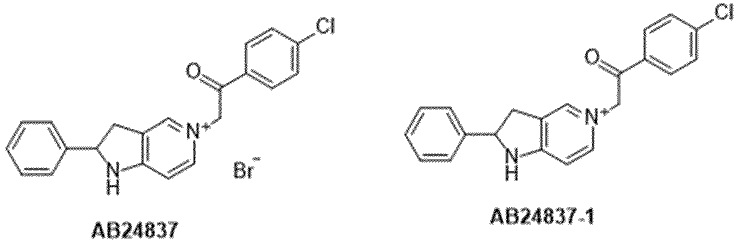

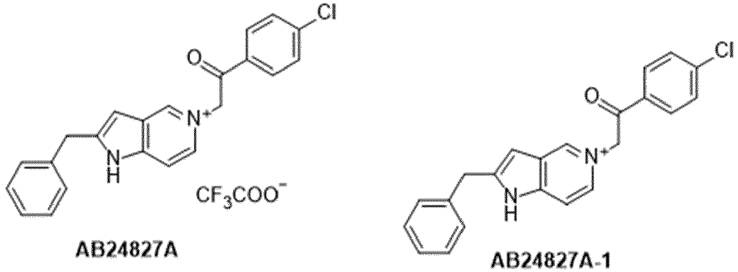

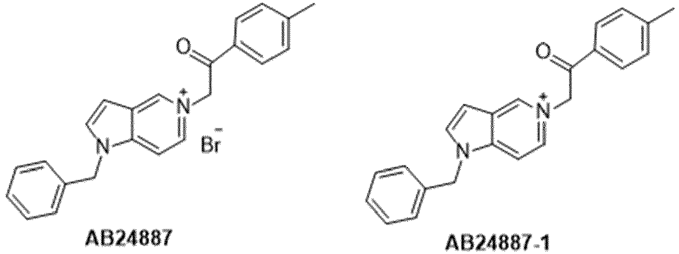

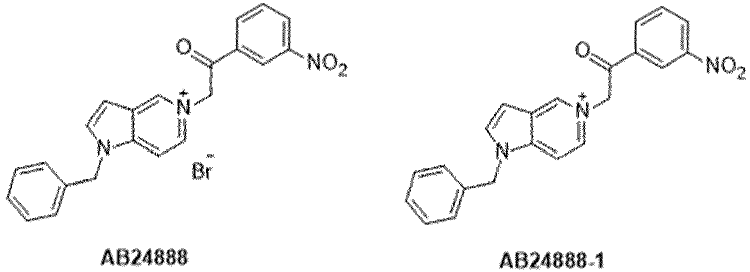

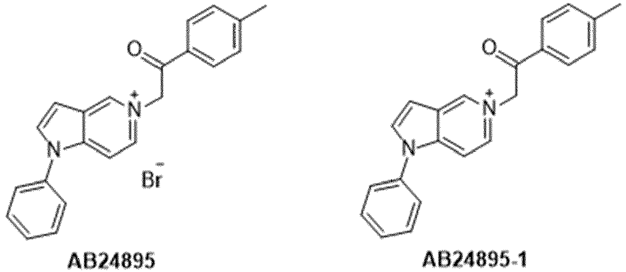

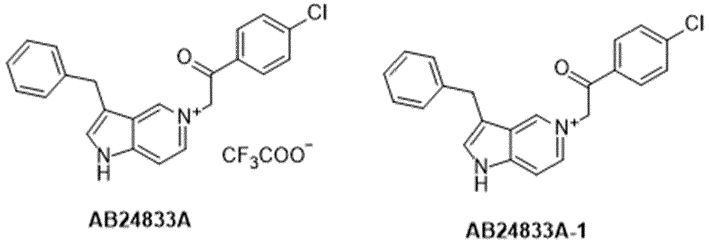

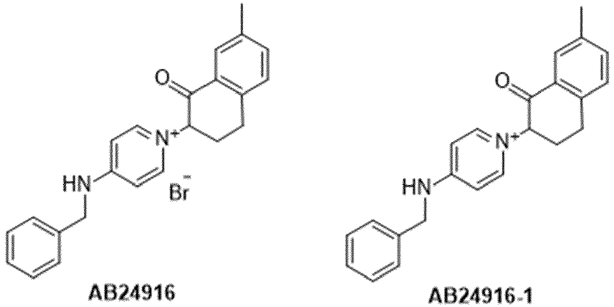

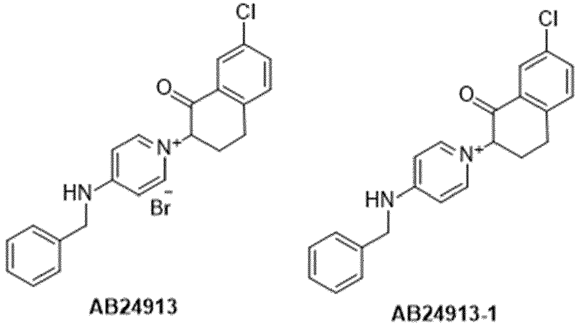

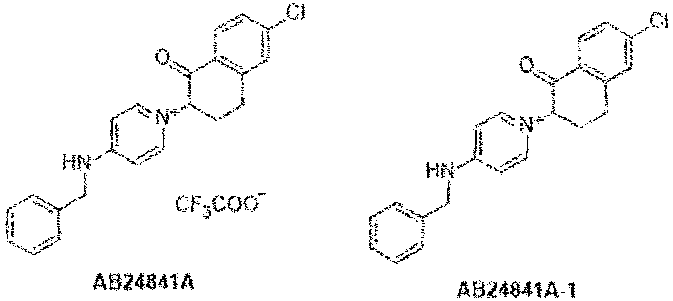

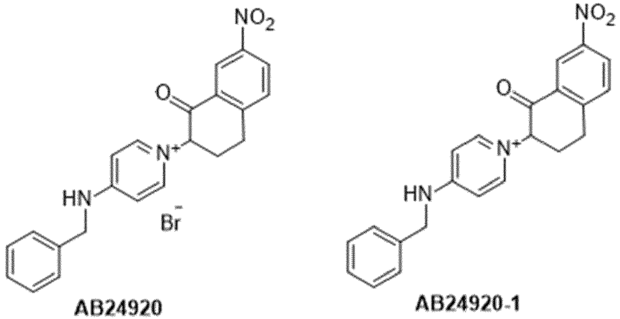

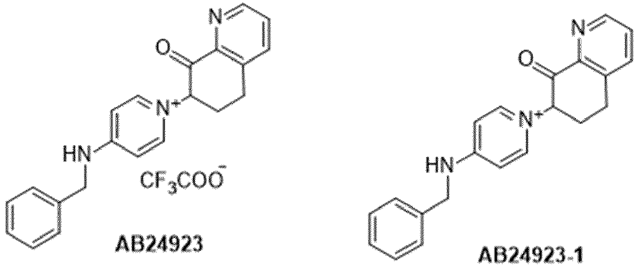

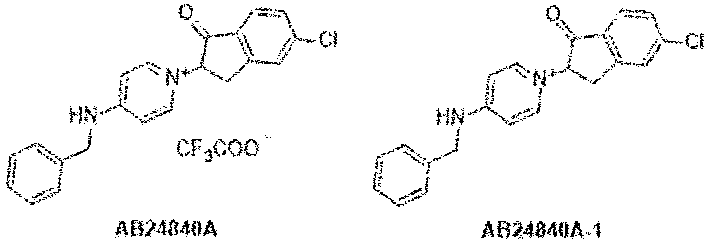

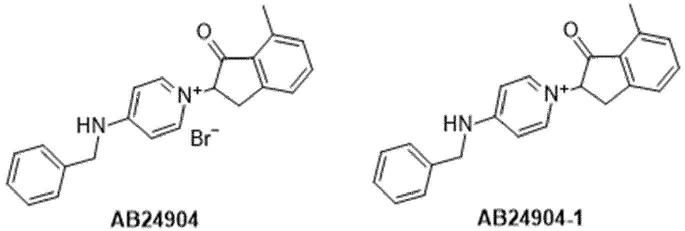

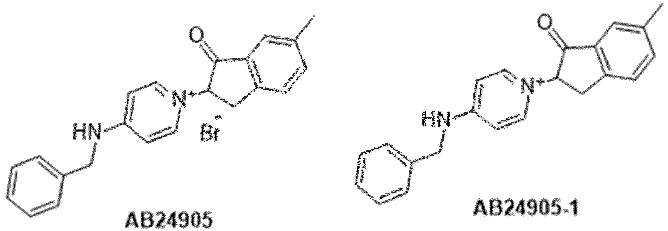

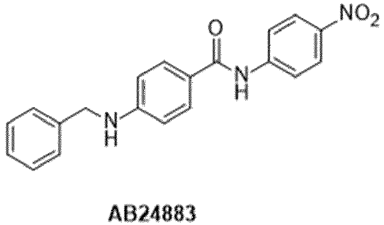

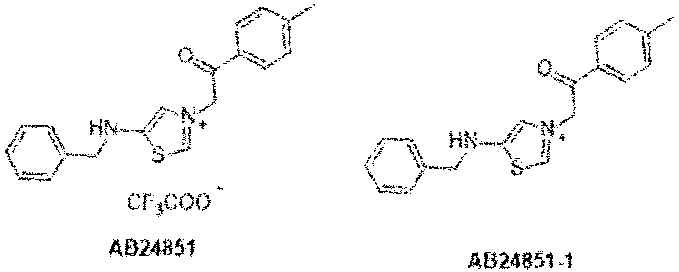

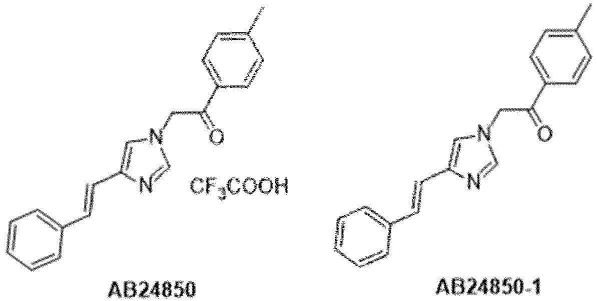

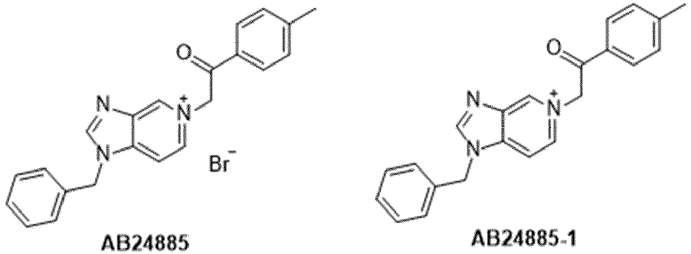

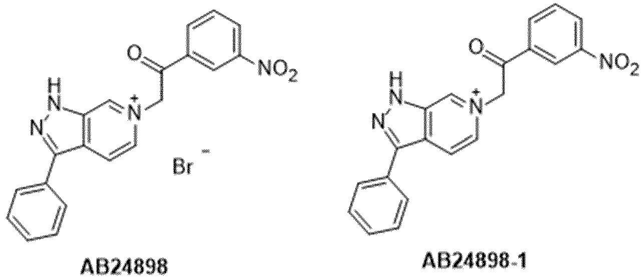

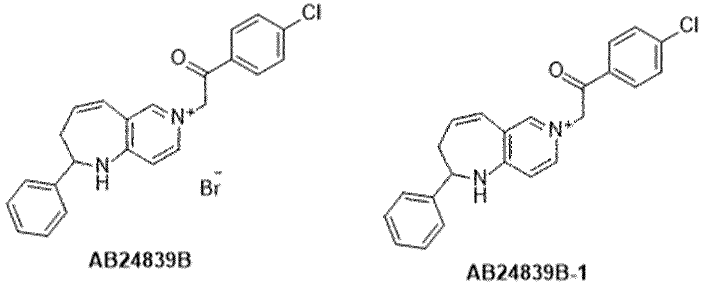

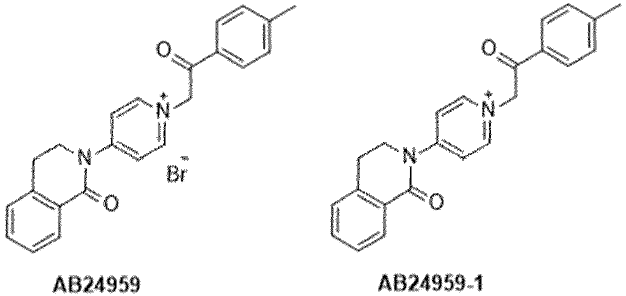

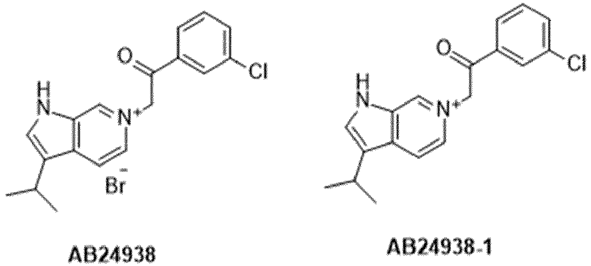

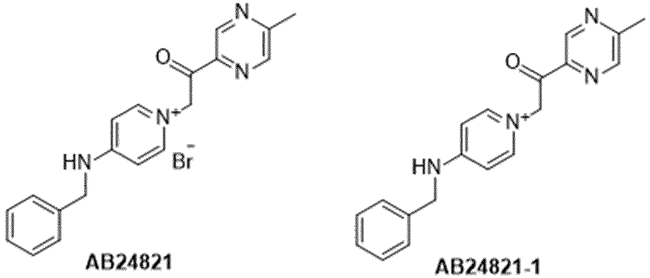

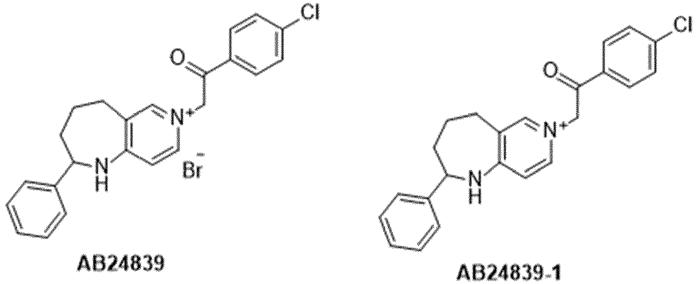

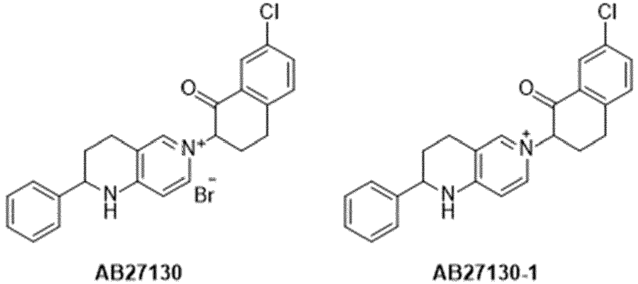

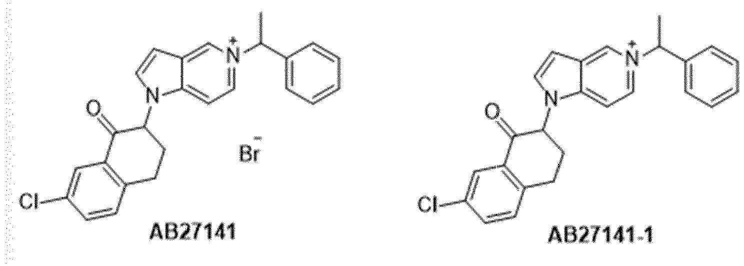

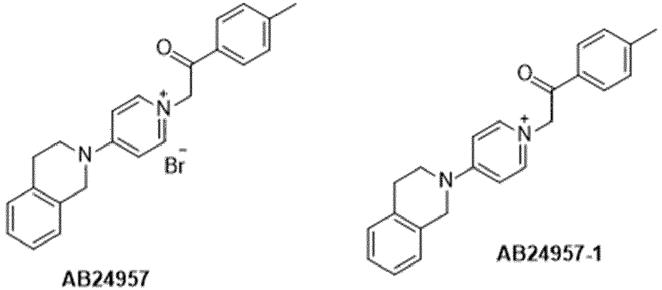

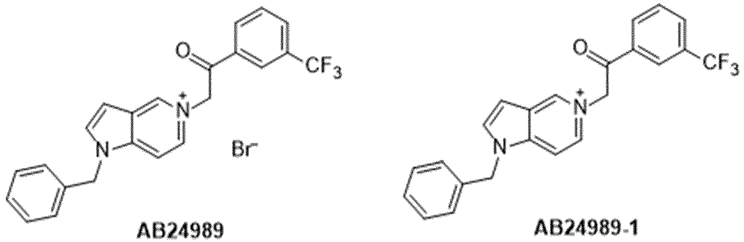

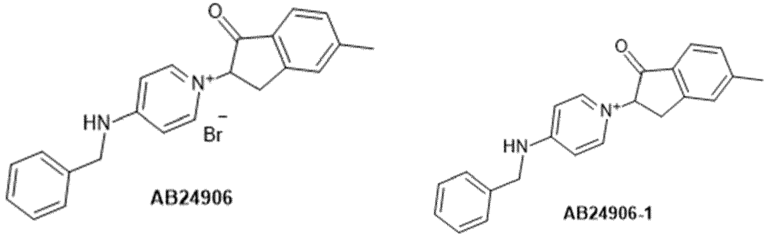

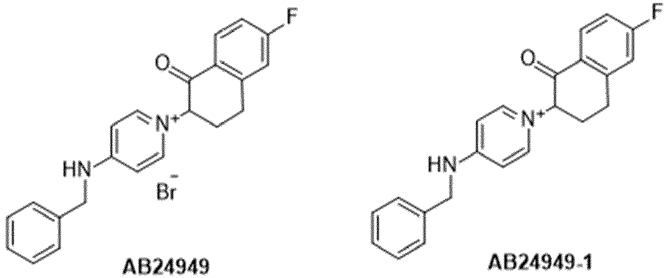

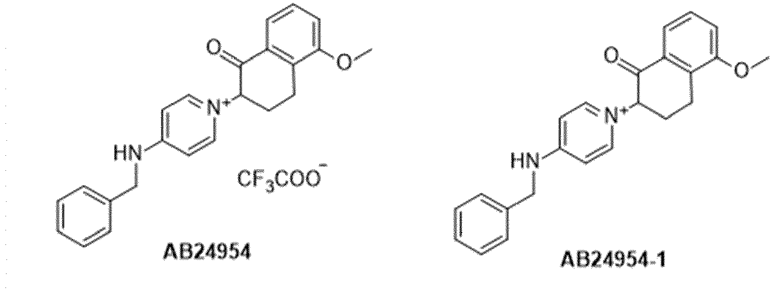

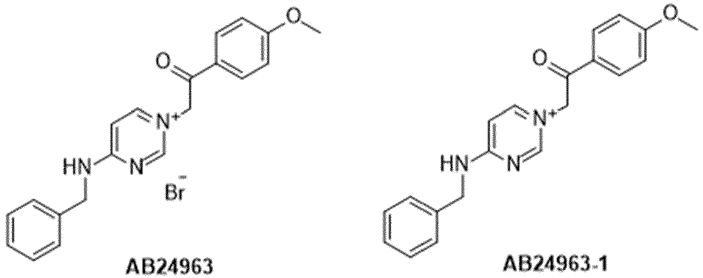

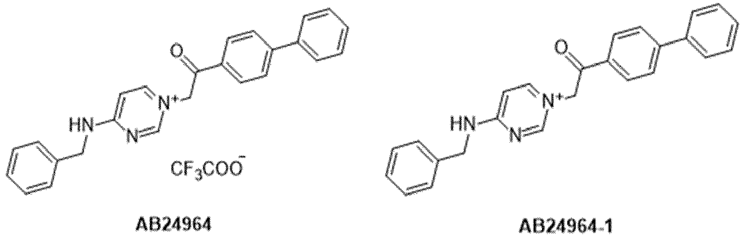

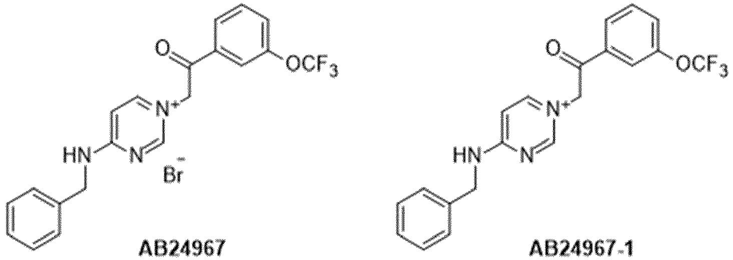

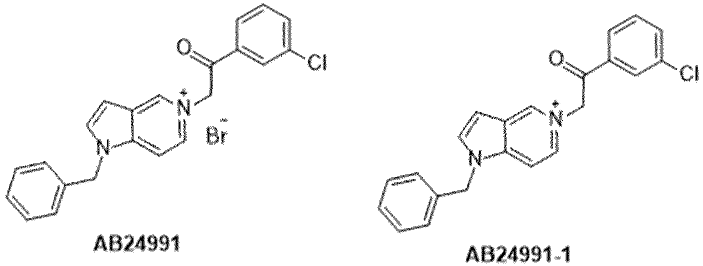

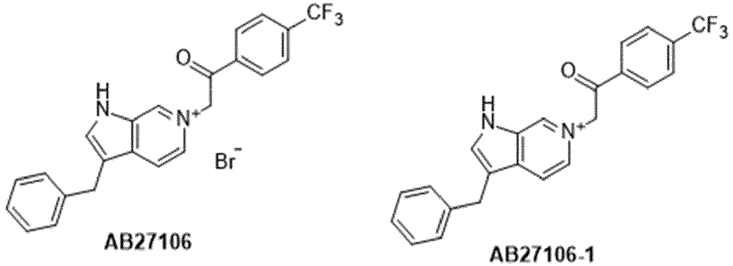

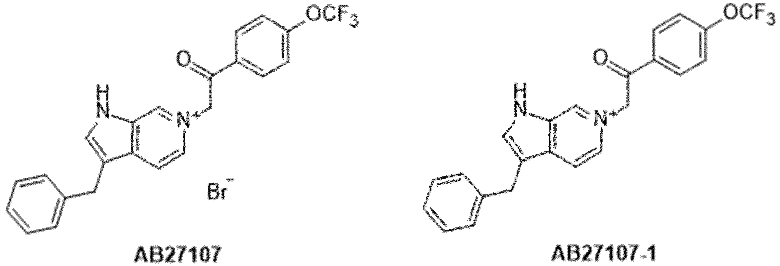

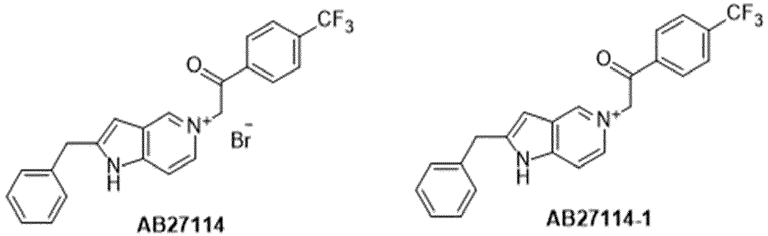

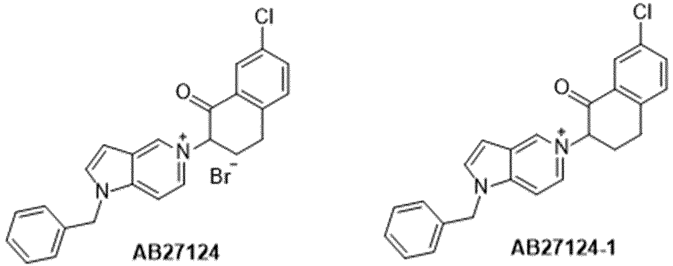

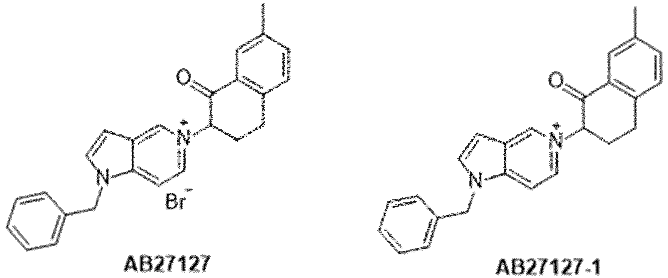

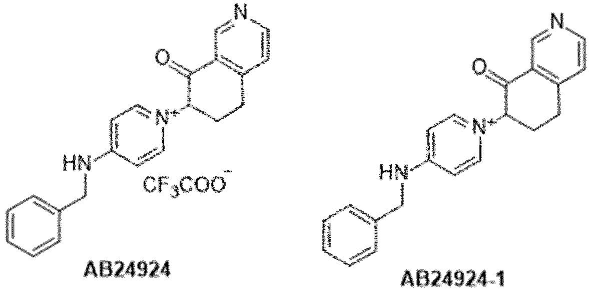

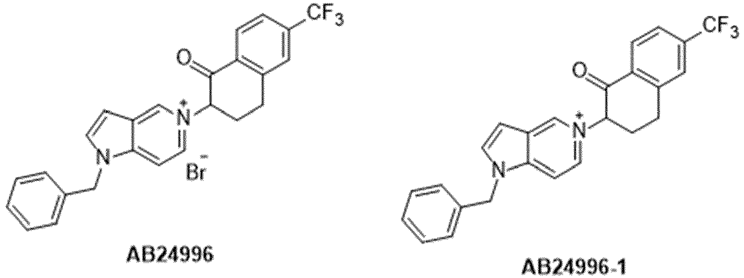

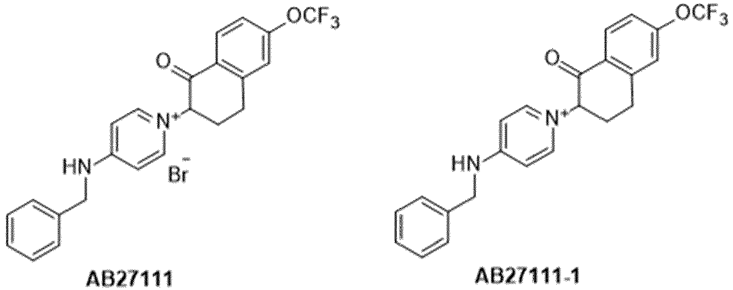

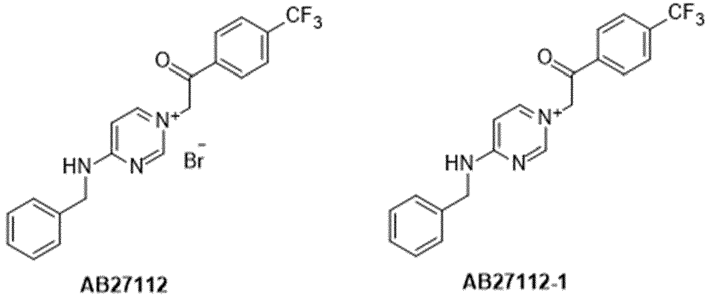

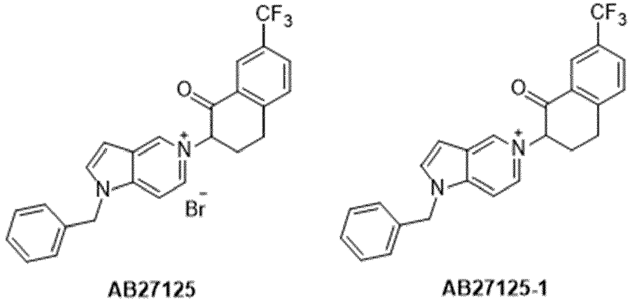

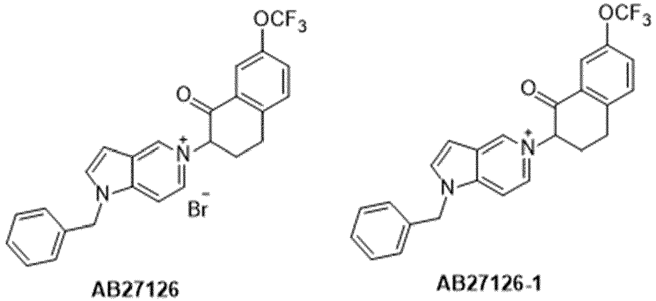

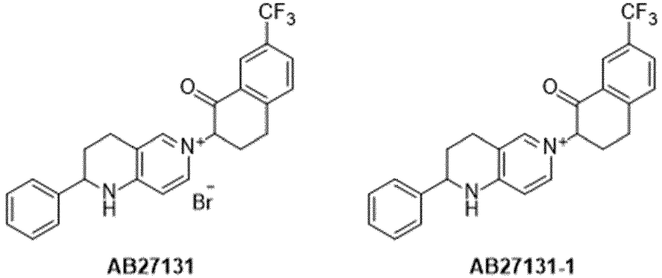

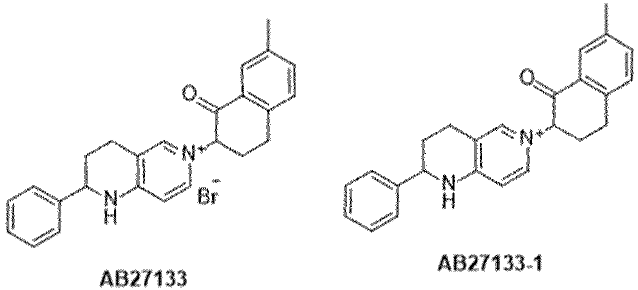

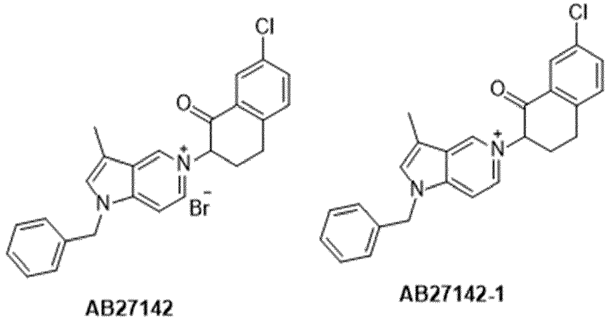

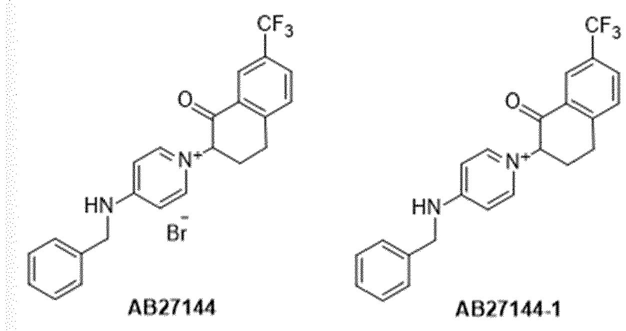

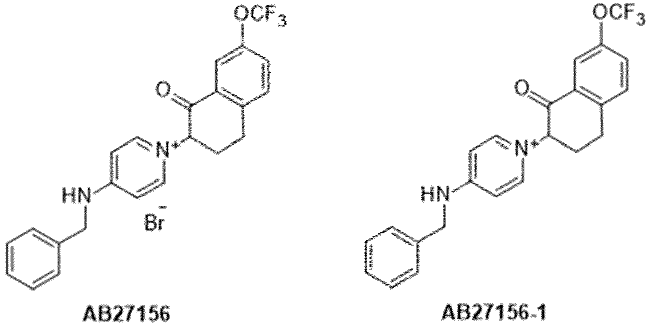

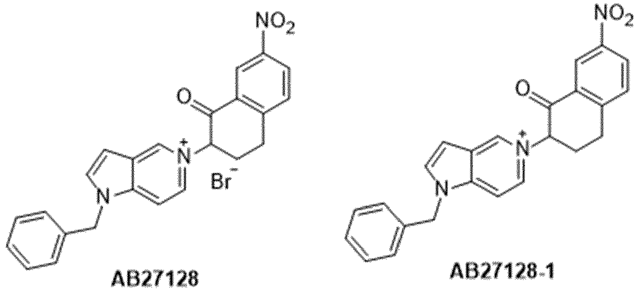

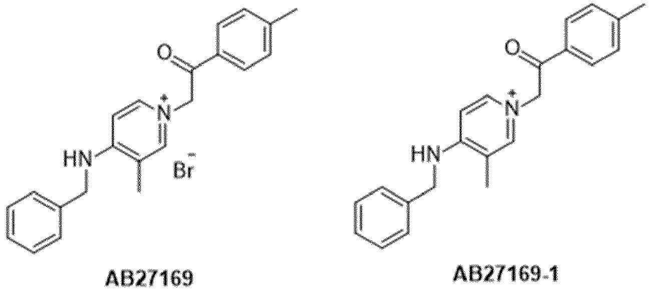

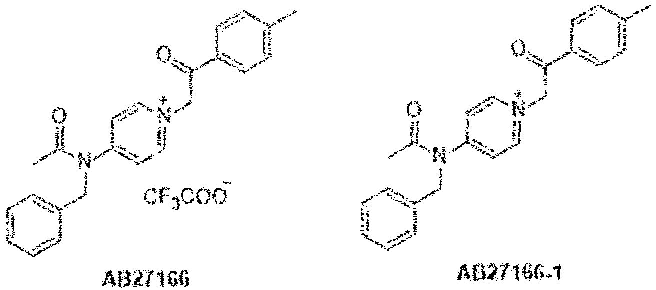

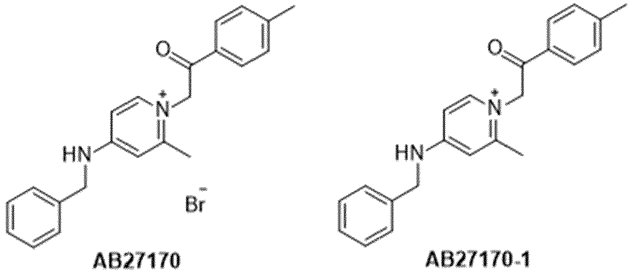

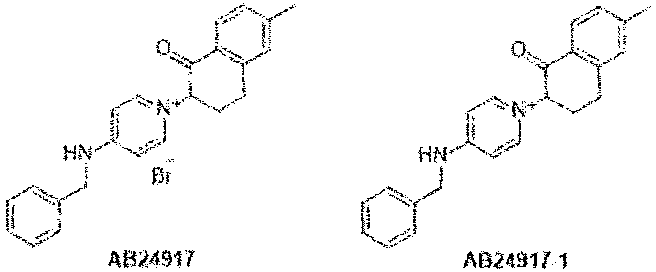

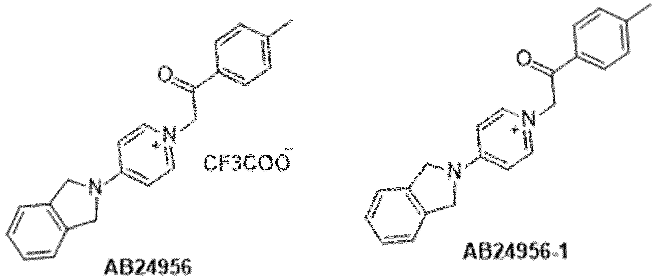

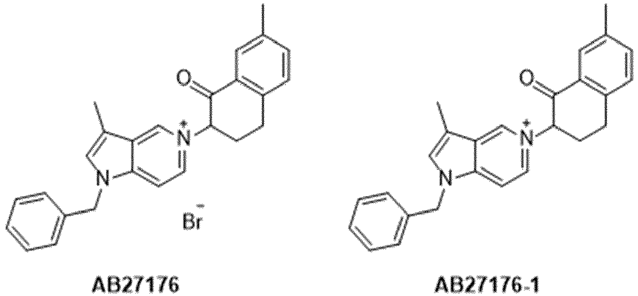

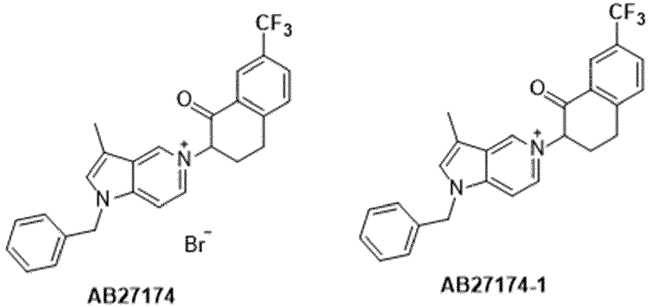

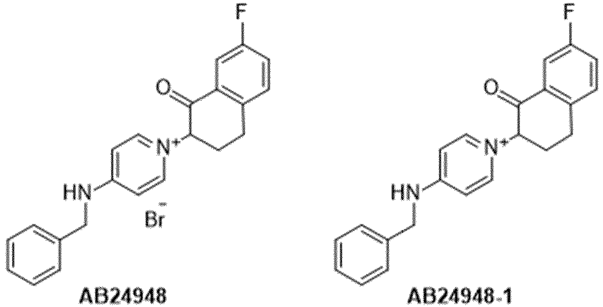

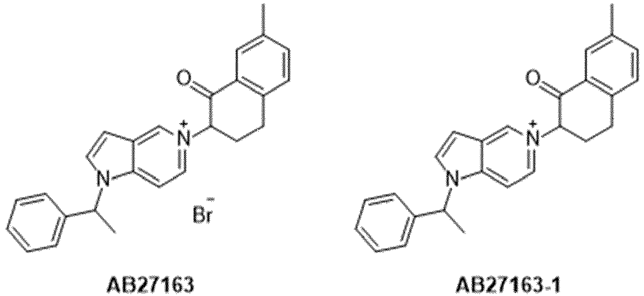

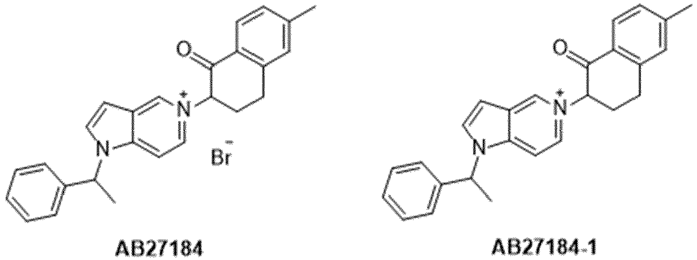

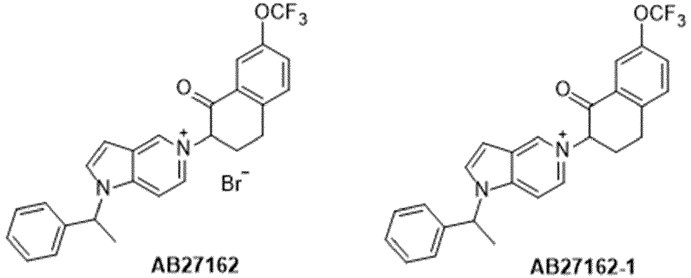

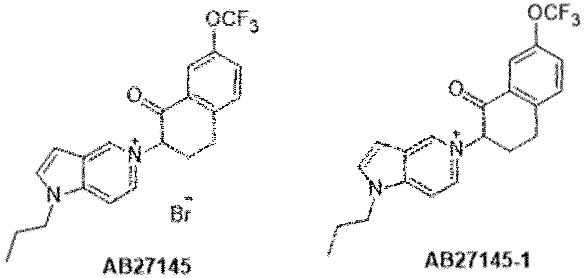

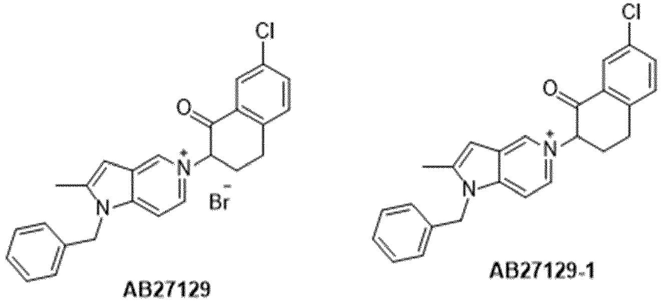

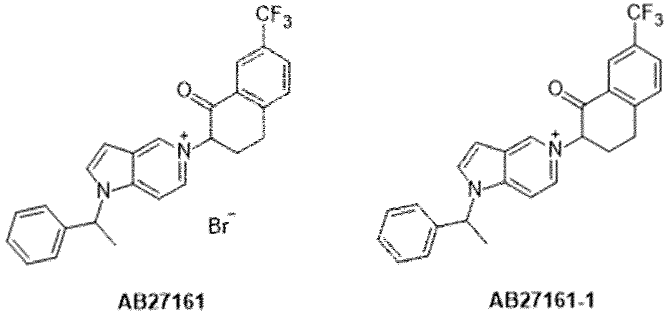

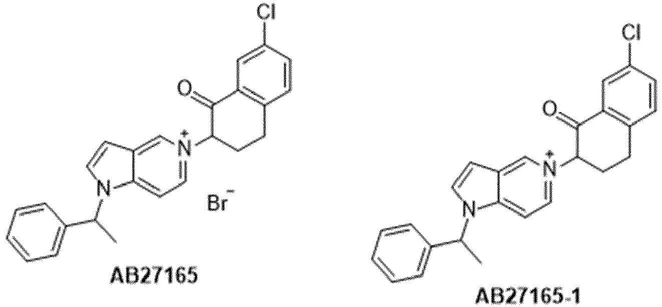

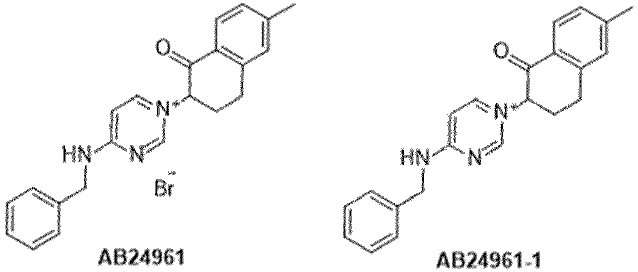

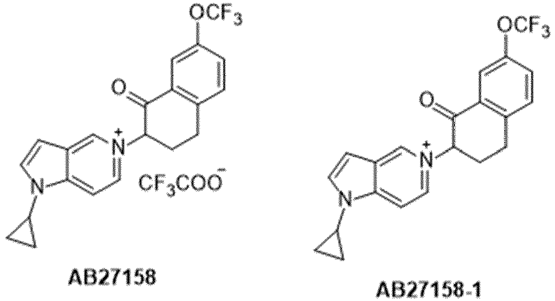

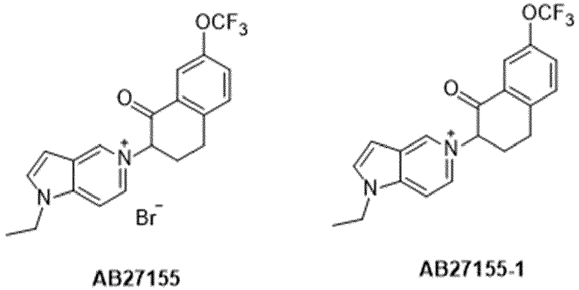

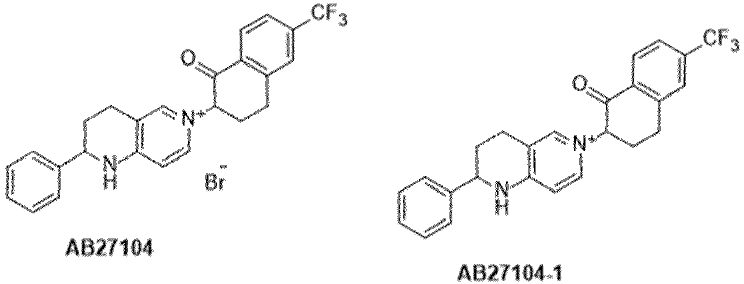

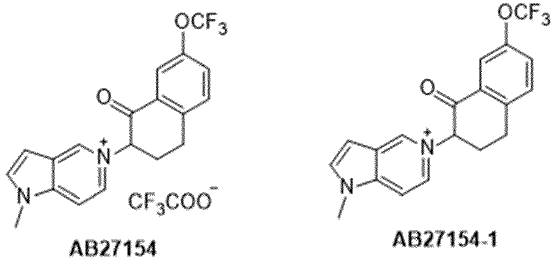

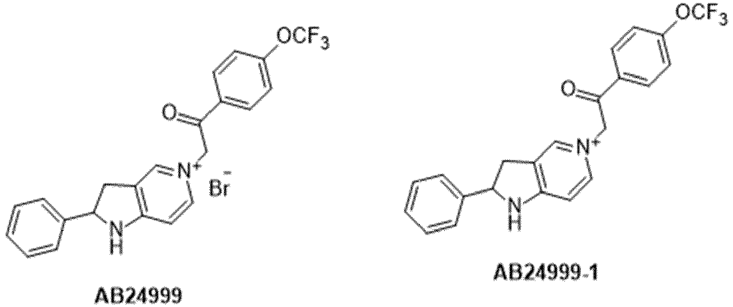

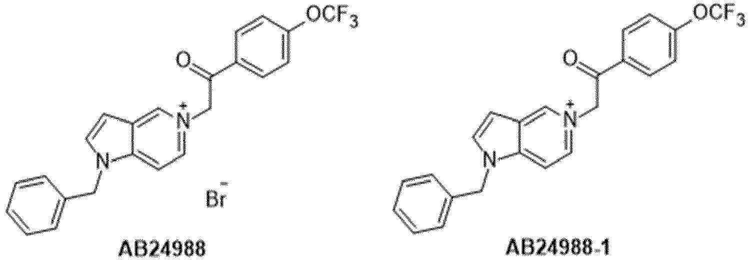

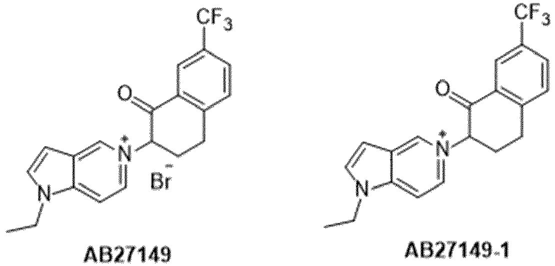

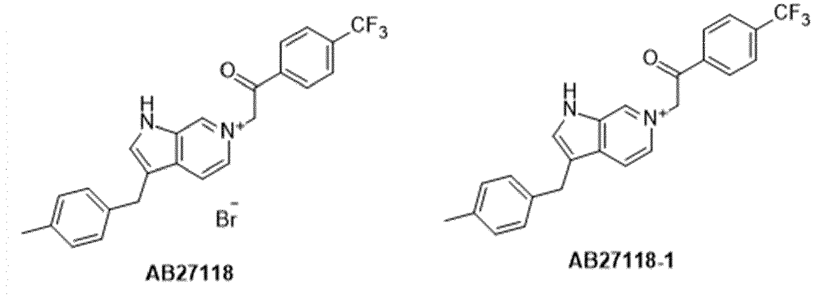

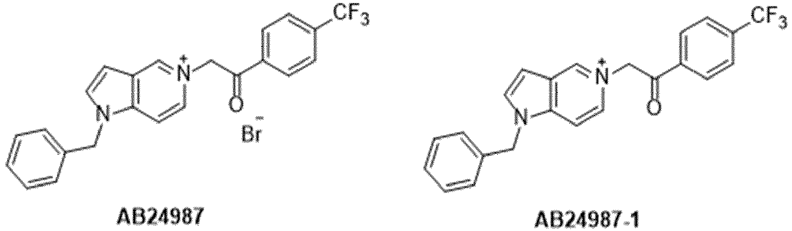

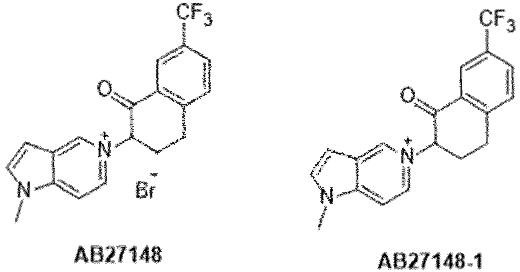

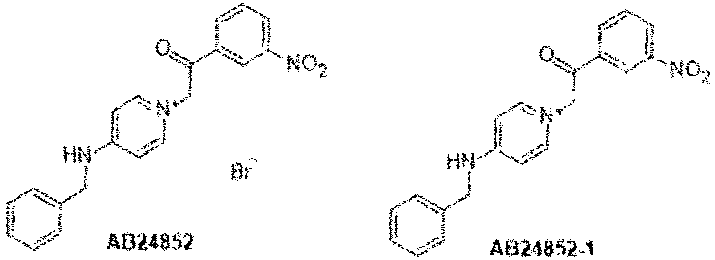

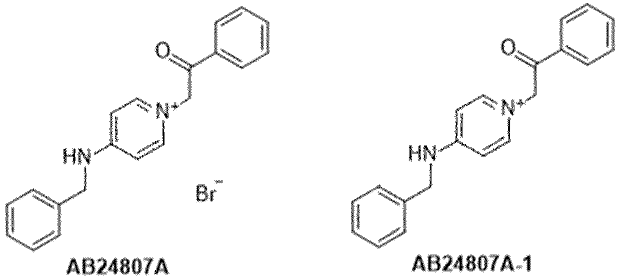

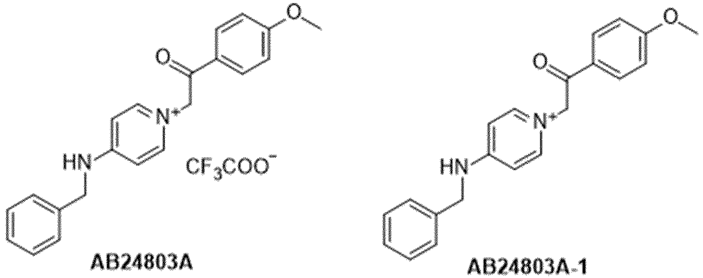

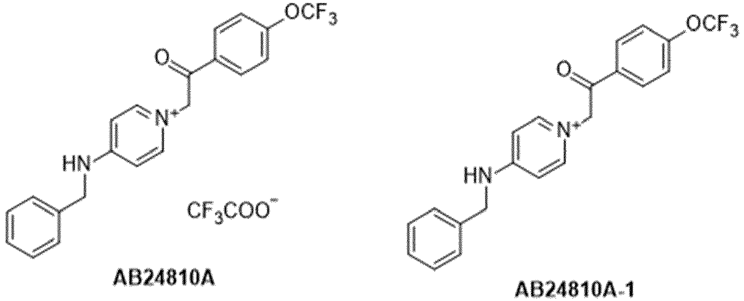

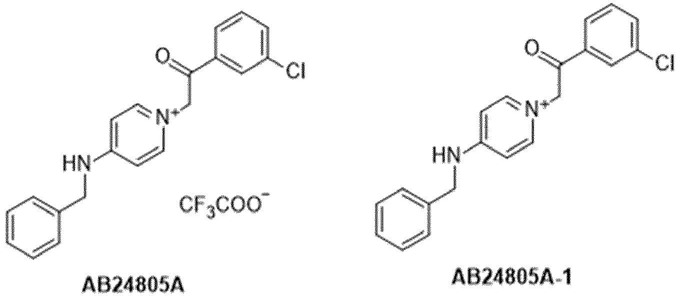

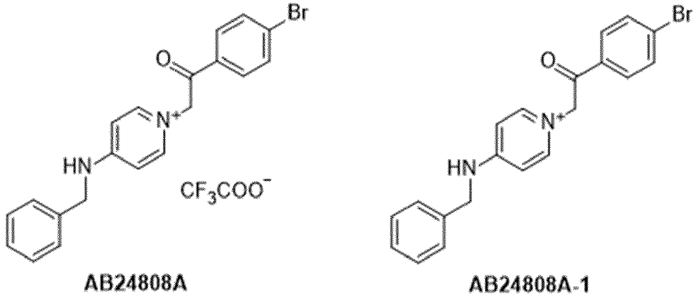

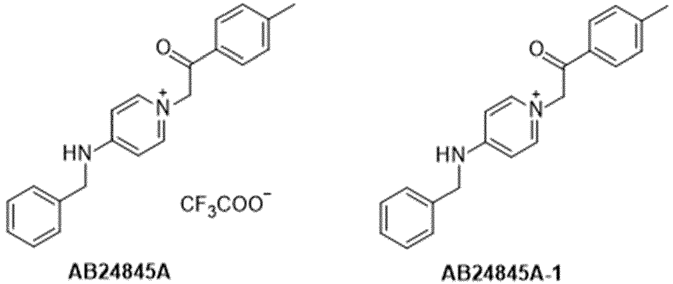

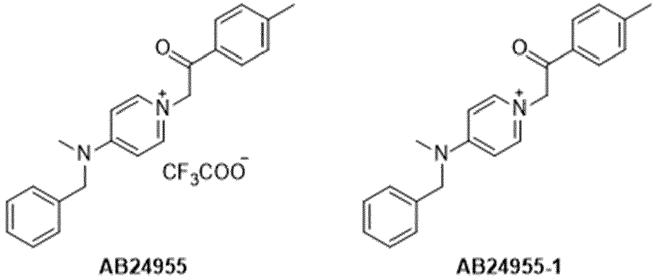

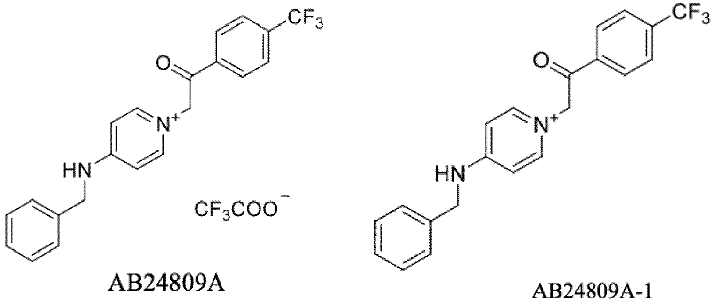

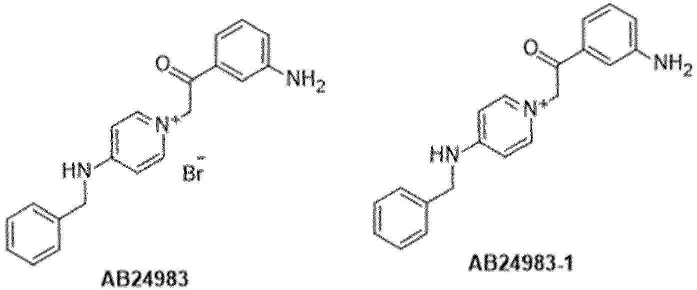

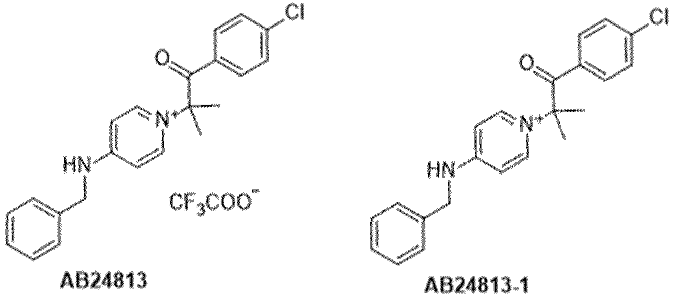

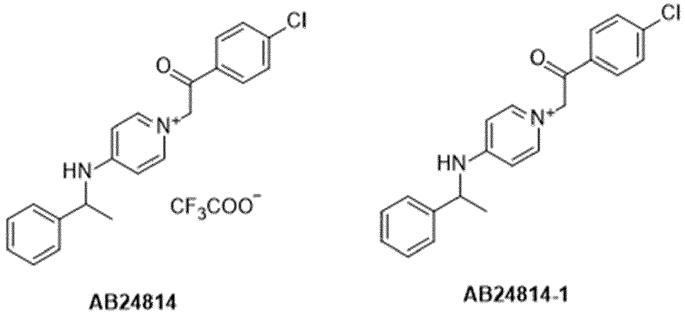

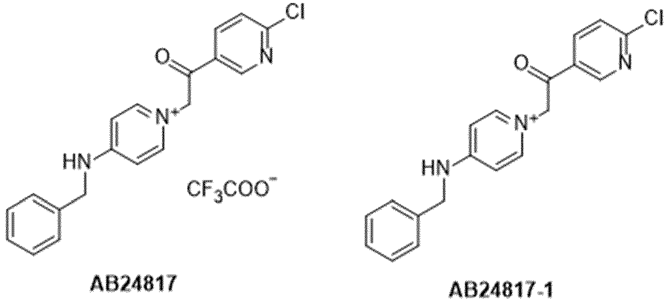

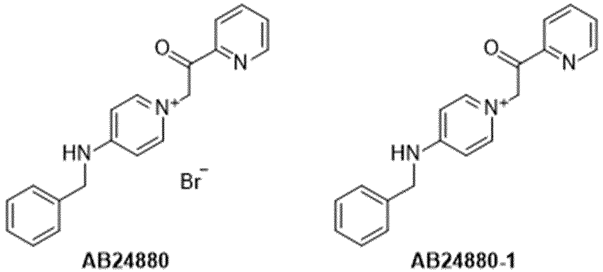

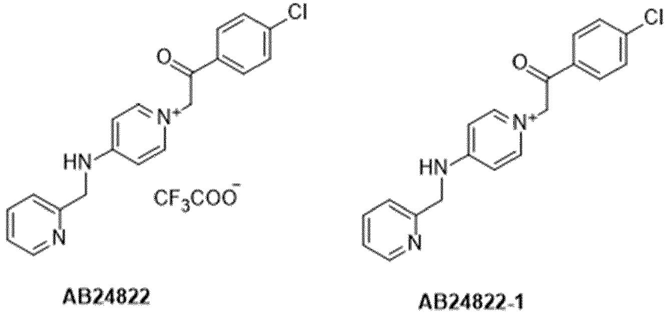

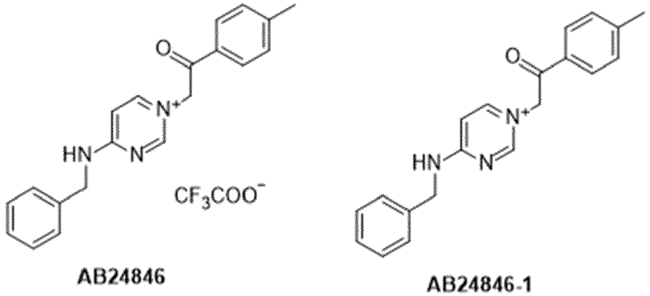

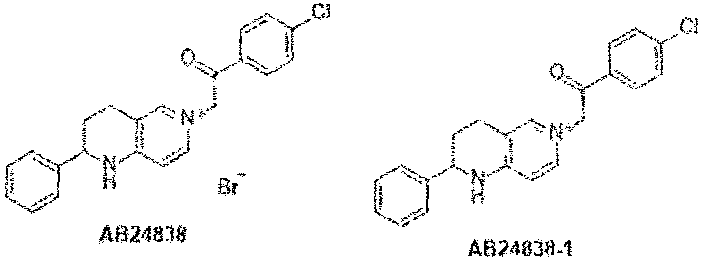

In the second aspect of the present invention, it provides a pharmaceutical composition, the pharmaceutical composition comprises (a) the compound of formula I, or an optical isomer thereof, or a racemate thereof, or a solvate thereof, or a pharmaceutically acceptable salt thereof according to the first aspect of the present invention; and (b) a pharmaceutically acceptable carrier.

In the third aspect of the present invention, it provides a use of the compound of formula I, or an optical isomer thereof, or a racemate thereof, or a solvate thereof, or a pharmaceutically acceptable salt thereof according to the first aspect of the present invention in the preparation of a composition or a preparation for preventing and/or treating cancer.

In another preferred embodiment, the cancer is selected from the group consisting of lung cancer, pancreatic cancer, breast cancer, lymphoma, prostate cancer, brain cancer, leukemia, liver cancer, melanoma, intestine cancer, renal carcinoma, colon cancer, and combinations thereof.

In another preferred embodiment, the tumor is human-derived tumor.

In another preferred embodiment, the tumor is human tumor.

In another preferred embodiment, the colon cancer comprises colon adenocarcinoma.

In another preferred embodiment, the mitochondria permeability transition pore of the cancer cell is low activity.

In another preferred embodiment, the cancer comprises the cancer with low activity of mitochondria permeability transition pore.

In another preferred embodiment, the low activity of mitochondria permeability transition pore means that the ratio (A1/A0) of the activity level or expression level A1 of mitochondria permeability transition pore in a cell (e.g., cancer cell) to the activity level or expression level A0 of mitochondria permeability transition pore in a normal cell (the same type of cell) is <1.0, preferably ≤0.8, preferably ≤0.7, more preferably ≤0.6, more preferably ≤0.5, more preferably ≤0.4, more preferably ≤0.3, more preferably ≤0.2, more preferably ≤0.1, most preferably ≤0.05.

In another preferred embodiment, the same type of cell refers to the cell ( e.g., the same type of tumor cell ) with normal activity level of mitochondria permeability transition pore.

In another preferred embodiment, the same type of cell refers to the same type of cell with normal activity level of mitochondria permeability transition pore.

In another preferred embodiment, the normal cell refers to normal tissue cell (e.g., tumor origin cell, tumor-adjacent cell or para-tumor tissue cell) with normal activity level of mitochondria permeability transition pore.

In another preferred embodiment, the breast cancer comprises triple negative breast cancer.

In another preferred embodiment, the liver cancer is anaplastic and lowly differentiated liver cancer.

In another preferred embodiment, the leukemia comprises acute myeloid leukemia.

In another preferred embodiment, the leukemia comprises type M4 of acute myeloid leukemia.

In another preferred embodiment, the lung cancer is selected from group consisting of small cell lung cancer, non-small cell lung cancer, and combinations thereof.

In another preferred embodiment, the lymphoid carcinoma comprises B cell lymphoma.

In another preferred embodiment, the intestine cancer comprises colorectal adenocarcinoma.

In another preferred embodiment, the colorectal adenocarcinoma comprises Duke's type C, grade IV of colorectal adenocarcinoma.

In another preferred embodiment, the brain cancer is selected from the group consisting of medulloblastoma, glioblastoma, and combination thereof.

In another preferred embodiment, the renal carcinoma comprises clear cell renal cell adenocarcinoma.

In another preferred embodiment, the cancer is cancer with up-regulation of mitochondrial oxidative phosphorylation pathway and/or low activity of mitochondria permeability transition pore in cancer cell.

In another preferred embodiment, the cancer is cancer with up-regulation of mitochondrial oxidative phosphorylation pathway in cancer cell.

In another preferred embodiment, the cancer comprises cancer with up-regulation of mitochondrial oxidative phosphorylation pathway.

In another preferred embodiment, the cancer is cancer with low activity of mitochondria permeability transition pore in cancer cell.

In another preferred embodiment, the up-regulation of mitochondrial oxidative phosphorylation pathway means that the ratio (E1/E0) of the level or expression E1 of mitochondrial oxidative phosphorylation pathway in a cell (e.g., cancer cell) to the level or expression E0 of mitochondrial oxidative phosphorylation pathway in a normal cell (the same type of cell) is >1.0, preferably ≥1.2, preferably ≥1.5, more preferably ≥2, more preferably ≥3, more preferably ≥5.

In another preferred embodiment, the same type of cell refers to the cell ( e.g., the same type of tumor cell ) with normal expression of mitochondrial oxidative phosphorylation pathway.

In another preferred embodiment, the same type of cell refers to the same type of cell with normal expression of mitochondrial oxidative phosphorylation pathway.

In another preferred embodiment, the normal cell refers to normal tissue cell (e.g., tumor origin cell, tumor-adjacent cell or para-tumor tissue cell) with normal expression of mitochondrial oxidative phosphorylation pathway.

In another preferred embodiment, the normal cell (the same type of cell) comprises the cell that is not sensitive to the compound of formula I, or an optical isomer thereof, or a racemate thereof, or a solvate thereof, or a pharmaceutically acceptable salt thereof.

In another preferred embodiment, the low activity of the mitochondria permeability transition pore means that the ratio (A1/A0) of the activity level or expression level A1 of mitochondria permeability transition pore in a cell (e.g., the cancer cell) to the activity level or expression level A0 of mitochondria permeability transition pore in a normal cell (the same type of cell) is <1.0, preferably ≤0.8, preferably ≤0.7, more preferably ≤0.6, more preferably ≤0.5, more preferably ≤0.4, more preferably ≤0.3, more preferably ≤0.2, more preferably ≤0.1, most preferably ≤0.05.

In another preferred embodiment, the cancer patient is administered mitochondria permeability transition pore inhibitor to make mitochondria permeability transition pore of cancer cell low activity.

In another preferred embodiment, the low activity of mitochondria permeability transition pore is made by administering mitochondria permeability transition pore inhibitor.

In another preferred embodiment, the level is protein level and/or mRNA level.

In another preferred embodiment, the expression is protein expression and/or mRNA expression.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the tumor (cancer) comprises tumor with low or no expression of NNMT gene.

In another preferred embodiment, the tumor (cancer) comprises tumor with high methylation level of nucleotide site of NNMT gene and/or high methylation level of DNA CpG site of NNMT gene.

In another preferred embodiment, the tumor comprises tumor with high methylation level of nucleotide site of NNMT gene.

In another preferred embodiment, the tumor (cancer) comprises tumor with high methylation level of DNA CpG site of NNMT gene.

In another preferred embodiment, the NNMT gene is human-derived NNMT gene.

In another preferred embodiment, the NNMT gene is human NNMT gene.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same type of cell or a normal cell (e.g., para-tumor tissue cell).

In another preferred embodiment, the tumor with low or no expression of NNMT gene means the ratio (E1/E0) of the expression level E1 of NNMT gene in the tumor cell to the expression level E0 of NNMT gene in the same type of cell or a normal cell (e.g., para-tumor tissue cell) is <1.0.

In another preferred embodiment, the low or no expression of NNMT gene means the ratio (E1/E0) of the expression E1 of NNMT gene in a cell ( e.g., cancer cell ) to the expression E0 of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the same type of cell refers to the cell ( e.g., the same type of tumor cell ) with normal expression of NNMT gene.

In another preferred embodiment, the same type of cell refers to the same type of cell with normal expression of NNMT gene.

In another preferred embodiment, the normal cell refers to normal tissue cell (e.g., tumor origin cell, tumor-adjacent cell or para-tumor tissue cell) with normal expression of NNMT gene.

In another preferred embodiment, E0 refers to the expression level of NNMT gene in the cell with normal expression of NNMT gene.

In another preferred embodiment, the cell with normal expression of NNMT gene comprises the cell that is not sensitive to the compound of formula I, or an optical isomer thereof, or a racemate thereof, or a solvate thereof, or a pharmaceutically acceptable salt thereof.

In another preferred embodiment, the high methylation level of nucleotide site of NNMT gene means the methylation level of nucleotide site of NNMT gene in a cell ( e.g., tumor cell) is higher than that in the same type of cell or a normal cell (e.g., para-tumor tissue cell).

In another preferred embodiment, the high methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the high methylation level of nucleotide site of NNMT gene means the methylation level of nucleotide site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the same type of cell refers to the cell ( e.g., the same type of tumor cell ) with normal methylation level of nucleotide site of NNMT gene.

In another preferred embodiment, the same type of cell refers to the same type of cell with normal methylation level of nucleotide site of NNMT gene.

In another preferred embodiment, the normal cell refers to normal tissue cell (e.g., tumor origin cell, tumor-adjacent cell or para-tumor tissue cell) with normal methylation level of nucleotide site of NNMT gene.

In another preferred embodiment, the cell with normal methylation level of nucleotide site of NNMT gene comprises the cell that is not sensitive to the compound of formula I, or an optical isomer thereof, or a racemate thereof, or a solvate thereof, or a pharmaceutically acceptable salt thereof.

In another preferred embodiment, the high methylation level of nucleotide site of NNMT gene means the methylation level (M%) of nucleotide site of NNMT gene in a cell ( e.g., tumor cell) is ≥ 3% and ≤ M1%, wherein M1 is any positive integer from 3 to 100.

In another preferred embodiment, M1 is 5, 10, 15, 20, 25, 30, 35, 40, 45 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the high methylation level of DNA CpG site of NNMT gene means the methylation level of DNA CpG site of NNMT gene in a cell ( e.g., tumor cell) is higher than that in the same type of cell or a normal cell (e.g., para-tumor tissue cell).

In another preferred embodiment, the high methylation level of DNA CpG site of NNMT gene means the ratio (W1/W0) of the methylation level W1 of DNA CpG site of NNMT gene in a cell ( e.g., tumor cell ) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the high methylation level of DNA CpG site of NNMT gene means the methylation level of DNA CpG site of NNMT gene in a cell ( e.g., tumor cell) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the same type of cell refers to the cell ( e.g., the same type of tumor cell ) with normal methylation level of DNA CpG site of NNMT gene.

In another preferred embodiment, the same type of cell refers to the same type of cell with normal methylation level of DNA CpG site of NNMT gene.

In another preferred embodiment, the normal cell refers to normal tissue cell (e.g., tumor origin cell, tumor-adjacent cell or para-tumor tissue cell) with normal methylation level of DNA CpG site of NNMT gene.

In another preferred embodiment, the cell with normal methylation level of DNA CpG site of NNMT gene comprises the cell that is not sensitive to the compound of formula I, or an optical isomer thereof, or a racemate thereof, or a solvate thereof, or a pharmaceutically acceptable salt thereof.

In another preferred embodiment, the high methylation level of DNA CpG site of NNMT gene means the methylation level (M%) of DNA CpG site of NNMT gene in a cell ( e.g., tumor cell) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of CpG site refers to the ratio of the number of methylated CpG nucleotides to the number of all nucleotides in a gene.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of CpG site refers to the ratio of the number of methylated CpG nucleotides to the number of all CpG nucleotides in a gene.

In another preferred embodiment, the methylation level of DNA CpG site of NNMT gene refers to the ratio of the number of methylated CpG nucleotides to the number of all CpG nucleotides in the NNMT gene.

In another preferred embodiment, the methylation level of DNA CpG site refers to the ratio of the number of methylated CpG sites to the number of all CpG sites in a DNA.

In another preferred embodiment, the methylation level of DNA CpG site refers to the ratio of the number of methylated CpG nucleotides to the number of all nucleotides in a DNA.

In another preferred embodiment, the methylation level of DNA CpG site refers to the ratio of the number of methylated CpG nucleotides to the number of all CpG nucleotides in a DNA.

In another preferred embodiment, the methylation level of DNA CpG site of NNMT gene refers to the ratio of the number of methylated CpG sites to the number of all CpG sites in the NNMT gene.

In another preferred embodiment, the methylation level of DNA CpG site of NNMT gene refers to the ratio of the number of methylated CpG nucleotides to the number of all CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the cancer is selected from the group consisting of lung cancer, renal carcinoma, breast cancer, colon cancer, lymphoma, leukemia, pancreatic cancer, liver cancer, prostate cance, and combinations thereof.

In another preferred embodiment, the expression comprises protein expression and/or mRNA expression.

In another preferred embodiment, the composition is a pharmaceutical composition.

In another preferred embodiment, the composition or preparation further comprises a pharmaceutically acceptable carrier.

In another preferred embodiment, the expression is mRNA expression or protein expression.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In another preferred embodiment, the composition is a pharmaceutical composition.

In another preferred embodiment, the composition or preparation further comprises a pharmaceutically acceptable carrier.

In the fourth aspect of the present invention, it provides a use of a mitochondria permeability transition pore inhibitor in the preparation of a composition or preparation for enhancing the anticancer effect of anticancer drug.

In another preferred embodiment, the anticancer drug is the compound of formula I, or an optical isomer thereof, or a racemate thereof, or a solvate thereof, or a pharmaceutically acceptable salt thereof according to the first aspect of the present invention.

In another preferred embodiment, the cancer is as described in the first aspect of the present invention.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the CyP-D protein inhibitor is SfA, BKA and ADP (small molecule that regulate the activity of ANT protein).

In another preferred embodiment, the peroxide scavenger is selected from the group consisting of propofol, pyruvate, MCI-186, and combinations thereof.

In the fifth aspect of the present invention, it provides an active ingredient combination, the active ingredient combination comprises:
(1) a first active ingredient, the first active ingredient is anticancer drug; and
(2) a second active ingredient, the second active ingredient is mitochondria permeability transition pore inhibitor.

In another preferred embodiment, the molar ratio of the first active ingredient to the second active ingredient is 0.01-600:1, preferably 0.05-500:1, more preferably 0.1-400:1, more preferably 0.2-200:1, more preferably 0.5-100:1, more preferably 0.5-80:1, most preferably 1-50:1.

In another preferred embodiment, at least one active ingredient is independent in the active ingredient combination.

In another preferred embodiment, the first active ingredient and the second active ingredients are independent of each other in the active ingredient combination.

In the sixth aspect of the present invention, it provides a composition, the composition comprises:
(1) a first active ingredient, the first active ingredient is anticancer drug; and
(2) a second active ingredient, the second active ingredient is mitochondria permeability transition pore inhibitor.

In another preferred embodiment, the composition is a pharmaceutical composition.

In another preferred embodiment, the pharmaceutical composition further comprises a pharmaceutically acceptable carrier.

In another preferred embodiment, the content of the first active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In another preferred embodiment, the content of the second active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In the seven aspect of the present invention, it provides a medicine kit, the medicine kit comprises:
(A) a first preparation comprising a first active ingredient, the first active ingredient is anticancer drug; and
(B) a second preparation comprising a second active ingredient, the second active ingredient is mitochondria permeability transition pore inhibitor.

In another preferred embodiment, the medicine kit further comprises a user manual.

In another preferred embodiment, the first preparation and the second preparation are independent preparation of each other.

In another preferred embodiment, the first preparation and the second preparation are combined preparation.

In another preferred embodiment, the user manual records that the first preparation and the second preparation are used together to enhance anti-tumor activity of anticancer drug.

In another preferred embodiment, the method for using together means the second preparation comprising the mitochondria permeability transition pore inhibitor is administered firstly, then the anticancer drug is administered.

In the eighth aspect of the present invention, it provides a method for non-therapeutically and non-diagnostically inhibiting cancer cell in vitro, which comprises contacting the cancer cell with the compound of formula I, or an optical isomer thereof, or a racemate thereof, or a solvate thereof, or a pharmaceutically acceptable salt thereof according to the first aspect of the present invention, thereby inhibiting cancer cell.

In another preferred embodiment, the contact is performed in vitro culture.

In the ninth aspect of the present invention, it provides a method for preventing and/or treating cancers, which comprises administering the compound of formula I, or an optical isomer thereof, or a racemate thereof, or a solvate thereof, or a pharmaceutically acceptable salt thereof according to the first aspect of the present invention, or the active ingredient combination according to the fifth aspect of the present invention, or the composition according to the sixth aspect of the present invention, or the medicine kit according to the seventh aspect of the present invention to a subject in need, thereby preventing and/or treating cancer.

In another preferred embodiment, the subject is human and non-human mammals (rodent, rabbit, monkey, livestock, dog, cat, and the like).

In the tenth aspect of the present invention, it provides a marker for determining whether the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor, the marker comprises mitochondrial oxidative phosphorylation pathway, mitochondria permeability transition pore, NNMT gene, methylation level of nucleotide site of NNMT gene, and/or methylation level of DNA CpG site of NNMT gene.

In another preferred embodiment, the marker comprises the expression level of mitochondrial oxidative phosphorylation pathway, the activity of mitochondria permeability transition pore, the expression level of NNMT gene, the methylation level of nucleotide site of NNMT gene, and/or the methylation level of DNA CpG site of NNMT gene

In another preferred embodiment, the methylation level of DNA CpG site of NNMT gene comprises the methylation level of DNA CpG site in promoter region of NNMT gene.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with up-regulation of mitochondrial oxidative phosphorylation pathway.

In another preferred embodiment, the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low activity of mitochondria permeability transition pore.

In another preferred embodiment, the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low or no expression of NNMT gene, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt thereof according to the first aspect of the present invention is not suitable for use in the prevention and/or treatment of patient tumor with high expression of NNMT gene, low methylation level of nucleotide site of NNMT gene, and/or low methylation level of DNA CpG site of NNMT gene.

In the eleventh aspect of the present invention, it provides a detection kit, the detection kit comprises:
(i) a detection reagent for detecting the level of mitochondrial oxidative phosphorylation pathway, level of mitochondria permeability transition pore, the expression of NNMT gene, and/or methylation level of DNA CpG site of NNMT gene

In another preferred embodiment, the level comprises level of protein and/or level of mRNA.

In another preferred embodiment, the test sample of the detection kit comprises tumor cell.

In another preferred embodiment, the expression of NNMT gene is the expression of mRNA and protein.

In another preferred embodiment, the methylation level of DNA CpG site of NNMT gene is the methylation level of DNA CpG site in promoter region of NNMT gene.

In another preferred embodiment, the methylation level of DNA CpG site of NNMT gene is the methylation level of DNA CpG sites from 1050 bp before the transcription start site to 499 bp after the transcription start site in NNMT gene.

In another preferred embodiment, the methylation level of DNA CpG site of NNMT gene is the methylation level of DNA CpG sites from 1050 bp to 193 bp before the transcription start site in NNMT gene.

In the twelfth aspect of the present invention, it provides a use of the detection kit according to the twelfth aspect of the present invention in the preparation of concomitant diagnose kit for determining whether the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor.

In another preferred embodiment, the concomitant diagnose kit further comprises instruction or label.

In another preferred embodiment, the instruction or label records that the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with up-regulation of mitochondrial oxidative phosphorylation pathway.

In another preferred embodiment, the instruction or label records that the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low activity of mitochondria permeability transition pore.

In another preferred embodiment, the instruction or label records that the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low or no expression of NNMT gene, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof according to the first aspect of the present invention is not suitable for use in the prevention and/or treatment of patient tumor with high expression of NNMT gene, low methylation level of nucleotide site of NNMT gene, and/or low methylation level of DNA CpG site of NNMT gene.

In the thirteenth aspect of the present invention, it provides a medicine kit, the medicine kit comprises:
(i) a detection reagent for detecting the level of mitochondrial oxidative phosphorylation pathway, level of mitochondria permeability transition pore, expression level of NNMT gene, methylation level of nucleotide site of NNMT gene, and/or methylation level of DNA CpG site of NNMT gene; and
(ii) the compound of formula I, or an optical isomer thereof, or a racemate thereof, or a solvate thereof, or a pharmaceutically acceptable salt thereof according to the first aspect of the present invention.

In another preferred embodiment, the medicine kit further comprises instruction or label.

In another preferred embodiment, the instruction or label records that the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with up-regulation of mitochondrial oxidative phosphorylation pathway.

In another preferred embodiment, the instruction or label records that the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low activity of mitochondria permeability transition pore.

In another preferred embodiment, the instruction or label records that the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low or no expression of NNMT gene, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof according to the first aspect of the present invention is not suitable for use in the prevention and/or treatment of patient tumor with high expression of NNMT gene, low methylation level of nucleotide site of NNMT gene, and/or low methylation level of DNA CpG site of NNMT gene.

In the fourteenth aspect of the present invention, it provides a method for preventing and/or treating tumor, which comprises administering the compound of formula I, or an optical isomer thereof, or a racemate thereof, or a solvate thereof, or a pharmaceutically acceptable salt thereof according to the first aspect of the present invention to a subject in need.

In another preferred embodiment, the tumor is as described in the first aspect of the invention.

In another preferred embodiment, the cancer is cancer with up-regulation of mitochondrial oxidative phosphorylation pathway and/or low activity of mitochondria permeability transition pore in cancer cell.

In another preferred embodiment, the tumor of the subject comprises tumor with low or no expression of NNMT gene.

In another preferred embodiment, the tumor of the subject comprises tumor with high methylation level of nucleotide site of NNMT gene.

In another preferred embodiment, the tumor of the subject comprises tumor with high methylation level of DNA CpG site of NNMT gene.

In another preferred embodiment, the subject is human and non-human mammals (rodent, rabbit, monkey, livestock, dog, cat, and the like).

In the fifteenth aspect of the present invention, it provides a device or system, the device or system comprises:
(i) a detection module, the detection module is used to detect the level of mitochondrial oxidative phosphorylation pathway, level of mitochondria permeability transition pore, expression level of NNMT gene, methylation level of nucleotide site of NNMT gene, and/or methylation level of DNA CpG site of NNMT gene;
(ii) a output module, the output module comprises the output of the information as follows:
   the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with up-regulation of mitochondrial oxidative phosphorylation pathway;
   the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low activity of mitochondria permeability transition pore;
   the compound of formula I, or an optical isomer thereof, or a racemate thereof, or a solvate thereof, or a pharmaceutically acceptable salt thereof according to the first aspect of the present invention is suitable for use in the prevention and/or treatment of patient tumor with low or no expression of NNMT gene, high methylation level of nucleotide site of NNMT gene, and/or high methylation level of DNA CpG site of NNMT gene; and/or
   the compound of formula I, or an optical isomer thereof, or a racemate thereof, or a solvate thereof, or a pharmaceutically acceptable salt thereof according to the first aspect of the present invention is not suitable for use in the prevention and/or treatment of patient tumor with high expression of NNMT gene, low methylation level of nucleotide site of NNMT gene, and/or low methylation level of DNA CpG site of NNMT gene.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level of NNMT gen and/or the methylation level of DNA CpG site of NNMT gene can be obtained by the procession of the data processing module.

In another preferred embodiment, the expression level of NNMT gene and/or the methylation level of DNA CpG site in promoter region of NNMT gene can be obtained by the procession of the data processing module.

In another preferred embodiment, the expression level of NNMT gene and/or the methylation level of DNA CpG sites from 1050 bp before the transcription start site to 499 bp after the transcription start site in NNMT gene can be obtained by the procession of the data processing module.

In another preferred embodiment, the expression level of NNMT gene and/or the methylation level of DNA CpG sites from 1050 bp to 193 bp before the transcription start site in NNMT gene can be obtained by the procession of the data processing module.

It should be understood that, in the present invention, each of the technical features specifically described above and below (such as those in the Examples) can be combined with each other, thereby constituting new or preferred technical solutions which need not be redundantly described one-by-one.

### DESCRIPTION OF THE DRAWINGS

Fig.1 shows the expression of NNMT gene in sensitive and insensitive tumor cells.
Fig.2 shows the methylation level of DNA CpG site of promoter region of NNMT gene in sensitive and insensitive tumor cells.
Fig.3 shows the methylation level of DNA CpG sites from 1050 bp before the transcription start site to 499 bp after the transcription start site in NNMT gene in sensitive and insensitive tumor cells.
Fig.4 shows the methylation level of DNA CpG sites from 1050 bp to 193 bp before the transcription start site in NNMT gene in sensitive and insensitive tumor cell.

### EMBODIMENTS FOR CARRYING OUT THE INVENTION

Based on an extensive and intensive research, the inventors have unexpectedly found the specified compound of formula I, or an optical isomer thereof, or a racemate thereof, or a solvate thereof, or a pharmaceutically acceptable salt thereof of the present invention can effectively inhibit tumor. Furthermore, the inventors have unexpectedly found the compound of the present invention can significantly inhibit the activity of tumor cell with low activity of mitochondria permeability transition pore (mPTP) at a low concentration (IC50), indicating the compound of the present invention has more significant inhibitory effect on tumor cells with lowly active or inactive mPTP, while the inhibitory effect of the compound of the present invention on cell with highly active mPTP is weak. Generally, the mPTP activity of normal somatic cell is higher, thus the compound of the present invention has relatively little impact on normal somatic cell, and the drug is safe.

The inventors have unexpectedly found the compound of present invention has significant inhibitory effect on tumor with low or no expression of NNMT gene, high methylation level of nucleotide site of NNMT gene, and/or high methylation level of DNA CpG site of NNMT gene. The expression level of NNMT gene, the methylation level of nucleotide site of NNMT gene, and/or the methylation level of DNA CpG site in the promoter region of NNMT gene can be used as a marker for determining whether the compound of present invention is suitable for use in the prevention and/or treatment of patient tumor. On this basis, the inventors has completed the present invention.

### Terms

As used herein, the term "comprise", " comprising", and "containing" are used interchangeably, which not only comprise closed definitions, but also semi-closed and open definitions. In other words, the term comprises "consisting of" and "essentially consisting of".

As used herein, the term "anticancer drug", and "antitumor drug" are used interchangeably.

As used herein, the term "cancer", and "tumor" are used interchangeably.

As used herein, the term "a cell" refers to a cell (e.g., single tumor cell) or a group of cells containing multiple similar cells ((e.g., a tumor tissue).

As used herein, the term "IC50" refers to 50% inhibiting concentration, ie, the concentration of the inhibitor when 50% inhibitory effect is achieved.

As used herein, the term "IC50" and "IC50" are used interchangeably, and refers to 50% inhibiting concentration, ie, the concentration of the inhibitor when 50% inhibitory effect is achieved.

The term "mitochondria permeability transition pore" is abbreviated as mPTP.

The term "Oxidative Phosphorylation Pathway" is abbreviated as OXPHOS (Oxidative Phosphorylation), also known as oxidative phosphorylation.

As used herein, the term "high methylation level of DNA CpG site", "high level of DNA CpG site methylation" and "high methylation of DNA CpG site" are used interchangeably.

As used herein, the term "low methylation level of DNA CpG site", "low level of DNA CpG site methylation" and "low methylation of DNA CpG site" are used interchangeably.

As used herein, the term "methylation level of DNA CpG site" refers to the ratio of the number of methylated CpG sites to the number of all CpG sites in a DNA.

As used herein, the term "methylation of CpG site", "methylation of CpG nucleotide" and "CpG methylation" are used interchangeably.

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, the term "NNMT" refers to Nicotinamide N-Methyltransferase.

As used herein, the term "bp" refers to base pair.

As used herein, the term "SST" refers to the transcription start site.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by non-hydrogen atom group, but it needs to meet its valence requirements and the substituted compound is chemically stable, that is, the substituted compound does not spontaneously undergo transformations such as cyclization and elimination, etc.

As used herein, "R₁", "R1" and "R¹" have the same meaning and can be used interchangeably. The other similar definitions have the same meaning.

As used herein, " 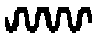 " denotes the linking site of the group.

As used herein, the term "alkyl" refers to a saturated hydrocarbon group with linear chain (ie, unbranched chain) or branched chain, or a combination of linear and branched chains. When the number of carbon atoms is limited in front of the alkyl (e.g., C1-C6 alkyl), it means that the alkyl has 1-6 carbon atoms, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "C2-C4 alkenyl" refers to a hydrocarbon group formed by the loss of one hydrogen atom connected to the double bond in linear or branched alkene with 2-4 carbon atoms and one or more double bonds, such as CH₂=CH-, C (CH₃) ₂=CH-, or the like.

As used herein, the term "C2-C4 alkynyl" refers to a hydrocarbon group formed by the loss of one hydrogen atom connected to the triple bond in linear or branched alkyne with 2-4 carbon atoms and one or more double bonds, such as (CH≡CH-), (H₃C-C≡CH-), or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the alkyl (e.g., C1-C8 haloalkyl), it means that the alkyl has 1-8 carbon atoms, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkane ring" refers to a cyclic ring having a saturated or partially saturated monocyclic ring, bicyclic ring or polycyclic ring (fused ring, bridged ring or spiro ring). When the number of carbon atoms is limited in front of the cycloalkane ring (e.g., C3-C12 cycloalkane ring), it means the cycloalkane ring has 3-12 ring carbon atoms. In some preferred embodiments, the term "C3-C8 cycloalkane ring" refers to a saturated or partially saturated monocycloalkane ring or dicycloalkane ring having 3-8 ring carbon atoms, comprising cyclopropane ring, cyclobutane ring, cyclopentane ring, cycloheptane ring, or the like. The term "spirocycloalkane ring" refers to a bicyclic or polycyclic ring that shares a carbon atom (referred to spiro-atom) between single rings, the spirocycloalkane ring can contain one or more double bonds, but no ring has a fully conjugated π electron system. Fused cycloalkane ring refers to all carbon bicyclic or polycyclic rings in which each ring in the system shares an adjacent pair of carbon atoms with other rings, one or more rings can contain one or more double bonds, but the shared adjacent pair of carbon atoms connecting the parent body is an adjacent pair of carbon atoms on an all carbon bicyclic or polycyclic ring with non-conjugated π electron system. Bridged cycloalkane ring refers to all carbon polycyclic ring in which any two rings share two non-directly connected carbon atoms, the bridged cycloalkane ring can contain one or more double bonds, but no ring has a fully conjugated π electron system. The representative examples of cycloalkane ring comprise but are not limited to cyclopropane ring, cyclobutane ring, cyclopentane ring, cycloheptane ring, or the like.

As used herein, the term "cycloalkyl" refers to a cyclic group having a saturated or partially saturated monocyclic ring, bicyclic ring or polycyclic ring (fused ring, bridged ring or spiro ring). When the number of carbon atoms is limited in front of the cycloalkyl (e.g., C3-C12 cycloalkyl), it means the cycloalkyl has 3-12 ring carbon atoms. In some preferred embodiments, the term "C3-C8 cycloalkyl" refers to a saturated or partially saturated monocycloalkyl or dicycloalkyl having 3-8 ring carbon atoms, comprising cyclopropyl, cyclobutyl, cyclopentane , cycloheptyl, or the like. The term "spirocycloalkyl" refers to a bicyclic or polycyclic group that shares a carbon atom (referred to spiro-atom) between single rings, the spirocycloalkyl can contain one or more double bonds, but no ring has a fully conjugated π electron system. Fused cycloalkyl refers to all carbon bicyclic or polycyclic groups in which each ring in the system shares an adjacent pair of carbon atoms with other rings, one or more rings can contain one or more double bonds, but the linking site connecting the parent must be on the carbon atom on the ring with a non conjugated π electron system. Bridged cycloalkyl refers to all carbon polycyclic group in which any two rings share two non-directly connected carbon atoms, the bridged cycloalkyl can contain one or more double bonds, but no ring has a fully conjugated π electron system. The representative examples of cycloalkyl comprise but are not limited to

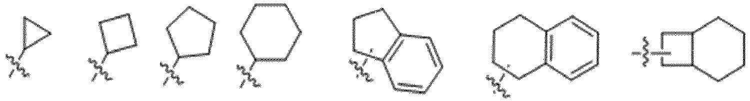

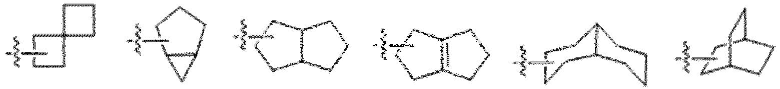

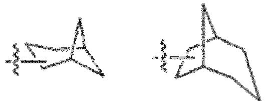

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the cycloalkyl (e.g, C3-C8 haloalkyl), it means that the cycloalkyl has 3-8 ring carbon atoms, for example, C3-C8 haloalkyl refers to an halocycloalkyl having 3-6 carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "alkylthio" refers to R-O- group, wherein R is alkyl, the alkyl is as defined above. When the number of carbon atoms is limited in front of the alkylthio, for example, C1-C8 alkylthio means that the alkyl in the alkylthio has 1-8 carbon atoms. Representative examples of alkylthio comprise but are not limited to methylthio, ethylthio, n-propylthio, isopropylthio, tert-butylthio, or the like.

As used herein, the term "haloalkoxyl" refers to haloalkyl-O-, wherein the haloalkyl is as defined above, for example, C1-C6 haloalkoxyl refers to a haloalkoxyl having 1-6 carbon atoms. Representative examples of haloalkoxyl comprise but are not limited to monofluoromethoxyl, monofluoroethoxyl, bisfluorobutoxyl, or the like.

As used herein, the term "haloalkylthio" refers to haloalkyl-S-, wherein the haloalkyl is as defined above, for example, C1-C6 haloalkylthio refers to a haloalkylthio having 1-4 carbon atoms. Representative examples of haloalkylthio comprise but are not limited to monofluoromethylthio, monofluoroethylthio, difluorobutylthio, or the like.

The term "heterocycloalkane ring" refers to fully saturated or partially unsaturated ring (comprising but not limited to such as 3-7 membered monocyclic ring, 7-11 membered bicyclic ring, or 8-16 membered tricyclic ring), at least one heteroatom is present in a ring with at least one carbon atom. When the number of members is limited in front of the heterocycloalkane ring, it refers to the number of ring atoms in the heterocycloalkane ring, for example, 3-16 membered heterocycloalkane ring refers to a heterocycloalkane ring having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Heterocycloalkane ring can be linked to any heteroatom or carbon atom residue of ring or ring system molecule. Representative examples of monocyclic heterocycloalkyl comprise but are not limited to azetidine ring, oxetane ring, imidazoline ring, tetrahydrofuran ring, piperidine ring, piperazine ring, 2-oxypiperazine ring, 2-oxypiperidine ring, 4-piperidone ring, tetrahydropyran ring, morpholine ring, thiomorpholine ring, thiomorpholine sulfoxide ring, thiomorpholine sulfone ring, 1,3-dioxane ring, and tetrahydro-1,1-dioxothiophene ring. Polycyclic heterocycloalkane ring comprises spiro, fused and bridged heterocycloalkane ring, the spiro, fused and bridged heterocycloalkyl is optionally linked with other rings by single bond, or further linked with other cycloalkyl rings, heterocyclic rings, aromatic ring or heteroaromatic ring by any two or more atoms on the ring, the linking site connecting the parent must be on the carbon atom or heteroatom on the ring with a non conjugated π electron system.

The term "heterocycloalkyl" refers to fully saturated or partially unsaturated cyclic group (comprising but not limited to such as 3-7 membered monocyclic ring, 7-11 membered bicyclic ring, or 8-16 membered tricyclic ring) , at least one heteroatom is present in a ring with at least one carbon atom. When the number of members is limited in front of the heterocycloalkyl, it refers to the number of ring atoms in the heterocycloalkyl, for example, 3-16 membered heterocycloalkyl refers to a heterocycloalkyl having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Heterocycloalkyl can be linked to any heteroatom or carbon atom residue of ring or ring system molecule. Representative examples of monocyclic heterocycloalkyl comprise but are not limited to azetidinyl, oxetanyl, imidazolidinyl, tetrahydrofuranyl, piperidinyl, piperazinyl, 2-oxypiperazinyl, 2-oxypiperidinyl, 4-piperidone group, tetrahydropyranyl, morpholine, thiomorpholine, thiomorpholine sulfoxide, thiomorpholine sulfone, 1,3-dioxane group, and tetrahydro-1,1-dioxothiophene. Polycyclic heterocycloalkyl comprises spiro, fused and bridged heterocyclyl, the spiro, fused and bridged heterocycloalkyl is optionally linked with other groups by single bond, or further linked with other cycloalkyl rings and heterocyclic rings by any two or more atoms on the ring. Polycyclic heterocycloalkyl can have fused aromatic ring, but the linking site connecting the parent must be on the carbon atom or heteroatom on the ring with a non conjugated π electron system.

The term "aromatic ring" refers to an all carbon monocyclic ring or fused polycyclic ring (i.e., a ring that share adjacent carbon atom pairs) with a conjugated π electron system, which is aromatic cyclic hydrocarbon compound. When the number of carbon atoms is limited in front of the aromatic ring, for example, C6-C12 aromatic ring means that the aromatic ring has 6-12 ring carbon atoms, such as benzene ring and naphthalene ring. The aromatic ring can be fused with other carbon ring (comprising saturated or unsaturated rings), but cannot have heteroatoms such as nitrogen, oxygen, or sulfur, and the linking site connecting the parent must be on the carbon atom of the ring with a conjugated π electron system. The representative examples of aromatic ring are benzene ring and naphthalene ring, or the like.

The term "aryl" refers to an all carbon monocyclic ring or fused polycyclic ring (i.e., a ring that share adjacent carbon atom pairs) group with a conjugated π electron system, which is aromatic cyclic hydrocarbon compound group. When the number of carbon atoms is limited in front of the aryl, for example, C6-C12 aryl means that the aryl has 6-12 ring carbon atoms, such as phenyl and naphthyl. The aromatic ring can be fused with other cyclic groups (comprising saturated or unsaturated rings), but cannot have heteroatoms such as nitrogen, oxygen, or sulfur, and position connecting the parent must be on the carbon atom of the ring with a conjugated π electron system. The representative examples of aryl comprises but are not limited to:

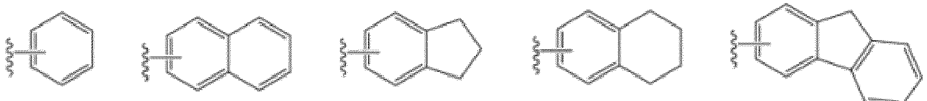

The term "heteroaromatic ring" refers to aromatic heterocyclic ring having one to more (preferably 1, 2, 3 or 4) heteroatoms, the heteroaromatic ring can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaromatic ring, it refers to the number of ring atoms on the heteroaromatic ring, for example, 5-12 membered heteroaromatic ring refers to a heteroaromatic ring having 5-12 ring atoms. Representative examples comprise but are not limited to pyrrole ring, pyrazole ring, imidazole ring, oxazole ring, isoxazole ring, thiazole ring, thiadiazole ring, isothiazole ring, furan ring, pyridine ring, pyrazine ring, pyrimidine ring, pyridazine ring, triazine ring, triazole ring, and tetrazolel ring, etc.

The term "heteroaryl" refers to aromatic heterocyclic ring group having one to more (preferably 1, 2, 3 or 4) heteroatoms, the heteroaryl can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaryl, it refers to the number of ring atoms of the heteroaryl, for example, 5-12 membered heteroaryl refers to a heteroaryl having 5-12 ring atoms. Representative examples comprise but are not limited to pyrrolyl, pyrazolyl, imidazolyl, oxazolyl, isoxazolyl, thiazolyl, thiadiazolyl, isothiazolyl, furanyl, pyridyl, pyrazinyl, pyrimidinyl, pyridazinyl, triazinyl, triazolyl and tetrazolyl, etc.

As used herein, the term "carboxyl" refers to -COOH or -alkyl-COOH, the alkyl is as defined above. For example, "C2-C4 carboxyl" refers to -C1-C3 alkyl-COOH. Representative examples of carboxyl comprise but are not limited to -COOH, - CH₂COOH, -C₂H₄COOH, or the like.

As used herein, the term "ester group" refers to R-CO-O- or -CO-O-R, wherein, R is alkyl, the alkyl is defined as above. For example, "C2-C4 ester group" refers to C1-C3 alkyl-CO-O- or -COO-C1-C3 alkyl. Representative examples of ester group comprise but are not limited to CH₃COO-, C₂H₅COO-, C₃H₈COO-, (CH₃)₂CHCOO-, -COOCH₃, -COOC₂H₅, -COOC₃H₈, or the like.

As used herein, the term "amide group" refers to R-C)-N- or -CO-N-R , wherein, R is alkyl, the alkyl is defined as above. For example, "C2-C4 amide group" refers to C1-C3 alkyl-CO-N- or -CON-C1-C3 alkyl. Representative examples of amide group comprise but are not limited to CH₃CON-, C₂H₅CO-N-, C₃H₈CO-N-, (CH₃)₂CHCO-N-, -CO-N-CH₃, -CO-N-C₂H₅, -CO-N-C₃H₈, or the like.

As used alone or as part of other substituent, the term "amino" refers to -NH₂.

As used alone or as part of other substituent, the term 'nitro' refers to -NO₂.

As used alone or as part of other substituent, the term "cyano" refers to -CN.

As used alone or as part of other substituent, the term "hydroxyl" refers to -OH.

As used alone or as part of other substituent, the term "sulfhydryl" refers to -SH.

In present invention, it should be understood that all substituents are unsubstituted, unless explicitly described herein as "substituted". The term "substituted" means that one or more hydrogen atoms on the specified group are substituted by specified substituent. The specific substituent is the substituent as described above, or the substituent in each example. Preferably, the "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl. Unless otherwise specified, each substituted group can have a substituent selected from a specified group at any substituted position of the group, the substitution can be the same or different at each substituted position.

In the present invention, the term "prevention" refers to a method for preventing the occurrence of disease and/or its accompanying symptoms, or protecting a subject from getting disease. The term 'prevention' further comprises delaying the occurrence of disease and/or its accompanying symptoms and reducing the risk of getting disease for subject.

In the present invention, the term "treatment" comprises delaying and terminating the progression of the disease, or eliminating the disease, and it does not require 100% inhibition, elimination and reversal. In some embodiments, compared to the level observed in the absence of the composition, medicine kit, food kit, or health product kit, or active ingredient combination of the present invention, the composition or pharmaceutical composition of present invention alleviates, inhibits and/or reverses tumor such as at least about 10%, at least about 30%, at least about 50%, or at least about 80%

In the present invention, the term "prevention" refers to a method for preventing the occurrence of disease and/or its accompanying symptoms, or protecting a subject from getting disease.

In the present invention, the term "treatment" comprises delaying and terminating the progression of the disease, or eliminating the disease, and it does not require 100% inhibition, elimination and reversal. In some embodiments, compared to the level observed in the absence of the compound of the present invention, the compound of present invention alleviates, inhibits and/or reverses related diseases (e.g., tumor) and its complications such as at least about 10%, at least about 30%, at least about 50%, at least about 80% ,at least about 90%, or 100%.

### Mitochondrial oxidative phosphorylation pathway and mitochondria permeability transition pore

Oxidative Phosphorylation (OXPHOS) is one of the most important pathways in mitochondria, which utilizes NADH and FADH derived from tricarboxylic acid cycle and fat oxidation, etc to produce ATP. The mitochondrial oxidative phosphorylation pathway is composed of more than 90 proteins, which form five protein complexes, complexes I, II, III, IV and V.

Mitochondrial oxidative phosphorylation pathway is regulated by mitochondria permeability transition pore (mPTP). The compound of the present invention have better inhibitory effect on cell with inactive mPTP.

As used herein, the term "the up-regulation of mitochondrial oxidative phosphorylation pathway" and "positive mitochondrial oxidative phosphorylation pathway" are used interchangeably, which means that the level or expression of mitochondrial oxidative phosphorylation pathway in a cell (e.g.,cancer cell) is higher than the that in a normal cell (the same type of cell). Preferably, the term "up-regulation of mitochondrial oxidative phosphorylation pathway" or "positive mitochondrial oxidative phosphorylation pathway" means that the ratio (E1/E0) of the level or expression E1 ofmitochondrial oxidative phosphorylation pathway in a cell (e.g., tumor cell) to the level or expression E0 of mitochondrial oxidative phosphorylation pathway in a normal cell (the same type of cell) is >1.0, preferably ≥1.2, preferably ≥ 1.5, more preferably ≥2, more preferably ≥3, more preferably ≥5.

In the present invention, "up-regulation of mitochondrial oxidative phosphorylation pathway" and "positive mitochondrial oxidative phosphorylation pathway" can also be characterized by the activity of mPTP. Inactive mPTP means "up-regulation of mitochondrial oxidative phosphorylation pathway" and "positive mitochondrial oxidative phosphorylation pathway ", for example, in the case that the ratio (A1/A0) of the activity level or expression level A1 of mPTP in a cell (e.g., cancer cell) to the activity level or expression level A0 of mPTP in a normal cell (the same type of cell) is <1.0, preferably ≤0.8, preferably ≤0.7, more preferably ≤0.6, more preferably ≤0.5, more preferably ≤0.4, more preferably ≤0.3, more preferably ≤0.2, more preferably ≤0.1, most preferably ≤0.05, it can be considered that the mitochondrial oxidative phosphorylation pathway of the cell is up-regulated and positive.

In a preferred embodiment, the level is protein level and/or mRNA level.

In a preferred embodiment, the expression is protein expression and/or mRNA expression.

In the present invention, the level or expression of oxidative phosphorylation pathway, activity level or expression level of mPTP can be measured by conventional method, such as measuring the activity of mPTP, or measuring the expression level of mPTP at the protein level or mRNA level.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF_000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) to the transcription start site itself and 499 bp after the transcription start site (underlined section) in NNMT gene, the total length of promoter region of NNMT gene is 2500 bp, The nucleotide sequence of the promoter region of NNMT gene is as shown in SEQ ID NO: 1 as follows:
**SEQ ID NO: 1:**

TATCCAAGAGCTATCAGCACTCCCATGTTTATTGTAGCACTGTTCACAATA
GCCAAGATTTGGAAGTACTCTAAGTGTCCATTAGCAGATGAATGGATAAAGAC
AATGTGGTAATACACATAATGGAGTACTATTCAGTCATAAAGAAGAATTAGAT
CCTGTCATTTGCAATAACATGGATGGAACTGGAGGTCATAATGTTGAGTGAAA

TAAACCAGGCACAGAAAGACAAACTTTGCATGTTCTCACTTATTTATGGGAGC
TAAAAACTAAAATAACTGAACTCACAGAGATAGAGAGTAGAAGGATGGTTACG
AGAGGATGGGAAGGGTAGCGAGGTGGGTAGGGGGGATGTGGGGATCATTAA
TGGGTATAAAAAATAGTTAGAGGCCAGGCGCAGTGGCTCACGCCTGTAATCC
CAGCACTTTGGGAGGCCGAGGTAGGCGGAACACCTGAGGAGTTCAAGACCAG
CCTGGCCAATATGATGAAACCCCGTCTCTACTAAAAATACAAAAATTAGCTGG
GCGTGATGGTGTGCACCTGTAGTCCCAGCTGCTTGGGAGGCTGAGGCAGGAG
AATCGCTGGAACCCAAGAGGTGAAGGTTGCAGTGAGCTGAGATCGCGTCACT
GCACTCCAGCCTGGGTGACAGAGTGAGACTCCACATCAAAAAAAAAAAAAAA
AAGTTAGAAAGATTGAATAAGACCTAATATTTGCTAGCACAACAGGGTGAATA
TAGTAAAAAATAATTTATTTGTACCTTCAAAAATAACTAGACAAGTATAATTGG
GTTGTTTGTAACACACAAAAAATAAGTACTTGAAGTGGTGGATACCCCATTTA
CCCTGATGTGATTATTTTGTATTGCAGGCCTCTATCAGAATATCTCATGTAACC
CATAAATATATACACCTACTCTGTACCCACAAAAAGTTTTTAAAAAGAAAAATA
AATAGCAACCGAAAAAAAAAGAGAGGGAGAAAAGAAAAAAGAAAAAAAAATC
AAGTGCCTGGCTGGGTAGAATAAATTCTAAGGCCACAATGTTACTGACCATGG
GTTTTTTGGCTCTCAGTGTATAGAAATTGACACAAGGCCAATAGTCTTCCCAA
ACATGCTTTACTGGAACTTACGCCCTGGCATAAGGGCCACAACAAAAGAGAGA
GCGAATTCTCTGGCTTGCTGACTCCTTGGAAAAAACCGGTAGGGATTTTTTTA
TTAGGCAAAGCACAGGAATTGACGTCAGAGGCAGGATGTGCTGCTGGGCAAA
GCATACGAGAAGTGGGGTATGCAGGTCAGCATTACTTGGTTGCAATGGTTATC
TTGAGGAATGGGCCAACTGGTGGTCTGGCCAGTGGCAACAAGGCTGTAAATC
AATTATTCAGCATTCCTTCCCAAGGTGGGACACCCGGCAACATTGTTTATCTC
CTAAGGCCAGTTCCTGGAATTAAGTGAAAGGATGACTAATGGACATGTTGTCA
GTGAGGTAGTGGTGTGGGTTTTGTGACCAGTGGGAATGCACGAAAGAATGCT
TTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGA
CAGAGTCTTGCTCTGTCGCCCAGGCTGGAGTGCAGTGGCGCCATCTTGGCTC
ACTGCAAGCTCCGCCCCCCGGGTTCATGCCATTCTCCTGCCTCAGCCTCCCAA
GTAGCTGGGACTGCAGGTGCCTACCACCAAGCCCAGCTAATTTTTTGTATTTTT
TTTTTAGTAGAGACGGGGTTTCACCATGTTAGCCAGGATGGTCTCGATCTCC
TGACCTCGTGATCTGCCCGCCTTAGCCTCCCAAAGTGCTGGGATTACAGGCAT
GAGCCTCCGCCCCCGGCCTTAAATAATTCTTAAAGGAAGTAAAGTTAACTTTG
AAAGAACTATCAGGATTTGGATTGACTGAAAGGAGTGGGGAAGCTTAGGGAG
GAGGTGCTTGCCAGACACTGGGTCATGGCAGTGGTCGGTGAAGCTGCAGTTGCCTA
GGGCAGGGATGGAGAGAGAGTCTGGGCATGAGGAGAGGGTCTCGGGATGTTTGGC
TGGACTAGATTTTACAGAAAGCCTTATCCAGGCTTTTAAAATTACTCTTTCCAGACT
TCATCTGAGACTCCTTCTTCAGCCAACATTCCTTAGCCCTGAATACATTTCCTATCC
TCATCTTTCCCTTCTTTTTTTTCCTTTCTTTTACATGTTTAAATTTAAACCATTCTTCG
TGACCCCTTTTCTTGGGAGATTCATGGCAAGAACGAGAAGAATGATGGTGCTTGTT
AGGGGATGTCCTGTCTCTCTGAACTTTGGGGTCCTATGCATTAAATAATTTTCCTGA
CGAGCTCAAGTGCTCCCTCTGGTCTACAATCCCTGGCGGCTGGCCTTCATCCCTTGG
GCAAGCATTGCATACAGCTCATGGCCCTCCCTCTACCATACCC.

In the present invention, the nucleotide sites from 1050 bp before the transcription start site to 499 bp after the transcription start site in NNMT gene is sites 951-2500 of nucleotide sequence as shown in SEQ ID NO: 1.

In the present invention, the nucleotide sites from 1050 bp to 193 bp before the transcription start site in NNMT gene is sites 951-1808 of nucleotide sequence as shown in SEQ ID NO: 1.

### DNA methylation

DNA methylation is a form of chemical modification of DNA, which can change genetic performance under no change of DNA sequence. The DNA methylation means that a methyl is covalently linked on at position C-5 of cytosine in the CpG dinucleotide of genome under the action of DNA methyltransferase. Many studies have shown that DNA methylation can cause changes in chromatin structure, DNA conformation, DNA stability and the way DNA interacts with protein, thereby regulating gene expression.

DNA methylation is one of the earliest discovered and most deeply studied epigenetic regulatory mechanisms. Broadly speaking, DNA methylation refers to the chemical modification process in which a specific base in the DNA sequence is modified with a methyl by covalent bonding with S-adenosyl methionine (SAM) as methyl donor under the catalysis of DNA methyltransferase (DNMT). This DNA methylation can occur at position C-5 of cytosine, position N-6 of adenine and position N-7 of guanine. DNA methylation in general studies mainly refers to the methylation process that occurs at the carbon atom of position C-5 on cytosine in CpG dinucleotides, the product is called 5-methylcytosine (5-mC). The 5-methylcytosine (5-mC) is the main form of DNA methylation in eukaryotic organisms such as plant and animal. DNA methylation, as a relatively stable modification state, can be passed on to new generations of DNA during DNA replication process under the action of DNA methyltransferase, which is an important epigenetic mechanism.

There are two types of DNA methylation reactions. One type is that the DNA with two unmethylated strands is methylated, which is called denovo methylation; The other type is that the unmethylated strand of double-stranded DNA with one methylated strand and one unmethylated strand is methylated, which is called maintenance methylation.

Typically, DNA methylation is the methylation of DNA CpG site. The distribution of CpG dinucleotide is very uneven in the human genome, while CpG remains or is higher than normal level in some regions of the genome. The CpG site enrichment region (also known as CpG island ) is mainly located in the promoter region and exon regions of the gene, which is a region rich in CpG dinucleotide. About 60% of promoter of the gene contains CpG island. The CpG is the abbreviation of cytosine (C)-phosphate (p)-guanine (G).

Gene expression is regulated by various signaling pathways, transcription factors and epigenetic modifications in the cell. DNA methylation modification is an important way in which epigenetic modifications regulate gene expression. The level of DNA methylation in a specific gene region often affects the expression level of gene. Compared to the regulation of gene expression induced by signal transduction pathways and transcription factors, the effect of DNA methylation modification on gene expression is more stable in epigenetic modification, which is not easily affected by the extracellular environment. DNA methylation modification can be easily and accurately detected using existing technologies, so the DNA methylation is an ideal biomarker.

### Compound

As used herein, the terms "compound of the present invention", "compound of formula I of the present invention" and "compound of formula I" are used interchangeably, and refer to a compound of formula I, or an optical isomer thereof, or a racemate thereof, or a solvate thereof, or a pharmaceutically acceptable salt thereof. It should be understood that the term also comprises a mixture of the above components.

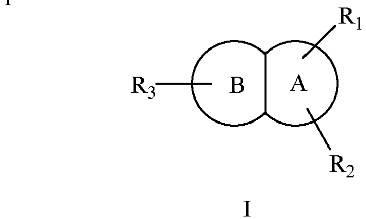

Specifically, the compound of formula I is as described above in the first aspect of the present invention.

Representative, the compound of formula I of the present invention is the compound (comprising salt thereof or free form without salt root) prepared in Examples 1-105.

In the present invention, it should be understood that when the ring B is absent, R₃ is none, ie, when the ring B is absent the compound of formula I has the following structure:

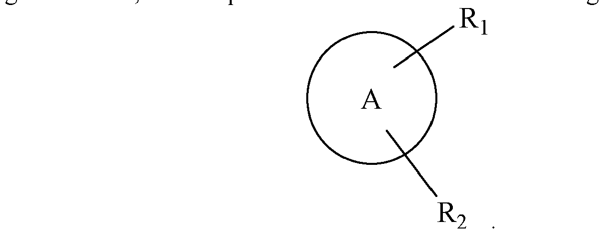

The compound of formula I of the present invention can be prepared using the known organic synthesis method in the art.

The compound of the present invention has excellent inhibitory effect on tumor cell, and has more significant inhibitory effect on tumor cell with up-regulation of mitochondrial oxidative phosphorylation pathway or low activity of mPTP, especially on tumor cell with always closed mPTP. At the same time, due to the high activity of mPTP in normal somatic cell, the compound of the present invention has weaker inhibition on normal somatic cells, and has less toxic and side effects, so the compound of the present invention can be developed into a relatively safe and effective anticancer drug.

The term "pharmaceutically acceptable salt" refers to a salt formed by the compound of the present invention and an acid or a base suitable for use as a drug. Pharmaceutically acceptable salt comprises inorganic salt and organic salt. A preferred type of salt is the salt formed by the compound of the present invention and an acid. Acids suitable for salt formation comprise but are not limited to inorganic acid such as hydrochloric acid, hydrobromic acid, hydrofluoric acid, sulfuric acid, nitric acid, phosphoric acid and the like; organic acid such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid and the like; and acidic amino acid such as aspartic acid and glutamic acid. A preferred type of salt is a metal salt formed by the compound of the present invention and a base. Suitable bases for salt formation comprise but are not limited to inorganic base such as sodium hydroxide, potassium hydroxide, sodium carbonate, sodium bicarbonate, sodium phosphate and the like; and organic base such as ammonia, triethylamine, diethylamine and the like.

The compound of formula I of the present invention can be converted into its pharmaceutically acceptable salt using conventional methods. For example, a solution of corresponding acid can be added into the solution of above compounds, and the solvent is removed after the salt is formed, thereby forming the corresponding salt of the compound of the present invention.

The compound of the present invention is preferably prepared in Examples.

### Cancer

The study of the present invention has shown that the compound of the present invention has significant inhibitory effect on tumor cell. Especially the compound of the present invention has more significant inhibitory effect on tumor cell with up-regulation of mitochondrial oxidative phosphorylation pathway, low activity of mPTP, low or no expression of NNMT gene, high methylation level of nucleotide site of NNMT gene, and/or high methylation level of DNA CpG site of NNMT gene.

Specifically, the tumor of present invention is as described above in the first aspect of the present invention.

### Anticancer drug

In the present invention, the anticancer drug can be the compound of formula I, or an optical isomer thereof, or a racemate thereof, or a solvate thereof, or a pharmaceutically acceptable salt thereof

### Mitochondria permeability transition pore inhibitor and use thereof

In the present invention, the mitochondria permeability transition pore inhibitor comprises but are not limited to Cyclosporin A, CyP-D protein inhibitor, peroxide scavenger, and combinations thereof.

Representatively, the CyP-D protein inhibitor is SfA, BKA and ADP (small molecule that regulate the activity of ANT protein).

Representatively, the peroxide scavenger comprises but are not limited to propofol, pyruvate, MCI-186, and combinations thereof.

### Marker

The present invention provides a marker for determining whether the compound of present invention is suitable for use in the prevention and/or treatment of patient tumor, the marker comprises mitochondrial oxidative phosphorylation pathway, mitochondria permeability transition pore, NNMT gene, methylation level of nucleotide site of NNMT gene, and/or methylation level of DNA CpG site of NNMT gene.

### Composition or preparation, active ingredient combination, medicine kit and administration method

The present invention provides a composition or preparation, active ingredient combination, and medicine kit, the composition or preparation, active ingredient combination, and medicine kit can be used for preventing and/or treating cancer.

Preferably, the composition of the present invention is pharmaceutical composition. The compositions of the present invention can comprise a pharmaceutically acceptable carrier.

The term "pharmaceutically acceptable carrier" refers to one or more compatible solid, semi-solid, liquid or gel fillers, which are suitable for use in human or animal and must have sufficient purity and sufficiently low toxicity. The "compatible" means each component and drug active ingredient in the pharmaceutical composition can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carrier are cellulose and its derivatives (e.g., methylcellulose, ethylcellulose, hydroxypropylmethylcellulose, sodium carboxymethylcellulose, etc.), gelatin, talc, solid lubricants (e.g., stearic acid, magnesium stearate), calcium sulfate, plant oil (e.g., soybean oil, sesame oil, peanut oil, olive oil, etc.), polyols (e.g., propylene glycol, glycerin, mannitol, sorbitol, etc.), emulsifier (e.g., Tween), wetting agent (e.g., sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In vitro study and in vivo administration (e.g., intratumoral administration), mitochondria permeability transition pore inhibitor can enhance the therapeutic effect of anti-tumor drug by lowing the activity of mPTP and up-regulating mitochondrial oxidative phosphorylation pathway in tumor cell. Therefore, anti-tumor drug and mPTP inhibitor can exert synergistic anti-tumor effect.

The present invention provides an active ingredient combination, composition and medicine kit comprising anticancer drug and mPTP inhibitor for synergistic anti-tumor.

The present invention provides an active ingredient combination, the active ingredient combination comprises:
(1) a first active ingredient, the first active ingredient is anticancer drug; and
(2) a second active ingredient, the second active ingredient is mitochondria permeability transition pore inhibitor.

In another preferred embodiment, at least one active ingredient is independent in the active ingredient combination.

In another preferred embodiment, the first active ingredient and the second active ingredients are independent of each other in the active ingredient combination.

The present invention further provides a composition, the composition comprises:
(1) a first active ingredient, the first active ingredient is anticancer drug; and
(2) a second active ingredient, the second active ingredient is mitochondria permeability transition pore inhibitor.

In another preferred embodiment, the pharmaceutical composition further comprises a pharmaceutically acceptable carrier.

In another preferred embodiment, the content of the first active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In another preferred embodiment, the content of the second active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

The present invention further provides a medicine kit, the medicine kit comprises:
(A) a first preparation comprising a first active ingredient, the first active ingredient is anticancer drug; and
(B) a second preparation comprising a second active ingredient, the second active ingredient is mitochondria permeability transition pore inhibitor.

In another preferred embodiment, the medicine kit further comprises a user manual.

In another preferred embodiment, the first preparation and the second preparation are independent preparation of each other.

In another preferred embodiment, the first preparation and the second preparation are combined preparation.

In another preferred embodiment, the user manual records that the first preparation and the second preparation are used together to enhance anti-tumor activity of anticancer drug.

In another preferred embodiment, the method for using together means the second preparation comprising the mitochondria permeability transition pore inhibitor is administered firstly, then the anticancer drug is administered.

Preferably, in the active ingredient combination, composition and/or medicine kit, the molar ratio of the first active ingredient to the second active ingredient is 0.01-600:1, preferably 0.05-500:1, more preferably 0.1-400:1, more preferably 0.2-200:1, more preferably 0.5-100:1, more preferably 0.5-80:1, and 1-50:1

In the present invention, the dosage form of the composition or preparation comprises but is not limited to a oral preparation, injection preparation or external preparation.

Typically, the dosage form of the composition or preparation comprises but is not limited to tablet, injection, infusion, paste, gel, solution, microsphere or film.

Typically, the injection is an intratumoral injection.

Typically, the injection is intratumoral injection.

The pharmaceutical preparation should be matched with the mode of administration. Preferred mode of administration is oral administration, injection administration (e.g. intratumoral injection), When used, a therapeutically effective amount of drug is administered to a subject in need (e.g. human or non-human mammal). As used herein, the term "therapeutically effective amount" refers to an amount that has a function or activity in human and/or animal and is acceptable to human and/or animal. It should be understand in the art that the "therapeutically effective amount" can vary depending on the form of the pharmaceutical composition, the route of administration, the excipients of the drug, the severity of the disease, and the combination with other drugs, etc.

In a mode of administration, the safe and effective daily dose of the first active ingredient is usually at least about 0.1 mg, and does not exceed about 2500 mg in most case. Preferably, the dose is 1 mg to 500 mg. The safe and effective amount of the second active ingredient is usually at least about 0.01 mg, and does not exceed about 2500 mg in most case. Preferably, the dose range is 0.1 mg to 2500 mg. Of course, the specific dose should also take into account the route of administration, the patient's health and other factors, which are within the skill range of skilled doctors.

### The main advantages of the present invention comprise:

The present invention has firstly developed a novel compound, the compound can efficiently and safely inhibit tumor cell. Especially, the compound of the present invention has significant inhibitory effect on tumor cell with up-regulation of mitochondrial oxidative phosphorylation pathway or low activity of mPTP. Furthermore, the compound of the present invention has significant inhibitory effect on tumor with low or no expression of NNMT gene, and/or high methylation level of DNA CpG site of NNMT gene.

The present invention will be further illustrated below with reference to the specific examples. It should be understood that these examples are only to illustrate the invention but are not to limit the scope of the invention. The experimental methods with no specific conditions described in the following examples are generally performed under the conventional conditions, or according to the manufacturer's instructions. Unless indicated otherwise, parts and percentage are calculated by weight.

### Example 1

### Synthesis of compound AB24831A

### Step 1):

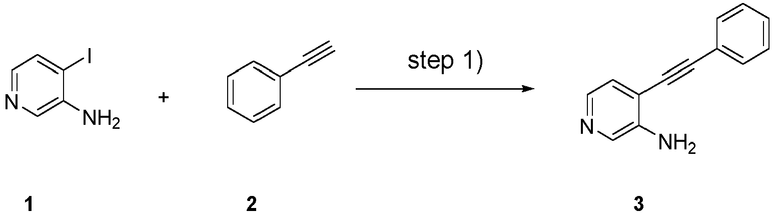

Compound 1 (440 mg, 2 mmol) was dissolved in dry N,N-dimethylformamide (20 mL), and compound 2 (428.4 mg, 4.2 mmol), bis(triphenylphosphine)palladium(II) chloride (42 mg, 0.06 mmol), N, N-diisopropylethylamine (825 mg, 6.4 mmol) and copper(I) iodide (19 mg, 0.1 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by column chromatography (ethyl acetate: petroleum ether=0-50%) to afford compound 3 (130 mg, 33% yield).

### Step 2):

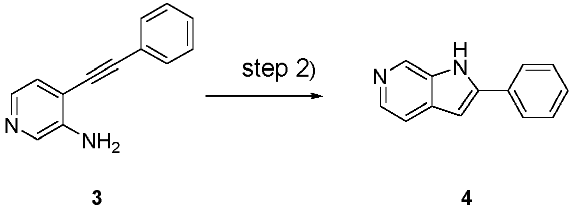

Compound 3 (130 mg, 0.47 mmol) was dissolved in dioxane (6 mL), and potassium tert-butoxide (336.6 mg, 3 mmol) was added. The mixture was replaced with N₂ three times, and stirred at 110°C for 16 h, new spot was detected using plate. The reaction mixture was diluted with water (20 ml) and extracted with ethyl acetate (10 mL x 3). The combined organic phase was washed with saturated brine (30 mL), dried over anhydrous sodium sulfate, filtered, and concentrated under reduced pressure to remove organic solvent. The residue was purified by thin layer chromatography (methanol: dichloromethane=0-10%) to afford compound 4 (50 mg, 50% yield).

### Step 3):

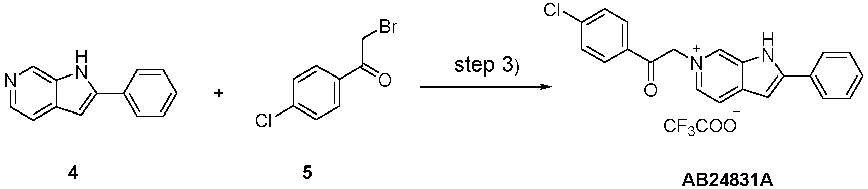

Compound 4 (100 mg, 0.67 mmol) was dissolved in acetonitrile (10 mL), and compound 5 (312 mg, 1.34 mmol) was added. The mixture was replaced with N₂ three times, and stirred at 80°C for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by preparation purification to afford compound AB24831A (25.2 mg, 8.1% yield).

### Compound AB24831A

¹H NMR (400 MHz, DMSO-d6) δ 13.53 (s, 1H), 9.06 (s, 1H), 8.24 (d, J = 7.1 Hz, 1H), 8.14 (d, J = 6.5 Hz, 1H), 8.08 (d, J = 7.4 Hz, 4H), 7.74 (d, J = 7.6 Hz, 2H), 7.64 - 7.55 (m, 3H), 7.48 (s, 1H), 6.36 (s, 2H).

MS-ESI: Calculated.: [M-CF₃COO]⁺347.82, Found: 347.25.

### Example 2

### Synthesis of compound AB24828A

### Step 1:

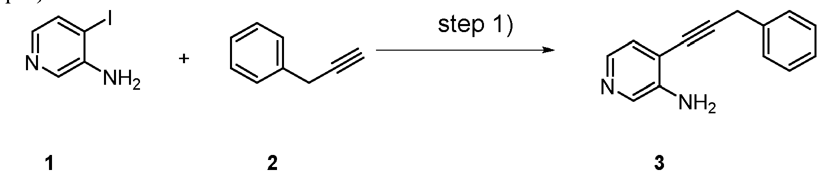

Compound 1 (140 mg, 0.66 mmol) was dissolved in dry N,N-dimethylformamide (10 mL), and compound 2 (161.4 mg, 1.39 mmol), bis(triphenylphosphine)palladium(II) chloride (14 mg, 0.02 mmol), N, N-diisopropylethylamine (272 mg, 2.11 mmol) and copper(I) iodide (5.7 mg, 0.03 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by thin layer chromatography (ethyl acetate: petroleum ether=0-50%) to afford compound 3 (100 mg, 75.7% yield).

### Step 2):

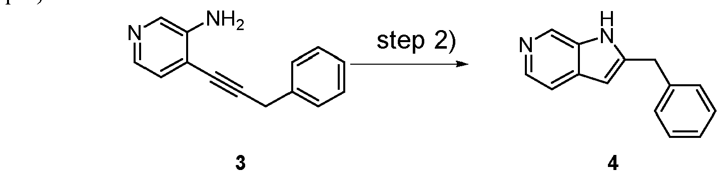

Compound 3 (100 mg, 0.47 mmol) was dissolved in dioxane (30 mL), and potassium tert-butoxide (160 mg, 1.43 mmol) was added. The mixture was replaced with N₂ three times, and stirred at 110°C for 1 h, new spot was detected using plate. The reaction mixture was diluted with water (20 ml) and extracted with ethyl acetate (10 mL x 3). The combined organic phase was washed with saturated brine (30 mL), dried over anhydrous sodium sulfate, filtered, and concentrated under reduced pressure to remove organic solvent. The residue was purified by thin layer chromatography (methanol: dichloromethane=0-10%) to afford compound 4 (50 mg, 50% yield).

### Step 3):

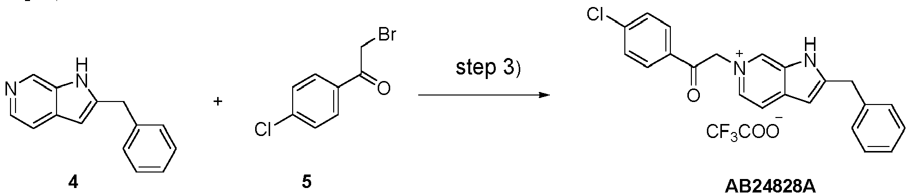

Compound 4 (50 mg, 0.67 mmol) was dissolved in acetonitrile (10 mL), and compound 5 (312 mg, 1.34 mmol) was added. The mixture was replaced with N₂ three times, and stirred at 80°C for overnight, new spot was detected using plate. The reaction mixture was concentrated under reduced pressure to remove organic solvent. The residue was purified by preparation purification to afford compound AB24828A (34.7 mg, 10.9% yield).

### Compound AB24828A

¹H NMR (400 MHz, DMSO-d6) δ 13.15 (s, 1H), 9.02 (s, 1H), 8.21 (d, J = 6.1 Hz, 1H), 8.07 (d, J = 8.4 Hz, 3H), 7.74 (d, J = 7.0 Hz, 2H), 7.31 (d, J = 29.2 Hz, 5H), 6.74 (s, 1H), 6.33 (s, 2H), 4.33 (s, 2H).

MS-ESI: Calculated.: [M-CF₃COO]⁺361.84, Found: 361.25.

### Example 3

### Synthesis of compound AB24861

### Step :

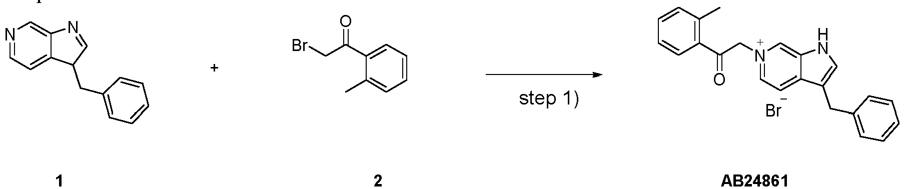

Compound 1 (100 mg, 0.48 mmol) and compound 2 (123 g, 0.577 mmol) were dissolved in acetonitrile (10 mL). The mixture was stirred at room temperature for 4 h, new spot was detected using plate. The reaction mixture was filtered, and filter cake was washed with acetonitrile to afford compound AB24861 (110.0mg, 67.2% yield),

### Compound AB24861

¹H NMR (400 MHz, DMSO-d6) δ 12.96 (s, 1H), 9.15 (s, 1H), 8.27 (s, 1H), 8.22 (s, 1H), 8.11 (s, 1H), 8.04 (d, J = 7.4 Hz, 1H), 7.56 (d, J = 7.1 Hz, 1H), 7.47 (d, J = 7.8 Hz, 1H), 7.39 (d, J = 6.5 Hz, 1H), 7.29 (d, J = 12.7 Hz, 4H), 7.17 (s, 1H), 6.27 (s, 2H), 4.18 (s, 2H), 2.43 (s, 3H).

MS-ESI: Calculated.: [M-Br]⁺ 341.43, Found: 341.30.

### Example 4

### Synthesis of compound AB24859

### Step 1:

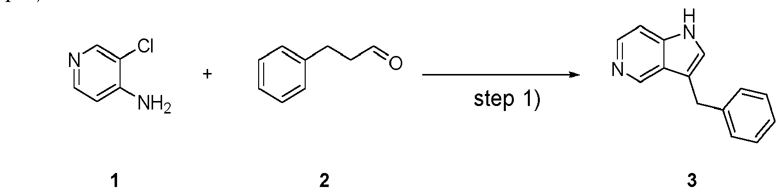

Compound 1 (2.0 g, 15.5 mmol compound 2 (5.6 g, 46.6 mmol), bis(dibenzylideneacetone)palladium (771.0 mg, 0.5 mmol), tri-tert-butylphosphine tetrafluoroborate (451.0 mg, 1 mmol) and triethylenediamine (5.24 g, 30 mmol) were dissolved in N,N-dimethylacetamide (50 mL). The mixture was replaced with N₂ three times, and stirred at 120°C for 16 h, new spot was detected using plate. The reaction mixture was cooled to room temperature, diluted with water and extracted with ethyl acetate (100 mL x 3). The combined organic phase was washed with saturated brine (100 mL), dried over anhydrous sodium sulfate and concentrated under reduced pressure to remove organic solvent. The residue was purified by column chromatography (ethyl acetate: petroleum ether=66.6%) to afford compound 3 (70 mg, 21.6% yield).

### Step (2):

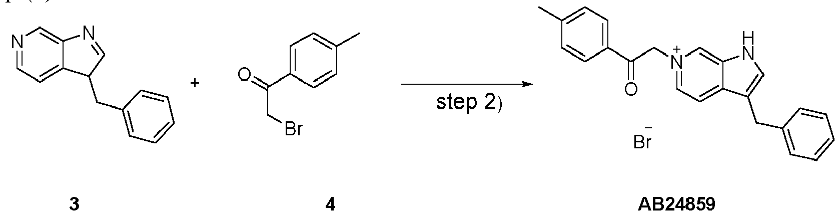

Compound 3 (97 mg, 0.5 mmol) and compound 4 (244 mg, 1 mmol) were dissolved in acetonitrile (10 mL). The mixture was stirred at room temperature for 16 h, new spot was detected using plate. The reaction mixture was filtered, and filter cake was washed with acetonitrile to afford compound AB24859 (89 mg, 52.1% yield).

### Compound AB24859

¹H NMR (400 MHz, DMSO-d6) δ 12.98 (s, 1H), 9.17 (s, 1H), 8.28 - 8.21 (m, 2H), 8.09 (d, *J* = 6.6 Hz, 1H), 7.94 (d, *J* = 7.7 Hz, 2H), 7.43 (d, *J* = 7.8 Hz, 2H), 7.34 - 7.24 (m, 4H), 7.18 (d, *J* = 7.2 Hz, 1H), 6.36 (s, 2H), 2.41 (s, 3H).

MS-ESI: Calculated.: [M-Br]⁺ 341.43, Found: 341.30.

### Example 5

### Synthesis of compound AB24835A

### Step (1):

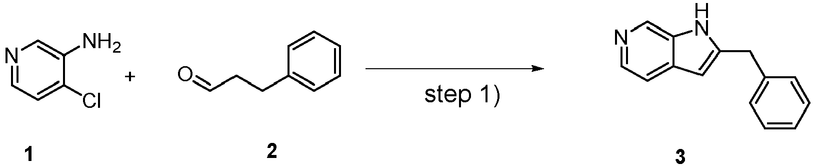

Compound 1 (1.28 g, 10 mmol), compound 2 (4.02 g, 30 mmol), bis(dibenzylideneacetone)palladium (457.5 mg, 0.5 mmol), tri-tert-butylphosphine tetrafluoroborate (290.13 mg, 1 mmol) and triethylenediamine (3.36 g, 30 mmol) were dissolved in N,N-dimethylacetamide (50 mL). The mixture was replaced with N₂ three times, and stirred at 120°C for 16 h, new spot was detected using plate. The reaction mixture was cooled to room temperature, diluted with water and extracted with ethyl acetate (100 mL x 3). The combined organic phase was washed with saturated brine (100 mL), dried over anhydrous sodium sulfate and concentrated under reduced pressure to remove organic solvent. The residue was purified by column chromatography (ethyl acetate: petroleum ether=66.6%) to afford compound 3 (1.1 g, 52.88% yield).

MS-ESI: Calculated.: [M+1]⁺208.26, Found: 209.30.

### Step (2):

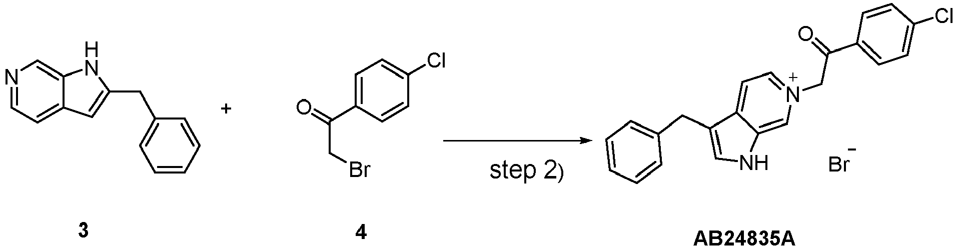

Compound 3 (208 mg, 1 mmol) and compound 4 (233.5 g, 1 mmol) were dissolved in acetonitrile (10 mL). The mixture was stirred at 80°C for 3 h, new spot was detected using plate. The reaction mixture was cooled to room temperature, filtered, and filter cake was washed with ethyl acetate to afford compound AB24835A (150 mg, 41.55% yield).

### Compound AB24835A

¹H NMR (400 MHz, DMSO-d6) δ 12.98 (s, 1H), 9.14 (s, 1H), 8.30 - 8.19 (m, 2H), 8.08 (dd, *J* = 22.2, 7.3 Hz, 3H), 7.72 (d, *J =* 7.9 Hz, 2H), 7.29 (dt, *J =* 14.9, 7.5 Hz, 4H), 7.18 (d, *J =* 7.0 Hz, 1H), 6.35 (s, 2H), 4.18 (s, 2H).

MS-ESI: Calculated.: [M-Br]⁺ 361.84, Found: 361.30.

### Example 6

### Synthesis of compound AB24863

### Step 1:

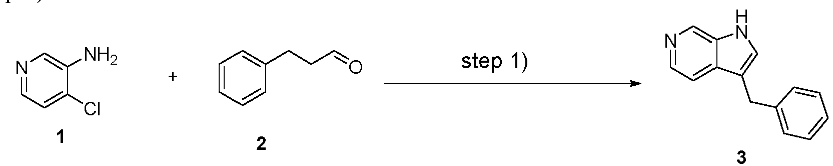

Compound 1 (3.86 g, 30 mmol) was dissolved in N,N-dimethylacetamide (250ml), and compound 2 (12.1 g, 90 mmol), triethylenediamine (10.0 g, 90 mmol), tri-tert-butylphosphine tetrafluoroborate (870 mg, 3 mmol) and bis(dibenzylideneacetone)palladium (1.37 mg, 1.5 mmol) were successively added. The mixture was replaced with N₂ three times, and stirred at 120°C for overnight, new spot was detected using plate. The reaction mixture was diluted with water (200ml), and extracted with ethyl acetate (100 mL x 3). The combined organic phase was washed with saturated brine (100 mL), dried over anhydrous sodium sulfate, filtered, and concentrated under reduced pressure to remove organic solvent. The residue was purified by column chromatography (methanol: dichloromethane=10:1) to afford compound 3 (2.0 g, 32% yield).

### Step 2:

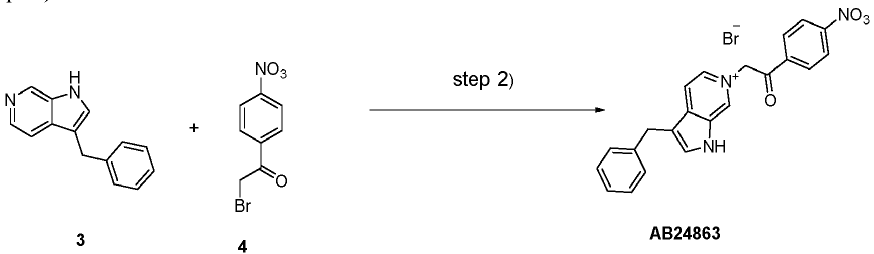

Compound 3 (100 mg, 0.5 mmol) was dissolved in acetonitrile (10 mL), and compound 4 (244 g, 1 mmol) was added. The mixture was replaced with N₂ three times, and stirred at 80°C for overnight, new spot was detected using plate. The reaction mixture was concentrated under reduced pressure to remove organic solvent. The residue was purified by thin layer chromatography (methanol: dichloromethane=10:1) to afford compound AB24863 (140 mg, 59.8% yield).

### Compound AB24863

¹H NMR (400 MHz, DMSO-d6) δ 13.01 (s, 1H), 9.16 (s, 1H), 8.46 (d, *J =* 7.9 Hz, 2H), 8.26 (dd, *J=* 16.4, 8.2 Hz, 4H), 8.14 (d, *J* = 6.9 Hz, 1H), 7.31 (dd, *J* = 20.1, 7.4 Hz, 4H), 7.20 (d, *J* = 7.3 Hz, 1H), 6.43 (s, 2H), 4.20 (s, 2H).

MS-ESI: Calculated.: [M-Br]⁺ 388.40, Found: 388.25

### Example 7

### Synthesis of compound AB24878

### Step 1:

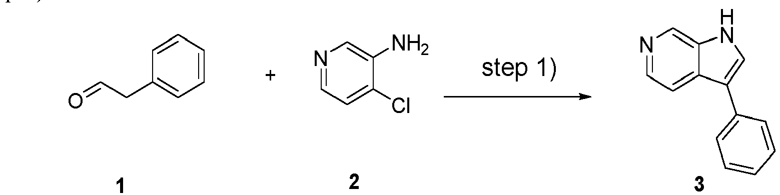

Compound 1 (2.7 g, 23 mmol) and compound 2 (2.5 g, 19 mmol) were dissolved in N,N-dimethylacetamide (30ml), and triethylenediamine (6.5 g, 58 mmol), bis(dibenzylideneacetone)palladium (890 mg, 0.97 mmol) and tri-tert-butylphosphine tetrafluoroborate (561 mg, 1.94 mmol) were added. The mixture was stirred at 120°C for 16 h in sealed tube, new spot was detected using plate. The reaction mixture was extracted with water and ethyl acetate (60 mL). The combined organic phase was concentrated under reduced pressure, the residue was purified by column chromatography (methanol: dichloromethane=5%) to afford compound 3 (800 mg, 17.9% yield).

### Step (2):

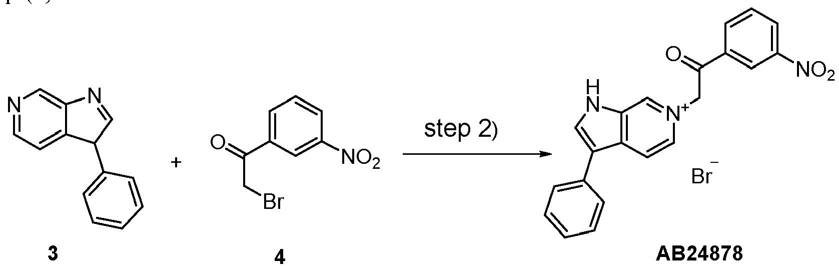

Compound 3 (92 mg, 0.5 mmol) and compound 4 (244 mg, 1 mmol) were dissolved in acetonitrile (10 mL). The mixture was stirred at room temperature for 16 h, new spot was detected using plate. The reaction mixture was filtered, and filter cake was washed with acetonitrile to afford compound AB24878 (116.2 mg, 53.02% yield).

### Compound AB24878

¹H NMR (400 MHz, DMSO-d6) δ 9.28 (s, 1H), 8.77 (d, J = 11.6 Hz, 2H), 8.59 (d, J = 8.2 Hz, 1H), 8.48 (s, 2H), 8.37 (d, J = 6.3 Hz, 1H), 7.96 (t, J = 7.8 Hz, 1H), 7.80 (d, J = 7.2 Hz, 2H), 7.52 (t, J = 7.2 Hz, 2H), 7.38 (d, J = 7.1 Hz, 1H), 6.53 (s, 2H).

MS-ESI: Calculated.: [M-Br]⁺ 358.37, Found: 358.30.

### Example 8

### Synthesis of compound AB24872

### Step 1):

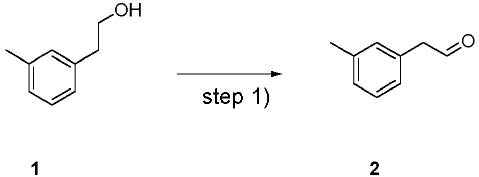

Compound 1 (500 mg, 3.6 mmol) was dissolved in dichloromethane (20 mL), Dess-Martin periodinane (2.02 g, 4.7 mmol) was added in an ice bath. The mixture was stirred at room temperature for 2 h, new spot was detected using plate. The reaction mixture was filtered, and the filtrate was concentrated under reduced pressure to remove organic solvent. The residue was purified by column chromatography (ethyl acetate: petroleum ether=0-10%) to afford compound 2 (340 mg, 69.1% yield).

### Step 2:

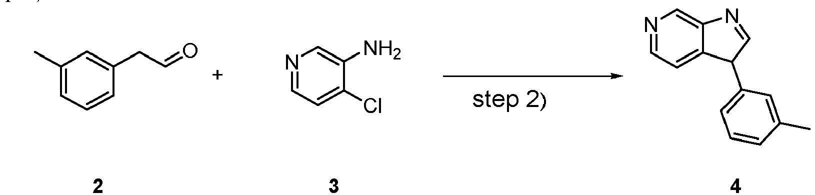

Compound 2 (340 mg, 2.67 mmol), compound 3 (286 g, 2.22 mmol), bis(dibenzylideneacetone)palladium (102 mg, 0.11 mmol), tri-tert-butylphosphine tetrafluoroborate (64 mg, 0.22 mmol) and triethylenediamine (750 mg, 6.68 mmol) were dissolved in N,N-dimethylacetamide (50 mL). The mixture was replaced with N₂ three times, and stirred at 120°C for 16 h, new spot was detected using plate. The reaction mixture was cooled to room temperature and extracted with water and ethyl acetate (100 mL x 3). The combined organic phase was washed with saturated brine (100 mL), dried over anhydrous sodium sulfate and concentrated under reduced pressure to remove organic solvent. The residue was purified by column chromatography (ethyl acetate: petroleum ether=66.6%) to afford compound 4 (200 mg, 37.9% yield).

### Step 3:

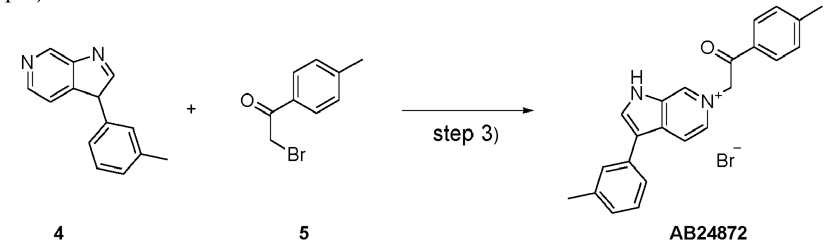

Compound 4 (200 mg, 0.96 mmol) and compound 5 (245 mg, 1.15 mmol) were dissolved in acetonitrile (10 mL). The mixture was replaced with N₂ three times, and stirred at room temperature for 5 h, new spot was detected using plate. The reaction mixture was concentrated under reduced pressure to remove organic solvent. The residue was purified by column chromatography (methanol: dichloromethane=0-10%) to afford compound AB 24872 (92.6 mg, 28.3% yield).

### Compound AB24872

¹H NMR (400 MHz, CD₃OD) δ 10.11 (s, 1H), 9.54 (s, 1H), 9.20 (dd, J = 39.1, 6.4 Hz, 2H), 8.78 (d, J = 7.5 Hz, 2H), 8.46 - 8.36 (m, 2H), 8.22 (dd, J = 29.1, 7.7 Hz, 3H), 7.98 (d, J = 6.7 Hz, 1H), 7.24 (s, 2H), 3.22 (d, J = 11.7 Hz, 6H).

MS-ESI: Calculated.: [M-Br]⁺ 341.43, Found: 341.25.

### Example 9

### Synthesis of compound AB24854

### Step (1):

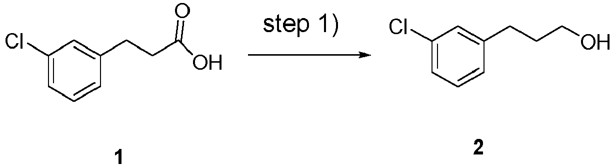

Compound 1 (8 g, 47.1 mmol) was dissolved in tetrahydrofuran (80 mL), and a solution of lithium aluminium hydride in tetrahydrofuran (2.5 N, 56 mL, 141.2 mmol) was added dropwise under ice bath. The mixture was stirred at room temperature for 30 min and heated to 75°C at reflux for overnight, new spot was detected using plate. The reaction mixture was cooled to room temperature, water (56 mL), sodium hydroxide aqueous solution (15%, 56 mL) and water (158 mL) were successively added dropwise. The mixture was filtered with diatomite to remove aluminum salt, and extracted with ethyl acetate (100 mL x 3). The combined organic phase was washed with saturated brine (50 mL), dried over anhydrous sodium sulfate and concentrated under reduced pressure to remove organic solvent to afford compound 2 (5.6 g, 75.76% yield).

### Step 2):

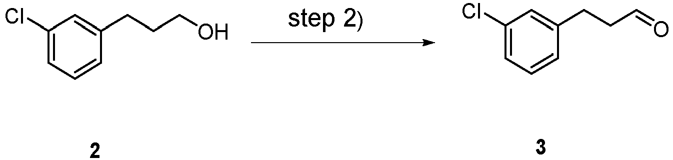

Compound 2 (3 g, 17.64 mmol) was dissolved in dichloromethane (50 mL), Dess-Martin periodinane (8.97 g, 21.18 mmol) was added in ice bath. The mixture was stirred at room temperature for 1 h, new spot was detected using plate, dichloromethane (50 mL) was added. The reaction mixture was filtered with diatomite, the filtrate was concentrated under reduced pressure to remove organic solvent, the residue was purified by column chromatography (ethyl acetate: petroleum ether=0-10%) to afford compound 3 (2.4g, 80.9% yield).

### Step 3:

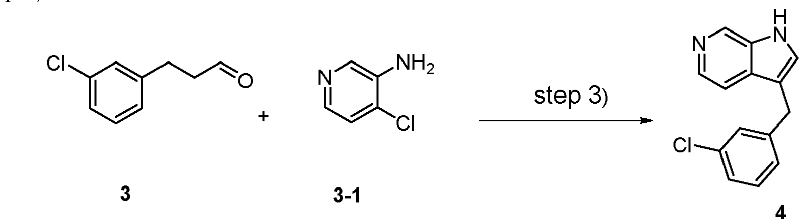

Compound 3-1 (1.67 g, 12.99 mmol) and compound 3 (2.4 g, 14.29 mmol) were dissolved in N,N-dimethylacetamide (30 mL), and triethylenediamine (4.37 g, 38.97 mmol), bis(dibenzylideneacetone)palladium (595 mg, 0.65 mmol) and tri-tert-butylphosphine tetrafluoroborate (377 mg, 1.31 mmol) were added. The mixture was stirred at 120°C for 16 h, new spot was detected using plate. The reaction mixture was extracted with water and ethyl acetate (10 mL). The combined organic phase was concentrated under reduced pressure, the residue was purified by column chromatography (methanol: dichloromethane=10%) to afford compound 4 (1.2 g, 50% crude).

### Step 4):

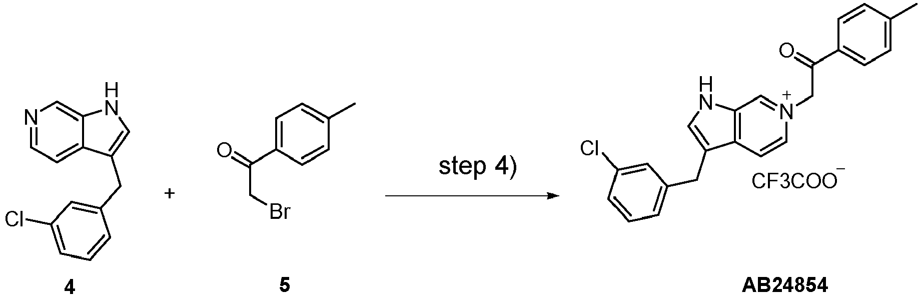

Compound 4 (500 mg, 2.06 mmol) was dissolved in acetonitrile (5 mL), and compound 5 (660 mg, 3.09 mmol) was added. The mixture was replaced with N₂ three times, and stirred at room temperature for overnight, new spot was detected using plate. The reaction mixture was dried, and the crude was purified by preparation purification to afford compound AB24854 (20 mg, 2.59% yield).

### Compound AB24854

¹H NMR (400 MHz, CD₃OD) δ 9.02 (s, 1H), 8.09 (s, 2H), 7.97 (dd, *J=* 13.7, 7.1 Hz, 3H), 7.42 (d, *J=* 8.0 Hz, 2H), 7.32 - 7.19 (m, 4H), 6.27 (s, 2H), 4.25 (s, 2H), 2.45 (s, 3H).

MS-ESI: Calculated.: [M-CF₃COO]⁺ 375.12, Found: 375.20.

### Example 10

### Synthesis of compound AB24945

### Step 1:

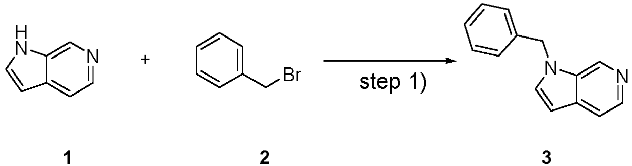

Compound 1 (200 mg, 1.69 mmol) was dissolved in dry N,N-dimethylformamide (20 mL), NaH (203 mg, 5.07 mmol) was added in ice bath. The mixture was stirred for 30 min in ice bath, compound 2 (289 mg, 1.86 mmol) was added, then the mixture was stirred at room temperature for 3 h, new spot was detected using plate. The reaction mixture was diluted with water (10 ml) and extracted with ethyl acetate (10ml x 3). The combined organic phase was washed with saturated brine, dried over anhydrous sodium sulfate, and concentrated under reduced pressure. The residue was purified by column chromatography (ethyl acetate: petroleum ether=0-10%) to afford compound 3 (260 mg, 73.8% yield).

### Step 2:

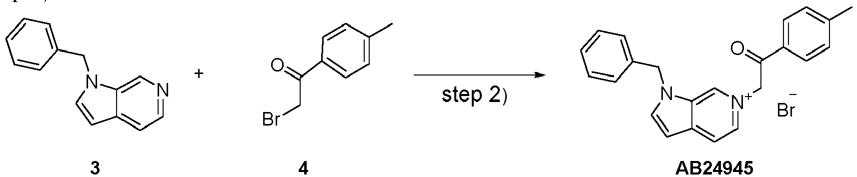

Compound 3 (130 mg, 0.625 mmol) was dissolved in acetonitrile (10 mL), and compound 4 (200 mg, 0.94 mmol) was added. The mixture was replaced with N₂ three times, and stirred at room temperature for overnight, new spot was detected using plate, solid was precipitated and filtered to afford compound AB24945 (70 mg, 32.8% yield).

### Compound AB24945

¹H NMR (400 MHz, CDCl3) δ 10.96 (s, 1H), 8.05 (d, J = 7.4 Hz, 2H), 7.87 (s, 2H), 7.78 (s, 1H), 7.42 (s, 2H), 7.36 - 7.28 (m, 5H), 6.81 (d, J = 11.9 Hz, 3H), 5.70 (s, 2H), 2.41 (s, 3H).

MS-ESI: Calculated.: [M-Br]⁺ 341.43, Found: 341.10.

### Example 11

### Synthesis of compound AB24837

### Step 1:

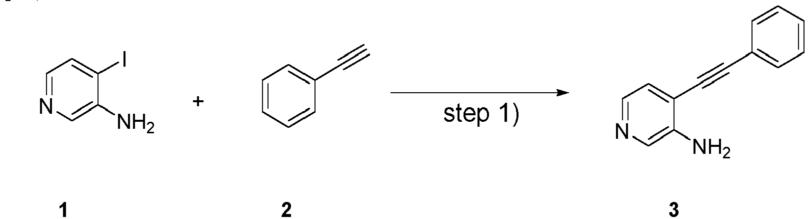

Compound 1 (440 mg, 2 mmol) was dissolved in dry N,N-dimethylformamide (20 mL), and compound 2 (428.4 mg, 4.2 mmol), bis(triphenylphosphine)palladium(II) chloride (42 mg, 0.06 mmol), N, N-diisopropylethylamine (825 mg, 6.4 mmol) and copper(I) iodide (19 mg, 0.1 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by column chromatography (ethyl acetate: petroleum ether=0-50%) to afford compound 3 (130 mg, 33% yield).

### Step 2):

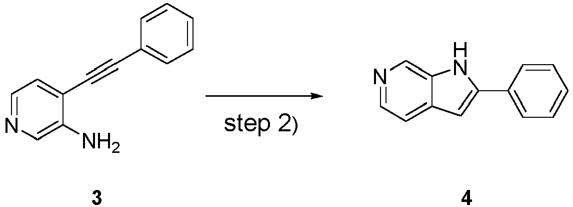

Compound 3 (130 mg, 0.47 mmol) was dissolved in dioxane (6 mL), and potassium tert-butoxide (336.6 mg, 3 mmol) was added. The mixture was replaced with N₂ three times, and stirred at 110°C for 16 h, new spot was detected using plate. The reaction mixture was diluted with water (20 ml) and extracted with ethyl acetate (10 mL x 3). The combined organic phase was washed with saturated brine (30 mL), dried over anhydrous sodium sulfate, filtered, and concentrated under reduced pressure to remove organic solvent. The residue was purified by thin layer chromatography (methanol: dichloromethane=0-10%) to afford compound 4 (50 mg, 50% yield).

### Step 3):

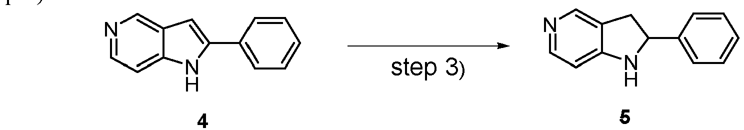

Compound 4 (340 mg, 1.75 mmol) was dissolved in acetic acid (5 mL), palladium carbon (100 mg, 3 mmol) was added. The mixture was replaced with H₂ three times, and stirred at room temperature for 12 h, new spot was detected using plate. The reaction mixture was filtered, the filtrate was dried, the residue was purified by preparation purification to afford compound 5 (50 mg, 50% yield).

### Step 4):

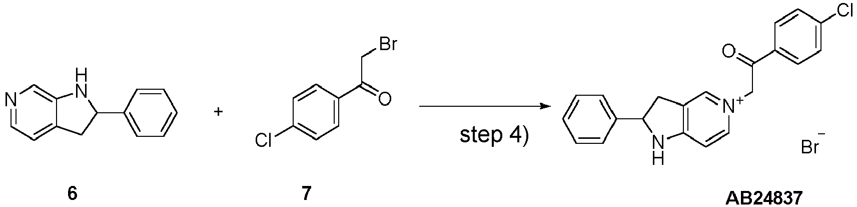

Compound 6 (50 mg, 0.26 mmol) was dissolved in acetonitrile (10 mL), and compound 7 (182mg, 0.78 mmol) was added. The mixture was replaced with N₂ three times, and stirred at 80 °C for overnight, new spot was detected using plate.The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by preparation purification to afford compound AB24837 (30 mg, 33.71% yield).

### Compound AB24837

¹H NMR (400 MHz, DMSO-d6) δ 9.40 (s, 1H), 8.05 (d, J = 6.6 Hz, 1H), 8.00 (d, J = 8.0 Hz, 2H), 7.93 (s, 1H), 7.69 (d, J = 7.9 Hz, 2H), 7.38 (d, J = 6.5 Hz, 2H), 7.32 (d, J = 6.5 Hz, 3H), 6.82 (d, J = 6.5 Hz, 1H), 5.87 (s, 2H), 5.36 (d, J = 8.9 Hz, 1H), 3.74 - 3.65 (m, 1H), 2.99 (s, 1H).

MS-ESI: Calculated.: [M-CF₃COO]⁺349.84, Found: 349.00.

### Example 12

### Synthesis of compound AB24827A

### Step 1:

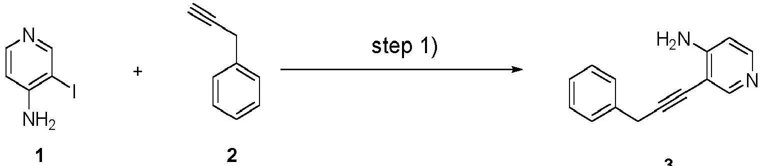

Compound 1 (440 mg, 2 mmol) was dissolved in N,N-dimethylformamide (20 mL), and compound 2 (486 mg, 4.2 mmol), copper(I) iodide (19 mg, 0.1 mmol), N, N-diisopropylethylamine (824 mg, 6.4 mmol) and bis(triphenylphosphine)palladium(II) chloride (42 mg, 0.06 mmol) were successively added. The mixture was replaced with N₂ three times, and stirred at room temperature for 16 h, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by thin layer chromatography (methanol: dichloromethane=10:1) to afford compound 3 (332 mg, 79.8% yield).

### Step 2:

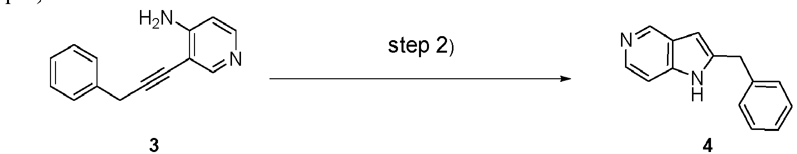

Compound 3 (332 mg, 1.6 mmol) was dissolved in 1,4-dioxane (10 mL), and potassium tert-butoxide (538 mg, 4.8 mmol) was added. The mixture was replaced with N₂ three times, and stirred at 100°C for overnight, new spot was detected using plate. The reaction mixture was concentrated under reduced pressure to remove organic solvent. The residue was purified by thin layer chromatography (methanol: dichloromethane=10:1) to afford compound 4 (189 mg, 56.7% yield).

### Step 3):

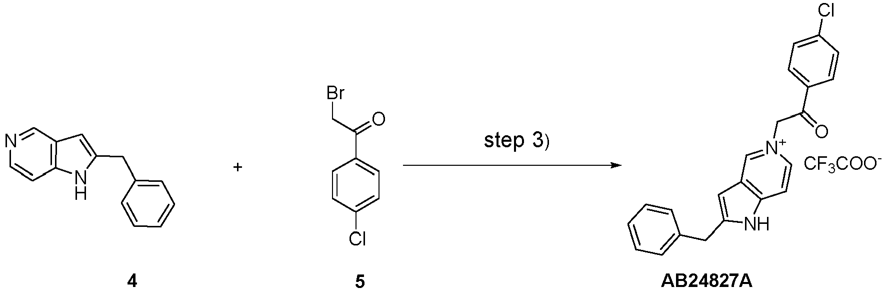

Compound 4 (189 mg, 0.9 mmol) was dissolved in acetonitrile (10 mL), and compound 5 (233mg, 1.1 mmol) was added. The mixture was replaced with N₂ three times, and stirred at 80°C for overnight, new spot was detected using plate. The reaction mixture was concentrated under reduced pressure to remove organic solvent. The residue was purified by thin layer chromatography (methanol: dichloromethane=10:1) to afford compound AB24827A (9.3% yield).

### Compound AB24827A

¹H NMR (400 MHz, DMSO-d6) δ 13.00 (s, 1H), 9.08 (s, 1H), 8.33 (d, *J* = 6.5 Hz, 1H), 8.05 (d, *J =* 8.5 Hz, 2H), 7.93 (d, *J =* 6.8 Hz, 1H), 7.72 (d, *J =* 8.3 Hz, 2H), 7.28 (d, *J =* 28.2 Hz, 5H), 6.80 (s, 1H), 6.32 (s, 2H), 4.23 (s, 2H).

MS-ESI: Calculated.: [M-CF₃COO]⁺361.84, Found: 361.25.

### Example 13

### Synthesis of compound AB24887

### Step 1):

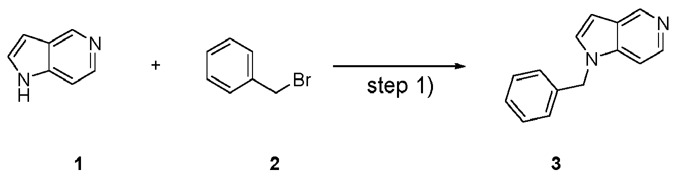

Compound 1 (200 mg, 1.69 mmol) was dissolved in dry N,N-dimethylformamide (20 mL), NaH (203 mg, 5.07 mmol) was added in ice bath. The mixture was stirred at 0 °C for 30 min, and compound 2 (318 mg, 1.86 mmol) was added, then the mixture was stirred at room temperature for 3 h, new spot was detected using plate. The reaction mixture was diluted with water (30 ml) and extracted with ethyl acetate (30 ml x 3). The combined organic phase was washed with saturated brine (50 ml), dried over anhydrous sodium sulfate, and concentrated under reduced pressure to remove organic solvent. The residue was purified by column chromatography (methanol: dichloromethane=0-10%) to afford compound 3 (280 mg, 79.5% yield).

### Step 2):

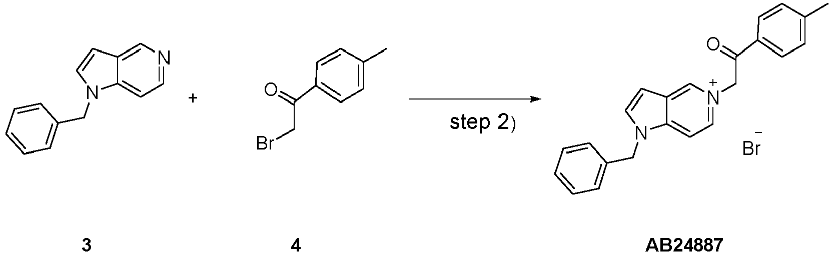

Compound 3 (280 mg, 1.3 mmol) was dissolved in acetonitrile (5 mL), and compound 4 (430 mg, 2.0 mmol) was added. The mixture was replaced with N₂ three times, and stirred at room temperature for overnight, new spot was detected using plate. The reaction mixture was dried, the residue was purified by column chromatography methanol: dichloromethane=0-10% to afford compound AB24887 (220 mg, 47.9% yield).

### Compound AB24887

¹H NMR (400 MHz, DMSO,d6) δ 9.28 (s, 1H), 8.47 (s, 1H), 8.32 (s, 1H), 8.15 (s, 1H), 7.95 (d, J = 7.6 Hz, 2H), 7.44 (d, J = 7.7 Hz, 2H), 7.34 (s, 5H), 7.17 (s, 1H), 6.37 (s, 2H), 5.66 (s, 2H), 2.41 (s, 3H).

MS-ESI: Calculated.: [M-Br]⁺341.43, Found: 341.25.

### Example 14

### Synthesis of compound AB24888

### Step 1:

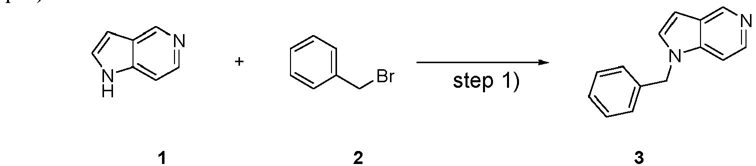

Compound 1 (200 mg, 1.69 mmol) was dissolved in dry N,N-dimethylformamide (20 mL), NaH (203 mg, 5.07 mmol) was added in ice bath. The mixture was stirred at 0°C for 30 min, and compound 2 (318 mg, 1.86 mmol) was slowly added, then the mixture was stirred at room temperature for 3 h, new spot was detected using plate. The reaction mixture was diluted with water (30 ml) and extracted with ethyl acetate (30 ml x 3). The combined organic phase was washed with saturated brine (50 ml), dried over anhydrous sodium sulfate, and concentrated under reduced pressure to remove organic solvent. The residue was purified by column chromatography (methanol: dichloromethane=0-10%) to afford compound 3 (280 mg, 79.5% yield).

### Step 2):

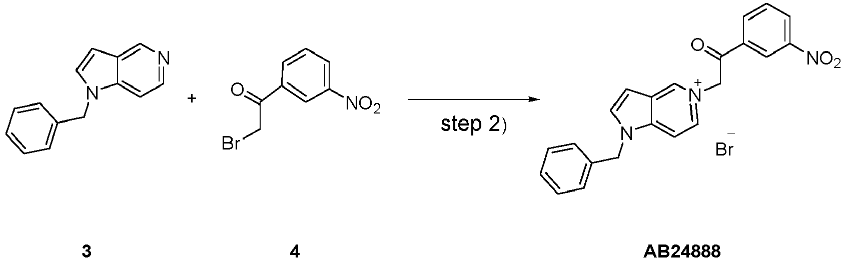

Compound 3 (250 mg, 1.2 mmol) was dissolved in acetonitrile (5 mL), and compound 4 (440 mg, 1.8 mmol) was added. The mixture was replaced with N₂ three times, and stirred at room temperature for overnight, new spot was detected using plate, solid was precipitated and filtered, and filter cake was washed with acetonitrile three times and dried to afford compound AB24888 (300mg, 67.1% yield),

### Compound AB24888

¹H NMR (400MHz, DMSO,d6) δ 9.26 (s, 1H), 8.73 (s, 1H), 8.58 (d, J = 8.1 Hz, 1H), 8.46 (t, J = 7.4 Hz, 2H), 8.35 (d, J = 6.8 Hz, 1H), 8.16 (s, 1H), 7.94 (t, J = 7.9 Hz, 1H), 7.33 (s, 5H), 7.19 (s, 1H), 6.47 (s, 2H), 5.66 (s, 2H).

MS-ESI: Calculated.: [M-Br]⁺ 372.40, Found: 372.25.

### Example 15

### Synthesis of compound AB24895

### Step (1):

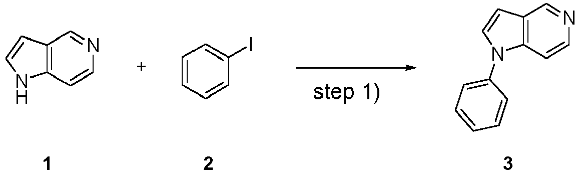

Compound 1 (500 mg, 4.23 mmol), iodobenzene (963 mg, 4.23 mmol), copper(I) iodide (16.2 mg, 0.085 mmol), tripotassium phosphate (1.79 g, 8.46 mmol) and diaminocyclohexane (99 mg, 0.87 mmol) were dissolved in N,N- dimethylacetamide (20 mL). The mixture was replaced with N₂ three times, and stirred at 110°C for 16 h, new spot was detected using plate. The reaction mixture was concentrated under reduced pressure to remove organic solvent. The residue was purified by column chromatography (methanol: dichloromethane=0-10%) to afford compound 3 (580 mg).

### Step 2:

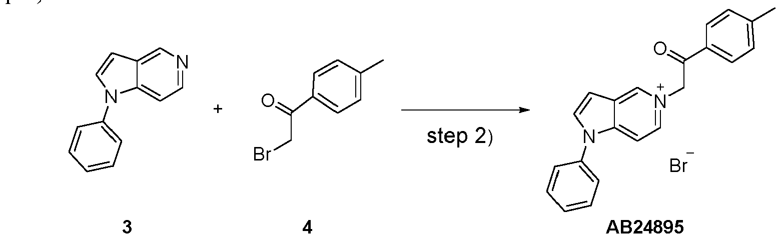

Compound 3 (290 mg, 1.49 mmol) and compound 4 (477 mg, 2.24 mmol) were dissolved in acetonitrile (10 mL). The mixture was replaced with N₂ three times, and stirred at room temperature for 3 h, new spot was detected using plate. The reaction mixture was concentrated under reduced pressure to remove organic solvent. The residue was purified by column chromatography (methanol: dichloromethane=0-10%) to afford compound AB24895 (377 mg, 77.3% yield).

### Compound AB24895

¹H NMR (400 MHz, DMSO-d6) δ 9.45 (s, 1H), 8.56 (d, *J=* 5.9 Hz, 1H), 8.31 (s, 1H), 8.14 (d, *J=* 6.5 Hz, 1H), 7.96 (d, *J=* 7.3 Hz, 2H), 7.70 (dd, *J* = 22.8, 7.0 Hz, 4H), 7.57 (d, *J=* 6.7 Hz, 1H), 7.43 (d, *J=* 7.3 Hz, 2H), 7.36 (s, 1H), 6.50 (s, 2H), 2.40 (s, 3H).

MS-ESI: Calculated.: [M-Br]⁺ 327.41, Found: 327.20.

### Example 16

### Synthesis of compound AB24833A

### Step 1):

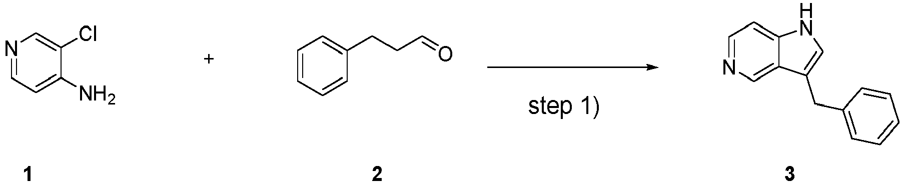

Compound 1 (2.0 g, 15.5 mmol), compound 2 (5.6 g, 46.6 mmol), bis(dibenzylideneacetone)palladium (771.0 mg, 0.5 mmol), tri-tert-butylphosphine tetrafluoroborate (451.0 mg, 1 mmol) and triethylenediamine (5.24 g, 30 mmol) were dissolved in N,N-dimethylacetamide (50 mL). The mixture was replaced with N₂ three times, and stirred at 120°C for 16 h, new spot was detected using plate. The reaction mixture was cooled to room temperature, diluted with water and extracted with ethyl acetate (100 mL x 3). The combined organic phase was washed with saturated brine (100 mL), dried over anhydrous sodium sulfate and concentrated under reduced pressure to remove organic solvent. The residue was purified by column chromatography (ethyl acetate: petroleum ether=66.6%) to afford compound 3 (70 mg, 21.6% yield).

### Step 2):

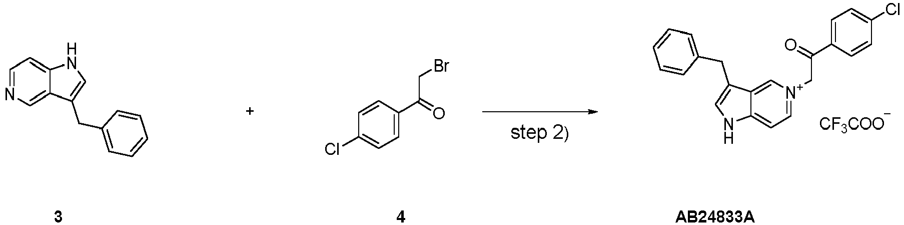

Compound 3 (70 mg, 0.336 mmol) and compound 4 (157 mg, 0.673 mmol) were dissolved in acetonitrile (10 mL). The mixture was stirred at 80°C for 3 h, new spot was detected using plate. The reaction mixture was cooled to room temperature and filtered, filter cake was washed with ethyl acetate to afford compound AB24833A (40 mg, 32.8% yield).

### Compound AB24833A

¹H NMR (399 MHz, DMSO-d6) δ 12.76 (s, 1H), 9.26 (s, 1H), 8.36 (d, J = 7.0 Hz, 1H), 8.06 (s, 2H), 8.02 (d, J = 7.2 Hz, 1H), 7.82 (s, 1H), 7.75 (s, 2H), 7.29 (d, J = 7.8 Hz, 4H), 7.18 (s, 1H), 6.33 (s, 2H), 4.14 (s, 2H).

MS-ESI: Calculated.: [M- CF₃COO]⁺ 361.85, Found: 361.25.

### Example 17

### Synthesis of compound AB24916

### Step 1:

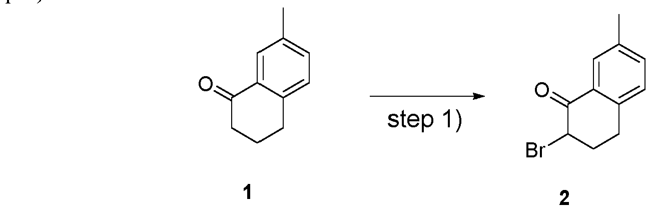

Compound 1 (480mg, 3.0mmol) was dissolved in acetic acid (7mL), two drops of liquid bromine (480mg, 3.0mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 2 (400 mg, 55.8% yield).

### Step 2):

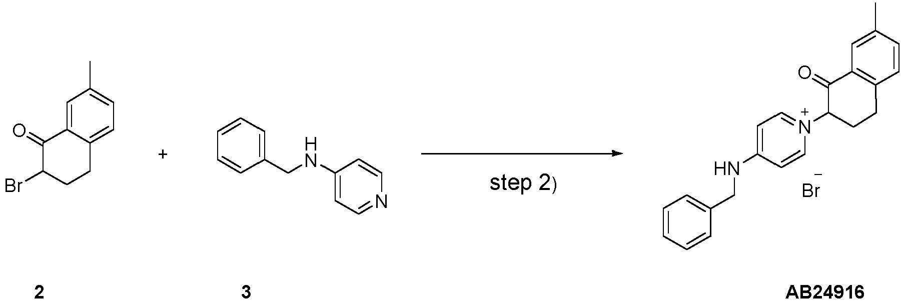

Compound 2 (150 mg, 0.63 mmol) was dissolved in acetonitrile (10 mL), and compound 3 (150 mg, 0.82 mmol) was added. The mixture was replaced with N₂ three times, and stirred at room temperature for overnight, new spot was detected using plate, solid was precipitated and filtered, and filter cake was washed with acetonitrile (2ml) three times and dried to afford compound AB24916 (50 mg, 23.1% yield).

### Compound AB24916

¹H NMR (400 MHz, DMSO-d6) δ 9.55 (s, 1H), 8.26 (d, J = 7.0 Hz, 1H), 8.15 (d, J = 7.1 Hz, 1H), 7.69 (s, 1H), 7.47 (d, J = 7.6 Hz, 1H), 7.35 (dd, J = 20.0, 5.6 Hz, 6H), 7.07 (d, J = 7.0 Hz, 1H), 6.98 (d, J = 6.7 Hz, 1H), 5.69 (d, J = 10.6 Hz, 1H), 4.57 (d, J = 5.5 Hz, 2H), 3.21 (d, J = 12.6 Hz, 1H), 3.11 (d, J = 16.3 Hz, 1H), 2.75 (d, J = 10.4 Hz, 1H), 2.46 - 2.41 (m, 1H), 2.32 (s, 3H).

MS-ESI: Calculated.: [M-Br]⁺ 343.44, Found: 343.10.

### Example 18

### Synthesis of compound AB24913

### Step 1):

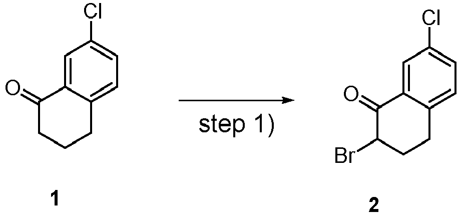

Compound 1 (500mg, 2.77 mmol) was dissolved in acetic acid (7mL), two drops of liquid bromine (443mg, 2.77mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 2 (200 mg, 27.8% yield).

### Step 2):

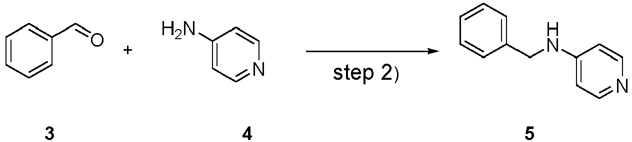

Compound 3 (5.6 g, 53.13 mmol) was dissolved in ethanol (10 mL), and compound 4 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with ice water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 5 (2.6g, 26.8% yield).

### Step 3):

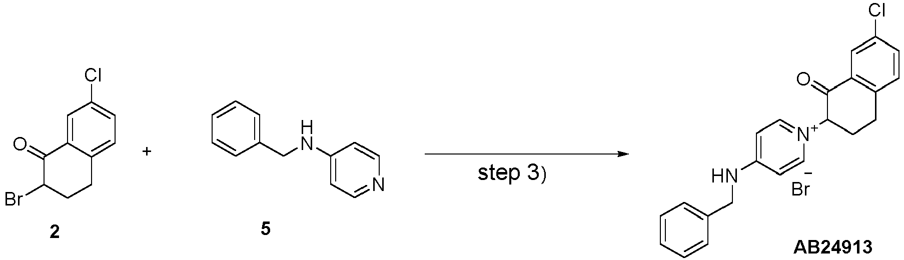

Compound 2 (200 mg, 0.77 mmol) was dissolved in acetonitrile (10 mL), and compound 5 (212 mg, 1.12 mmol) was added. The mixture was replaced with N₂ three times, and stirred at room temperature for overnight, new spot was detected using plate, solid was precipitated and filtered, and filter cake was washed with acetonitrile (2 mL) three times and dried to afford compound AB249163 (50 mg, 17.5% yield),

### Compound AB24913

¹H NMR (400 MHz, DMSO,d6) δ 9.34 (s, 1H), 8.22 (s, 1H), 8.12 (d, *J =* 6.5 Hz, 1H), 7.82 (s, 1H), 7.72 (d, *J =* 8.0 Hz, 1H), 7.50 (d, *J* = 7.9 Hz, 1H), 7.34 (d, *J =* 25.3 Hz, 5H), 7.01 (d, *J =* 6.4 Hz, 2H), 5.68 (d, *J=* 14.0 Hz, 1H), 4.57 (d, *J=* 5.3 Hz, 2H), 3.19 (t, *J=* 17.3 Hz, 3H), 2.77 (d, *J=* 9.0 Hz, 1H).

MS-ESI: Calculated.: [M-Br]⁺363.86, Found: 363.30.

### Example 19

### Synthesis of compound AB24841A

### Step 1:

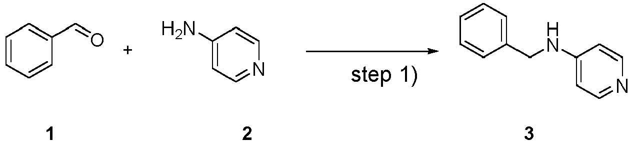

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with ice water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2:

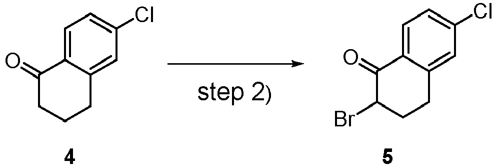

Compound 4 (500mg, 2.7 mmol) was dissolved in acetic acid (50 mL), two drops of liquid bromine (442 mg, 2.7 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 5 (700 mg, 97.3% yield).

### Step 3):

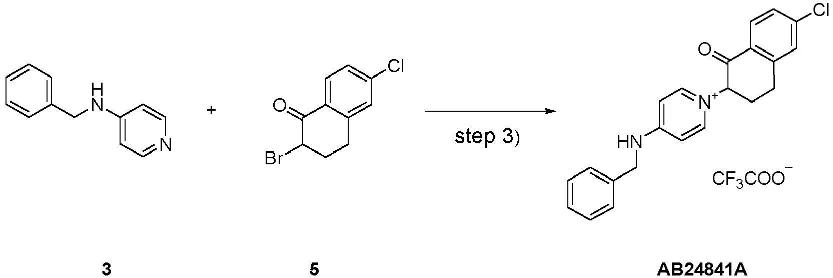

Compound 5 (400 mg, 1.5 mmol) and compound 3 (200 mg, 1.1 mmol) were dissolved in acetonitrile (10 mL). The mixture was replaced with N₂ three times, and stirred at room temperature for 5 h, new spot was detected using plate. The reaction mixture was concentrated under reduced pressure to remove organic solvent. The residue was purified by column chromatography (methanol: dichloromethane=0-10%) to afford compound AB24841A (37 mg, 9.3% yield).

### Compound AB24841A

¹H NMR (400 MHz, CDCl3) δ 9.96 (s, 1H), 7.90 - 7.52 (m, 3H), 7.25 (s, 8H), 6.50 (s, 1H), 5.50 (s, 1H), 4.43 (s, 2H), 3.34 (s, 1H), 3.05 (s, 1H), 2.50 (d, J = 38.1 Hz, 2H).

MS-ESI: Calculated.: [M-CF₃C00]⁺363.86, Found: 363.25.

### Example 20

### Synthesis of compound AB24920

### Step 1:

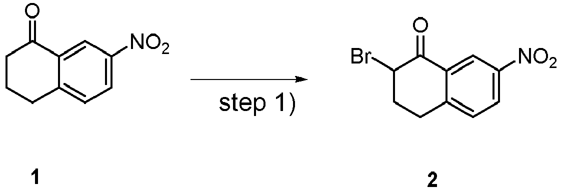

Compound 1 (500 mg, 2.6mmol) was dissolved in tetrahydrofuran (10 mL), liquid bromine (418mg, 2.6mmol) was added at 0 °C. The mixture was stirred at 0 °C for 2 h, new spot was detected using plate. The reaction mixture was diluted with sodium bicarbonate aqueous solution (2M, 40mL) and ethyl acetate (40mL), and the organic phase was dried to afford compound 2 (400mg, crude).

### Step 2):

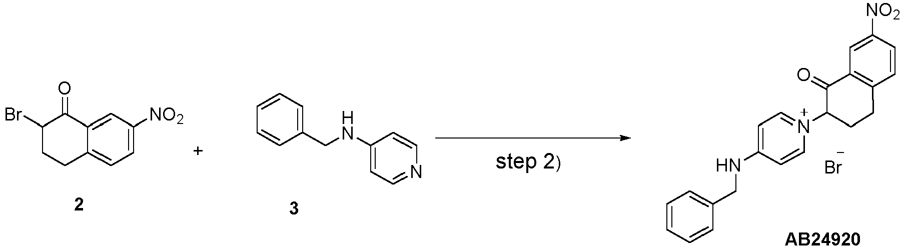

Compound 2 (200 mg, 0.70 mmol) and compound 3 (130 mg, 0.70 mmol) were dissolved in acetonitrile (10 mL). The mixture was stirred at room temperature for 4 h, new spot was detected using plate. The reaction mixture was dried and purified by column chromatography (DCM: MeOH=10:1) to afford compound AB24920 (41mg, 14.8% yield).

### Compound AB24920

¹H NMR (400 MHz, DMSO,d6) δ = 9.44 (s, 1H), 8.54 (s, 1H), 8.46 (d, *J*=8.1, 1H), 8.26 (d, *J*=6.5, 1H), 8.14 (d, *J*=6.0, 1H), 7.76 (d, *J*=8.3, 1H), 7.37 (s, 4H), 7.31 (s, 1H), 7.04 (s, 2H), 5.79 (d, *J*=12.9, 1H), 5.73 (s, 1H), 4.58 (d, J=4.9, 2H), 2.83 (s, 1H), 2.51 (s, 1H).

MS-ESI: Calculated.: [M-Br]⁺374.41, Found: 374.30

### Example 21

### Synthesis of compound AB24923

### Step 1:

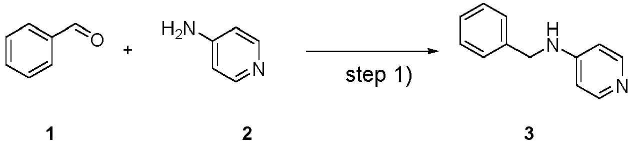

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

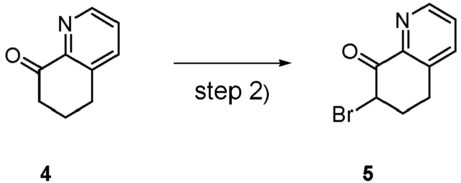

Compound 4 (300mg, 2.03 mmol) was dissolved in acetic acid (10 mL), two drops of liquid bromine (326 mg, 2.03 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 5 (400 mg, 86.7% yield).

### Step 3):

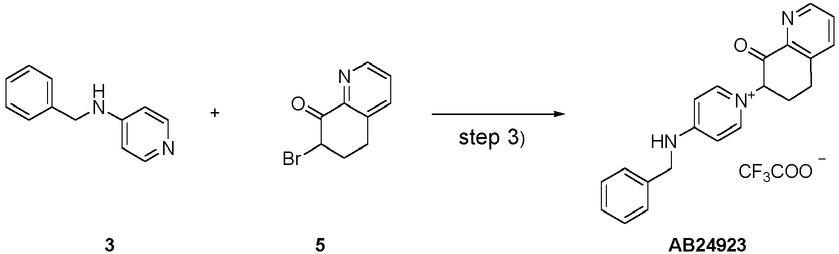

Compound 5 (200 mg, 0.88 mmol) was dissolved in acetonitrile (10 mL), and compound 3 (109 mg, 0.59 mmol) were added. The mixture was replaced with N₂ three times, and stirred at room temperature for overnight, new spot was detected using plate. The reaction mixture was dried to afford compound AB24923 (25 mg, 8.6% yield).

### Compound AB24923

¹H NMR (400 MHz, CD3OD) δ 8.68 (s, 1H), 8.14 (d, J = 6.6 Hz, 1H), 8.06 (t, J = 6.7 Hz, 3H), 7.71 (s, 1H), 7.37 (s, 7H), 7.31 (d, J = 3.5 Hz, 1H), 6.97 (dd, J = 26.9, 6.0 Hz, 3H), 4.93 - 4.91 (m, 2H), 4.60 (s, 2H), 3.44 (d, J = 13.3 Hz, 1H), 2.85 (t, J = 9.2 Hz, 1H), 2.64 (d, J = 10.5 Hz, 1H).

MS-ESI: Calculated.: [M-CF₃COO]⁺ 330.40, Found: 330.30.

### Example 22

### Synthesis of compound AB24840A

### Step 1):

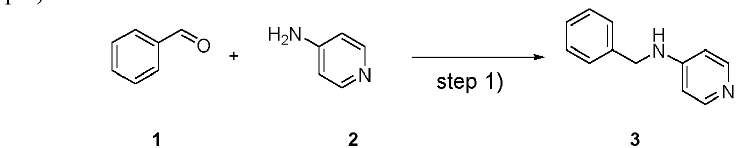

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

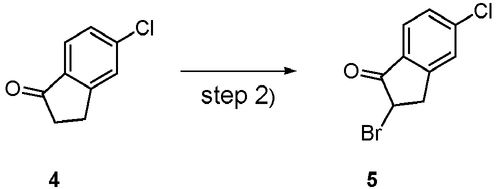

Compound 4 (500mg, 3.0 mmol) was dissolved in acetic acid (50 mL), two drops of liquid bromine (480 mg, 3.0 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 5 (600 mg, 38.46% yield).

### Step 3):

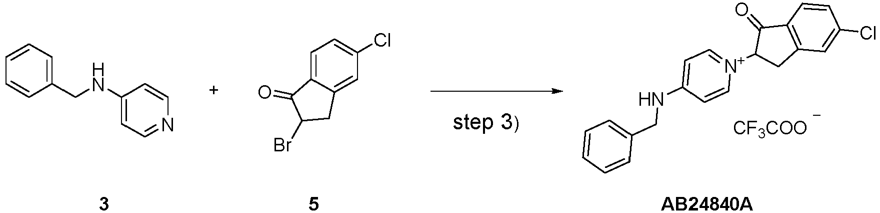

Compound 5 (300 mg, 1.2 mmol) and compound 3 (200 mg, 1.1 mmol) were dissolved in acetonitrile (10 mL). The mixture was replaced with N₂ three times, and stirred at room temperature for 5 h, new spot was detected using plate. The reaction mixture was concentrated under reduced pressure to remove organic solvent. The residue was purified by column chromatography (methanol: dichloromethane=0-10%) to afford compound AB24840A (27.6 mg, 7.2% yield).

### Compound AB24840A

¹H NMR (400 MHz, DMSO, d6) δ 9.38 (s, 1H), 8.31 (d, J = 7.1 Hz, 1H), 8.16 (d, J = 7.0 Hz, 1H), 7.84 - 7.75 (m, 2H), 7.60 (d, J = 8.1 Hz, 1H), 7.34 (dd, J = 23.4, 6.5 Hz, 5H), 6.99 (d, J = 6.6 Hz, 2H), 5.62 (t, J = 6.7 Hz, 1H), 4.58 (d, J = 5.4 Hz, 2H), 3.85 (dd, J = 17.3, 8.2 Hz, 1H), 3.45 (s, 1H).

MS-ESI: Calculated.: [M-CF₃COO]⁺349.84, Found: 349.20.

### Example 23

### Synthesis of compound AB24904

### Step 1:

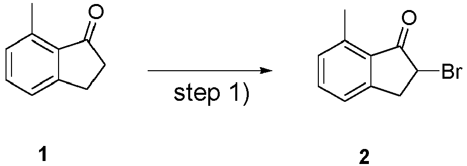

Compound 1 (146mg, 1.0mmol) was dissolved in acetic acid (5 mL), two drops of liquid bromine (160 mg, 1.0 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 2 (180 mg, 97.3% yield).

### Step 2:

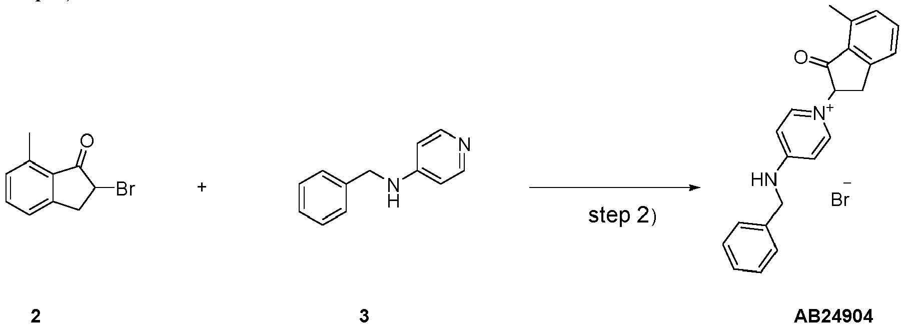

Compound 2 (180 mg, 0.79 mmol) was dissolved in acetonitrile (10 mL), and compound 3 (161 mg, 0.87 mmol) was added. The mixture was replaced with N₂ three times, and stirred at room temperature for overnight, new spot was detected using plate, solid was precipitated and filtered, and filter cake was washed with acetonitrile (2 mL) three times and dried to afford compound AB24904 (40 mg, 15.4 % yield),

### Compound AB24904

¹H NMR (400 MHz, DMSO-d6) δ 9.76 (s, 1H), 8.34 (d, J = 6.1 Hz, 1H), 8.20 (d, J = 6.0 Hz, 1H), 7.64 (d, J = 6.7 Hz, 1H), 7.45 (d, J = 6.7 Hz, 1H), 7.37 (s, 4H), 7.30 (d, J = 5.3 Hz, 2H), 7.11 (s, 1H), 6.97 (s, 1H), 5.67 (s, 1H), 4.57 (s, 2H), 3.77 (dd, J = 16.2, 8.1 Hz, 1H), 2.53 (s, 3H), 2.48 (s, 1H).

MS-ESI: Calculated.: [M-Br]⁺ 329.42, Found: 329.50.

### Example 24

### Synthesis of compound AB24905

### Step 1):

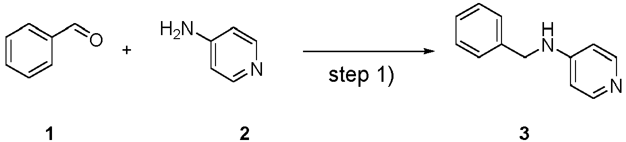

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

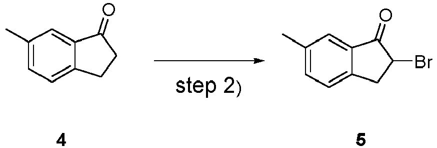

Compound 4 (300mg, 2.05 mmol) was dissolved in acetic acid (10 mL), two drops of liquid bromine (328 mg, 2.05 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent. The residue was purified by column chromatography (ethyl acetate: petroleum ether=0-20%) to afford compound 5 (300 mg, 65 % yield).

### Step 3:

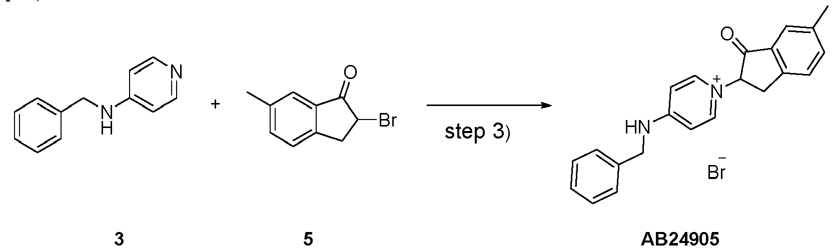

Compound 5 (200 mg, 0.89 mmol) was dissolved in acetonitrile (10 mL), and compound 3 (110 mg, 0.60 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-10%) to afford compound AB24905 (110 mg, 56 % yield).

### Compound AB24905

¹H NMR (400 MHz, DMSO-d6) δ 9.43 (s, 1H), 8.30 (d, *J =* 6.9 Hz, 1H), 8.15 (d, *J =* 6.8 Hz, 1H), 7.63 (d, *J =* 7.6 Hz, 1H), 7.55 (d, *J =* 8.9 Hz, 2H), 7.36 (s, 4H), 7.30 (d, *J =* 5.6 Hz, 1H), 6.99 (t, *J =* 8.0 Hz, 2H), 5.62 (s, 1H), 4.57 (d, *J =* 5.5 Hz, 2H), 3.78 (dd, *J =* 16.7, 8.4 Hz, 1H), 3.37 (s, 1H), 2.38 (s, 3H).

MS-ESI: Calculated.: [M-Br]⁺329.41, Found: 329.25.

### Example 25

### Synthesis of compound AB24883

### Step 1:

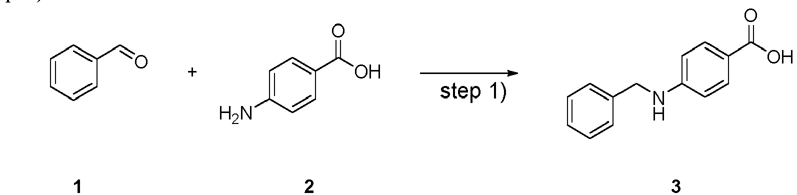

Compound 1 (1.06 g, 10 mmol) was dissolved in ethanol (50 mL), and compound 2 (1.37 g, 10 mmol) was added. The mixture was replaced with N₂ three times, and stirred at 90°C for 2 h, new spot was detected using plate. The reaction mixture was cooled to 0 °C, sodium borohydride (1.13 g, 30 mmol) was slowly added, and the system naturally rose to room temperature and was stirred for 1 h, new spot was detected using plate. The reaction mixture was diluted with water (20 mL) and extracted with ethyl acetate (20 mL x 3). The combined organic phase was washed with saturated brine (20 mL), dried over anhydrous sodium sulfate, filtered and concentrated under reduced pressure to remove organic solvent. The residue was purified by column chromatography (methanol: dichloromethane=10:1) to afford compound 3 (600 mg, 26.4% yield).

### Step 2):

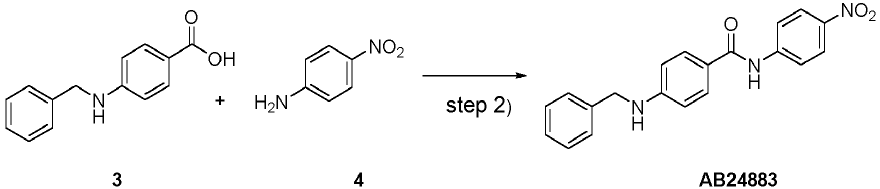

Compound 3 (50mg, 0.22mmol) was dissolved in N,N-dimethylformamide (5 mL), and 2- (7-azabenzotriazole)-N,N,N',N'-tetramethyluronium hexafluorophosphate (125.55mg, 0.33mmol), N,N-diisopropylethylamine (85.2 mg, 0.66 mmol) and compound 4 (25.9 mg, 0.24mmol) were added. The mixture was replaced with N₂ three times, and stirred at room temperature for overnight, new spot was detected using plate. The reaction mixture was diluted with water (10 ml) and extracted with ethyl acetate (10 mL x 3). The combined organic phase was washed with saturated brine, dried over anhydrous sodium sulfate, filtered, and concentrated under reduced pressure. The residue was purified by column chromatography (methanol: dichloromethane=0-10%) to afford compound AB24883 (27.9 mg, 36.7% yield).

### Compound AB24883

¹H NMR (400 MHz, DMSO,d6) δ 8.79 (s, 1H), 8.70 (d, J = 8.2 Hz, 1H), 7.94 (d, J = 8.4 Hz, 2H), 7.77 (s, 1H), 7.62 (s, 1H), 7.36 (s, 4H), 7.26 (s, 1H), 6.78 (d, J = 8.1 Hz, 2H), 4.43 (d, J = 5.0 Hz, 2H).

MS-ESI: Calculated.: [M+1]⁺348.30, Found: 348.20.

### Example 26

### Synthesis of compound AB24851

### Step 1):

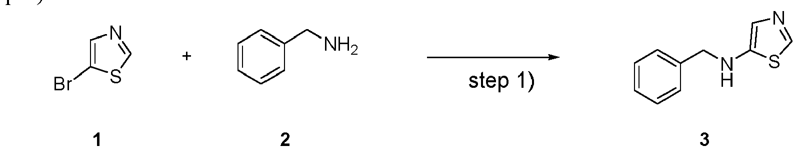

Compound 1 (3.0g, 18.3mmol) was dissolved in toluene (50mL), and compound 2 (5.8g, 54.8 mmol), caesium carbonate (12.0g, 36.5mmol),tri-(diphenylene-BASE acetone) dipalladium (334mg, 0.36mmol) and 2-dicyclohexylphosphate-2,4,6-triisopropylbiphenyl (262 mg, 0.54 mmol) were added. The mixture was replaced with N₂ three times, and stirred at 110°C for 16h, new spot was detected using plate. The reaction mixture was cooled to room temperature, diluted with water (50 ml) and extracted with ethyl acetate (50 mL x 3). The combined organic phase was washed with saturated brine, dried over anhydrous sodium sulfate, filtered, and concentrated under reduced pressure to remove organic solvent. The residue was purified by column chromatography (methanol: dichloromethane=10:1) to afford compound 3 (150 mg, 4.4 % yield).

### Step 2:

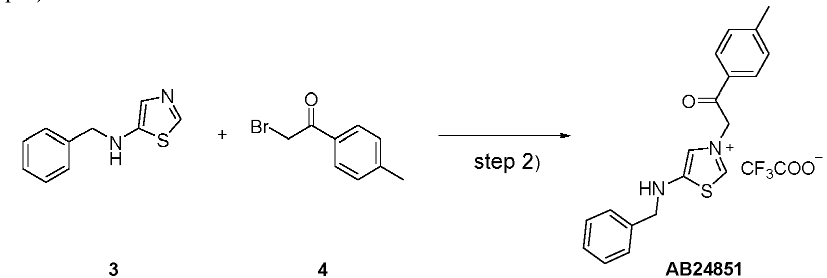

Compound 3 (150 mg, 0.78 mmol) was dissolved in acetonitrile (5 mL), and compound 4 (151 mg, 0.71 mmol) was added. The mixture was replaced with N₂ three times, and stirred at 80°C for overnight, new spot was detected using plate. The reaction mixture was cooled to room temperature and dried, the residue was purified by preparation purification to afford compound AB24851 (10 mg, 3.9 % yield).

### Compound AB24851

¹H NMR (400 MHz,CD30D) δ 7.93 (d, J = 7.8 Hz, 2H), 7.34 (dd, J = 47.3, 19.8 Hz, 9H), 4.58 (s, 2H), 4.37 (s, 2H), 2.45 (s, 3H).

MS-ESI: Calculated.: [M-CF₃COO]⁺323.43, Found: 323.20.

### Example 27

### Synthesis of compound AB24850

### Step 1):

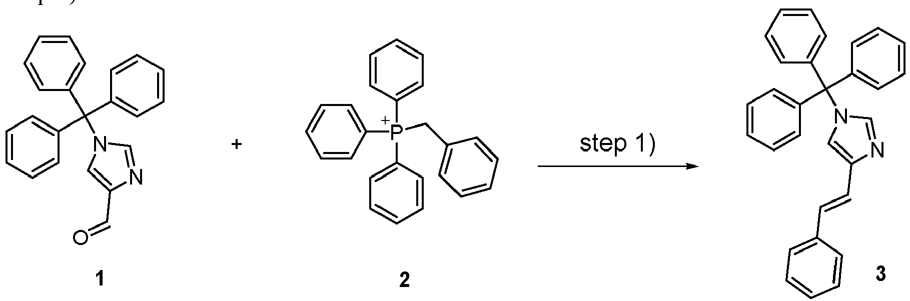

Compound 1 (2g, 5.91mmol), compound 2 (2.51g, 7.09mmol) and sodium methoxide (958mg, 17.73mmol) were dissolved in anhydrous ethanol (30ml). The mixture was stirred at room temperature for 16h, new spot was detected using plate. The reaction mixture was cooled to room temperature, diluted with water (50 ml) and extracted with ethyl acetate (50 mL x 3). The combined organic phase was washed with saturated brine (50ml), dried over anhydrous sodium sulfate, and concentrated under reduced pressure to remove organic solvent. The residue was purified by column chromatography (ethyl acetate: petroleum ether=0-30%) to afford compound 3 (2.1g, 86.24 % yield).

### Step 2):

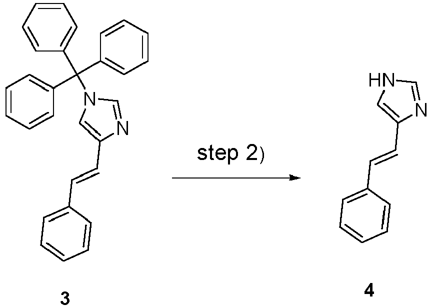

Compound 3 (1.2g, 2.91mmol) was dissolved in tetrahydrofuran (6mL) and water (6mL), and concentrated hydrochloric acid (3mL) was added. The mixture was stirred at 100°C for 3 h, new spot was detected using plate. The reaction mixture was cooled to room temperature, and saturated sodium bicarbonate aqueous solution was added to adjust alkali content. The mixture was xtracted with ethyl acetate (50 mL x 3). The combined organic phase was washed with saturated brine (50mL), dried over anhydrous sodium sulfate, and concentrated under reduced pressure to remove organic solvent to afford compound 4 (500 mg, 83.21 % yield).

### Step 3:

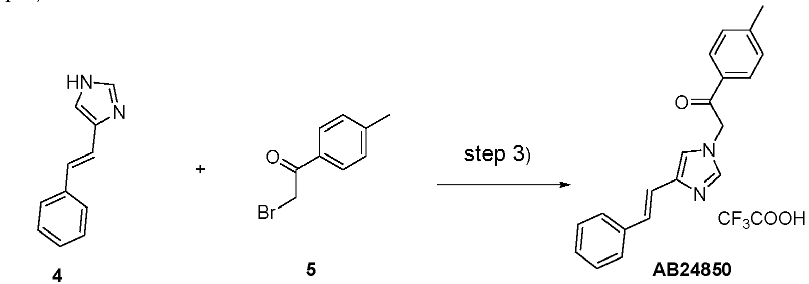

Compound 4 (200 mg, 1.17 mmol) and compound 5 (249 mg, 1.17 mmol) were dissolved in acetonitrile (10 mL), and triethylamine (356mg, 3.51mmol) was added. The mixture was stirred at 80°C for 16 h, new spot was detected using plate. The reaction mixture was cooled to room temperature and dried, the residue was purified by preparation purification to afford compound AB24850 (40 mg, 11.32 % yield).

### Compound AB24850

¹H NMR (400 MHz, CD₃OD) δ 8.83 (s, 0H), 7.93 (d, *J=* 7.4 Hz, 1H), 7.43 - 7.27 (m, 2H), 7.22 (s, 0H), 6.99 (s, 0H), 6.48 (s, 0H), 5.84 (s, 1H), 2.44 (s, 3H).

MS-ESI: Calculated.: [M+1]⁺303.14, Found: 303.00.

### Example 28

### Synthesis of compound AB24885

### Step 1):

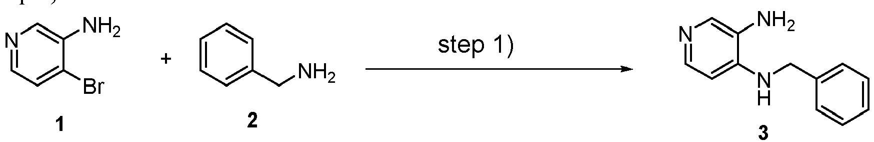

Compound 1 (519mg, 3 mmol) was dissolved in tetrahydrofuran (30mL), and compound 2 (481mg, 4.5mmol) and methanesulfonic acid (2-dicyclohexylphosphine)-3,6-dimethoxyl-2',4',6'-triisopropyl-1,1'-biphenyl)(2'-amino-1,1'-biphenyl-2-yl) palladium (II) (163 mg, 0.18mmol) were added, the mixture was cooled to 0°C, and lithium bis(trimethylsilyl)amide (7.5mL) was injectly added. The mixture was replaced with N₂ three times, and stirred at 40°C for 16h, new spot was detected using plate. The reaction mixture was diluted with water (50mL) and extracted with ethyl acetate (50 mL x 3). The combined organic phase was washed with saturated brine (50 mL), dried over anhydrous sodium sulfate, filtered, and concentrated under reduced pressure to remove organic solvent. The residue was purified by column chromatography (methanol: dichloromethane=10:1) to afford compound 3 (80 mg, 13 % yield).

### Step 2):

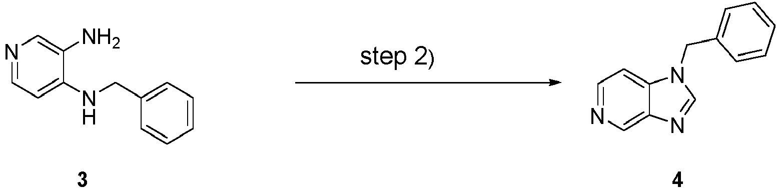

Compound 3 (160mg, 0.8mmol) was dissolved in n-tert-butanol (10mL), and triethyl orthoformate (296mg, 2mmol) was added, then formic acid (37mg, 0.8mmol) was added. The mixture was replaced with N₂ three times, and stirred at 110°C for 18 h, new spot was detected using plate. The reaction mixture was concentrated under reduced pressure to remove organic solvent. The residue was purified by thin layer chromatography to afford compound 4 (100 mg, 63.8 % yield).

### Step 3):

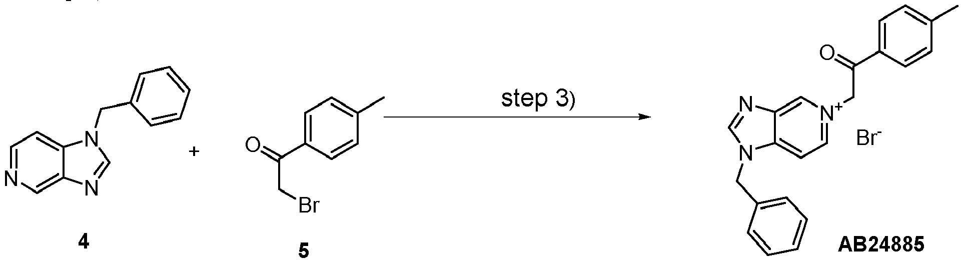

Compound 4 (100 mg, 0.5 mmol) was dissolved in acetonitrile (10 mL), and compound 5 (127 mg, 0.6 mmol) was added. The mixture was replaced with N₂ three times, and stirred at room temperature for overnight, new spot was detected using plate. The reaction mixture was concentrated under reduced pressure to remove organic solvent. The residue was purified by thin layer chromatography to afford compound AB24885 (20 mg, 9.4 % yield).

### Compound AB24885

¹H NMR (400 MHz, DMSO-d6) δ 9.39 (s, 1H), 8.92 (s, 1H), 8.54 (d, *J=* 6.4 Hz, 1H), 8.17 (d, *J =* 6.9 Hz, 1H), 7.99 (d, *J =* 7.7 Hz, 2H), 7.42 (d, *J =* 10.2 Hz, 7H), 5.72 (s, 2H), 4.85 - 4.84 (m, 2H), 2.46 (s, 3H).

MS-ESI: Calculated.: [M-Br]⁺342.41, Found: 342.30.

### Example 29

### Synthesis of compound AB24898

### Step 1:

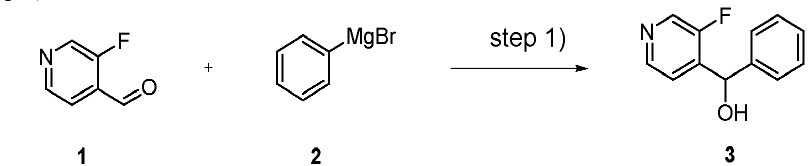

Compound 2 (53.5 mg, 150 mmol, 3N) was dissolved in tetrahydrofuran, and compound 1 (6.25g, 50 mmol) was added at 0°C. The mixture was stirred at 0°C for 16h, new spot was detected using plate. The reaction mixture was quenched with saturated ammonium chloride at 0°C, filtered with diatomite, extracted with ethyl acetate, and concentrated under reduced pressure to remove organic solvent. The residue was purified by column chromatography (ethyl acetate: petroleum ether=1:2) to afford compound 3 (4.2g, 41.37 % yield).

### Step 2):

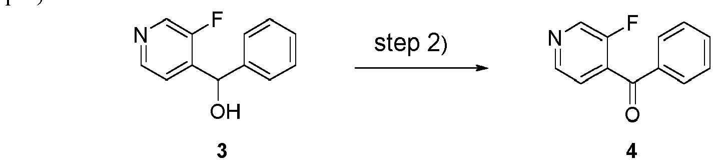

Compound 3 (4.06 g, 20 mmol) was dissolved in tetrahydrofuran, Dess-Martin periodinane (10.17 g, 24 mmol) was added at 0°C. The mixture was stirred at 0°C for 2 h, new spot was detected using plate, The reaction mixture was purified by column chromatography (ethyl acetate: petroleum ether=1:3) to afford compound 4 (4.0g, 99.0% yield).

### Step 3):

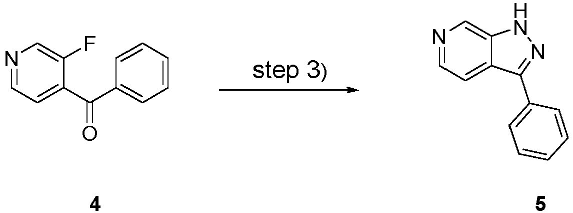

Compound 4 (402 mg, 2 mmol) was dissolved in ethanol, and hydrazine hydrate (200 mg, 4 mmol) was added. The mixture was stirred at 90°C for 16 h, new spot was detected using plate. The reaction mixture was dried and purified by column chromatography (ethyl acetate: petroleum ether=1:1) to afford compound 5 (60 mg, 15.30% yield).

### Step 4):

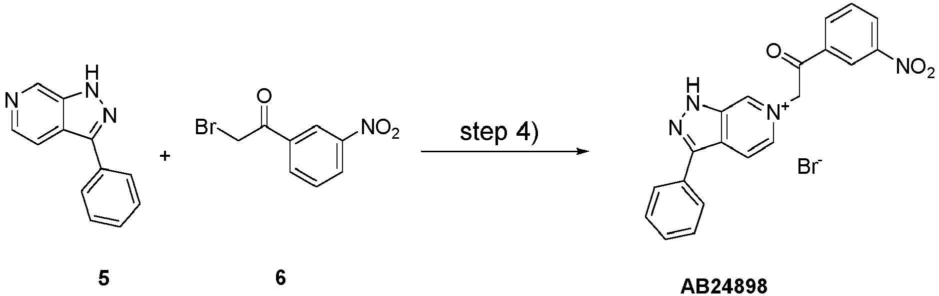

Compound 5 (100 mg, 0.5 mmol) and compound 6 (123.93 mg, 0.5 mmol) was dissolved in acetonitrile (5 mL). The mixture was stirred at room temperature for 3 h, new spot was detected using plate. The reaction mixture was concentrated under reduced pressure to remove organic solvent. The residue was purified by column chromatography (methanol: dichloromethane=0-30%) to afford compound AB24898 (100 mg, 54.61% yield).

### Compound AB24898

¹H NMR (400 MHz, DMSO-d6) δ 9.77 (s, 1H), 8.89 (d, *J =* 6.4 Hz, 1H), 8.78 (s, 1H), 8.65 - 8.47 (m, 3H), 8.11 (d, *J=* 7.4 Hz, 2H), 7.97 (t, *J=* 7.9 Hz, 1H), 7.65 - 7.50 (m, 3H), 6.65 (s, 2H).

MS-ESI: Calculated.: [M-Br]⁺ 359.36, Found: 359.25.

### Example 30

### Synthesis of compound AB24839B

### Step 1):

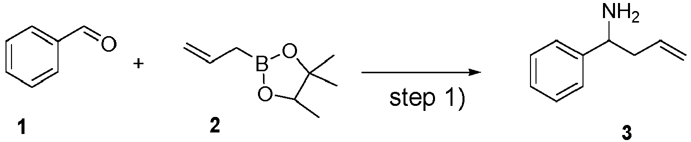

Compound 1 (2.1g, 20mmol) was dissolved in ammonia and ethanol (40:40 mL). The mixture was stirred at -78 °C to -10 °C for 3 h, and compound 2 (6.72g, 40mmol) was added. The mixture was stirred at room temperature for overnight, new spot was detected using plate, and concentrated hydrochloric acid was added to adjust pH to 1. The reaction mixture was extracted with water and ethyl acetate (80 mL), the pH of water phase was adjust to 14 with 50% sodium hydroxide, then the mixture was extracted with water and dichloromethane (2 x 80 mL), the organic phase was dried to afford compound 3 (1.6 g, 54.42% yield).

### Step 2:

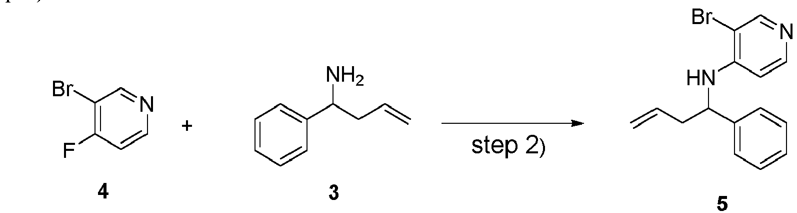

Compound 3 (5g, 28.57mmol), compound 4 (21g, 142.85mmol) and triethylamine (28.85g, 285.7mmol) were dissolved in acetonitrile (60 mL). The mixture was stirred at 80°C for 16 h, new spot was detected using plate. The reaction mixture was extracted with water and ethyl acetate (200 mL x2), the organic phase was dried and the residue was purified by column chromatography (PE:EA=5:1) to afford compound 5 (2.2g, 25.41% yield).

### Step 3):

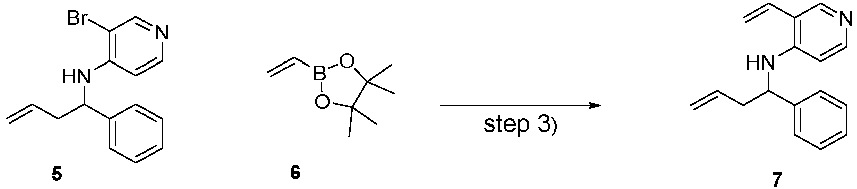

Compound 5 (2.2 g, 7.26 mmol), compound 6 (1.68 g, 10.89 mmol), sodium carbonate (2.1 g, 19.8 mmol) and [1,1'-bis(diphenylphosphine)ferrocene]palladium(II)chloride (495.6 mg, 0.66 mmol) were dissolved in dioxane: water (21:7 mL). The mixture was stirred at 95°C for 16 h in sealed tuble, new spot was detected using plate. The reaction mixture was extracted with water and ethyl acetate (200 mL x2), the organic phase was dried and the residue was purified by column chromatography (PE:EA=3:1) to afford compound 7 (200 mg, 11.02% yield).

### Step 4):

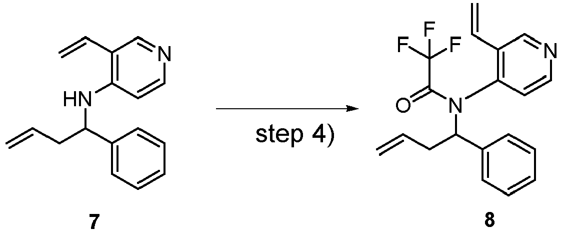

Compound 7 (500 mg, 2 mmol), dimethylaminopyridine (48.8 mg, 0.4 mmol), triethylamine (404 mg, 4 mmol) and trifluoroacetic anhydride (2.1 g, 10 mmol) were dissolved in dichloromethane (10 mL). The mixture was stirred at room temperature for 16 h, new spot was detected using plate. The reaction mixture was extracted with water and dichloromethane (50 mLx2), the organic phase was dried and the residue was purified by column chromatography (PE:EA=3:1) to afford compound 8 (200 mg, 28.82 % yield).

### Step 5):

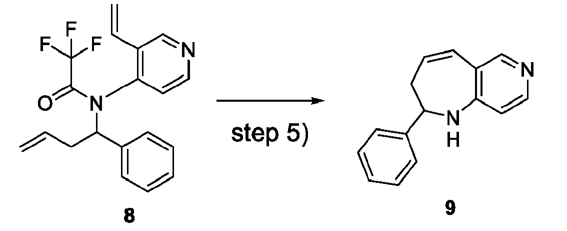

Compound 8 (300 mg, 0.86 mmol) and Grubbs Catalyst 2ND (100 mg) were dissolved in dichloromethane (10 mL). The mixture was stirred at 40 °C for 16 h, and potassium hydroxide aqueous solution (1N, 5mL) was added, then the mixture was stirred for 1 h, new spot was detected using plate. The reaction mixture was extracted with water and dichloromethane (50 mLx2), the organic phase was dried and the residue was purified by column chromatography (DCM: MeOH=10:1) to afford compound 9 (120 mg, 62.57 % yield).

### Step 6):

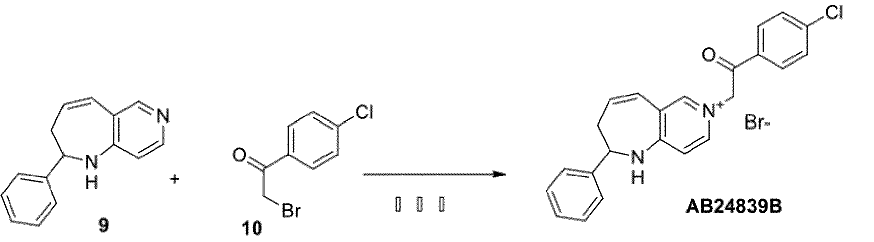

Compound 9 (60mg, 0.25mmol) was dissolved in acetonitrile (5mL), and th compound 10 (57mg, 0.25 mmol) was added. The mixture was replaced with N₂ three times, and stirred at room temperature for overnight, new spot was detected using plate. The reaction mixture was dried, the residue was purified by column chromatography (methanol: dichloromethane=0-10%) to afford compound AB24839B (6 mg, 9.94 % yield).

### Compound AB24839B

¹H NMR (400 MHz, CD₃OD) δ 8.16 (s, 1H), 8.05 (d, *J=* 7.1 Hz, 2H), 7.83 (s, 1H), 7.62 (d, *J* = 7.3 Hz, 2H), 7.37 (d, *J* = 13.9 Hz, 6H), 7.09 (d, *J* = 6.1 Hz, 1H), 6.40 (d, *J* = 11.0 Hz, 1H), 6.20 (s, 1H), 4.80 - 4.74 (m, 2H), 3.62 (s, 1H), 3.03 (s, 1H), 2.81 (d, *J =* 10.6 Hz, 1H).

MS-ESI: Calculated.: [M-Br]⁺ 375.87, Found: 375.30.

### Example 31

### Synthesis of compound AB24959

### Step 1):

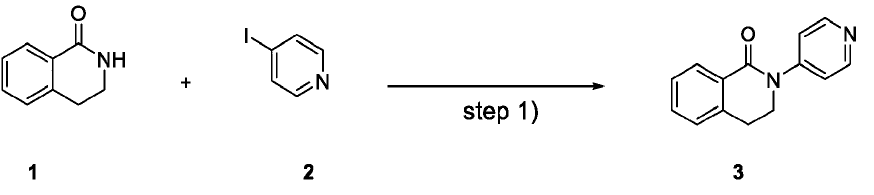

Compound 1 (294 mg, 2 mmol) was dissolved in dimethyl sulfoxide (10 mL), caesium carbonate (1.64 g, 5 mmol), copper(I) iodide (76.2 mg, 0.4 mmol) and five drops of N, N-2 methyl ethylenediamine were added, the mixture was stirred at room temperature for 10 min, and compound 2 (650 mg, 3 mmol) was added. The mixture was stirred at 120 °C for 2 h, new spot was detected using plate. The reaction mixture was extracted with water and ethyl acetate (10 mL). The organic phase was concentrated under reduced pressure, the residue was purified by column chromatography (methanol: dichloromethane=10%) to afford compound 3 (200 mg, 47.62% yield).

### Step 2:

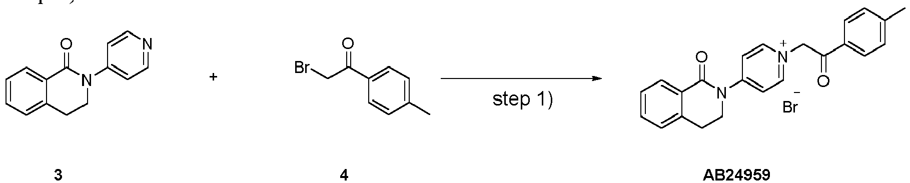

Compound 3 (200 mg, 0.89 mmol) was dissolved in acetonitrile (10 mL), and compound 4 (189.57 mg, 0.89 mmol) was added. The mixture was replaced with N₂ three times, and stirred at room temperature for overnight, new spot was detected using plate, solid was precipitated, filtered and wased with acetonitrile (5mL) to afford compound AB24959 (70 mg, 22.03% yield).

### Compound AB24959

¹H NMR (400 MHz, DMSO-d6) δ 8.83 (d, *J =* 5.4 Hz, 2H), 8.27 (d, *J =* 5.5 Hz, 2H), 8.04 (d, *J =* 7.2 Hz, 1H), 7.95 (d, *J =* 7.2 Hz, 2H), 7.63 (s, 1H), 7.45 (d, *J =* 5.7 Hz, 4H), 6.31 (s, 2H), 4.24 (s, 2H), 3.21 (s, 2H), 2.42 (s, 3H).

MS-ESI: Calculated.: [M-Br]⁺ 357.43, Found: 357.10.

### Example 32

### Synthesis of compound AB24938

### Step 1:

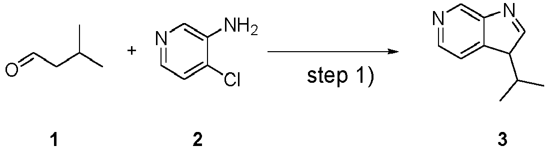

Compound 1 (2 g, 23 mmol) and compound 2 (2.5 g, 19 mmol) were dissolved in N,N-dimethylacetamide (30ml), and triethylenediamine (6.5 g, 58 mmol), bis(dibenzylideneacetone)palladium (890 mg, 0.97 mmol) and tri-tert-butylphosphine tetrafluoroborate (561 mg, 1.94 mmol) were added. The mixture was stirred at 120°C for 16 h in sealed tube, new spot was detected using plate. The reaction mixture was extracted with water and ethyl acetate (10 mL). The combined organic phase was concentrated under reduced pressure. The residue was purified by column chromatography (methanol: dichloromethane=5%) to afford compound 3 (800 mg, 17.9% yield).

### Step 2:

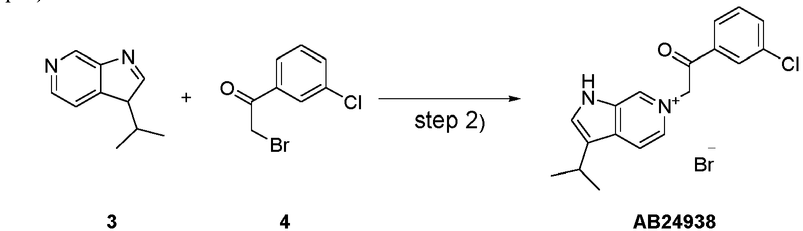

Compound 3 (100 mg, 0.625 mmol) was dissolved in acetonitrile (5 mL), and compound 4 (200 mg, 0.938 mmol) was added. The mixture was replaced with N₂ three times, and stirred at room temperature for overnight, new spot was detected using plate. The reaction mixture was dried, the residue was purified by column chromatography (methanol: dichloromethane=0-10%) to afford compound AB24938 (78 mg, 39.8% yield).

### Compound AB24938

¹H NMR (400 MHz, DMSO-d6) δ 9.14 (s, 1H), 8.23 (d, *J=* 9.1 Hz, 3H), 8.08 (s, 1H), 8.00 (d, *J* = 7.0 Hz, 1H), 7.84 (d, *J =* 7.3 Hz, 1H), 7.68 (t, *J =* 7.1 Hz, 1H), 6.39 (s, 2H), 3.14 (s, 1H), 1.33 (d, *J=* 5.7 Hz, 6H).

MS-ESI: Calculated.: [M-Br]⁺ 313.80, Found: 313.25.

### Example 33

### Synthesis of compound AB24821

### Step 1):

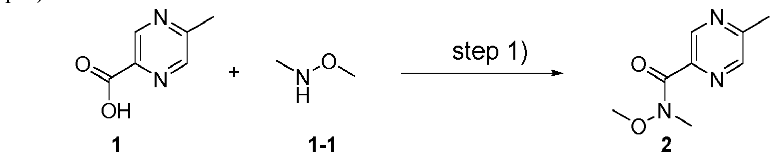

Compound 1 (2.7 g, 20 mmol) was dissolved in N,N-dimethylformamide (5 mL), and compound 1-1 (5.3g, 40mmol), 2-(7-azabenzotriazole)-N,N,N',N'-tetramethyluronium hexafluorophosphate (11.4g, 30mmol) and N,N-diisopropylethylamine (9 g, 70 mmol) were added. The mixture was stirred at room temperature for 1h, new spot was detected using plate. The reaction mixture was diluted with water (50 ml) and extracted with ethyl acetate (50 mL x 3). The combined organic phase was washed with saturated brine (50 mL), dried over anhydrous sodium sulfate, and concentrated under reduced pressure to remove organic solvent. The residue was purified by column chromatography (ethyl acetate: petroleum ether=0-30%) to afford compound 2 (3.6 g, 91.1% yield).

### Step 2):

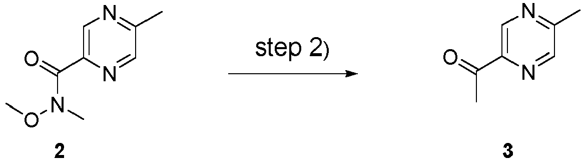

Compound 2 (200 mg, 1 mmol) was dissolved in dry tetrahydrofuran (5 mL), and a solution of methylmagnesium bromide in tetrahydrofuran (1N, 2 mL) was added at 0°C. The mixture was stirred at 0 °C for 3h, new spot was detected using plate. The reaction mixture was extracted with saturated sodium bicarbonate aqueous solution (6 mL), water (20 mL) and ethyl acetate (20mLx3). The combined organic phase was washed with saturated brine (30 mL), dried over anhydrous sodium sulfate, and concentrated under reduced pressure to remove organic solvent to afford compound 3 (67 mg, 37.4% yield).

### Step 3):

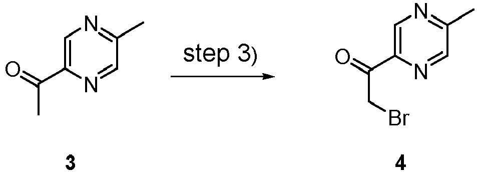

Compound 3 (363 mg, 3 mmol) was dissolved in acetic acid (5 mL), two drops of liquid bromine (383 mg, 3.8 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 4 (100 mg, 97.3% yield).

### Step 4):

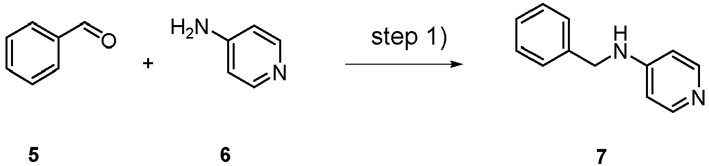

Compound 5 (5.6 g, 53.13 mmol) was dissolved in ethanol (10 mL), and compound 6 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 7 (2.6g, 26.8% yield).

### Step 5):

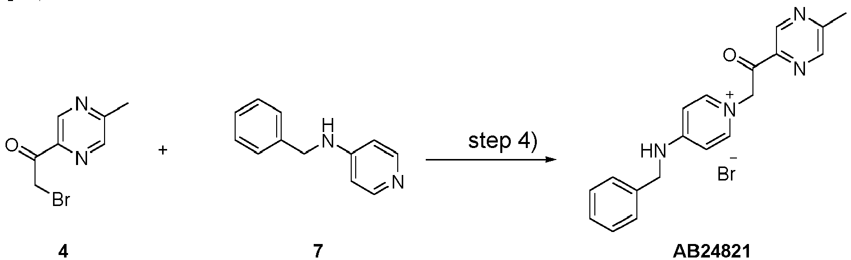

Compound 4 (100 mg, 0.46 mmol) and compound 7 (86 mg, 0.46 mmol) were dissolved in acetonitrile (10 mL). The mixture was stirred at room temperature for 5h, new spot was detected using plate. The reaction mixture was concentrated under reduced pressure to remove organic solvent. The residue was purified by column chromatography (methanol: dichloromethane=0-10%) to afford compound AB24821 (15 mg, 10.1% yield).

### Compound AB24821

¹H NMR (400 MHz, DMSO-d6) δ 9.45 (s, 1H), 9.03 (s, 1H), 8.77 (s, 1H), 8.16 (d, J = 7.0 Hz, 1H), 8.05 (d, J = 7.1 Hz, 1H), 7.36 (s, 4H), 7.30 (s, 1H), 7.05 (d, J = 8.3 Hz, 1H), 7.01 (d, J = 7.3 Hz, 1H), 5.92 (s, 2H), 4.56 (d, J = 5.4 Hz, 2H), 2.63 (s, 3H).

MS-ESI: Calculated.: [M-Br]⁺319.38, Found: 319.30.

### Example 34

### Synthesis of compound AB24839

### Step 1:

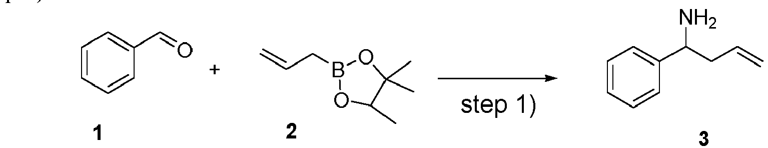

Compound 1 (2.1g, 20mmol) was dissolved in ammonia and ethanol (40:40 mL). The mixture was stirred at -78 °C to -10 °C for 3 h, and compound 2 (6.72g, 40mmol) was added. The mixture was stirred at room temperature for overnight, new spot was detected using plate, and concentrated hydrochloric acid was added to adjust pH to 1. The reaction mixture was extracted with water and ethyl acetate (80 mL), the pH of water phase was adjust to 14 with 50% sodium hydroxide, then the mixture was extracted with water and dichloromethane (2 x 80 mL), the organic phase was dried to afford compound 3 (1.6 g, 54.42% yield).

### Step 2):

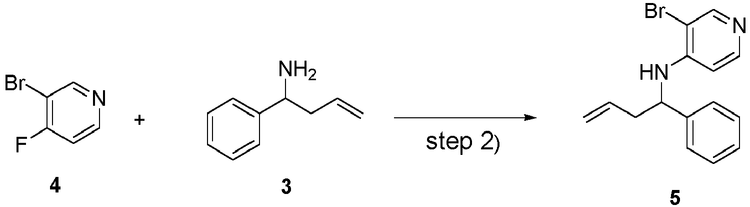

Compound 3 (5g, 28.57mmol), compound 4 (21g, 142.85mmol) and triethylamine (28.85g, 285.7mmol) were dissolved in acetonitrile (60 mL). The mixture was stirred at 80°C for 16 h, new spot was detected using plate. The reaction mixture was extracted with water and ethyl acetate (200 mLx2), the organic phase was dried and the residue was purified by column chromatography (PE:EA=5:1) to afford compound 5 (2.2g, 25.41% yield).

### Step 3):

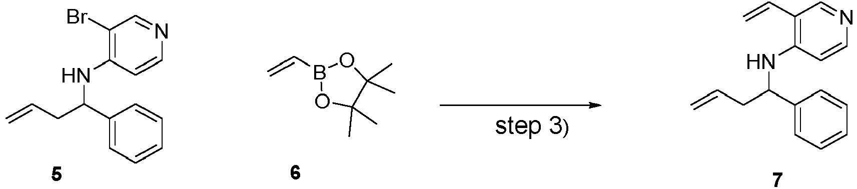

Compound 5 (2.2 g, 7.26 mmol), compound 6 (1.68 g, 10.89 mmol), sodium carbonate (2.1 g, 19.8 mmol) and [1,1'-bis(diphenylphosphine)ferrocene]palladium(II)chloride (495.6 mg, 0.66 mmol) were dissolved in dioxane: water (21:7 mL). The mixture was stirred at 95°C for 16 h in sealed tube, new spot was detected using plate. The reaction mixture was extracted with water and ethyl acetate (200 mL x2), the organic phase was dried and the residue was purified by column chromatography (PE:EA=3:1) to afford compound 7 (200 mg, 11.02% yield).

### Step 4):

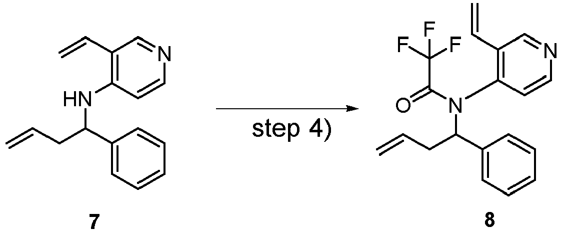

Compound 7 (500 mg, 2 mmol), dimethylaminopyridine (48.8 mg, 0.4 mmol), triethylamine (404 mg, 4 mmol) and trifluoroacetic anhydride (2.1 g, 10 mmol) were dissolved in dichloromethane (10 mL). The mixture was stirred at room temperature for 16 h, new spot was detected using plate. The reaction mixture was extracted with water and dichloromethane (50 mLx2), the organic phase was dried and the residue was purified by column chromatography (PE:EA=3:1) to afford compound 8 (200 mg, 28.82 % yield).

### Step 5):

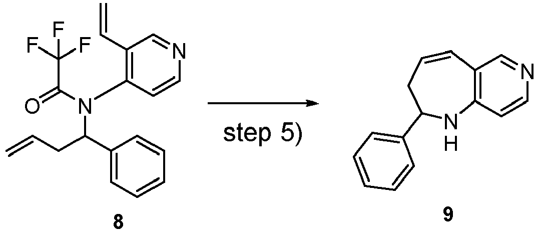

Compound 8 (300 mg, 0.86 mmol) and Grubbs Catalyst 2ND (100 mg) were dissolved in dichloromethane (10 mL). The mixture was stirred at 40 °C for 16 h, and a solution of potassium hydroxide aqueous solution (1N, 5mL) was added, then the mixture was stirred for 1 h, new spot was detected using plate. The reaction mixture was extracted with water and dichloromethane (50 mLx2), the organic phase was dried and the residue was purified by column chromatography (DCM: MeOH=10:1) to afford compound 9 (120 mg, 62.57 % yield).

### Step 6:

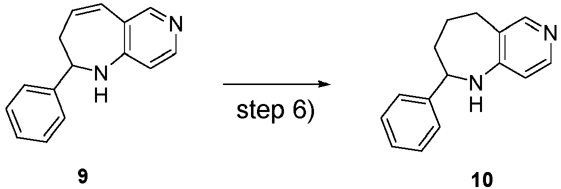

Compound 9 (120 mg, 0.53 mmol) was dissolved in tetrahydrofuran (5 mL), and palladium carbon (12 mg) was added. The mixture was stirred for 4 h under H₂ condition, new spot was detected using plate. The reaction mixture was filtered with diatomite, the filtrate was dried to afford compound 10 (20 mg, 16.77 % yield).

### Step7):

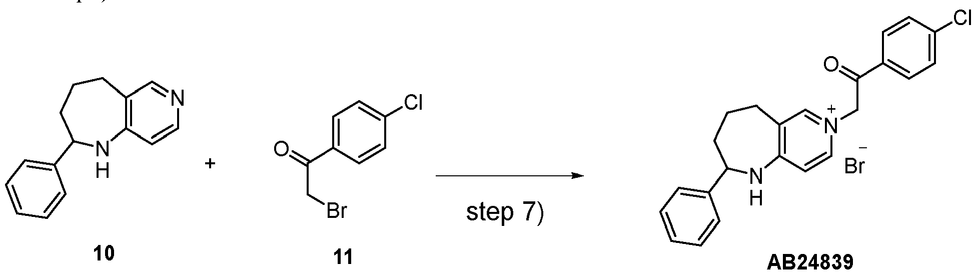

Compound 10 (20 mg, 0.08mmol) was dissolved in acetonitrile (5 mL), and compound 11 (20.71 mg, 0.08 mmol) was added. The mixture was replaced with N₂ three times, and stirred at room temperature for overnight, new spot was detected using plate. The reaction mixture was dried, the residue was purified by column chromatography (methanol: dichloromethane=0-10%) to afford compound AB24839 (3.1 mg, 9.94% yield).

### Compound AB24839

¹H NMR (400 MHz, CD3OD) δ 8.05 (d, *J =* 7.7 Hz, 2H), 7.92 - 7.82 (m, 2H), 7.62 (d, *J =* 7.7 Hz, 2H), 7.40 (dd, *J=* 30.0, 8.5 Hz, 6H), 6.92 (d, *J=* 6.7 Hz, 1H), 5.80 (s, 2H), 5.21 (d, *J=* 10.2 Hz, 1H), 2.69 - 2.45 (m, 2H), 2.13 (s, 2H), 1.99 (s, 2H).

MS-ESI: Calculated.: [M-Br]⁺ 377.89, Found: 377.25.

### Example 35

### Synthesis of compound AB27130

### Step 1):

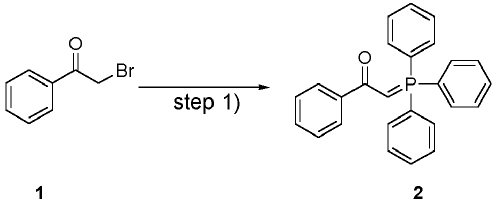

Compound 1 (200 mg, 1.01 mmol) and triphenylphosphine (400 mg, 3.54 mmol) dissolved in tetrahydrofuran (10 mL). The mixture was replaced with N₂ three times, and stirred at 80°C for 16h, new spot was detected using plate. The reaction mixture was cooled to room temperature, filtered, and the filter cake was washed with tetrahydrofuran three times to afford crude compound 2 (350 mg, 91.1 % yield).

### Step 2):

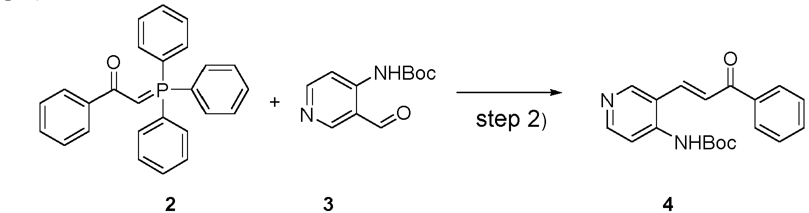

Compound 2 (200 mg, 0.52 mmol) and compound 3 (108 mg, 0.46 mmol) were dissolved in tetrahydrofuran (10 mL), and triethylamine (2 ml) was added. The mixture was replaced with N₂ three times, and stirred at 70°C for 16h, new spot was detected using plate. The reaction mixture was cooled to room temperature, concentrated under reduced pressure. The residue was purified by column chromatography (methanol: dichloromethane=0-10%) to afford compound 4 (150 mg, 89 % yield).

### Step 3:

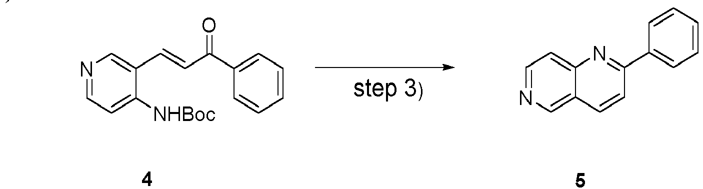

Compound 4 (200 mg, 0.61 mmol) was dissolved in ethyl acetates (5 mL), the mixture was stirred at room temperature for 3h, new spot was detected using plate. The reaction mixture was concentrated under reduced pressure to afford crude compound 5 (210 mg).

### Step 4):

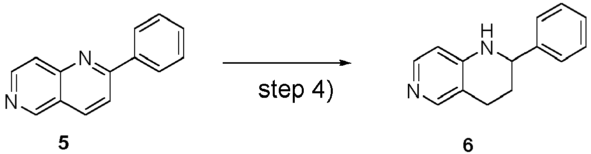

Compound 5 (200 mg, 0.97 mmol) was dissolved in ethanol (5 mL), palladium carbon (20 mg) was added. The mixture was replaced with N₂ three times, and stirred at room temperature for overnight, new spot was detected using plate. The reaction mixture was filtered, concentrated under reduced pressure. The residue was purified by column chromatography (methanol: dichloromethane=0-10%) to afford compound 6 (60 mg, 29.5 % yield).

### Step 5):

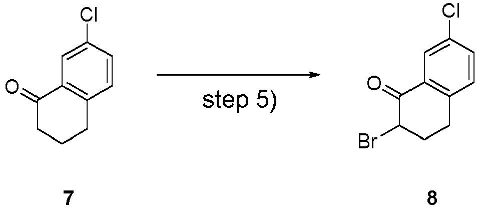

Compound 7 (200 mg, 1.11 mmol) was dissolved in dichloromethane (10 mL), and bis(N,N-dimethylacetamide) hydrogen dibromobromate (456 mg, 1.11 mmol) was added. The mixture was stirred at room temperature for 1 h, new spot was detected using plate. The reaction mixture was dried, and the residue was purified by thin layer chromatography to afford compound 8 (250 mg, 87.29% yield).

### Step 6:

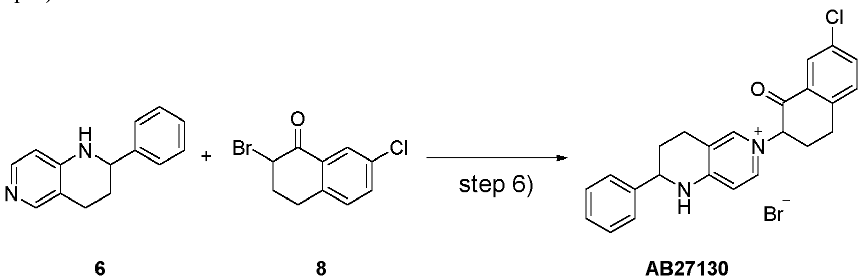

Compound 6 (100 mg, 0.48 mmol) and compound 8 (148 mg, 0.57 mmol) were dissolved in acetonitrile (10 mL), and potassium carbonate (66 mg, 0.48 mmol) was added. The mixture was stirred at room temperature for 16 h, new spot was detected using plate.The reaction mixture was concentrated under reduced pressure to remove organic solvent. The residue was purified by column chromatography (methanol: dichloromethane=0-10%) to afford compound AB21730 (55 mg, 29.4% yield).

### Compound AB21730

¹H NMR (400 MHz, DMSO-d6) δ 9.37 (s, 1H), 8.18 (s, 1H), 8.09 (s, 1H), 7.79 (d, *J=* 45.7 Hz, 2H), 7.53 (s, 1H), 7.38 (d, *J =* 20.0 Hz, 5H), 6.93 (s, 1H), 5.71 (d, *J =* 9.8 Hz, 1H), 4.77 (s, 1H), 3.22 (s, 2H), 2.79 (s, 2H), 2.52 (s, 2H), 2.10 (s, 1H), 1.89 (s, 1H).

MS-ESI: Calculated.: [M-Br]⁺389.90, Found: 389.10.

### Example 36

### Synthesis of compound AB27141

### Step 1):

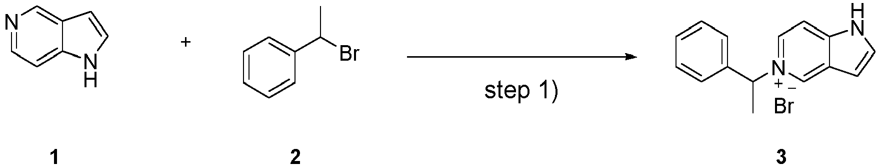

Compound 1 (240 mg, 2 mmol) was dissolved in dry N,N-dimethylformamide (10 mL), and compound 2 (555 mg, 1.5 mmol) and triethylamine (606.0 mg, 6.0 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by column chromatography (ethyl acetate: petroleum ether=0-50%) to afford compound 3 (260 mg, 58.47% yield).

### Step 2):

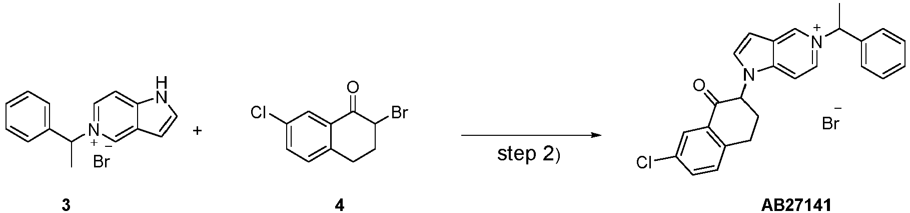

Compound 3 (111 mg, 0.50 mmol) was dissolved in acetonitrile (10 mL), and compound 4 (130 mg, 0.5 mmol) was added. The mixture was replaced with N₂ three times, and stirred at room temperature for overnight, new spot was detected using plate. The reaction mixture was filtered, and concentrated under reduced pressure to remove organic solvent. The residue was purified by thin layer chromatography (methanol: dichloromethane=0-10%) to afford compound AB27141 (40 mg, 19.95 % yield).

### Compound AB27141

¹H NMR (400 MHz, DMSO-d6) δ 9.67 (s, 1H), 8.75 (d, J = 6.4 Hz, 1H), 8.22 (d, J = 6.5 Hz, 1H), 8.05 (s, 1H), 7.80 (s, 1H), 7.73 (d, J = 7.8 Hz, 1H), 7.57 - 7.50 (m, 3H), 7.40 (dd, J = 12.0, 7.2 Hz, 4H), 7.14 (s, 1H), 6.25 - 6.15 (m, 2H), 3.43 (d, J = 43.7 Hz, 2H), 3.20 (d, J = 16.7 Hz, 1H), 2.95 (d, J = 10.2 Hz, 1H), 2.06 (d, J = 6.2 Hz, 3H).

MS-ESI: Calculated.: [M-Br]⁺ 401.91, Found: 401.10.

### Example 37

### Synthesis of compound AB24957

### Step 1):

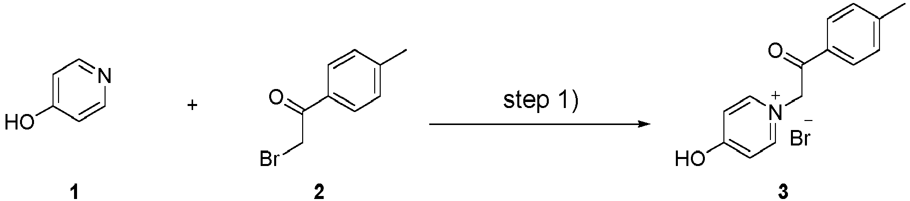

Compound 1 (300 mg, 3.15 mmol) was dissolved in acetonitrile (10 mL), and compound 2 (671 mg, 2.35 mmol) was added. The mixture was replaced with N₂ three times, and stirred at room temperature for overnight, new spot was detected using plate. The reaction mixture was dried, the residue was purified by thin layer chromatography to afford compound 3 (600 mg, 83.54 % yield).

### Step 2):

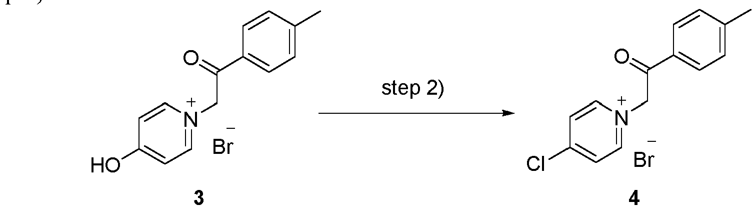

Compound 3 (200 mg, 0.87 mmol) was dissolved in phosphorus oxychloride (10 mL). The mixture was replaced with N₂ three times, and stirred at 90°C for 2h, new spot was detected using plate. The reaction mixture was dried to afford compound 4 (215 mg, crude) for the next step.

### Step 3):

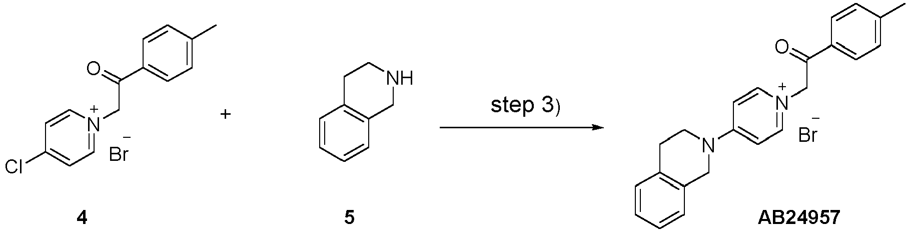

Compound 4 (215 mg, 0.88 mmol) was dissolved in N,N-dimethylformamide (10 mL), and compound 5 (117 mg, 0.88 mmol) and triethylamine (267 mg, 2.64 mmol) were added. The mixture was replaced with N₂ three times, and stirred at 80°C for overnight, new spot was detected using plate to afford compound AB24957 (80 mg, 26.50%).

### Compound AB24957

¹H NMR (400 MHz, DMSO, d6) δ 8.30 (s, 2H), 7.95 (d, *J =* 8.2 Hz, 2H), 7.45 (d, *J =* 8.1 Hz, 1H), 7.38 (s, 1H), 7.32 - 7.27 (m, 2H), 6.00 (s, 2H), 4.85 (s, 2H), 3.86 (t, *J=* 6.0 Hz, 2H), 3.04 (t, *J* = 5.9 Hz, 2H), 2.44 (s, 2H).

MS-ESI: Calculated.: [M-Br]⁺343.18, Found: 343.30.

### Example 38

### Synthesis of compound AB24989

### Step 1):

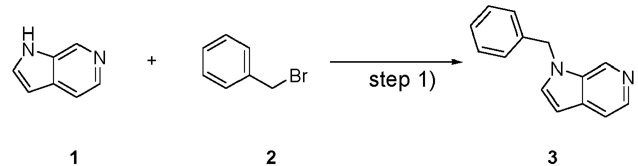

Compound 1 (200 mg, 1.69 mmol) was dissolved in N,N-dimethylformamide (20 mL), NaH (203 mg, 5.07 mmol) was added in ice bath. The mixture was stirred for 30 min in ice bath, compound 2 (289 mg, 1.86 mmol) was added, then the mixture was stirred at room temperature for 3 h, new spot was detected using plate. The reaction mixture was diluted with water (10 ml) and extracted with ethyl acetate (10 ml x 3). The combined organic phase was washed with saturated brine, dried over anhydrous sodium sulfate, and concentrated under reduced pressure. The residue was purified by column chromatography (ethyl acetate: petroleum ether=0-10%) to afford compound 3 (260 mg, 73.8% yield).

### Step 2):

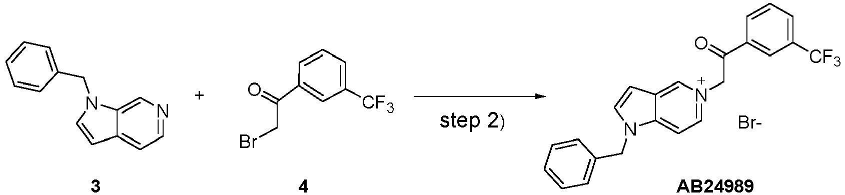

Compound 3 (300 mg, 1.44 mmol) was dissolved in acetonitrile (10 mL), and compound 4 (577 mg, 2.16 mmol) was added. The mixture was replaced with N₂ three times, and stirred at room temperature for overnight, new spot was detected using plate, solid was precipitated and filtered to afford compound AB24989 (30 mg, 5.3% yield).

### Compound AB24989

¹H NMR (400 MHz, DMSO, d6) δ 9.25 (s, 1H), 8.45 (s, 1H), 8.33 (s, 3H), 8.16 (s, 2H), 7.90 (s, 1H), 7.34 (s, 5H), 7.20 (s, 1H), 6.44 (s, 2H), 5.67 (s, 2H).

MS-ESI: Calculated.: [M-Br]⁺ 395.40, Found: 395.30.

### Example 39

### Synthesis of compound AB24906

### Step 1):

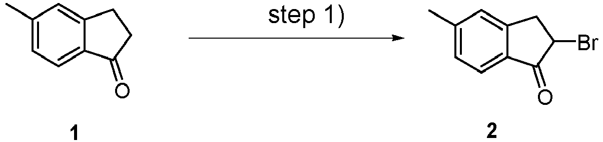

Compound 1 (438mg, 3.0mmol) was dissolved in acetic acid (5mL), a drop of hydrobromic acid were added, and a solution of liquid bromine (384 mg, 2.4 mmol) in acetic acid (2 mL) was added. The mixture was replaced with N₂ three times, and stirred at room temperature for 3h, new spot was detected using plate. The reaction mixture was filtered, and concentrated under reduced pressure, the residue was purified by thin layer chromatography to afford compound 2 (400 mg, 59.51 % yield).

### Step 2):

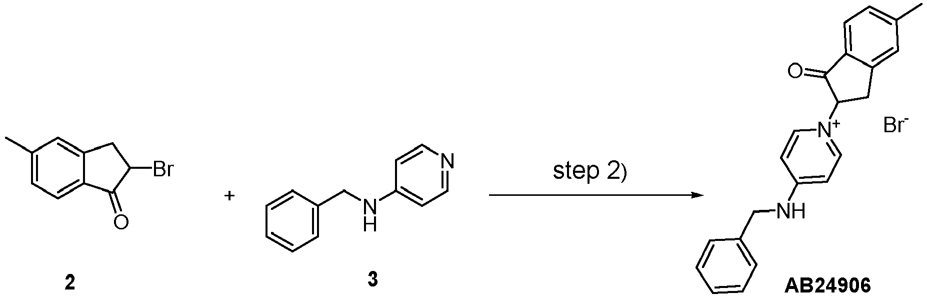

Compound 2 (200 mg, 0.9 mmol) was dissolved in acetonitrile (10 mL), and compound 3 (171 mg, 0.9 mmol) was added. The mixture was replaced with N₂ three times, and stirred at room temperature for 3 h, new spot was detected using plate. The reaction mixture was filtered, concentrated under reduced pressure to remove organic solvent. The residue was purified by thin layer chromatography to afford compound AB24906 (100 mg, 27.23 % yield).

### Compound AB24906

¹H NMR (400 MHz, DMSO-d6) δ 9.54 (s, 1H), 8.30 (d, *J =* 7.0 Hz, 1H), 8.15 (d, *J =* 7.0 Hz, 1H), 7.66 (d, *J* = 7.6 Hz, 1H), 7.48 (s, 1H), 7.33 (d, *J =* 25.3 Hz, 6H), 7.00 (dd, *J =* 22.7, 6.8 Hz, 2H), 5.62 (s, 1H), 4.57 (d, *J=* 4.8 Hz, 2H), 3.76 (d, *J=* 8.3 Hz, 1H), 3.39 (s, 1H), 2.44 (s, 3H).

MS-ESI: Calculated.: [M-Br]⁺329.16, Found: 329.16.

### Example 40

### Synthesis of compound AB24949

### Step 1):

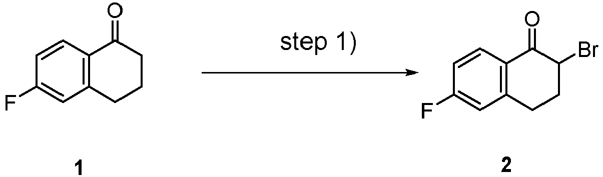

Compound 1 (200 mg, 1.22 mmol) was dissolved in acetic acid (5mL), a drop of hydrobromic acid were added, and a solution of liquid bromine (193 mg, 1.22 mmol) in acetic acid (2 mL) was injectly added. The mixture was replaced with N₂ three times, and stirred at room temperature for 3h, new spot was detected using plate. The reaction mixture was filtered, and concentrated under reduced pressure, the residue was purified by thin layer chromatography to afford compound 2 (300 mg, 101 % yield).

### Step 2:

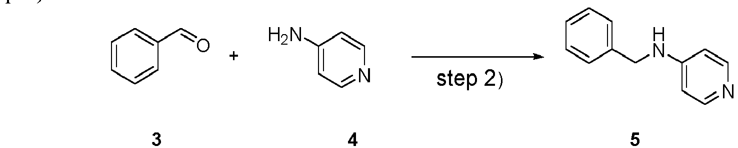

Compound 3 (5.6 g, 53.13 mmol) was dissolved in ethanol (10 mL), and compound 4 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 5 (2.6g, 26.8% yield).

### Step 3:

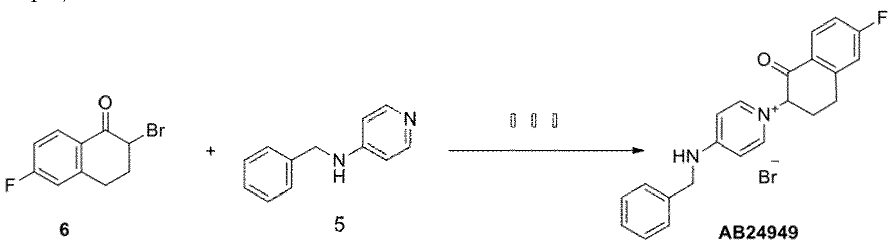

Compound 6 (300 mg, 1.24 mmol) was dissolved in acetonitrile (10 mL), and compound 5 (228 mg, 1.24 mmol) was added. The mixture was replaced with N₂ three times, and stirred at room temperature for 3 h, new spot was detected using plate. The reaction mixture was filtered and concentrated under reduced pressure to remove organic solvent. The residue was purified by thin layer chromatography (dichloromethane: methanol=10:1) to afford compound AB24949 (35 mg, 12.5% yield).

### Compound AB24949

¹H NMR (400 MHz, CD3OD) δ 8.16 - 7.98 (m, 3H), 7.35 (d, J = 21.1 Hz, 5H), 7.16 (s, 2H), 6.95 (d, J = 12.6 Hz, 2H), 4.59 (s, 2H), 3.39 (s, 1H), 3.24 - 3.19 (m, 1H), 2.76 (s, 1H), 2.59 (s, 1H).

MS-ESI: Calculated.: [M-Br]⁺347.40, Found: 347.10.

### Example 41

### Synthesis of compound AB24954

### Step 1):

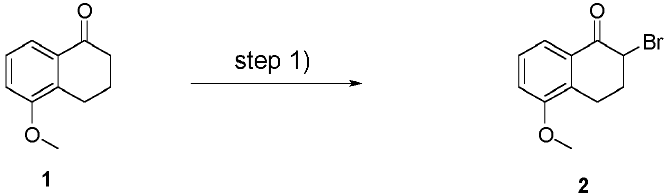

Compound 1 (176mg, 1.0 mmol) was dissolved in acetic acid (7mL), two drops of liquid bromine (160 mg, 1.0mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 2 (200 mg, 78.43% yield).

### Step 2):

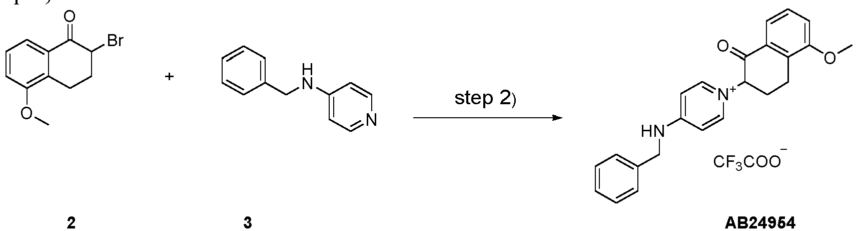

Compound 2 (200 mg, 0.78 mmol) was dissolved in acetonitrile (10 mL), and compound 3 (150 mg, 0.82 mmol) was added. The mixture was replaced with N₂ three times, and stirred at room temperature for overnight, new spot was detected using plate, solid was precipitated and filtered, and filter cake was washed with acetonitrile (2 mL) three times and dried to afford compound AB24954 (20 mg, 7.14% yield).

### Compound AB24954

¹H NMR (400 MHz, CD3OD) δ 8.11 (d, J = 7.4 Hz, 1H), 8.03 (d, J = 7.3 Hz, 1H), 7.58 (d, J = 8.0 Hz, 1H), 7.37 (s, 5H), 7.32 (s, 1H), 7.26 (d, J = 7.3 Hz, 1H), 6.98 (d, J = 7.2 Hz, 1H), 6.92 (d, J = 7.3 Hz, 1H), 4.86 - 4.84 (m, 1H), 4.59 (s, 2H), 3.90 (s, 3H), 3.40 (d, J = 18.1 Hz, 1H), 3.00 (t, J = 12.3 Hz, 1H), 2.69 (s, 1H), 2.60 (s, 1H).

MS-ESI: Calculated.: [M- CF₃COO]⁺ 359.45, Found: 359.10.

### Example 42

### Synthesis of compound AB24963

### Step 1:

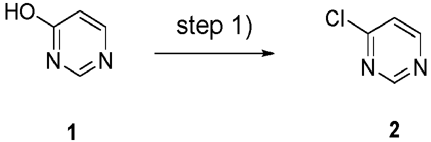

Compound 1 (8g, 83.33 mmol) was dissolved in phosphorus oxychloride (40 mL). The mixture was replaced with N₂ three times, and stirred at 90°C for overnight, new spot was detected using plate. Dichloromethane (50 mL) was added in the ice water bath, and ice water (50 mL) was slowly added. The mixture was extracted with dichloromethane (50 mL x 3). The combined organic phase was washed with saturated brine (100 mL), dried over anhydrous sodium sulfate, and concentrated under reduced pressure to remove organic solvent. The residue was purified by column chromatography (ethyl acetate: petroleum ether=30-80%) to afford compound 2 (5 g, 52.17% yield).

### Step 2):

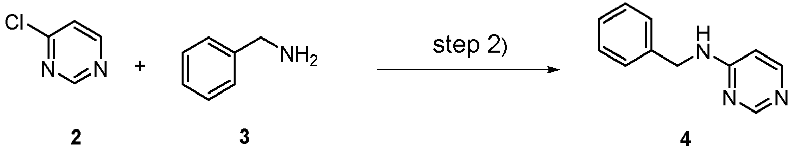

Compound 2 (2.5 g, 21.73 mmol) was dissolved in N,N-dimethylformamide, and compound 3 (7.43 g, 65.22 mmol) and potassium carbonate (9.01 g, 65.22 mmol) were added. The mixture was replaced with N₂ three times, and stirred at 80°C for overnight, new spot was detected using plate. The reaction mixture was extracted with water (50 mL) and ethyl acetate (50 mL x 3). The combined organic phase was washed with saturated brine (50 mL), dried over anhydrous sodium sulfate, and concentrated under reduced pressure to remove organic solvent. The residue was purified by column chromatography (ethyl acetate: petroleum ether=0-30%) to afford compound 4 (2.1 g, 52.24% yield).

### Step 3):

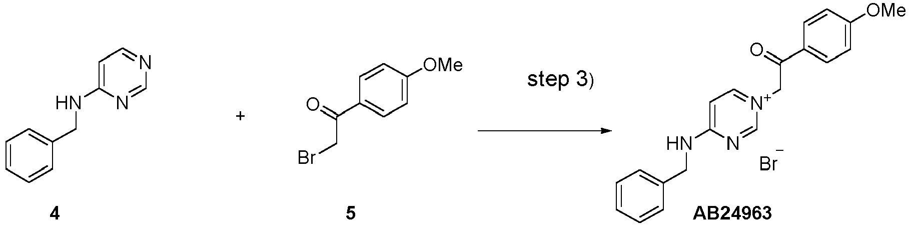

Compound 4 (200 mg, 1.08 mmol) was dissolved in acetonitrile (10 mL), and compound 5 (371 mg, 1.62 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-30%) to afford compound AB24963 (20 mg, 5.54% yield).

### Compound AB24963

¹H NMR (400 MHz, DMSO,d6) δ 10.18 (s, 1H), 8.77 (s, 1H), 8.14 (s, 1H), 7.98 (d, J = 8.2 Hz, 2H), 7.33 (d, J = 22.3 Hz, 5H), 7.13 (d, J = 8.2 Hz, 2H), 6.97 (s, 1H), 5.83 (s, 2H), 4.73 (s, 2H), 3.86 (s, 3H).

MS-ESI: Calculated.: [M-Br]⁺334.16, Found: 334.25.

### Example 43

### Synthesis of compound AB24964

### Step 1):

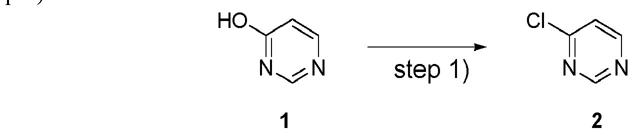

Compound 1 (8g, 83.33 mmol) was dissolved in phosphorus oxychloride (40 mL). The mixture was replaced with N₂ three times, and stirred at 90°C for overnight, new spot was detected using plate. The phosphorus oxychloride was dried, and dichloromethane (50 mL) was added in the ice water bath, and ice water (50 mL) was slowly added. The mixture was extracted with dichloromethane (50 mL x 3), the combined organic phase was washed with saturated brine (100 mL), dried over anhydrous sodium sulfate, and concentrated under reduced pressure to remove organic solvent. The residue was purified by column chromatography (ethyl acetate: petroleum ether=30-80%) to afford compound 2 (5 g, 52.17% yield).

### Step 2):

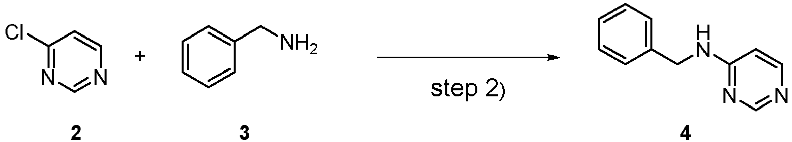

Compound 2 (2.5 g, 21.73 mmol) was dissolved in N,N-dimethylformamide (50 mL), and compound 3 (7.43 g, 65.22 mmol) and potassium carbonate (9.01 g, 65.22 mmol) were added. The mixture was replaced with N₂ three times, and stirred at 80°C for overnight, new spot was detected using plate. The reaction mixture was extracted with water (50 mL) and ethyl acetate (50 mL x 3 the combined organic phase was washed with saturated brine (50 mL), dried over anhydrous sodium sulfate, and concentrated under reduced pressure to remove organic solvent. The residue was purified by column chromatography (ethyl acetate: petroleum ether=0-30%) to afford compound 4 (2.1 g, 52.24% yield).

### Step 3):

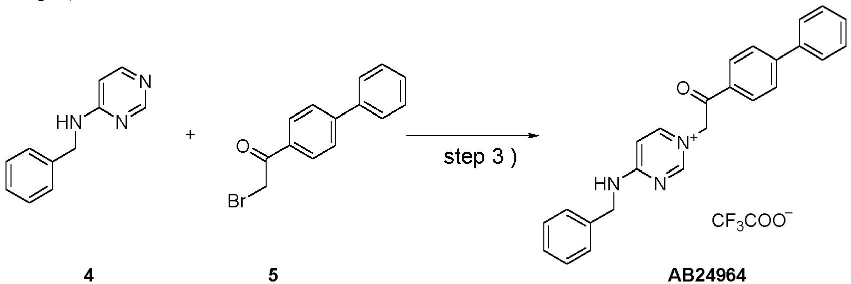

Compound 4 (200 mg, 1.08 mmol) was dissolved in acetonitrile (10 mL), and compound 5 (446 mg, 1.62 mmol) was added. The mixture was replaced with N₂ three times, and stirred at room temperature for overnight, new spot was detected using plate. The reaction mixture was dried, the residue was purified by column chromatography (methanol: dichloromethane=10%) to afford compound AB24964 (30 mg, 7.3% yield).

### Compound AB24964

¹H NMR (400 MHz, DMSO-d6) δ 10.13 (s, 1H), 8.79 (s, 1H), 8.12 (d, *J* = 25.9 Hz, 3H), 7.94 (d, *J=* 6.7 Hz, 2H), 7.77 (s, 2H), 7.51 (s, 1H), 7.44 (s, 1H), 7.35 (d, *J=* 17.5 Hz, 3H), 6.97 (s, 1H), 5.92 (s, 2H), 4.74 (s, 1H).

MS-ESI: Calculated.: [M-CF₃COO]⁺380.46, Found: 380.30.

### Example 44

### Synthesis of compound AB24967

### Step 1:

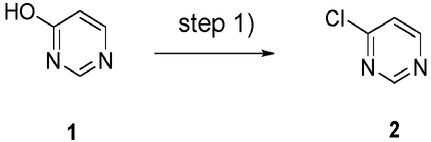

Compound 1 (8g, 83.33 mmol) was dissolved in phosphorus oxychloride (40 mL). The mixture was replaced with N₂ three times, and stirred at 90°C for overnight, new spot was detected using plate, the phosphorus oxychloride was dried. Dichloromethane (50 mL) was added in the ice water bath, and ice water (50 mL) was slowly added. The mixture was extracted with dichloromethane (50 mL x 3), the combined organic phase was washed with saturated brine (100 mL), dried over anhydrous sodium sulfate, and concentrated under reduced pressure to remove organic solvent. The residue was purified by column chromatography (ethyl acetate: petroleum ether=30-80%) to afford compound 2 (5 g, 52.17% yield).

### Step 2):

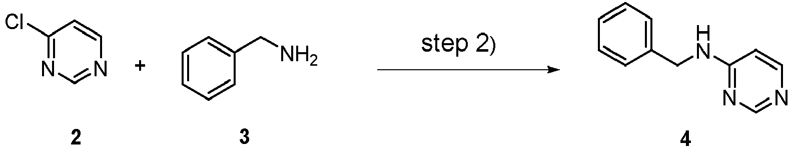

Compound 2 (2.5 g, 21.73 mmol) was dissolved in N,N-dimethylformamide (50ml), and compound 3 (7.43 g, 65.22 mmol) and potassim carbonate (9.01 g, 65.22 mmol) were added. The mixture was replaced with N₂ three times, and stirred at 80°C for overnight, new spot was detected using plate. The reaction mixture was extracted with water (50 mL) and ethyl acetate (50 mL x 3), the combined organic phase was washed with saturated brine (50 mL), dried over anhydrous sodium sulfate, and concentrated under reduced pressure to remove organic solvent. The residue was purified by column chromatography (ethyl acetate: petroleum ether=0-30%) to afford compound 4 (2.1 g, 52.24% yield).

### Step 3:

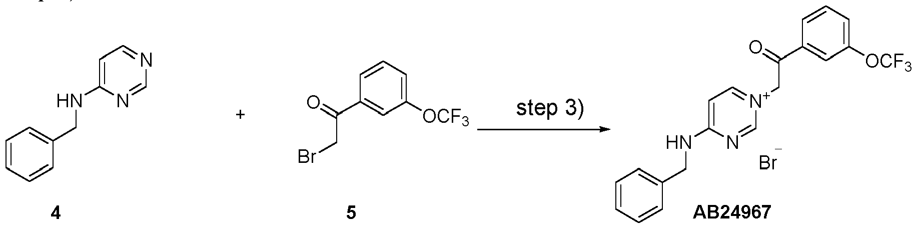

Compound 4 (200 mg, 1.08 mmol) was dissolved in acetonitrile (15 mL), and compound 5 (367 mg, 1.29 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-30%) to afford compound AB24967 (30 mg, 7.16 % yield).

### Compound AB24967

¹H NMR (400 MHz, DMSO,d6) δ 10.29 (s, 1H), 8.75 (s, 1H), 8.14 (s, 1H), 8.05 (s, 1H), 7.92 (s, 1H), 7.78 (s, 2H), 7.33 (d, *J=* 23.1 Hz, 6H), 6.99 (d, *J=* 6.7 Hz, 1H), 5.90 (s, 2H), 4.73 (s, 2H).

MS-ESI: Calculated.: [M-Br]⁺388.13, Found: 388.25.

### Example 45

### Synthesis of compound AB24991

### Step 1:

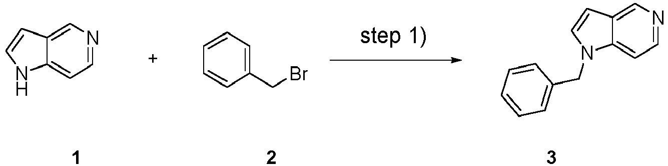

Compound 1 (200 mg, 1.69 mmol) was dissolved in N,N-dimethylformamide (20 mL), NaH (203 mg, 5.07 mmol) was added in ice bath. The mixture was stirred at 0°C for 30 min, compound 2 (318 mg, 1.86 mmol) was added, then the mixture was stirred at room temperature for 3 h, new spot was detected using plate. The reaction mixture was diluted with water (30 ml) and extracted with ethyl acetate ((30 ml x 3). The combined organic phase was washed with saturated brine (50 mL), dried over anhydrous sodium sulfate, and concentrated under reduced pressure to remove organic solvent. The residue was purified by column chromatography (methanol: dichloromethane=0-10%) to afford compound 3 (200 mg, 56.9 % yield).

### Step 2):

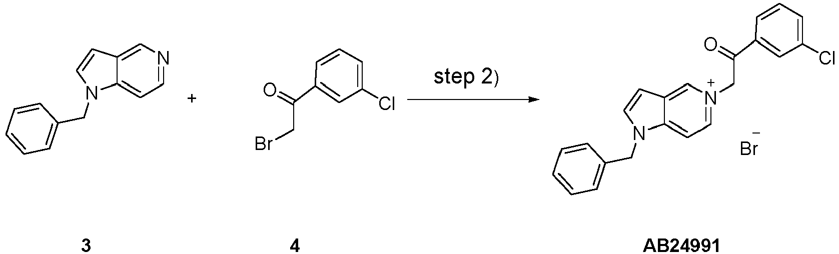

Compound 3 (200 mg, 0.96 mmol) was dissolved in acetonitrile (10 mL), and compound 4 (337 mg, 1.44 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-10%) to afford compound AB24991 (100 mg, 28.86 % yield).

### Compound AB24991

¹H NMR (400 MHz, DMSO,d6) δ 9.26 (s, 1H), 8.41 (d, *J =* 42.8 Hz, 2H), 8.06 (dd, *J =* 48.7, 20.6 Hz, 3H), 7.85 (d, *J=* 6.8 Hz, 1H), 7.69 (d, *J=* 7.0 Hz, 1H), 7.27 (d, *J=* 60.4 Hz, 6H), 6.40 (s, 2H), 5.67 (s, 2H).

MS-ESI: Calculated.: [M-Br]⁺ 361.11, Found: 361.05.

### Example 46

### Synthesis of compound AB27106

### Step:

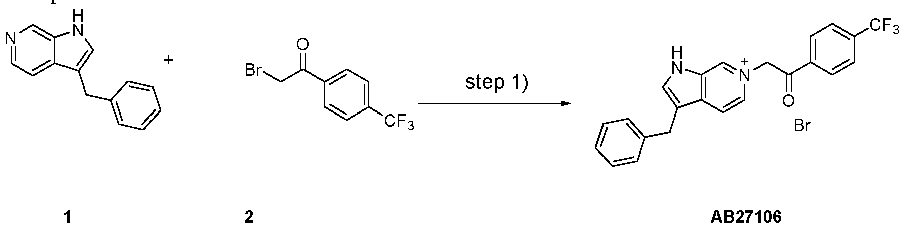

Compound 1 (80 mg, 0.38 mmol) and compound 2 (103 mg, 0.385 mmol) were dissolved in acetonitrile (5 mL). The mixture was stirred at room temperature for 3 h, new spot was detected using plate, solid was precipitated and filtered, and filter cake was washed with acetonitrile (2 ml) three times and dried to afford compound AB27106 (57mg, 37.97% yield),

### Compound AB27106

₁H NMR (400 MHz, DMSO-d6) δ 13.53 (s, 1H), 9.29 (s, 1H), 8.33 - 8.25 (m, 4H), 8.14 (d, J = 6.7 Hz, 1H), 8.05 (d, J = 8.3 Hz, 2H), 7.38 - 7.28 (m, 4H), 7.21 (dd, J = 7.2, 4.0, 1.4 Hz, 1H), 6.53 (s, 2H), 4.22 (s, 2H).

MS-ESI: Calculated.: [M-Br]⁺ 395.40, Found: 395.05.

### Example 47

### Synthesis of compound AB27107

### Step:

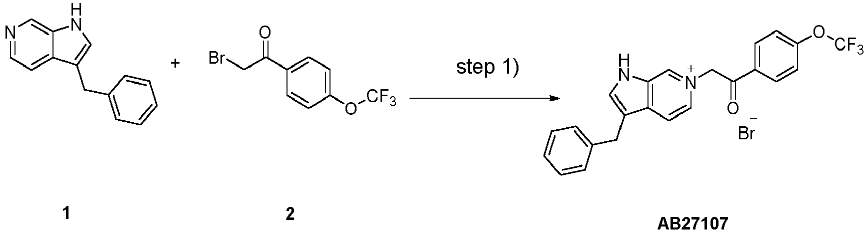

Compound 1 (100 mg, 0.50 mmol) and compound 2 (141 mg, 0.5 mmol) were dissolved in acetonitrile (5 mL). The mixture was stirred at room temperature for 3 h, new spot was detected using plate, solid was precipitated and filtered, and filter cake was washed with acetonitrile (2 ml) three times and dried to afford compound AB27107 (65 mg, 31.55 % yield),

### Compound AB27107

¹H NMR (400 MHz, DMSO-d6) δ 13.10 (s, 1H), 9.24 (s, 1H), 8.41 - 8.12 (m, 5H), 7.67 (d, J = 8.3 Hz, 2H), 7.40 - 7.27 (m, 4H), 7.21 (t, J = 7.1 Hz, 1H), 6.48 (s, 2H), 4.22 (s, 2H).

MS-ESI: Calculated.: [M-Br]⁺ 411.40, Found: 411.05.

### Example 48

### Synthesis of compound AB27114

### Step 1):

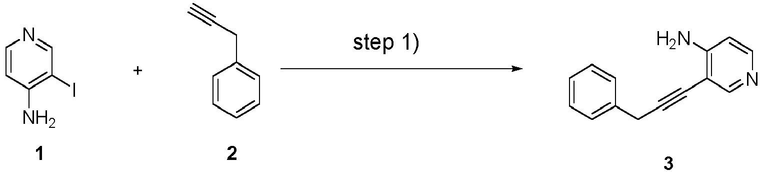

Compound 1 (4.4 g, 20 mmol) was dissolved in dry N,N-dimethylformamide (20 mL), and compound 2 (4.28 g, 42 mmol), copper(I) iodide (0.19 g, 1 mmol), N, N-diisopropylethylamine (8.24 g, 64 mmol) and bis(triphenylphosphine)palladium(II) chloride (190 mg, 0.6 mmol) were added successively. The mixture was replaced with N₂ three times, and stirred at room temperature for 16 h, new spot was detected using plate. The reaction mixture was diluted with water (100 ml) and extracted with ethyl acetate (100 mL x 3). The combined organic phase was washed with saturated brine (100 mL), dried over anhydrous sodium sulfate, filtered, and concentrated under reduced pressure to remove organic solvent. The residue was purified by column chromatography (ethyl acetate: petroleum ether=2:1) to afford compound 3 (3.5 g, 84.13% yield).

### Step 2:

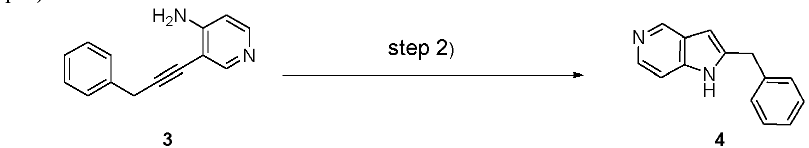

Compound 3 (1.5 g, 7.2 mmol) was dissolved in 1,4-dioxane (4 mL), and potassium tert-butoxide (2.4 mg, 21.6 mmol) was added. The mixture was replaced with N₂ three times, and stirred at 100°C for overnight, new spot was detected using plate. The reaction mixture was concentrated under reduced pressure to remove organic solvent. The residue was purified by thin layer chromatography (methanol: dichloromethane=10:1) to afford compound 4 (1.1g, 73.45% yield).

### Step 3):

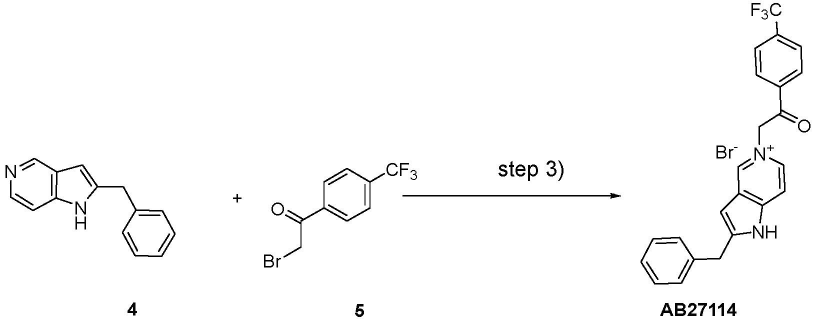

Compound 4 (104 mg, 0.5 mmol) was dissolved in acetonitrile (10 mL), and compound 5 (160 mg, 0.6 mmol) was added. The mixture was replaced with N₂ three times, and stirred at room temperature for overnight, new spot was detected using plate. The reaction mixture was concentrated under reduced pressure to remove organic solvent. The residue was purified by thin layer chromatography (dichloromethane: methanol=10:1) to afford compound AB27114 (141 mg, 59.49% yield).

### Compound AB27114

¹HNMR (400 MHz, DMSO-d6) δ 13.04 (s, 1H), 9.11 (s, 1H), 8.35 (d, J= 6.0 Hz, 1H), 8.24 (d, J = 7.2 Hz, 2H), 8.03 (d, J = 7.2 Hz, 2H), 7.96 (d, J = 6.2 Hz, 1H), 7.33 (s, 2H), 7.26 (s, 1H), 6.82 (s, 1H), 6.41 (s, 2H), 4.24 (s, 2H).

MS-ESI: Calculated.: [M-Br]⁺ 395.14, Found: 395.14.

### Example 49

### Synthesis of compound AB27124

### Step 1:

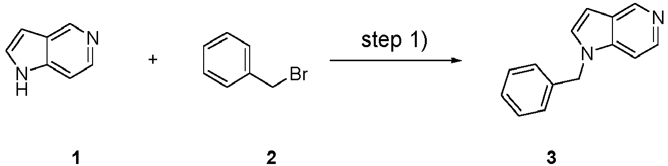

Compound 1 (200 mg, 1.69 mmol) was dissolved in dry N,N-dimethylformamide (20 mL), NaH (203 mg, 5.07 mmol) was added in ice bath. The mixture was stirred at 0 °C for 30 min, compound 2 (318 mg, 1.86 mmol) was slowly added, then the mixture was stirred at room temperature for 3 h, new spot was detected using plate. The reaction mixture was diluted with water (30 ml) and extracted with ethyl acetate (30 ml x 3). The combined organic phase was washed with saturated brine (50 ml), dried over anhydrous sodium sulfate, and concentrated under reduced pressure to remove organic solvent. The residue was purified by column chromatography (methanol: dichloromethane=0-10%) to afford compound 3 (200 mg, 56.9% yield).

### Step 2):

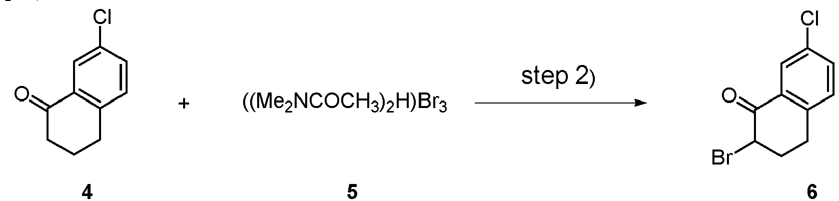

Compound 4 (200 mg, 1.11 mmol) was dissolved in dichloromethane (10 mL), and compound 5 (456 mg, 1.11 mmol) was added. The mixture was stirred at room temperature for 1 h, new spot was detected using plate. The reaction mixture was dried, the residue was purified by thin layer chromatography to afford compound 6 (250 mg, 87.29% yield)

### Step 3:

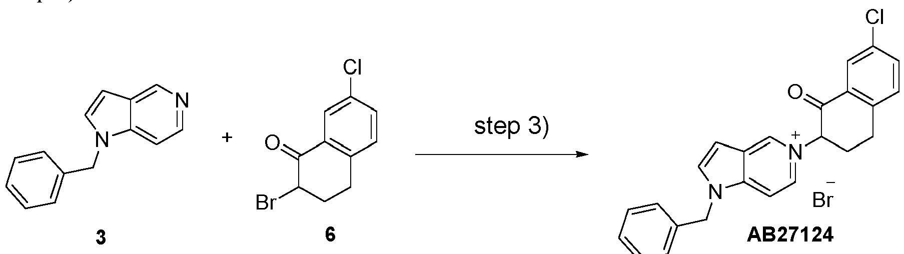

Compound 3 (150 mg, 0.58 mmol) was dissolved in acetonitrile (10 mL), and compound 6 (100 mg, 0.48 mmol) was added. The mixture was replaced with N₂ three times, and stirred at room temperature for overnight, new spot was detected using plate. The reaction mixture was dried, the residue was purified by thin layer chromatography to afford compound AB27124 (65 mg, 34.99% yield).

### Compound AB27124

¹H NMR (400 MHz, DMSO,d6) δ 9.45 (s, 1H), 8.62 (s, 1H), 8.40 (d, *J=* 6.8 Hz, 1H), 8.21 (d, *J =* 2.5 Hz, 1H), 7.88 (d, *J =* 2.0 Hz, 1H), 7.79 (dd, *J =* 8.3, 2.3 Hz, 1H), 7.58 (d, *J =* 8.3 Hz, 1H), 7.42 - 7.31 (m, 5H), 7.19 (d, *J =* 3.3 Hz, 1H), 5.71 (s, 2H), 3.30 (s, 3H), 3.04 (dd, *J=* 13.2, 4.6 Hz, 1H), 2.69 (d, *J =* 8.9 Hz, 1H).

MS-ESI: Calculated.: [M-Br]⁺387.13, Found: 387.20.

### Example 50

### Synthesis of compound AB27127

### Step 1:

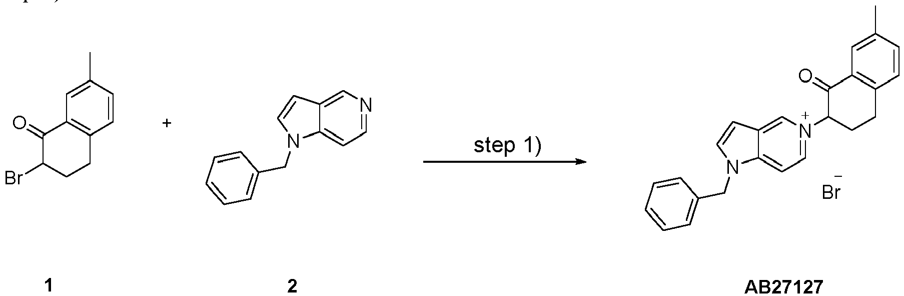

Compound 1 (100 mg, 0.42 mmol) was dissolved in acetonitrile (10 mL), and compound 2 (77.73 mg, 0.42 mmol) was added. The mixture was replaced with N₂ three times, and stirred at room temperature for overnight, new spot was detected using plate. The reaction mixture was dried, the residue was purified by thin layer chromatography to afford compound AB27127 (15 mg, 9.73% yield).

### Compound AB27127

¹H NMR (400 MHz, DMSO-d6) δ 9.50 (s, 1H), 8.65 (s, 1H), 8.40 (d, J = 6.5 Hz, 1H), 8.20 (s, 1H), 7.72 (s, 1H), 7.49 (s, 1H), 7.41 - 7.26 (m, 6H), 7.14 (s, 1H), 6.36 (d, J = 11.1 Hz, 1H), 5.70 (s, 2H), 3.31 (s, 1H), 3.21 - 3.06 (m, 1H), 2.95 (t, J = 30.6 Hz, 1H), 2.65 (s, 1H), 2.33 (s, 3H).

MS-ESI: Calculated.: [M-Br]⁺367.47, Found: 367.25.

### Example 51

### Synthesis of compound AB24924

### Step 1):

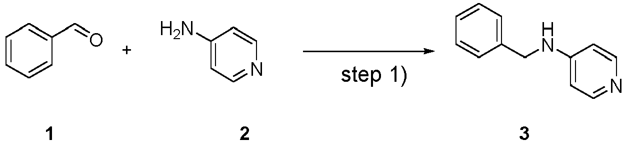

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

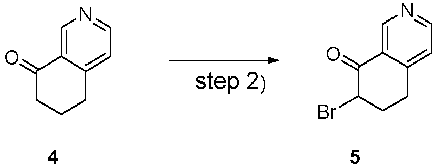

Compound 4 (400mg, 2.1 mmol) was dissolved in acetic acid (10 mL), two drops of liquid bromine (348 mg, 2.17 mmol) and hydrobromic acid aqueous solution were added. The mixture was replaced with N₂ three times, and stirred at room temperature for 3 h, new spot was detected using plate, solid was precipitated and filtered, and filter cake was washed with dichloromethane (5ml), the organic solvent in filter cake was removed to afford compound 5 (400 mg, 81.3% yield).

### Step 3:

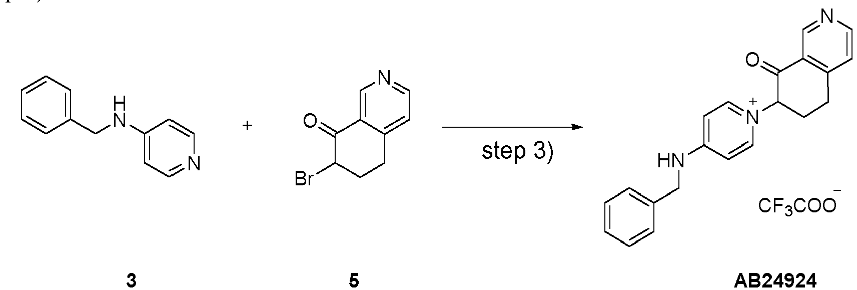

Compound 5 (300 mg, 1.16 mmol) and compound 3 (107 mg, 0.58 mmol) were dissolved in acetonitrile (10 mL). The mixture was stirred at room temperature for 3 h, new spot was detected using plate. The reaction mixture was concentrated under reduced pressure to remove organic solvent, the residue was purified by column chromatography (methanol: dichloromethane=10%) to afford compound AB24924 (15 mg, 3.9% yield).

### Compound AB24924

¹H NMR (400 MHz, CD3OD) δ 8.96 (s, 1H), 8.57 (d, *J =* 5.3 Hz, 1H), 8.05 (dd, *J =* 7.5, 1.9 Hz, 1H), 7.97 (dd, *J=* 7.4, 1.9 Hz, 1H), 7.41 (d, *J=* 5.3 Hz, 1H), 7.29 (t, *J=* 4.6 Hz, 4H), 7.27 - 7.20 (m, 1H), 6.92 (dd, *J =* 7.4, 2.9 Hz, 1H), 6.86 (dd, *J =* 7.5, 2.9 Hz, 1H), 4.52 (s, 2H), 3.35 (s, 1H), 3.24 - 3.21 (m, 1H), 3.20 - 3.17 (m, 1H), 2.72 (d, *J* = 4.5 Hz, 1H), 2.56 - 2.48 (m, 1H).

MS-ESI: Calculated.: [M-CF₃COO]⁺330.41, Found: 330.30.

### Example 52

### Synthesis of compound AB24996

### Step 1:

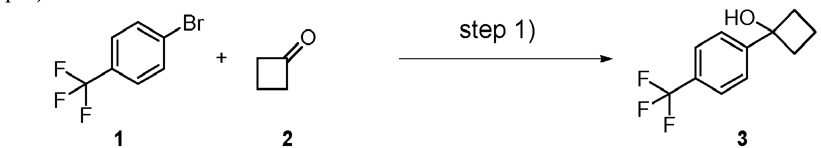

Compound 1 (22 g, 100 mmol) was dissolved in tetrahydrofuran (200 mL), compound 2 (10.5 g, 150 mmol) was added, n-butyllithium (80 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=50:1) to afford compound 3 (12 g, 55.2% yield).

### Step 2):

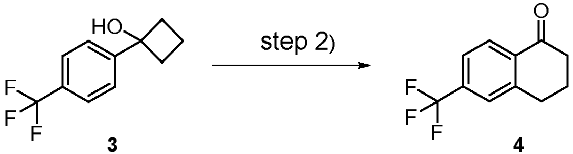

Compound 3 (12 g, 55.6 mmol) was dissolved in dichloromethane (100 mL) and water (100 mL), and AgNO₃ (1.89 g, 11.12 mmol) and potassium persulfate (37 g, 139 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 4 (3.1 g, 26.05 % yield).

### Step 3):

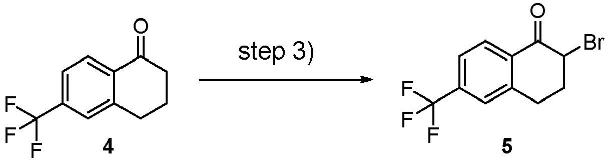

Compound 4 (1.5 mg, 6.9 mmol) was dissolved in dichloromethane (20 mL), and C₈H₁₈N₂O₂HBr₃ (2.9 g, 6.9 mmol) was added. The mixture was stirred at room temperature for 1 h, new spot was detected using plate. The reaction mixture was dried, the residue was purified by thin layer chromatography to afford compound 5 (2.2g, 85.48% yield)

### Step 4):

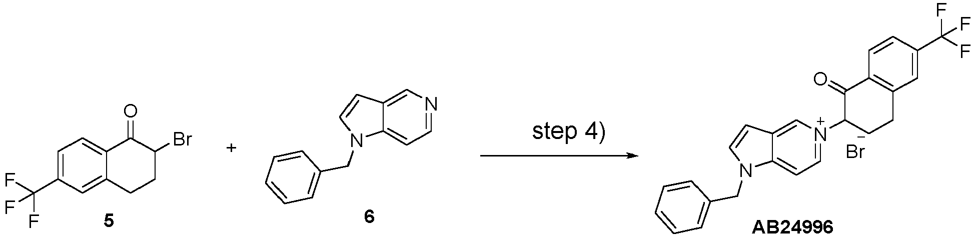

Compound 5 (150 mg, 0.5 mmol) was dissolved in acetonitrile (5 mL), and compound 6 (104 mg, 0.5 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB24996 (40 mg, 16.0% yield).

### Compound AB24996

¹H NMR (400 MHz, DMSO-d6) δ 9.38 (s, 1H), 8.56 (d, J = 6.7 Hz, 1H), 8.36 (d, J = 6.9 Hz, 1H), 8.17 (s, 1H), 8.11 (d, J = 8.0 Hz, 1H), 7.94 (s, 1H), 7.80 (d, J = 8.1 Hz, 1H), 7.35 (s, 5H), 7.17 (s, 1H), 6.20 (d, J = 9.8 Hz, 1H), 5.67 (s, 2H), 3.40 (s, 3H), 3.06 (s, 1H), 2.68 (s, 1H).

MS-ESI: Calculated.: [M-Br]⁺ 421.15, Found: 421.15.

### Example 53

### Synthesis of compound AB27111

### Step 1:

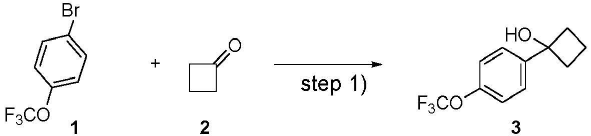

Compound 1 (36.3 g, 150 mmol) was dissolved in tetrahydrofuran (120 mL), compound 2 (12.6 g, 180 mmol) was added, n-butyllithium (120 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 3 (6.72 g, 18.66% yield).

### Step 2:

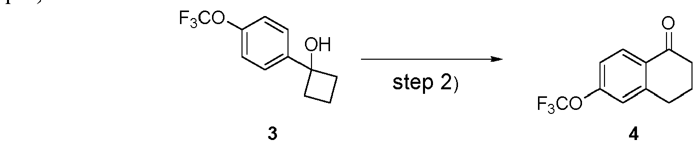

Compound 3 (6.72 g, 28 mmol) was dissolved in dichloromethane (200 mL) and water (200 mL), and AgNO₃ (952 mg, 5.6 mmol) and potassium persulfate (18.9g, 70 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 4 (2.35 g, 36.33 % yield).

### Step 3:

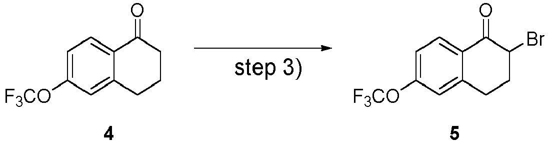

Compound 4 (2.0 g, 8.69 mmol) was dissolved in dichloromethane (50 mL), and bis(N,N-dimethylacetamide) hydrogen dibromobromate (3.65 g, 8.90 mmol) was added. The mixture was stirred at room temperature for 1 h, new spot was detected using plate. The reaction mixture was dried, the residue was purified by thin layer chromatography (petroleum ether: ethyl acetate=0-10%) to afford compound 5 (2.5g, 93.10% yield)

### Step 4):

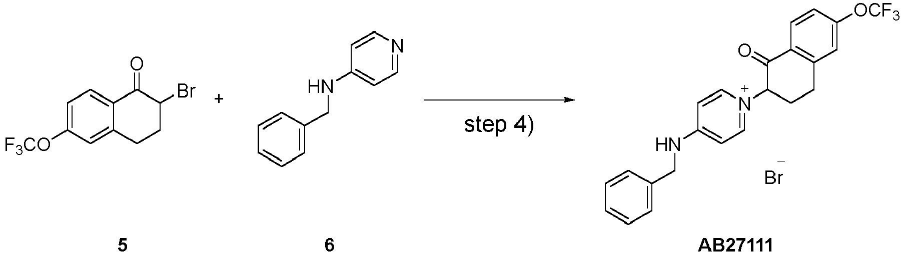

Compound 5 (240 mg, 0.78 mmol) was dissolved in acetonitrile (10 mL), and compound 6 (150 mg, 0.78 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27111 (88 mg, 27.31% yield).

### Compound AB27111

¹H NMR (400 MHz, DMSO-d6) δ 9.72 (s, 1H), 8.27 (d, *J =* 6.8 Hz, 1H), 8.17 (d, *J =* 6.7 Hz, 1H), 8.02 (d, J= 8.5 Hz, 1H), 7.48 (s, 1H), 7.40 (d, *J=* 11.9 Hz, 5H), 7.29 (s, 1H), 7.11 (d, J= 5.7 Hz, 1H), 6.99 (d, *J=* 5.6 Hz, 1H), 5.81 (d, *J=* 10.7 Hz, 1H), 4.57 (d, *J=* 4.9 Hz, 2H), 3.25 (s, 2H), 3.21 (s, 1H), 2.81 (d, *J=* 9.3 Hz, 1H).

MS-ESI: Calculated.: [M-Br]⁺ 413.42, Found: 413.20.

### Example 54

### Synthesis of compound AB27112

### Step 1):

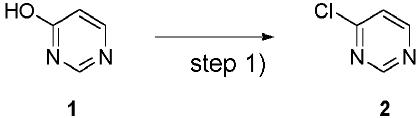

Compound 1 (8g, 83.33 mmol) was dissolved in phosphorus oxychloride (40 mL). The mixture was replaced with N₂ three times, and stirred at 90°C for overnight, new spot was detected using plate. Phosphorus oxychloride was dried, dichloromethane (50 mL) was added in the ice water bath, and ice water (50 mL) was slowly added. The mixture was extracted with dichloromethane (50 mL x 3), the combined organic phase was washed with saturated brine (100 mL), dried over anhydrous sodium sulfate, and concentrated under reduced pressure to remove organic solvent. The residue was purified by column chromatography (ethyl acetate: petroleum ether=30-80%) to afford compound 2 (5 g, 52.17% yield).

### Step 2):

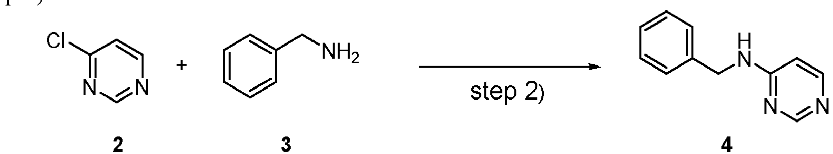

Compound 2 (2.5 g, 21.73 mmol) was dissolved in N,N-dimethylformamide (50ml), and compound 3 (7.43 g, 65.22 mmol) and potassium carbonate (9.01 g, 65.22 mmol) were added. The mixture was replaced with N₂ three times, and stirred at 80°C for overnight, new spot was detected using plate. The reaction mixture was extracted with water (50 mL) and ethyl acetate (50 mL x 3), the combined organic phase was washed with saturated brine (50 mL), dried over anhydrous sodium sulfate, and concentrated under reduced pressure to remove organic solvent. The residue was purified by column chromatography (ethyl acetate: petroleum ether=0-30%) to afford compound 4 (2.1 g, 52.24% yield).

### Step 3):

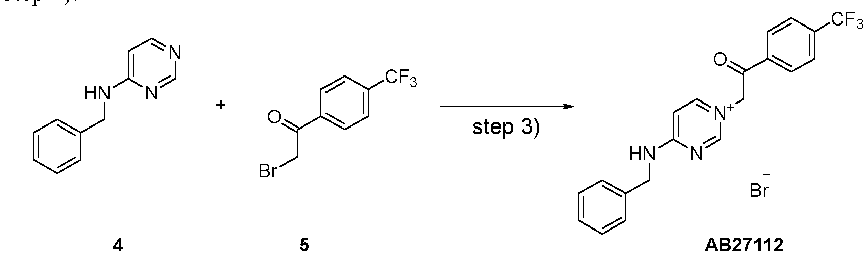

Compound 4 (100 mg, 0.54 mmol) was dissolved in acetonitrile (10 mL), and compound 5 (173 mg, 0.65 mmol) was added. The mixture was stirred at room temperature for 16 h, new spot was detected using plate. The reaction mixture was concentrated under reduced pressure to remove organic solvent, the residue was purified by column chromatography (methanol: dichloromethane=0-10%) to afford compound AB27112 (40 mg, 19.9% yield).

### Compound AB27112

¹H NMR (400 MHz, DMSO-d6) δ 10.47 (s, 1H), 8.79 (s, 1H), 8.20 (s, 3H), 8.01 (d, *J=* 6.7 Hz, 2H), 7.37 (s, 5H), 7.06 (d, *J=* 6.5 Hz, 1H), 5.97 (s, 2H), 4.73 (s, 2H).

MS-ESI: Calculated.: [M-Br]⁺372.36, Found: 372.10.

### Example 55

### Synthesis of compound AB27125

### Step 1:

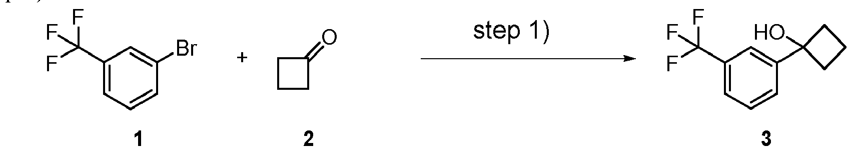

Compound 1 (22 g, 100 mmol) was dissolved in tetrahydrofuran (200 mL), compound 2 (10.5 g, 150 mmol) was added, n-butyllithium (80 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=50:1) to afford compound 3 (13 g, 60.3% yield).

### Step 2):

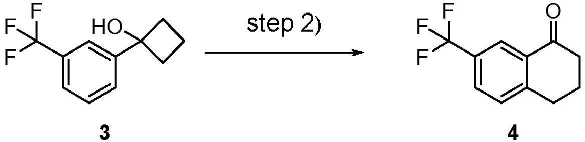

Compound 3 (6.48 g, 30 mmol) was dissolved in dichloromethane (100 mL) and water (100 mL), and AgNO₃ (1 g, 6 mmol) and potassium persulfate (20 g, 270 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 4 (584 mg, 9.0 % yield).

### Step 3):

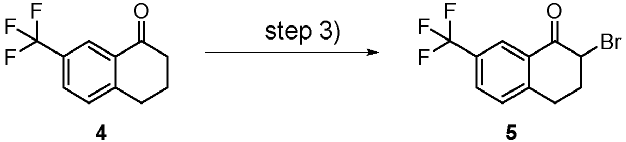

Compound 4 (584 mg, 1.98 mmol) was dissolved in dichloromethane (20 mL), and C₈H₁₈N₂O₂HBr₃ (820 mg, 1.98 mmol) was added. The mixture was stirred at room temperature for 1 h, new spot was detected using plate. The reaction mixture was dried, the residue was purified by thin layer chromatography to afford compound 5 (670 mg, 94.7% yield)

### Step 4):

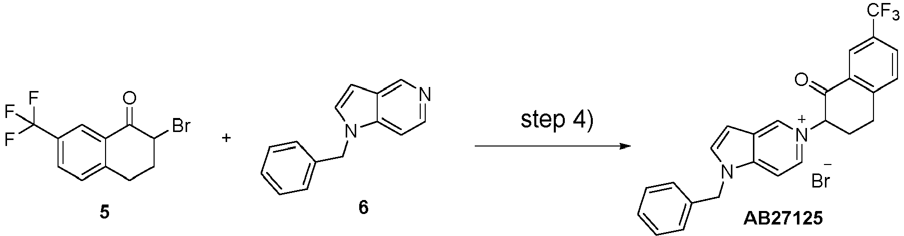

Compound 5 (200 mg, 0.65 mmol) was dissolved in acetonitrile (5 mL), and compound 6 (100 mg, 0.65 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27125 (53 mg, 16.3% yield).

### Compound AB27125

¹H NMR (400 MHz, DMSO-d6) δ 9.40 (s, 1H), 8.58 (d, *J=* 6.0 Hz, 1H), 8.37 (d, *J=* 6.2 Hz, 1H), 8.15 (d, *J =* 17.0 Hz, 2H), 8.06 (d, *J =* 7.5 Hz, 1H), 7.76 (d, *J =* 7.5 Hz, 1H), 7.35 (s, 5H), 7.17 (s, 1H), 6.24 (d, *J =* 12.0 Hz, 1H), 5.68 (s, 2H), 3.39 (s, 2H).

MS-ESI: Calculated.: [M-Br]⁺ 421.15, Found: 421.15

### Example 56

### Synthesis of compound AB27126

### Step 1):

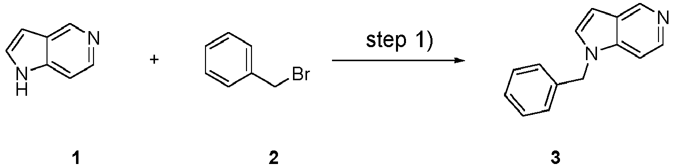

Compound 1 (200 mg, 1.69 mmol) was dissolved in dry N,N-dimethylformamide (20 mL), NaH (203 mg, 5.07 mmol) was added in ice bath. The mixture was stirred at 0°C for 30 min, and compound 2 (318 mg, 1.86 mmol) was slowly added, then the mixture was stirred at room temperature for 3 h, new spot was detected using plate. The reaction mixture was diluted with water (30 ml) and extracted with ethyl acetate (30 ml x 3). The combined organic phase was washed with saturated brine (50 ml), dried over anhydrous sodium sulfate, and concentrated under reduced pressure to remove organic solvent. The residue was purified by column chromatography (methanol: dichloromethane=0-10%) to afford compound 3 (200 mg, 56.9% yield).

### Step 2:

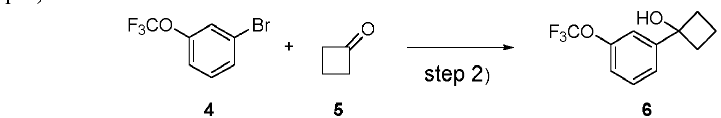

Compound 4 (30 g, 124 mmol) was dissolved in tetrahydrofuran (200 mL), and compound 5 (10.46 g, 149 mmol) was added. The mixture was replaced with N₂ three times, n-butyllithium (100 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 6 (11.2 g, 38.93% yield).

### Step 3:

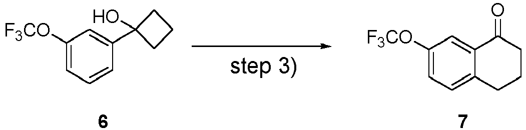

Compound 6 (11.2 g, 48 mmol) was dissolved in dichloromethane (100 mL) and water (100 mL), and AgNO₃ (1.65 g, 9.7 mmol) and potassium persulfate (32.6 g, 121 mmol) were added. The mixture was stirred at room temperature for 16 h, new spot was detected using plate. The reaction mixture was concentrated under reduced pressure to remove water and organic solvent, the residue was purified by column chromatography (petroleum ether: ethyl acetate=0-10%) to afford compound 7 (930 mg, 8.42 % yield).

### Step 4):

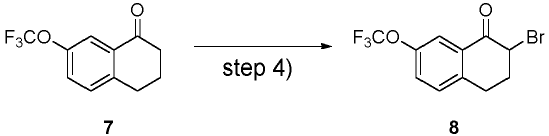

Compound 7 (930 mg, 4.04 mmol) was dissolved in dichloromethane (20 mL), and bis(N,N-dimethylacetamide) hydrogen dibromobromate (1.68 g, 4.04 mmol) was added. The mixture was stirred at room temperature for 1 h, new spot was detected using plate. The reaction mixture was dried, the residue was purified by column chromatography (petroleum ether: ethyl acetate=0-10%) to afford compound 8 (800 mg, 64.50% yield).

### Step 5):

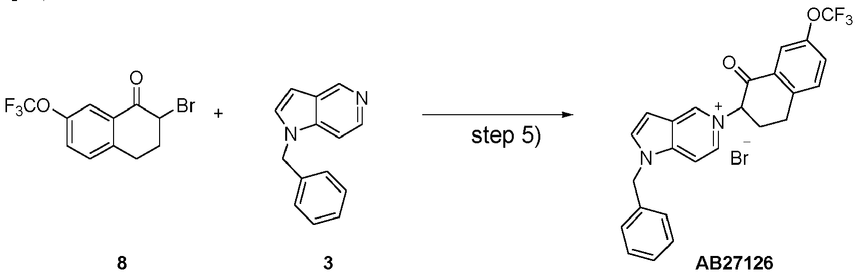

Compound 8 (200 mg, 0.65 mmol) was dissolved in acetonitrile (5 mL), and compound 3 (100 mg, 0.65 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27126 (20 mg, 7.04% yield).

### Compound AB27126

¹H NMR (400 MHz, DMSO-d6) δ 9.41 (s, 1H), 8.58 (s, 1H), 8.37 (d, *J=* 5.9 Hz, 1H), 8.18 (s, 1H), 7.70 (dd, *J=* 33.1, 12.2 Hz, 3H), 7.35 (s, 5H), 7.16 (s, 1H), 6.25 (s, 1H), 5.68 (s, 2H), 3.48 (s, 2H), 3.04 (d, *J =* 9.3 Hz, 1H), 2.65 (s, 1H).

MS-ESI: Calculated.: [M-Br]⁺437.15, Found: 437.20.

### Example 57

### Synthesis of compound AB27131

### Step 1:

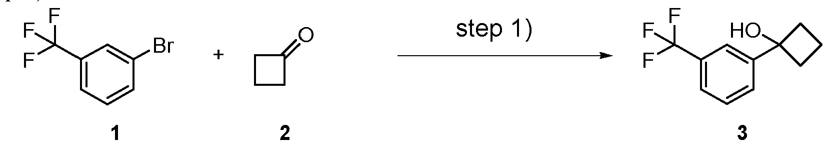

Compound 1 (22 g, 100 mmol) was dissolved in tetrahydrofuran (200 mL), and compound 2 (10.5 g, 150 mmol) was added, then n-butyllithium (80 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=50:1) to afford compound 3 (13 g, 60.1% yield).

### Step 2):

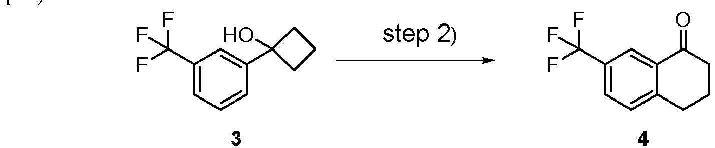

Compound 3 (6.48 g, 30 mmol) was dissolved in dichloromethane (100 mL) and water (100 mL), and AgNO3 (1 g, 6 mmol) and potassium persulfate (20 g, 270 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 4 (584 mg, 9.0 % yield).

### Step 3:

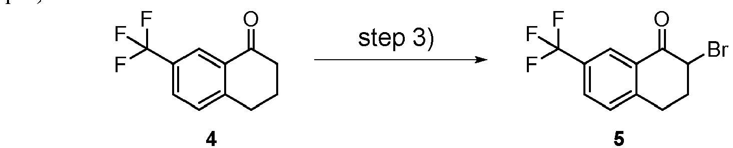

Compound 4 (584 mg, 1.98 mmol) was dissolved in dichloromethane (20 mL), and C₈H₁₈N₂O₂HBr₃ (820 mg, 1.98 mmol) was added. The mixture was stirred at room temperature for 1 h, new spot was detected using plate. The reaction mixture was dried, the residue was purified by thin layer chromatography to afford compound 5 (670 mg, 94.7 % yield)

### Step 4):

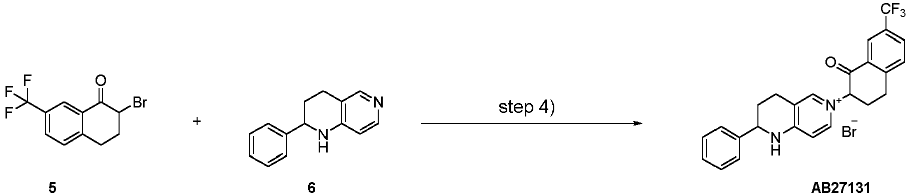

Compound 5 (150 mg, 0.5 mmol) and compound 6 (105 mg, 0.5 mmol) were dissolved in acetonitrile (10 mL). The mixture was stirred at room temperature for 3 h new spot was detected using plate. The reaction mixture was concentrated under reduced pressure to remove organic solvent, the residue was purified by thin layer chromatography (methanol: dichloromethane=10%) to afford compound AB27131 (30 mg, 11.9% yield).

### Compound AB27131

¹H NMR (400 MHz, DMSO-d6)δ 9.29 (s, 1H), 8.15 (d, *J* = 14.3 Hz, 2H), 8.05 (dd, *J* = 14.3, 7.1 Hz, 2H), 7.73 (d, *J* = 7.4 Hz, 1H), 7.45 - 7.30 (m, 5H), 6.90 (d, *J* = 6.3 Hz, 1H), 5.70 (d, *J=* 12.1 Hz, 1H), 4.77 (s, 1H), 2.81 (s, 2H), 2.59 (s, 4H), 2.10 (s, 1H), 1.88 (s, 1H).

MS-ESI: Calculated.: [M-Br]⁺ 423.17, Found: 423.17.

### Example 58

### Synthesis of compound AB27133

### Step 1:

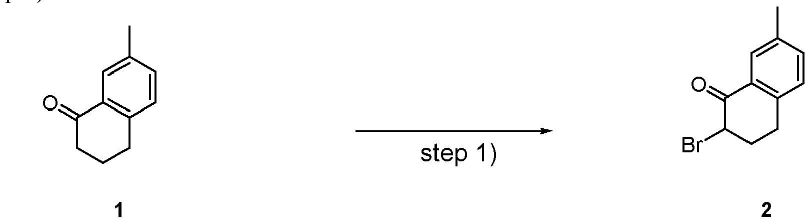

Compound 1 (160 mg, 1 mmol) was dissolved in dichloromethane (50 mL), and bis(N,N-dimethylacetamide) hydrogen dibromobromate (414 mg, 1 mmol) was added. The mixture was stirred at room temperature for 1 h, new spot was detected using plate. The reaction mixture was dried, and the residue was purified by thin layer chromatography (petroleum ether: ethyl acetate=0-10%) to afford compound 2 (240 mg, 100 % yield).

### Step 2):

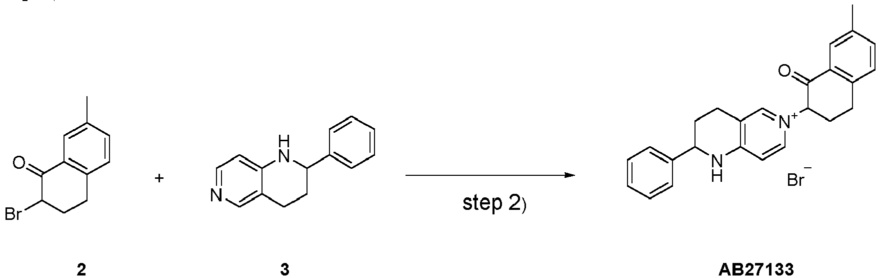

Compound 2 (240 mg, 1.0 mmol) were dissolved in acetonitrile (10 mL), and compound 3 (210 mg, 1.0mmol) was added. The mixture was replaced with N₂ three times, and stirred at room temperature for overnight, new spot was detected using plate.The reaction mixture was concentrated under reduced pressure to remove organic solvent. The residue was purified by column chromatography (methanol: dichloromethane=0-30%) to afford compound AB21733 (70.0 mg, 18.97% yield).

### Compound AB21733

¹H NMR (400 MHz, DMSO-d6) δ 9.47 (s, 1H), 8.20 (s, 1H), 8.12 (d, *J=* 6.4 Hz, 1H), 7.71 (s, 1H), 7.48 (d, *J* = 7.2 Hz, 1H), 7.39 (d, *J* = 6.3 Hz, 2H), 7.34 (d, *J* = 6.5 Hz, 4H), 6.98 (d, *J* = 6.7 Hz, 1H), 5.72 (d, *J* = 11.3 Hz, 1H), 4.77 (s, 1H), 3.33 - 3.27 (m, 1H), 3.23 (d, *J=* 12.8 Hz, 1H), 3.12 (d, *J=* 15.4 Hz, 1H), 2.77 (d, *J=* 9.3 Hz, 2H), 2.57 (s, 1H), 2.33 (s, 3H), 2.08 (s, 1H), 1.88 (s, 1H).

MS-ESI: Calculated.: [M-Br]⁺ 369.49, Found: 369.15.

### Example 59

### Synthesis of compound AB27142

### Step 1):

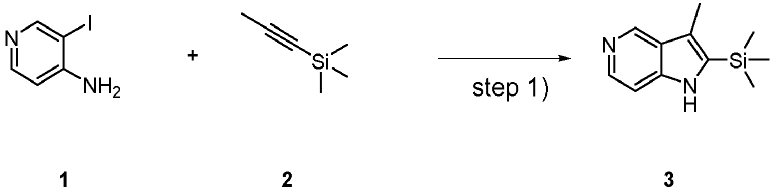

Compound 1 (5.0 g, 22.72 mmol) was dissolved in dry N-N-dimethylformamide (100 mL), and compound 2 (7.65 g, 68.16 mmol), dimethyl ferrocene palladium dichloride (1.0 g, 1.2 mmol), lithium chloride (0.96 mg, 22.72 mmol) and sodium carbonate (4.8 g, 45.44 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (100 mL), dried over anhydrous sodium sulfate, filtered, and concentrated under reduced pressure to remove organic solvent. The residue was purified by thin layer chromatography (ethyl acetate: petroleum ether=0-50%) to afford compound 3 (2.65 g, 56.89% yield).

### Step 2:

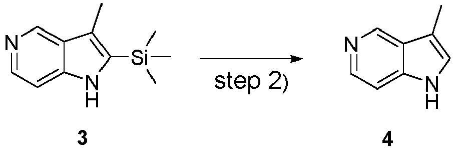

Compound 3 (2.65 g, 12.99 mmol) was dissolved in ethanol (100 mL), and sodium hydroxide (15.59 g, 389.70 mmol) was added. The mixture was replaced with N₂ three times, and stirred at 90°C for overnight, new spot was detected using plate. The reaction mixture was diluted with water (20 ml) and extracted with dichloromethane (30 mL x 3). The combined organic phase was washed with saturated brine (50 mL), dried over anhydrous sodium sulfate, filtered, and concentrated under reduced pressure to remove organic solvent. The residue was purified by thin layer chromatography (methanol: dichloromethane=0-10%) to afford compound 4 (1.1 g, 63.66% yield).

### Step 3:

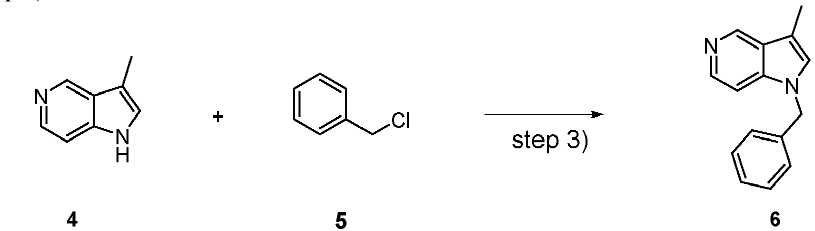

Compound 4 (260 mg, 2 mmol) was dissolved in dry N-N-dimethylformamide (10 mL), then compound 5 (253 mg, 1.5 mmol) and potassium carbonate (830 mg, 6 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by thin layer chromatography (ethyl acetate: petroleum ether=0-50%) to afford compound 6 (100 mg, 22.52 % yield).

### Step 4):

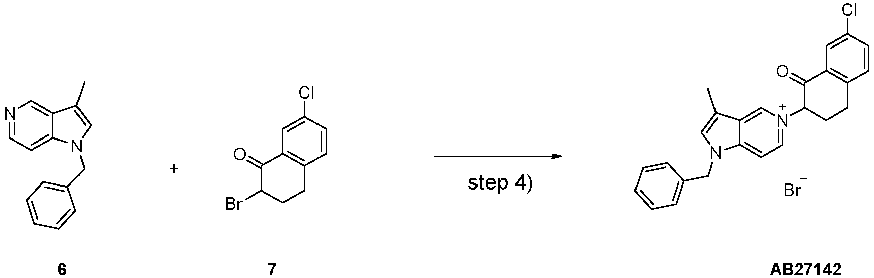

Compound 6 (100 mg, 0.45 mmol) were dissolved in acetonitrile (10 mL), and compound 7 (116 mg, 0.45 mmol) was added. The mixture was replaced with N₂ three times, and stirred at room temperature for overnight, new spot was detected using plate.The reaction mixture was filtered and concentrated under reduced pressure to remove organic solvent. The residue was purified by thin layer chromatography (methanol: dichloromethane=0-10%) to afford compound AB27142 (12.1 mg, 6.70% yield).

### Compound AB27142

¹H NMR (400 MHz, CD3OD-d6) δ 9.25 (s, 1H), 8.38 (d, J = 6.2 Hz, 1H), 8.06 - 7.96 (m, 2H), 7.66 (d, J = 14.5 Hz, 2H), 7.48 (d, J = 7.8 Hz, 1H), 7.33 (d, J = 16.3 Hz, 5H), 5.57 (s, 2H), 3.47 (t, J = 13.4 Hz, 1H), 3.33 (s, 1H), 3.04 (s, 1H), 2.75 (d, *J=* 10.2 Hz, 1H), 2.44 (s, 4H).

MS-ESI: Calculated.: [M-Br]⁺ 401.91, Found: 401.10.

### Example 60

### Synthesis of compound AB27144

### Step 1:

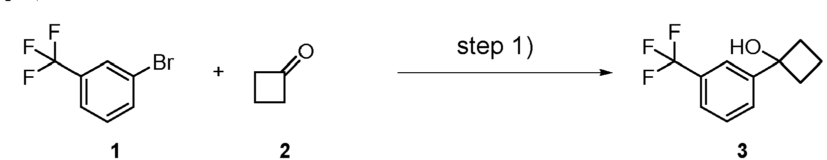

Compound 1 (22 g, 100 mmol) was dissolved in tetrahydrofuran (200 mL), compound 2 (10.5 g, 150 mmol) was added, n-butyllithium (80 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=50:1) to afford compound 3 (13 g, 60.1% yield).

### Step 2):

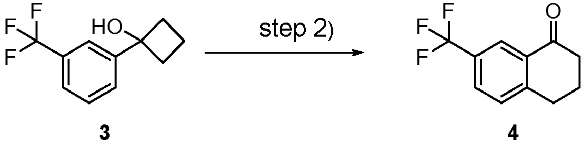

Compound 3 (6.48 g, 30 mmol) was dissolved in dichloromethane (100 mL) and water (100 mL), and AgNO₃ (1 g, 6 mmol) and potassium persulfate (20 g, 270 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 4 (584 mg, 9.0 % yield).

### Step 3):

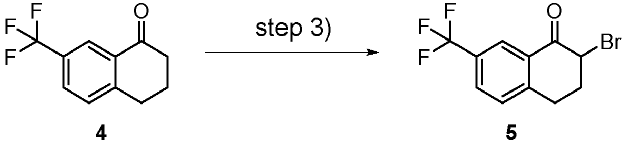

Compound 4 (584 mg, 1.98 mmol) was dissolved in dichloromethane (20 mL), and C₈H₁₈N₂O₂HBr₃ (820 mg, 1.98 mmol) was added. The mixture was stirred at room temperature for 1 h, new spot was detected using plate. The reaction mixture was dried, the residue was purified by thin layer chromatography to afford compound 5 (670 g, 94.7% yield)

### Step 4:

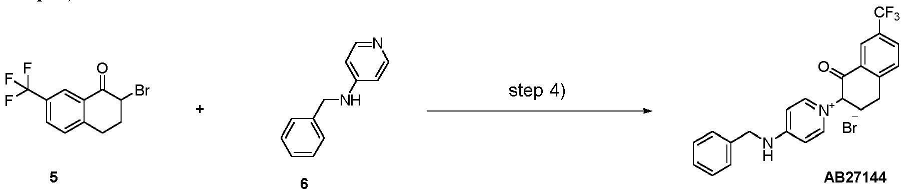

Compound 5 (150 mg, 0.5 mmol) and compound 6 (92 mg, 0.5 mmol) were dissolved in acetonitrile (10 mL). The mixture was stirred at room temperature for 3 h, new spot was detected using plate. The reaction mixture was concentrated under reduced pressure to remove organic solvent, the residue was purified by thin layer chromatography (dichloromethane: methanol=10:1) to afford compound AB27144 (30 mg, 12.6% yield).

### Compound AB27144

¹H NMR (400 MHz, DMSO-d6) δ 9.33 (s, 1H), 8.25 (d, *J* = 6.3 Hz, 1H), 8.12 (d, *J* = 11.7 Hz, 2H), 8.03 (d, *J* = 7.2 Hz, 1H), 7.71 (d, *J* = 7.9 Hz, 1H), 7.34 (d, *J* = 24.9 Hz, 5H), 7.02 (s, 2H), 5.72 (d, *J=* 10.7 Hz, 1H), 4.58 (d, *J=* 4.9 Hz, 2H), 3.39 (s, 1H), 2.83 (s, 1H), 2.50 (s, 2H).

MS-ESI: Calculated.: [M-Br]⁺ 397.15, Found: 397.15

### Example 61

### Synthesis of compound AB27156

### Step 1:

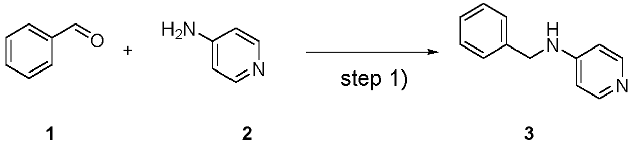

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2:

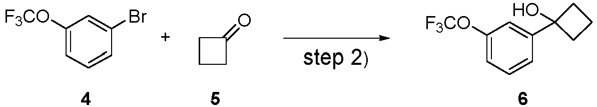

Compound 4 (30 g, 124 mmol) was dissolved in tetrahydrofuran (200 mL), and compound 5 (10.46 g, 149 mmol) was added. The mixture was replaced with N₂ three times, n-butyllithium (100 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 6 (11.2 g, 38.93% yield).

### Step 3:

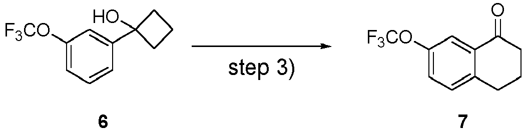

Compound 6 (11.2 g, 48 mmol) was dissolved in dichloromethane (100 mL) and water (100 mL), and AgNO₃ (1.65 g, 9.7 mmol) and potassium persulfate (32.6 g, 121 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 7 (930 g, 8.42 % yield).

### Step 4:

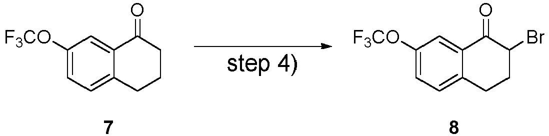

Compound 7 (930 g, 4.04 mmol) was dissolved in dichloromethane (20 mL), and bis(N,N-dimethylacetamide) hydrogen dibromobromate (1.68 g, 4.04 mmol) was added. The mixture was stirred at room temperature for 1 h, new spot was detected using plate. The reaction mixture was dried, the residue was purified by coloum chromatography (petroleum ether: ethyl acetate=0-10%) to afford compound 8 (800 mg, 64.50% yield)

### Step 5):

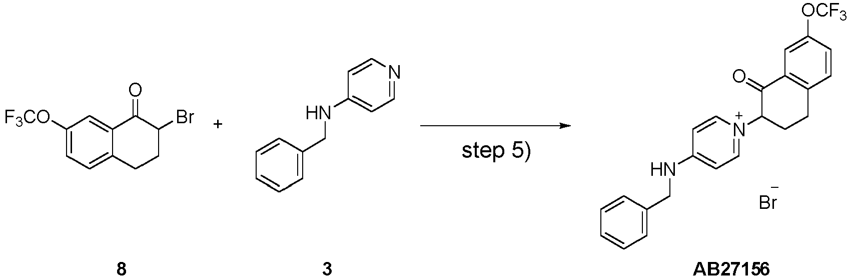

Compound 8 (200 mg, 0.65 mmol) was dissolved in acetonitrile (5 mL), and compound 3 (100 mg, 0.65 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27156 (12 mg, 4.5% yield).

¹H NMR (400 MHz, DMSO-d6) δ 9.50 (s, 1H), 8.25 (d, *J* = 6.7 Hz, 1H), 8.14 (d, *J* = 6.6 Hz, 1H), 7.76 - 7.66 (m, 2H), 7.61 (d, *J* = 8.2 Hz, 1H), 7.34 (d, *J* = 24.9 Hz, 5H), 7.03 (dd, *J* = 16.4, 6.5 Hz, 2H), 5.75 (d, *J=* 10.8 Hz, 1H), 4.58 (d, *J=* 4.8 Hz, 2H), 3.24 (s, 3H), 2.81 (d, *J=* 8.6 Hz, 1H).

MS-ESI: Calculated.: [M-Br]⁺ 413.41, Found: 413.30.

### Example 62

### Synthesis of compound AB27128

### Step 1:

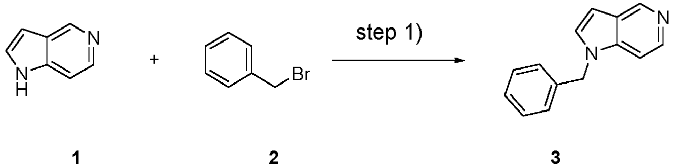

Compound 1 (200 mg, 1.69 mmol) was dissolved in dry N,N-dimethylformamide (20 mL), NaH (203 mg, 5.07 mmol) was added in ice bath. The mixture was stirred at 0°C for 30 min, and compound 2 (318 mg, 1.86 mmol) was slowly added, then the mixture was stirred at room temperature for 3 h, new spot was detected using plate. The reaction mixture was diluted with water (30 ml) and extracted with ethyl acetate (30 ml x 3). The combined organic phase was washed with saturated brine (50 ml), dried over anhydrous sodium sulfate, and concentrated under reduced pressure to remove organic solvent. The residue was purified by column chromatography (methanol: dichloromethane=0-10%) to afford compound 3 (200 mg, 56.9% yield).

### Step 2):

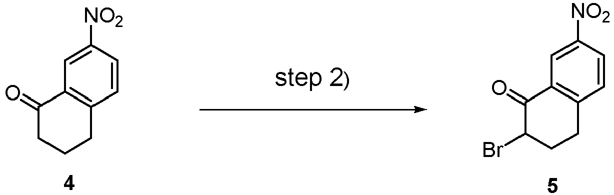

Compound 4 (200 mg, 1.05 mmol) was dissolved in dichloromethane (10 mL), and bis(N,N-dimethylacetamide) hydrogen dibromobromate (434 mg, 1.05 mmol) was added. The mixture was stirred at room temperature for 1 h, new spot was detected using plate. The reaction mixture was dried, the residue was purified by thin layer chromatography to afford compound 5 (260 mg, 92.05% yield).

### Step 3:

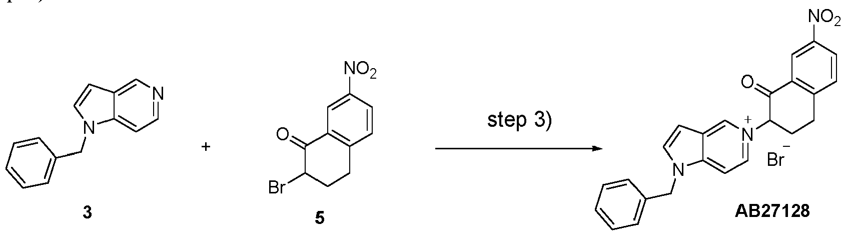

Compound 3 (125 mg, 0.65 mmol) was dissolved in acetonitrile (10 mL), and compound 5 (260 mg, 0.96 mmol) was added. The mixture was replaced with N₂ three times, and stirred at room temperature for overnight, new spot was detected using plate. The reaction mixture was dried, the residue was purified by thin layer chromatography to afford compound AB27128 (20 mg, 7.73% yield).

¹H NMR (400 MHz, DMSO-d6) δ 9.45 (s, 1H), 8.61 (dd, J = 10.6, 4.8 Hz, 2H), 8.52 (dd, J = 8.5, 2.5 Hz, 1H), 8.41 (d, J = 7.1 Hz, 1H), 8.21 (d, J = 3.3 Hz, 1H), 7.83 (d, J = 8.5 Hz, 1H), 7.42 - 7.31 (m, 5H), 7.21 (d, J = 3.2 Hz, 1H), 6.34 (d, J = 14.0 Hz, 1H), 5.71 (s, 2H), 3.47 (s, 2H), 3.09 (dd, J = 13.3, 5.7 Hz, 1H), 2.73 (s, 1H).

MS-ESI: Calculated.: [M-Br]⁺ 398.15, Found: 398.25.

### Example 63

### Synthesis of compound AB27169

### Step 1:

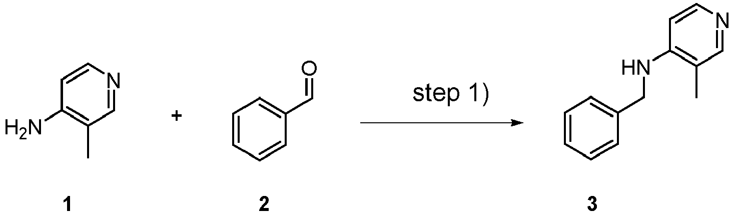

Compound 1 (200 mg, 1.85 mmol) was dissolved in anhydrous ethanol (30 mL), and compound 2 (236 mg, 236 mmol) was added. The mixture was replaced with N₂ three times, and stirred at 90°C for overnight, new spot was detected using plate, and sodium borohydride (210 mg, 5.55 mmol) was slowly added. The reaction mixture was stirred for at room temperature for 3h, new spot was detected using plate. The reaction mixture was quenched with water (20 mL), and extracted with ethyl acetate (30 mL x 3). The combined organic phase was washed with saturated brine (50 mL), dried over anhydrous sodium sulfate, filtered and concentrated under reduced pressure to remove organic solvent. The residue was purified by thin layer chromatography (methanol: dichloromethane=0-10%) to afford compound 3 (100 mg, 27.16% yield).

### Step 2:

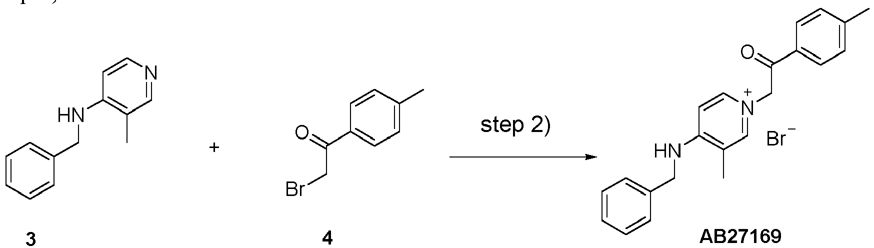

Compound 3 (100 mg, 0.5 mmol) was dissolved in acetonitrile (10 mL), and compound 4 (160 mg, 0.75 mmol) was added. The mixture was replaced with N₂ three times, and stirred at 80 °C for overnight, new spot was detected using plate. The reaction mixture was concentrated under reduced pressure to remove organic solvent. The residue was purified by column chromatography (methanol: dichloromethane=0-30%) to afford compound AB27169 (25 mg, 15.10% yield).

¹H NMR (400 MHz, DMSO-d6) δ 8.65 (s, 1H), 8.10 (s, 2H), 7.87 (s, 2H), 7.38 (d, J = 12.9 Hz, 8H), 7.27 (s, 1H), 6.89 (s, 1H), 5.86 (s, 2H), 4.64 (s, 2H), 2.39 (s, 4H), 2.20 (s, 3H).

MS-ESI: Calculated.: [M-Br]⁺ 331.18, Found: 331.30.

### Example 64

### Synthesis of compound AB27166

### Step 1):

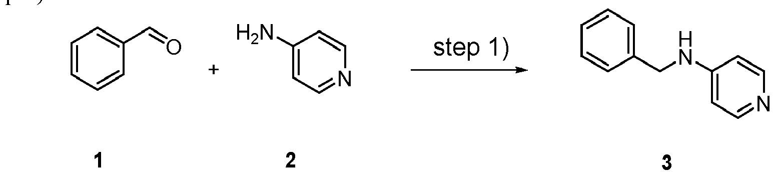

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

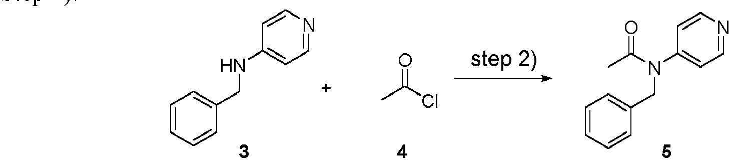

Compound 3 (184 mg, 1 mmol) was dissolved in N,N-dimethylhexanamide (5 mL), and ttriethylamine (303 mg, 3 mmol) was added. The mixture was replaced with N₂ three times, and stirred at 0°C for 30 min, compound 4 (157 mg, 2mmol) was added at 0°C. The mixture was reacted at room temperature, new spot was detected using plate. The reaction mixture was concentrated under reduced pressure to remove organic solvent. The residue was purified by thin layer chromatography (methanol: dichloromethane=0-10%) to afford compound 5 (180 mg, 79.2% yield).

### Step 3:

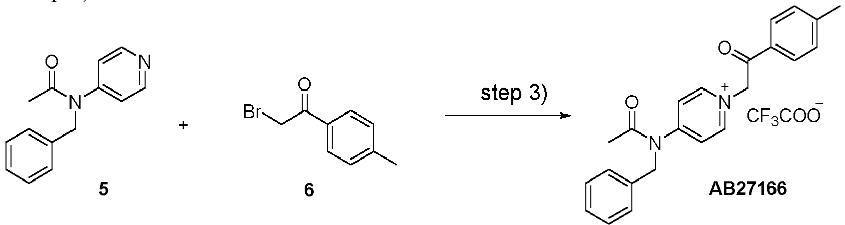

Compound 5 (180 mg, 0.8 mmol) was dissolved in acetonitrile (10 mL), and compound 6 (426 mg, 2 mmol) was added. The mixture was replaced with N₂ three times, and stirred at room temperature for overnight, new spot was detected using plate. The reaction mixture was concentrated under reduced pressure to remove organic solvent, the residue was purified by preparation purification to afford compound AB27166 (40 mg, 10.5 % yield).

¹H NMR (400 MHz, DMSO-d6) δ 8.78 (d, J = 6.3 Hz, 2H), 8.16 (d, J = 6.3 Hz, 2H), 7.91 (d, J = 7.6 Hz, 2H), 7.43 (d, J = 7.6 Hz, 2H), 7.35 (d, J = 7.1 Hz, 1H), 7.27 (d, J = 7.2 Hz, 3H), 6.29 (s, 2H), 5.32 (s, 2H), 2.40 (s, 3H), 2.38 (s, 2H).

MS-ESI: Calculated.: [M-CF₃COO]⁺ 359.44, Found: 359.20.

### Example 65

### Synthesis of compound AB27170

### Step 1:

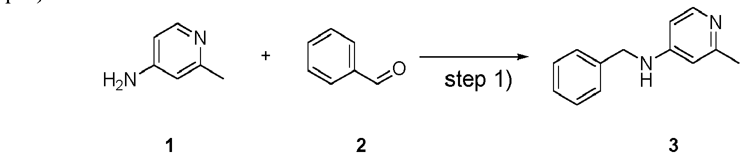

Compound 1 (1 g, 9.26 mmol) was dissolved in ethanol (10 mL), and compound 2 (2.9 g, 27.78 mmol) was added. The mixture was replaced with N₂ three times, and stirred at 90°C for overnight, new spot was detected using plate. The reaction mixture was cooled to 0 °C, sodium borohydride (422 mg, 11.11 mmol) was slowly added, and the system naturally rose to room temperature and was stirred for 1 h, new spot was detected using plate. The reaction mixture was diluted with water (30 mL) and extracted with ethyl acetate (20 mL x 3). The combined organic phase was washed with saturated brine (20 mL), dried over anhydrous sodium sulfate, filtered and concentrated under reduced pressure to remove organic solvent. The residue was purified by column chromatography (methanol: dichloromethane=10:1) to afford compound 3 (1.5 g, 81.84% yield).

### Step 2):

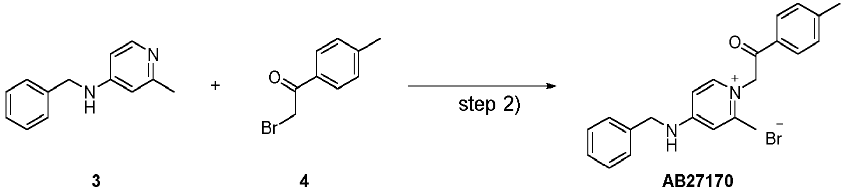

Compound 3 (100 mg, 0.51 mmol) and compound 4 (108 mg, 0.51 mmol) were dissolved in acetonitrile (10 mL). The mixture was stirred at 80°C for 16 h, new spot was detected using plate. The reaction mixture was concentrated under reduced pressure to remove organic solvent, the residue was purified by column chromatography (methanol: dichloromethane=0-10%) to afford compound AB27170 (75 mg, 29.4% yield).

¹H NMR (400 MHz, DMSO-d6) δ 9.20 (d, J = 24.3 Hz, 1H), 8.07 (dd, J = 48.5, 6.5 Hz, 1H), 7.92 (d, J = 6.8 Hz, 2H), 7.40 (dd, J = 22.1, 7.3 Hz, 5H), 7.31 (s, 1H), 7.03 - 6.84 (m, 2H), 5.96 (s, 2H), 4.55 (s, 2H), 2.40 (s, 3H), 2.32 (d, J = 7.7 Hz, 3H).

MS-ESI: Calculated.: [M-Br]⁺ 331.43, Found: 331.25.

### Example 66

### Synthesis of compound AB24917

### Step 1):

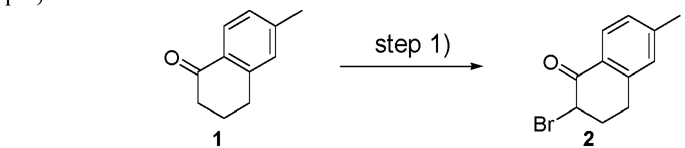

Compound 1 (160mg, 1 mmol) was dissolved in acetic acid (7 mL), two drops of liquid bromine (160 mg, 1.0 mmol) and hydrobromic acid aqueous solution were added. The mixture was stirred at room temperature for 3 h, new spot was detected using plate. The reaction mixture was diluted with ice water (30 mL) and extracted with ethyl acetate (30 mL x 3). The combined organic phase was washed with saturated brine (30 mL), dried over anhydrous sodium sulfate and concentrated under reduced pressure to remove organic solvent to afford compound 2 (260 mg, 90.0% yield).

### Step 2):

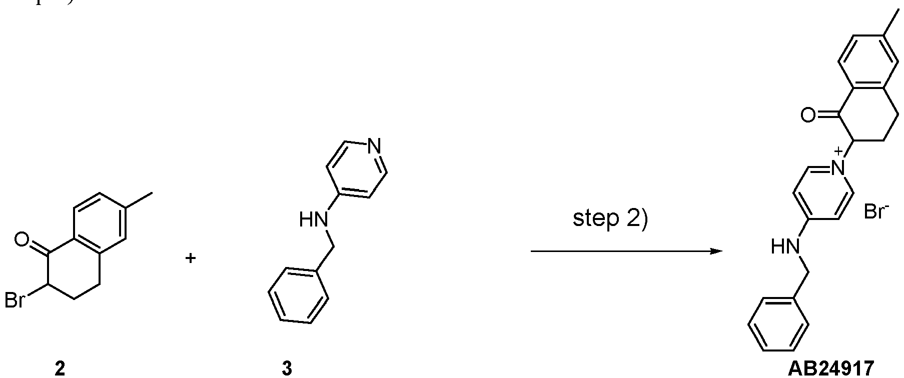

Compound 2 (260 mg, 0.9 mmol) were dissolved in acetonitrile (10 mL), and compound 3 (165 mg, 0.9 mmol) was added. The mixture was replaced with N₂ three times, and stirred at room temperature for overnight, new spot was detected using plate, solid was precipitated and filtered, and filter cake was washed with acetonitrile (2 mL) three times and dried to afford compound AB24917 (45 mg, 14.57 % yield).

¹H NMR (400 MHz, DMSO-d6) δ 9.76 (s, 1H), 8.30 (d, J = 7.2 Hz, 1H), 8.20 (d, J = 7.0 Hz, 1H), 7.82 (d, J = 7.8 Hz, 1H), 7.45 - 7.37 (m, 4H), 7.34 - 7.30 (m, 1H), 7.26 (d, J = 10.1 Hz, 2H), 7.17 - 7.11 (m, 1H), 7.03 - 6.97 (m, 1H), 5.76 (dd, J = 13.8, 4.1 Hz, 1H), 4.60 (d, J = 5.4 Hz, 2H), 3.33 - 3.22 (m, 2H), 3.12 (d, J = 16.4 Hz, 1H), 2.83 - 2.72 (m, 1H), 2.46 (s, 1H), 2.38 (s, 3H).

MS-ESI: Calculated.: [M-Br]⁺ 343.45, Found: 343.10.

### Example 67

### Synthesis of compound AB24956

### Step 1:

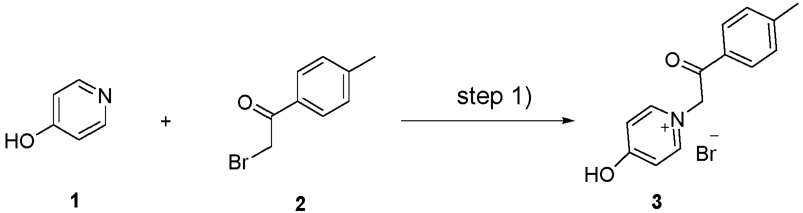

Compound 1 (300 mg, 3.15 mmol) was dissolved in acetonitrile (10 mL), and compound 2 (671 mg, 2.35 mmol) was added. The mixture was replaced with N₂ three times, and stirred at room temperature for overnight, new spot was detected using plate. The reaction mixture was dried, the residue was purified by thin layer chromatography to afford compound 3 (600 mg, 83.54 % yield).

### Step 2:

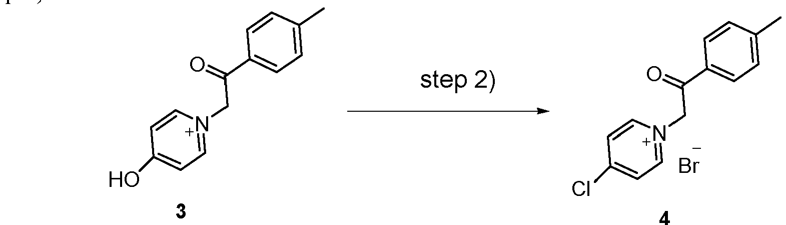

Compound 3 (200 mg, 0.87 mmol) was dissolved in phosphorus oxychloride (10 mL). The mixture was replaced with N₂ three times, and stirred at 90°C for 2h, new spot was detected using plate. The reaction mixture was dried to afford compound 4 (215 mg, crude) for the next step.

### Step 3):

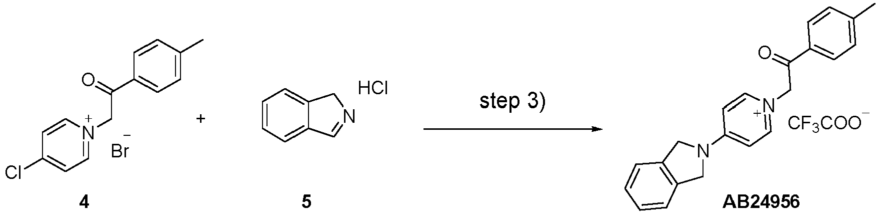

Compound 4 (215 mg, 0.88 mmol) was dissolved in N,N-dimethylformamide (10 mL), and compound 5 (137 mg, 0.88 mmol) and triethylamine (267 mg, 2.64 mmol) were added. The mixture was replaced with N₂ three times, and stirred at 80°C for overnight, new spot was detected using plate. The reaction mixture was dried, the residue was purified by preparation purification to afford compound AB24956 (20 mg, 6.9 % yield).

¹H NMR (400 MHz, DMSO-d6) δ 8.27 (s, 2H), 7.92 (d, J = 6.6 Hz, 2H), 7.40 (d, J = 18.2 Hz, 6H), 7.08 (s, 2H), 5.96 (s, 2H), 4.95 (s, 4H), 2.41 (s, 3H).

MS-ESI: Calculated.: [M-CF₃COO]⁺ 329.16, Found: 329.25.

### Example 68

### Synthesis of compound AB27176

### Step 1):

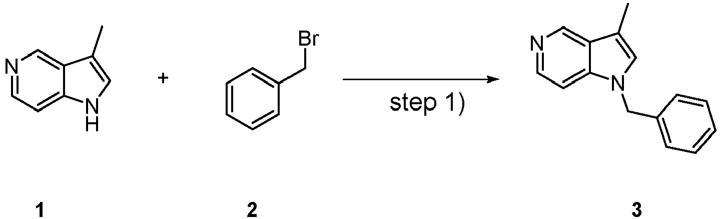

Compound 1 (100 mg, 0.76 mmol) was dissolved in dry N,N-dimethylformamide (10 mL), NaH (61 mg, 1.56 mmol) was added in ice bath. The mixture was stirred for 10 min in ice bath, compound 2 (130 mg, 0.76 mmol) was added. The mixture was replaced with N₂ three times, and the mixture was stirred at room temperature for 5 h, new spot was detected using plate. The reaction mixture was diluted with water (20 ml) and extracted with ethyl acetate (15 mLx 3). The combined organic phase was washed with saturated brine (20 ml), dried over anhydrous sodium sulfate, filtered, and concentrated under reduced pressure to remove organic solvent. The residue was purified by thin layer chromatography (methanol: dichloromethane=0-10%) to afford compound 3 (90 mg, 53.1 % yield).

### Step 2):

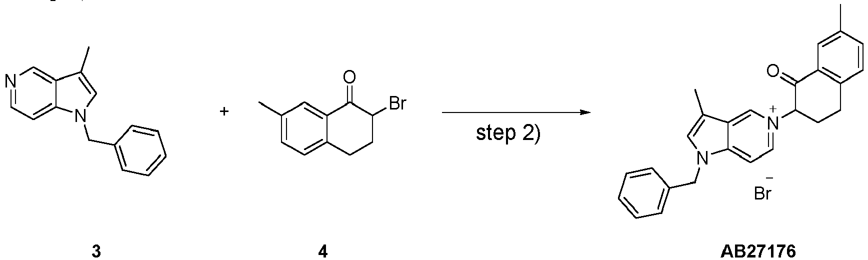

Compound 3 (90 mg, 0.4 mmol) was dissolved in acetonitrile (10 mL), and compound 4 (116 mg, 0.48 mmol) was added. The mixture was replaced with N₂ three times, and stirred at 70 °C for overnight, new spot was detected using plate. The reaction mixture was filtered, and concentrated under reduced pressure to remove organic solvent. The residue was purified by thin layer chromatography (methanol: dichloromethane=0-10%) to afford compound AB27176 (40 mg, 26.2 % yield).

¹H NMR (400 MHz, DMSO-d6) δ 9.43 (s, 1H), 8.54 (s, 1H), 8.30 (s, 1H), 7.89 (s, 1H), 7.73 (s, 1H), 7.50 (s, 1H), 7.34 (s, 5H), 6.13 (d, J = 12.3 Hz, 1H), 5.59 (s, 2H), 3.21 (s, 2H), 3.03 (d, J = 11.6 Hz, 1H), 2.62 (s, 1H), 2.49 (s, 1H), 2.48 (s, 3H), 2.36 (s, 1H), 2.34 (s, 3H).

MS-ESI: Calculated.: [M-Br]⁺ 381.49, Found: 381.10.

### Example 69

### Synthesis of compound AB27174

### Step 1):

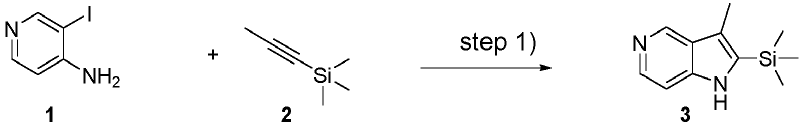

Compound 1 (10.0 g, 25.44 mmol) was dissolved in dry N-N-dimethylformamide (100 mL), and compound 2 (15.3 g, 136 mmol), dimethyl ferrocene palladium dichloride (2.0 g, 2.4 mmol), lithium chloride (1.82 mg, 45 mmol) and sodium carbonate (9.6 g, 90 mmol) were added. The mixture was replaced with N₂ three times, and stirred at room temperature for overnight, new spot was detected using plate. The reaction mixture was diluted with water (50 ml) and extracted with ethyl acetate (50 mL x 3). The combined organic phase was washed with saturated brine (100 mL), dried over anhydrous sodium sulfate, filtered, and concentrated under reduced pressure to remove organic solvent. The residue was purified by thin layer chromatography (ethyl acetate: petroleum ether=0-50%) to afford compound 3 (5.3 g, 56.89% yield).

### Step 2:

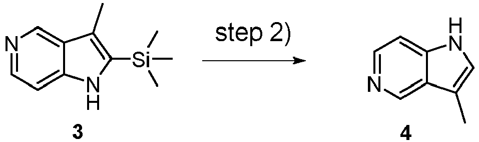

Compound 3 (3.2 g, 15.6 mmol) was dissolved in ethanol (100 mL), and sodium hydroxide (18.7 g, 468 mmol) was added. The mixture was replaced with N₂ three times, and stirred at 90°C for overnight, new spot was detected using plate. The reaction mixture was diluted with water (20 ml) and extracted with dichloromethane (30 mL x 3). The combined organic phase was washed with saturated brine (50 mL), dried over anhydrous sodium sulfate, filtered, and concentrated under reduced pressure to remove organic solvent. The residue was purified by thin layer chromatography (methanol: dichloromethane=0-10%) to afford compound 4 (1.9 g, 63.66% yield).

### Step 3:

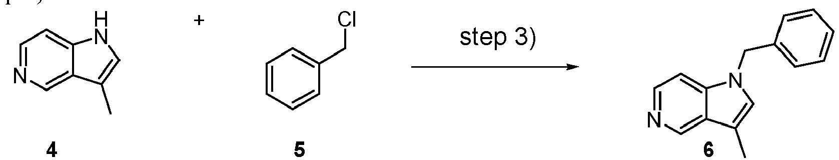

Compound 4 (100 mg, 0.751 mmol) was dissolved in dry N-N-dimethylformamide (10 mL), then compound 5 (95 mg, 0.751 mmol) and potassium carbonate (311 mg, 2.25 mmol) were added. The mixture was replaced with N₂ three times, and stirred at room temperature for overnight, new spot was detected using plate. The reaction mixture was diluted with water (50 ml) and extracted with ethyl acetate (30 mL x 3). The combined organic phase was washed with saturated brine (50 mL), dried over anhydrous sodium sulfate, filtered, and concentrated under reduced pressure to remove organic solvent. The residue was purified by thin layer chromatography (ethyl acetate: petroleum ether=0-50%) to afford compound 6 (60 mg, 35.8 % yield).

### Step 4):

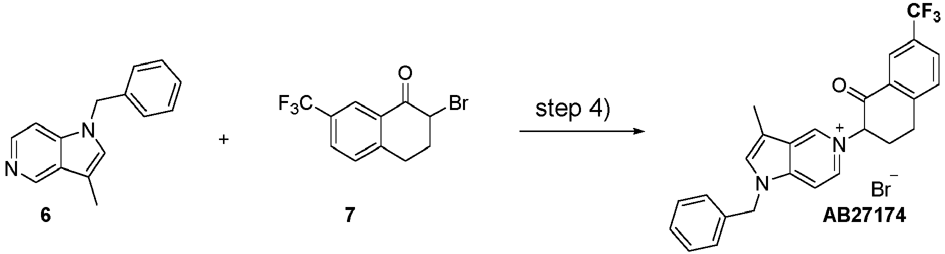

Compound 6 (60 mg, 0.269 mmol) were dissolved in acetonitrile (10 mL), and compound 7 (78 mg, 0.269 mmol) was added. The mixture was replaced with N₂ three times, and stirred at room temperature for overnight, new spot was detected using plate.The reaction mixture was filtered and concentrated under reduced pressure to remove organic solvent. The residue was purified by thin layer chromatography (methanol: dichloromethane=0-10%) to afford compound AB27174 (20 mg, 18.54% yield).

¹H NMR (400 MHz, DMSO-d6) δ 9.49 (s, 1H), 8.60 (d, J = 6.5 Hz, 1H), 8.34 (d, J = 6.5 Hz, 1H), 8.14 (s, 1H), 8.06 (d, J = 7.6 Hz, 1H), 7.90 (s, 1H), 7.77 (d, J = 7.7 Hz, 1H), 7.35 (s, 5H), 6.37 (d, J = 11.0 Hz, 1H), 5.61 (s, 2H), 3.09 (d, J = 10.1 Hz, 1H), 2.67 (d, J = 8.6 Hz, 1H), 2.35 (s, 3H).

MS-ESI: Calculated.: [M-Br]⁺ 435.41, Found: 435.35.

### Example 70

### Synthesis of compound AB24948

### Step 1):

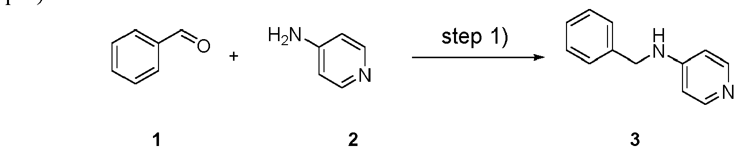

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

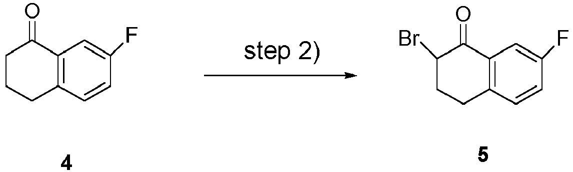

Compound 4 (200 mg, 1.22 mmol) was dissolved in dichloromethane (10 mL), and bis(N,N-dimethylacetamide) hydrogen dibromobromate (505 mg, 1.22 mmol) was added. The mixture was stirred at room temperature for 1 h, new spot was detected using plate. The reaction mixture was dried, and the residue was purified by thin layer chromatography to afford compound 5 (270 mg, 92.97 % yield).

### Step 3:

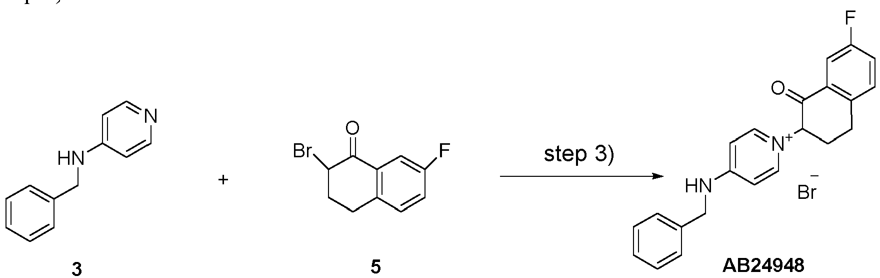

Compound 3 (140 mg, 0.74 mmol) was dissolved in acetonitrile (10 mL), and compound 5 (270mg, 1.11 mmol) was added. The mixture was replaced with N₂ three times, and stirred at room temperature for overnight, new spot was detected using plate to afford compound AB24948 (220 mg, 75.47% yield).

¹H NMR (400 MHz, CD3OD) δ 10.19 (s, 1H), 9.05 (s, 1H), 8.94 (s, 1H), 8.44 - 8.30 (m, 3H), 8.15 (d, J = 25.3 Hz, 5H), 7.83 (s, 2H), 6.52 (s, 1H), 5.38 (s, 2H), 4.03 (d, J = 15.5 Hz, 2H), 3.95 (s, 1H), 3.60 (d, J = 8.4 Hz, 1H).

MS-ESI: Calculated.: [M-Br]⁺ 347.16, Found: 347.10.

### Example 71

### Synthesis of compound AB27163

### Step 1):

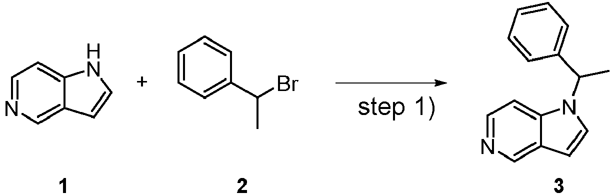

Compound 1 (1.19 mg, 10 mmol) was dissolved in N,N-dimethylhexanamide (50 mL), NaH (180 mg, 20 mmol) was added. The mixture was replaced with N₂ three times, and stirred at 0°C for 30 min, and compound 2 (1.85g, 10 mmol) was added at 0°C. The mixture was stirred at room temperature, new spot was detected using plate. The reaction mixture was concentrated under reduced pressure to remove organic solvent, the residue was purified by column chromatography (methanol: dichloromethane=0-30%) to afford compound 3 (1.2g, 53.8% yield).

### Step 2:

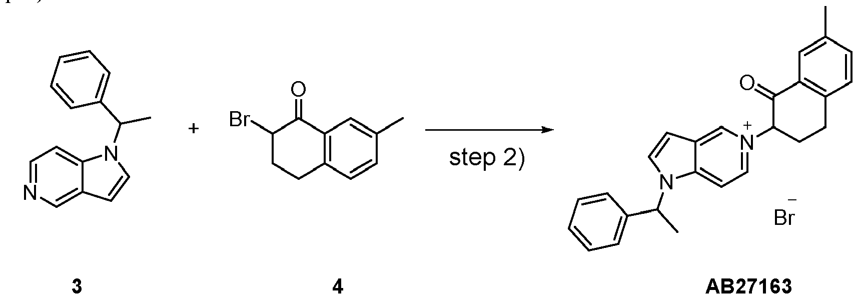

Compound 3 (100 mg, 0.45 mmol) was dissolved in acetonitrile (10 mL), and compound 4 (107.55 mg, 0.45 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27163 (30 mg, 17.49% yield).

¹H NMR (400 MHz, DMSO-d6) δ 9.37 (s, 1H), 8.54 (s, 1H), 8.37 - 8.29 (m, 2H), 7.72 (s, 1H), 7.51 (d, J = 7.5 Hz, 1H), 7.42 (d, J = 7.0 Hz, 2H), 7.37 (d, J = 8.0 Hz, 3H), 7.30 (d, J = 6.8 Hz, 1H), 7.18 (s, 1H), 6.14 (dd, J = 28.5, 8.4 Hz, 2H), 3.19 (d, J = 15.1 Hz, 2H), 2.98 (d, J = 9.9 Hz, 1H), 2.64 (s, 1H), 2.34 (s, 3H), 1.97 (d, J = 6.4 Hz, 3H).

MS-ESI: Calculated.: [M-Br]⁺ 381.49, Found: 381.20

### Example 72

### Synthesis of compound AB27184

### Step 1:

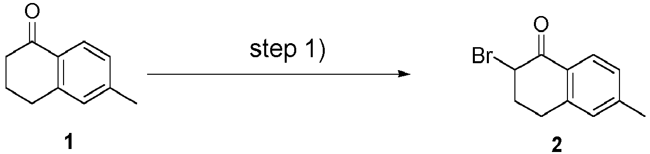

Compound 1 (360 mg, 2.25 mmol) was dissolved in dichloromethane (10 mL), and C₈H₁₈N₂O₂HBr₃ (929 mg, 2.25 mmol) was added, then a drop of methanol was added. The mixture was replaced with N₂ three times, and stirred at room temperature for 1 h, new spot was detected using plate. The reaction mixture was concentrated under reduced pressure to remove organic solvent, the residue was purified by thin layer chromatography (petroleum ether: ethyl acetate=5:1) to afford compound 2 (470 mg, 64.48% yield).

### Step 2):

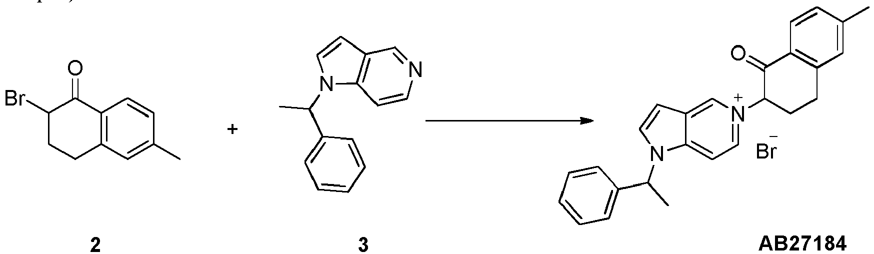

Compound 2(116 mg, 0.5 mmol) was dissolved in acetonitrile (10 mL), and compound 3 (111 mg, 0.5 mmol) was added. The mixture was replaced with N₂ three times, and stirred at 80°C for overnight, new spot was detected using plate. The reaction mixture was concentrated under reduced pressure to remove organic solvent, the residue was purified by thin layer chromatography (dichloromethane: methanol=10:1) to afford compound AB27184 (104 mg, 46.73 % yield).

¹H NMR (400 MHz, dmso-d6) δ 9.37 (s, 1H), 8.54 (s, 1H), 8.33 (d, J = 11.0 Hz, 2H), 7.81 (d, J = 6.6 Hz, 1H), 7.31 (dd, J = 58.4, 33.6 Hz, 9H), 6.17 (s, 1H), 6.10 (s, 1H), 3.18 (d, J = 18.2 Hz, 2H), 2.98 (s, 1H), 2.64 (s, 1H), 2.38 (s, 3H), 1.96 (s, 3H).

MS-ESI: Calculated.: [M-Br]⁺ 381.49, Found: 381.30.

### Example 73

### Synthesis of compound AB27162

### Step 1):

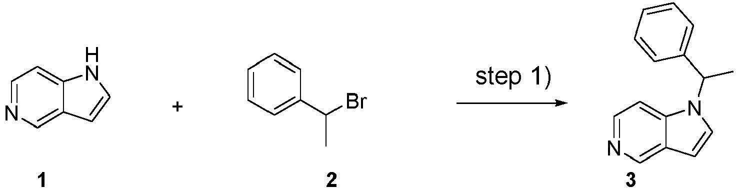

Compound 1 (1.19 mg, 10 mmol) was dissolved in N,N-dimethylhexanamide (50 mL), NaH (480 mg, 20 mmol) was added. The mixture was replaced with N₂ three times, and stirred at 0°C for 30 min, and compound 2 (1.85g, 10 mmol) was added at 0°C. The mixture was stirred at room temperature, new spot was detected using plate. The reaction mixture was concentrated under reduced pressure to remove organic solvent, the residue was purified by column chromatography (methanol: dichloromethane=0-30%) to afford compound 3 (1.2g, 53.8% yield).

### Step 2):

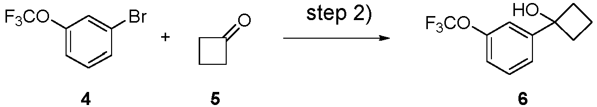

Compound 4 (30 g, 124 mmol) was dissolved in tetrahydrofuran (200 mL), compound 5 (10.46 g, 149 mmol) was added. The mixture was replaced with N₂ three times, n-butyllithium (100 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 6 (11.2 g, 38.93 % yield).

### Step 3):

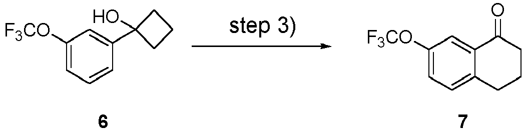

Compound 6 (11.2 g, 48 mmol) was dissolved in dichloromethane (100 mL) and water (100 mL), and AgNO₃ (1.65 g, 9.7 mmol) and potassium persulfate (32.6 g, 121 mmol) were added. The mixture was stirred at room temperature for 16 h, new spot was detected using plate. The reaction mixture was concentrated under reduced pressure to remove water and organic solvent, the residue was purified by column chromatography (petroleum ether: ethyl acetate=0-10%) to afford compound 7 (930 mg, 8.42 % yield).

### Step 4:

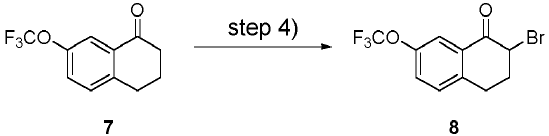

Compound 7 (930 mg, 4.04 mmol) was dissolved in dichloromethane (20 mL), and bis(N,N-dimethylacetamide) hydrogen dibromobromate (1.68 g, 4.04 mmol) was added. The mixture was stirred at room temperature for 1 h, new spot was detected using plate. The reaction mixture was dried, the residue was purified by column chromatography (petroleum ether: ethyl acetate=0-10%) to afford compound 8 (800 mg, 64.50% yield)

### Step 5):

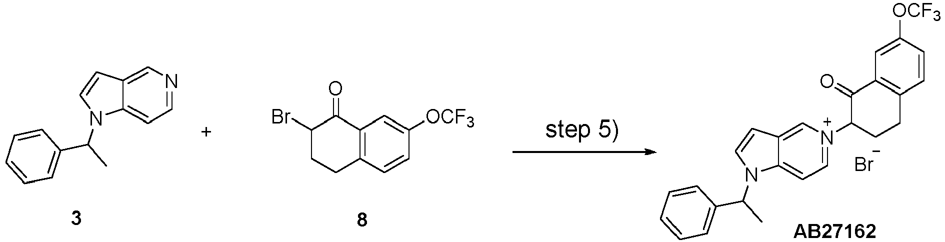

Compound 3 (100 mg, 0.45 mmol) was dissolved in acetonitrile (10 mL), and compound 8 (138 mg, 0.45 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27162 (30 mg, 14.78% yield).

¹H NMR (400 MHz, DMSO-d6) δ 9.39 (s, 1H), 8.56 (s, 1H), 8.36 (s, 2H), 7.70 (dd, J = 33.7, 12.2 Hz, 3H), 7.46 - 7.26 (m, 5H), 7.20 (s, 1H), 6.19 (s, 2H), 3.34 (s, 2H), 3.02 (d, J = 10.4 Hz, 1H), 2.64 (s, 1H), 1.97 (d, J = 6.4 Hz, 3H).

MS-ESI: Calculated.: [M-Br]⁺ 451.16, Found: 451.35.

### Example 74

### Synthesis of compound AB27145

### Step 1):

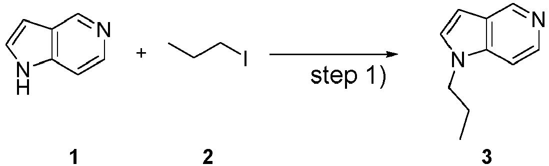

Compound 1 (236 mg, 2.0 mmol) was dissolved in dry N,N-dimethylformamide (10 mL), NaH (24 mg, 1.0 mmol) was added in ice bath. The mixture was replaced with N₂ three times, and the mixture was stirred for 30 min in ice bath, and compound 2 (408 mg, 2.4 mmol) was slowly added in ice bath. The mixture was stirred at room temperature for 90 min, new spot was detected using plate. The reaction mixture was diluted with ice water (20 ml) and extracted with ethyl acetate (30 mLx 3). The combined organic phase was washed with saturated brine (50 ml), dried over anhydrous sodium sulfate, filtered, and concentrated under reduced pressure to remove organic solvent. The residue was purified by thin layer chromatography (methanol: dichloromethane=0-10%) to afford compound 3 (200 mg, 62.5 % yield).

### Step 2):

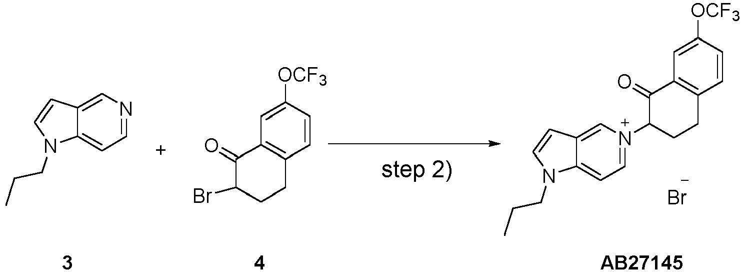

Compound 3 (60 mg, 0.31 mmol) was dissolved in acetonitrile (10 mL), and compound 4 (100 mg, 0.31 mmol) was added. The mixture was replaced with N₂ three times, and stirred at 60 °Cfor overnight, new spot was detected using plate. The reaction mixture was concentrated under reduced pressure to remove organic solvent. The residue was purified by thin layer chromatography to afford compound AB27145 (30 mg, 24.88 % yield).

¹H NMR (400 MHz, DMSO-d6) δ 9.37 (s, 1H), 8.57 (d, J = 6.7 Hz, 1H), 8.34 (d, J = 6.6 Hz, 1H), 8.07 (s, 1H), 7.80 - 7.71 (m, 2H), 7.67 (d, J = 8.1 Hz, 1H), 7.13 (s, 1H), 6.21 (d, J = 12.0 Hz, 1H), 4.37 (s, 2H), 3.33 (s, 2H), 3.06 (d, J = 10.1 Hz, 1H), 2.69 (s, 1H), 1.84 (d, J = 7.0 Hz, 2H), 0.84 (t, J = 6.8 Hz, 3H).

MS-ESI: Calculated.: [M-Br]⁺ 389.39, Found: 389.05.

### Example 75

### Synthesis of compound AB27129

### Step 1:

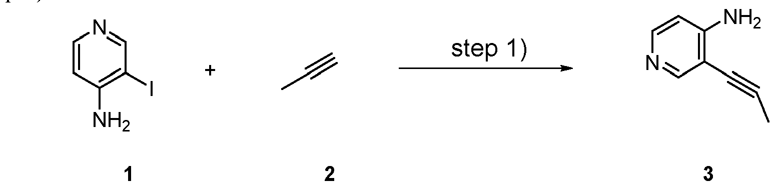

Compound 1 (5.5 g, 25 mmol) was dissolved in N,N-dimethylformamide (200 mL), and compound 2 (52.5 ml), copper(I) iodide (237 mg, 1.25 mmol), N, N-diisopropylethylamine (10.0 g, 80 mmol) and bis(triphenylphosphine)palladium(II) chloride (525.75 mg, 0.75 mmol) were added successively. The mixture was stirred at room temperature for 16 h, new spot was detected using plate. The reaction mixture was concentrated under reduced pressure to remove organic solvent, the residue was purified by column chromatography (methanol: dichloromethane=0-10%) to afford compound 3 (2.25 g, 67.67% yield).

### Step 2):

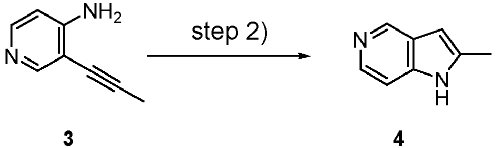

Compound 3 (2.25 g, 17 mmol) was dissolved in 1,4-dioxane (20 mL), and potassium tert-butoxide (51 ml) was added. The mixture was stirred at 110°C for 16h, new spot was detected using plate. The reaction mixture was concentrated under reduced pressure to remove organic solvent. The residue was purified by column chromatography (methanol: dichloromethane=0-10%) to afford compound 4 (1.2 g, 54.3% yield).

### Step 3:

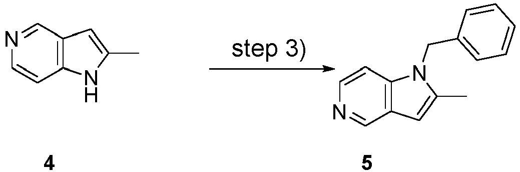

Compound 4 (550 mg, 4.2 mmol) was dissolved in N,N-dimethylformamide (10 mL), NaH (304 mg, 12.7 mmol) was added in ice bath. The mixture was stirred at room temperatute for 3 h, new spot was detected using plate. The reaction mixture was dried, the residue was purified by thin layer chromatography to afford compound 5 (294 mg, 31.53 % yield).

### Step 4:

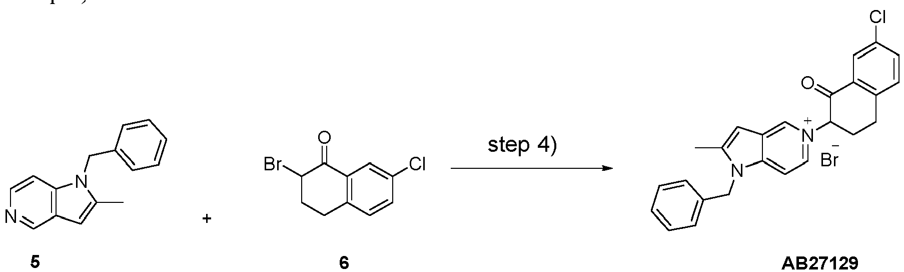

Compound 5 (100 mg, 0.5 mmol) was dissolved in acetonitrile (10 mL), and compound 6 (129 mg, 0.5 mmol) was added. The mixture was replaced with N₂ three times, and stirred at room temperature for overnight, new spot was detected using plate. The reaction mixture was concentrated under reduced pressure to remove organic solvent. The residue was purified by thin layer chromatography (methanol: dichloromethane=10%) to afford compound AB27129 (40 mg, 16.67% yield).

¹H NMR (400 MHz, dmso-d6) δ 9.11 (s, 1H), 8.36 (d, J = 6.4 Hz, 1H), 8.06 - 7.93 (m, 2H), 7.66 (d, J = 7.5 Hz, 1H), 7.48 (d, J = 7.8 Hz, 1H), 7.34 (d, J = 6.5 Hz, 3H), 7.08 (d, J = 6.2 Hz, 2H), 6.93 (s, 1H), 5.67 (s, 2H), 3.44 (d, J = 12.1 Hz, 1H), 3.33 (s, 1H), 3.00 (s, 1H), 2.77 (s, 1H), 2.53 (s, 3H).

MS-ESI: Calculated.: [M-Br]⁺ 401.91, Found: 401.20.

### Example 76

### Synthesis of compound AB27161

### Step 1):

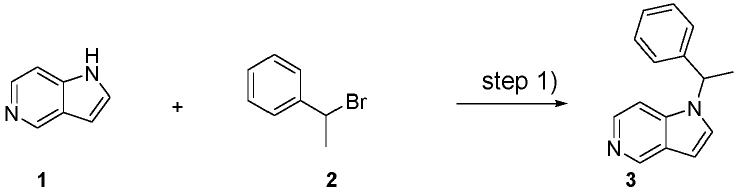

Compound 1 (1.19 mg, 10 mmol) was dissolved in N,N-dimethylhexanamide (50 mL), NaH (480 mg, 20 mmol) was added. The mixture was replaced with N₂ three times, and stirred at 0°C for 30 min, and compound 2 (1.85g, 10 mmol) was added at 0°C. The mixture was stirred at room temperature, new spot was detected using plate. The reaction mixture was concentrated under reduced pressure to remove organic solvent, the residue was purified by column chromatography (methanol: dichloromethane=0-30%) to afford compound 3 (1.2g, 53.8% yield).

### Step 2):

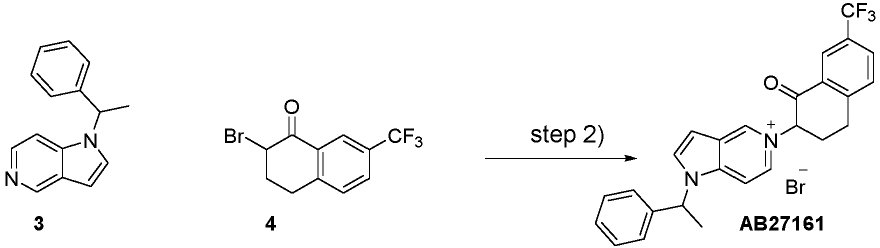

Compound 3 (111 mg, 0.5 mmol) was dissolved in acetonitrile (10 mL), and compound 4 (146 mg, 0.5 mmol) was added. The mixture was replaced with N₂ three times, and stirred at 80°C for overnight, new spot was detected using plate. The reaction mixture was concentrated under reduced pressure to remove organic solvent. The residue was purified by colum chromatography (methanol: dichloromethane=0-30%) to afford compound AB27161 (90 mg, 41.37% yield).

¹H NMR (400 MHz, DMSO-d6) δ 9.47 (s, 1H), 8.62 (d, J = 6.1 Hz, 1H), 8.38 (s, 2H), 8.21 - 8.01 (m, 2H), 7.76 (d, J = 7.7 Hz, 1H), 7.57 - 7.16 (m, 6H), 6.42 (d, J = 13.2 Hz, 1H), 6.21 (d, J = 5.8 Hz, 1H), 3.44 (d, J = 13.0 Hz, 2H), 3.05 (d, J = 11.5 Hz, 1H), 2.69 (s, 1H), 1.97 (d, J = 6.2 Hz, 3H).

MS-ESI: Calculated.: [M]⁺ 435.47, Found: 435.25.

### Example 77

### Synthesis of compound AB27165

### Step 1):

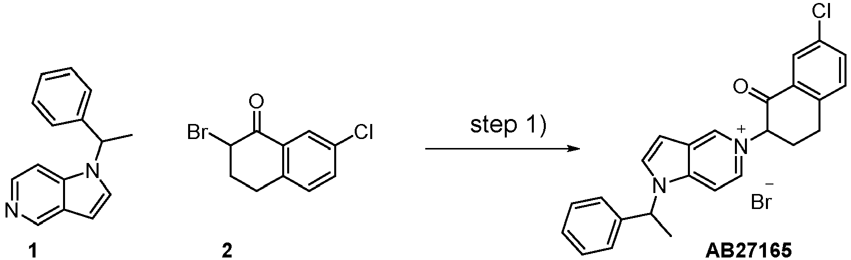

Compound 1 (100 mg, 0.5 mmol) was dissolved in acetonitrile (10 mL), and compound 2 (130.0 mg, 0.5 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-30%) to afford compound AB27165 (80 mg, 39.90% yield).

¹H NMR (400 MHz,DMSO-d6) δ 9.47 (s, 1H), 8.61 (d, J = 5.8 Hz, 1H), 8.45 - 8.30 (m, 2H), 7.83 (s, 1H), 7.75 (d, J = 7.7 Hz, 1H), 7.54 (d, J = 7.7 Hz, 1H), 7.44 (d, J = 6.1 Hz, 2H), 7.32 (dd, J = 22.8, 3.1 Hz, 3H), 7.19 (s, 1H), 6.38 (d, J = 12.2 Hz, 1H), 6.21 (d, J = 5.5 Hz, 1H), 3.33 - 3.11 (m, 2H), 3.02 (d, J = 11.2 Hz, 1H), 2.65 (s, 1H), 1.96 (d, J = 5.5 Hz, 3H).

MS-ESI: Calculated.: [M-Br]⁺ 401.90, Found: 401.10.

### Example 78

### Synthesis of compound AB24961

### Step 1):

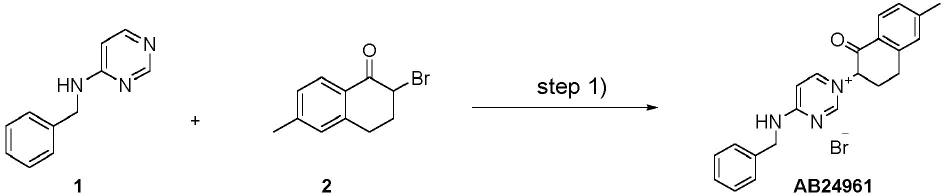

Compound 1 (202 mg, 1.09 mmol) was dissolved in acetonitrile (10 mL), and compound 2 (250 mg, 1.09 mmol) was added. The mixture was replaced with N₂ three times, and stirred at room temperature for overnight, new spot was detected using plate. The reaction mixture was dried, the residue was purified by thin layer chromatographyto afford compound AB24961 (40 mg, 10.65% yield).

¹H NMR (400 MHz, DMSO-d6) δ 10.47 (s, 1H), 8.88 (s, 1H), 8.27 (d, J = 6.7 Hz, 1H), 7.81 (d, J = 7.5 Hz, 1H), 7.37 (d, J = 3.3 Hz, 3H), 7.25 (d, J = 7.9 Hz, 2H), 7.03 (d, J = 7.2 Hz, 1H), 5.70 (d, J = 9.9 Hz, 1H), 4.73 (d, J = 5.0 Hz, 2H), 2.74 (d, J = 9.9 Hz, 1H), 2.37 (s, 4H), 2.06 (s, 3H).

MS-ESI: Calculated.: [M-Br]⁺ 344.44, Found: 344.10.

### Example 79

### Synthesis of compound AB27158

### Step 1:

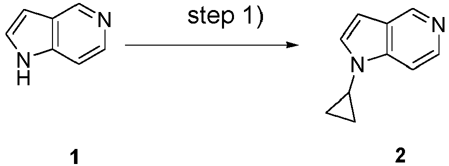

Compound 1 (700 mg, 6 mmol) was dissolved in dichloromethane (80 mL), and cyclopropyl boric acid (1.5 g, 18 mmol), triethylamine (1.2 g, 12 mmol) and copper acetate (1.2 g, 6 mmol) were added. The mixture was replaced with O₂ three times, and stirred at room temperature for overnight, new spot was detected using plate. The reaction mixture was diluted with water (20 mL) and extracted with dichloromethane (30 mL x 3). The combined organic phase was washed with saturated brine (50 mL), dried over anhydrous sodium sulfate, filtered and concentrated under reduced pressure to remove organic solvent. The residue was purified by thin layer chromatography (methanol: dichloromethane=0-10%) to afford compound 2 (170 mg, 15.58% yield).

### Step 2):

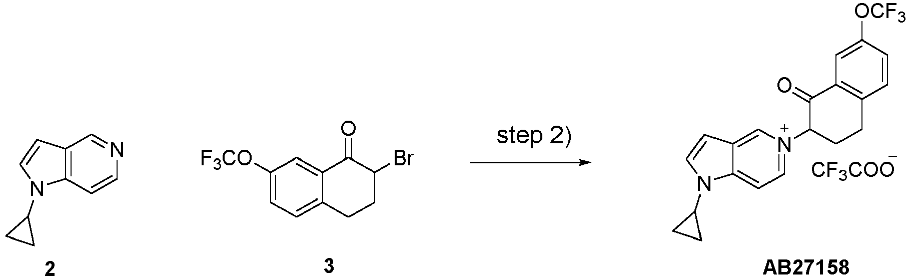

Compound 2 (85 mg, 0.5 mmol) was dissolved in acetonitrile (10 mL), and compound 3 (150 mg, 0.5 mmol) was added. The mixture was replaced with N₂ three times, and stirred at 80 °C for overnight, new spot was detected using plate. The reaction mixture was concentrated under reduced pressure to remove organic solvent. The residue was purified by preparation purification to afford compound AB27158 (40 mg, 15.99% yield).

¹H NMR (400 MHz, dmso-d6) δ 9.35 (s, 1H), 8.58 (d, J = 6.3 Hz, 1H), 8.25 (d, J = 6.3 Hz, 1H), 8.00 (s, 1H), 7.71 (dd, J = 35.0, 14.5 Hz, 3H), 7.07 (s, 1H), 6.18 (d, J = 12.2 Hz, 1H), 3.76 (s, 1H), 3.05 (s, 1H), 2.65 (s, 1H), 2.49 (s, 2H), 1.14 (d, J = 22.5 Hz, 4H).

MS-ESI: Calculated.: [M- CF₃COO]⁺ 387.37, Found: 387.10.

### Example 80

### Synthesis of compound AB27155

### Step 1):

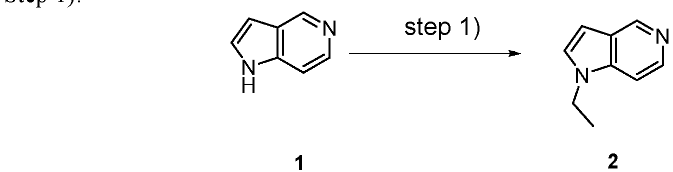

Compound 1 (236 mg, 2.0 mmol) was dissolved in N,N-dimethylformamide (10 mL), NaH (24 mg, 1.0 mmol) was added in ice bath. The mixture was replaced with N₂ three times, and the mixture was stirred for 30 min in ice bath, and iodoethane (374 mg, 2.4 mmol) was slowly added in ice bath. The mixture was stirred at room temperature for 90 min, new spot was detected using plate. The reaction mixture was diluted with ice water (20 ml), and concentrated under reduced pressure to remove organic solven, the crude product was filtered with methanol, and the filtrate was concentrated under reduced pressure to remove organic solvent, the residue was purified by thin layer chromatography (ethyl acetate: petroleum ether=0-30%) to afford compound 2 (185 mg, 62.9 % yield).

### Step 2):

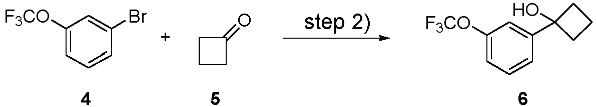

Compound 1 (30 g, 124 mmol) was dissolved in tetrahydrofuran (200 mL), compound 5 (10.46 g, 149 mmol) was added. The mixture was replaced with N₂ three times, n-butyllithium (100 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 6 (11.2 g, 38.93% yield).

### Step 3):

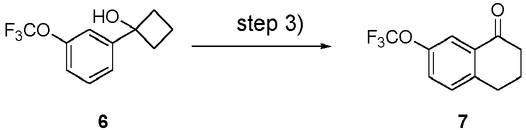

Compound 6 (11.2 g, 48 mmol) was dissolved in dichloromethane (100 mL) and water (100 mL), and AgNO₃ (1.65 g, 9.7 mmol) and potassium persulfate (32.6 g, 121 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 7 (930 mg, 8.42 % yield).

### Step 4):

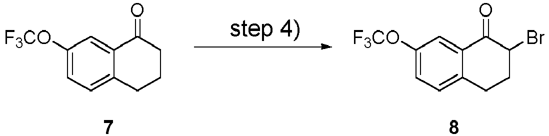

Compound 7 (930 mg, 4.04 mmol) was dissolved in dichloromethane (20 mL), and bis(N,N-dimethylacetamide) hydrogen dibromobromate (1.68 g, 4.04 mmol) was added. The mixture was stirred at room temperature for 1h, new spot was detected using plate. The reaction mixture was dried, and the residue was purified by colum chromatography (petroleum ether: ethyl acetate=0-10%) to afford compound 8(800 mg, 64.50% yield).

### Step 5):

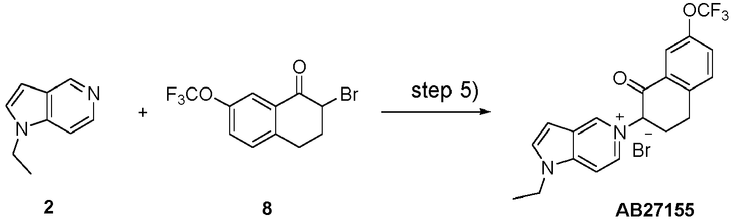

Compound 2 (90 mg, 0.61 mmol) was dissolved in acetonitrile (10 mL), and compound 8 (472 mg, 1.53 mmol) was added. The mixture was replaced with N₂ three times, and stirred at 80°C for overnight, new spot was detected using plate. The reaction mixture was concentrated under reduced pressure to remove organic solvent, the residue was purified by thin layer chromatography (methanol: dichloromethane=0-10%) to afford compound AB27155 (20 mg, 8.7% yield).

¹H NMR (400 MHz, DMSO-d6) δ 9.37 (s, 1H), 8.58 (d, J = 6.4 Hz, 1H), 8.32 (d, J = 6.6 Hz, 1H), 8.10 (s, 1H), 7.71 (dd, J = 34.7, 14.4 Hz, 3H), 7.13 (s, 1H), 6.23 (d, J = 9.9 Hz, 1H), 4.44 (d, J = 6.7 Hz, 2H), 3.33 (s, 2H), 3.06 (d, J = 12.7 Hz, 1H), 2.67 (s, 1H), 1.42 (dd, J = 6.5 Hz, 3H).

MS-ESI: Calculated.: [M-Br]⁺ 375.36, Found: 375.20.

### Example 81

### Synthesis of compound AB27104

### Step 1:

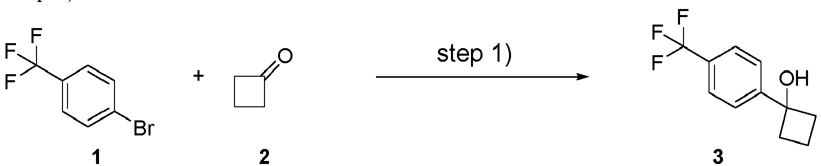

Compound 1 (22 g, 100 mmol) was dissolved in tetrahydrofuran (200 mL), compound 2 (10.5 g, 150 mmol) was added, n-butyllithium (80 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=50:1) to afford compound 3 (12 g, 55.30% yield).

### Step 2):

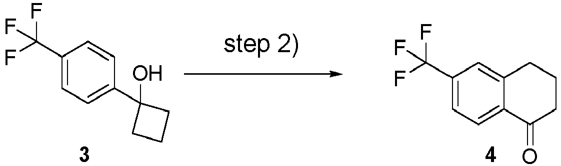

Compound 3 (12 g, 55.6 mmol) was dissolved in dichloromethane (100 mL) and water (100 mL), and AgNO₃ (1.89 g, 11.12 mmol) and potassium persulfate (37 g, 139 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=10:1) to afford compound 4 (3.1 g, 25.93 % yield).

### Step 3:

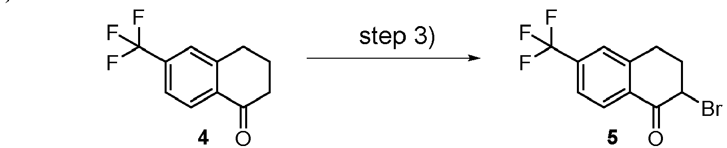

Compound 4 (1.5 g, 6.9 mmol) was dissolved in dichloromethane (20 mL), and C₈H₁₈N₂O₂HBr₃ (2.9 g, 6.9 mmol) was added. The mixture was stirred at room temperature for 1 h, new spot was detected using plate. The reaction mixture was dried, the residue was purified by thin layer chromatography to afford compound 5 (2.2g, 85.48% yield)

### Step 4:

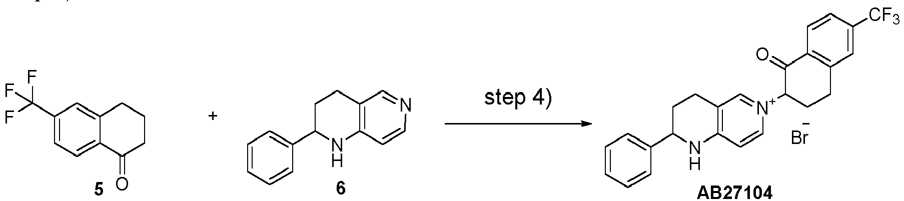

Compound 5 (150 mg, 0.5 mmol) was dissolved in acetonitrile (5 mL), and compound 6 (105 mg, 0.5 mmol) was added. The mixture was replaced with N₂ three times, and stirred at room temperature for overnight, new spot was detected using plate. The reaction mixture was concentrated under reduced pressure to remove organic solvent, the residue was purified by column chromatography (methanol: dichloromethane=0-30%) to afford compound AB27104 (20 mg, 7.96% yield).

### Compound AB27104

¹H NMR (400 MHz, DMSO-d6) δ 9.29 (s, 1H), 8.17 (s, 1H), 8.09 (s, 2H), 7.91 (s, 1H), 7.78 (d, J = 7.8 Hz, 1H), 7.38 (d, J = 21.0 Hz, 6H), 6.90 (d, J = 6.8 Hz, 1H), 5.70 (d, J = 13.2 Hz, 1H), 4.77 (s, 1H), 3.32 (s, 2H), 2.81 (s, 2H), 2.62 (s, 1H), 2.10 (s, 1H), 1.88 (s, 1H).

MS-ESI: Calculated.: [M-Br]⁺ 423.45, Found: 423.25.

### Example 82

### Synthesis of compound AB27154

### Step 1):

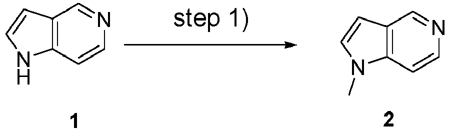

Compound 1 (500 mg, 4.32 mmol) was dissolved in dry N,N-dimethylformamide (10 mL), NaH (304.56 mg, 12.7 mmol) was added in ice bath. The mixture was replaced with N₂ three times, and stirred for 30 min in ice bath, and iodomethane (723.89 mg, 5.1 mmol) was slowly injected in ice bath, then the mixture was stirred at room temperature for 90 min, new spot was detected using plate. The reaction mixture was diluted with water (20 ml) and concentrated under reduced pressure to remove organic solvent, the crude product was filtered with methanol, and the filtrate was concentrated under reduced pressure to remove organic solvent, the residue was purified by thin layer chromatography (methanol: dichloromethane=10%) to afford compound 2 (479 mg, 85.78 % yield).

### Step 2:

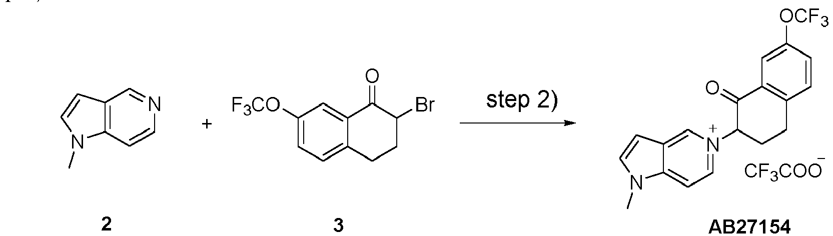

Compound 2 (100 mg, 0.76 mmol) was dissolved in acetonitrile (10 mL), and compound 3 (233 mg, 0.76 mmol) was added. The mixture was stirred at 80°C for overnight, new spot was detected using plate. The reaction mixture was concentrated under reduced pressure to remove organic solvent, the residue was purified by thin layer chromatography (methanol: dichloromethane=0-10%) to afford compound AB27154 (40 mg, 11.10% yield).

¹H NMR (400 MHz, dmso-d6) δ 9.35 (s, 1H), 8.56 (d, J = 6.8 Hz, 1H), 8.24 (d, J = 6.8 Hz, 1H), 7.99 (s, 1H), 7.81 - 7.70 (m, 2H), 7.67 (d, J = 8.2 Hz, 1H), 7.11 (s, 1H), 6.16 (d, J = 11.0 Hz, 1H), 4.01 (s, 3H), 3.33 (s, 2H), 3.06 (d, J = 8.6 Hz, 1H), 2.67 (d, J = 11.1 Hz, 1H).

MS-ESI: Calculated.: [M-CF₃COO]+ 361.34, Found: 361.05.

### Example 83

### Synthesis of compound AB24999

### Step 1:

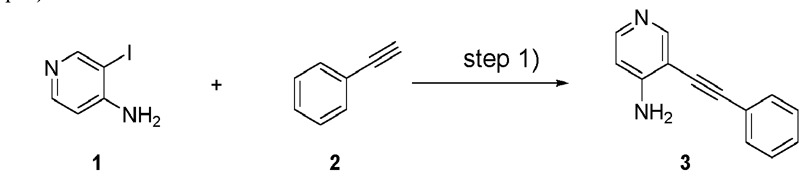

Compound 1 (4.4 g, 20 mmol) was dissolved in dry N,N-dimethylformamide (150 mL), and compound 2 (4.28 g, 42 mmol), bis(triphenylphosphine)palladium(II) chloride (421 mg, 0.6 mmol), N, N-diisopropylethylamine (8.25 g, 64 mmol) and copper(I) iodide (190 mg, 1 mmol) were added. The mixture was replaced with N₂ three times, and stirred at room temperature for overnight, new spot was detected using plate. The reaction mixture was diluted with water (250 ml) and extracted with ethyl acetate (100 mL x 3). The combined organic phase was washed with saturated brine (100 mL), dried over anhydrous sodium sulfate, filtered, and concentrated under reduced pressure to remove organic solvent. The residue was purified by column chromatography (ethyl acetate: petroleum ether=0-50%) to afford compound 3 (3.5, 89.74% yield).

### Step 2):

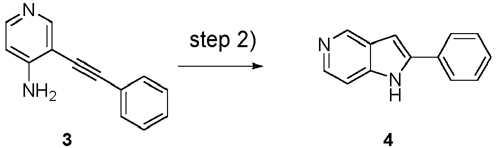

Compound 3 (3.5 g, 17.9 mmol) was dissolved in dioxane (100 mL), and a solution of potassium tert-butoxide in tetrahydrofuran (1 mol, 53.8ml) was added. The mixture was replaced with N₂ three times, and stirred at 110°C for 16 h, new spot was detected using plate. The reaction mixture was diluted with water (200 ml) and extracted with ethyl acetate (10 mL x 3). The combined organic phase was washed with saturated brine (300 mL), dried over anhydrous sodium sulfate, filtered, and concentrated under reduced pressure to remove organic solvent. The residue was purified by column chromatography (methanol: dichloromethane=0-10%) to afford compound 4 (2.2 g, 62.8 % yield).

### Step 3):

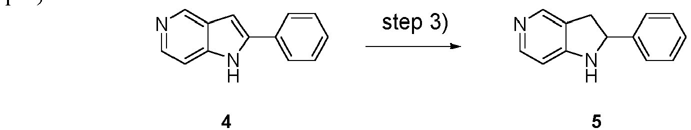

Compound 4 (800 mg, 4.12 mmol) was dissolved in acetic acid (50 mL), palladium carbon (100 mg,) was added. The mixture was stirred at 80 °C for 16 h under H₂ condition, new spot was detected using plate. The reaction mixture was filtered with diatomite, the filtrate was dried, the residue was purified by preparation purification to afford compound 5 (100 mg, 12.3% yield).

### Step 4):

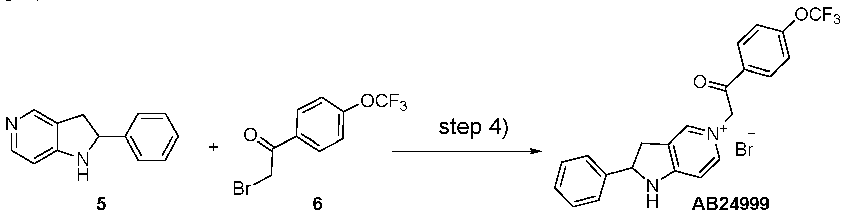

Compound 5 (70 mg, 0.35 mmol) was dissolved in acetone (10 mL), and compound 6 (286 mg, 0.76 mmol) and sodium acetate (143 mg, 1.75 mmol) were added.The mixture was replaced with N₂ three times, and stirred at 80°C for 2 h, new spot was detected using plate. The reaction mixture was concentrated under reduced pressure to remove organic solvent. The residue was purified by thin layer chromatography to afford compound AB24999 (21.8 mg, 15.5 % yield).

¹H NMR (400 MHz, DMSO-d6) δ 9.48 (s, 1H), 8.14 (d, J = 7.5 Hz, 2H), 8.08 (d, J = 7.1 Hz, 1H), 7.95 (s, 1H), 7.62 (d, J = 8.0 Hz, 2H), 7.40 (d, J = 6.7 Hz, 2H), 7.35 (s, 3H), 6.85 (d, J = 6.8 Hz, 1H), 5.93 (s, 2H), 5.39 (s, 1H), 3.76 - 3.66 (m, 1H), 2.99 (d, J = 18.1 Hz, 1H).

MS-ESI: Calculated.: [M-Br]⁺ 399.39, Found: 399.10.

### Example 84

### Synthesis of compound AB24988

### Step 1:

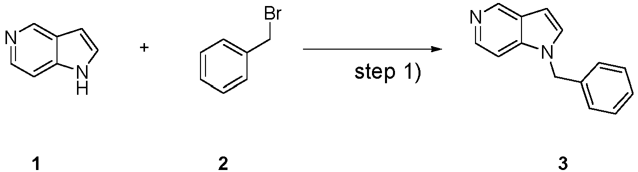

Compound 1 (100 mg, 0.85 mmol) was dissolved in dry N,N-dimethylformamide (10 mL), NaH (68 mg, 1.69 mmol) was added in ice bath. The mixture was stirred for 10 min in ice bath, compound 2 (145 mg, 0.85 mmol) was added. The mixture was replaced with N₂ three times, and was stirred at room temperature for 5 h, new spot was detected using plate. The reaction mixture was diluted with water (20 ml) and extracted with ethyl acetate (15 ml x 3). The combined organic phase was washed with saturated brine (20mL), dried over anhydrous sodium sulfate, filtered and concentrated under reduced pressure to remove organic solvent. The residue was purified by thin layer chromatography (methanol: dichloromethane=0-10%) to afford compound 3 (90 mg, 50.90 % yield).

### Step 2:

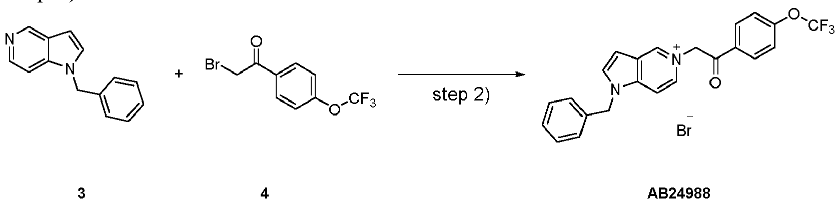

Compound 3 (100 mg, 0.48 mmol) was dissolved in acetonitrile (10 mL), and compound 4 (136 mg, 0.48 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-10%) to afford compound AB24988 (50 mg, 25.34 % yield).

¹H NMR (400 MHz, DMSO-d6) δ 9.26 (s, 1H), 8.46 (s, 1H), 8.35 (s, 1H), 8.18 (d, *J=* 8.9 Hz, 3H), 7.64 (d, *J=* 7.0 Hz, 2H), 7.34 (s, 5H), 7.19 (s, 1H), 6.39 (s, 2H), 5.67 (s, 2H).

MS-ESI: Calculated.: [M-Br]⁺ 411.40, Found: 411.30.

### Example 85

### Synthesis of compound AB27149

### Step 1):

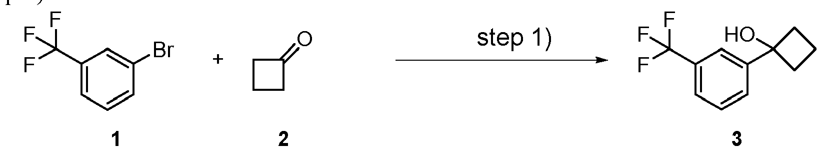

Compound 1 (22 g, 100 mmol) was dissolved in tetrahydrofuran (200 mL), compound 2 (10.5 g, 150 mmol) was added, n-butyllithium (80 mL, 2.5 mol/L) was added dropwise at -78°C. The mixture was stirred at room temperature for 16 h, new spot was detected using plate. The reaction mixture was concentrated under reduced pressure to remove organic solvent, the residue was purified by column chromatography (petroleum ether: ethyl acetate=50:1) to afford compound 3 (13 g, 60.3 % yield).

### Step 2:

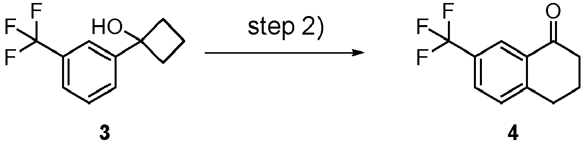

Compound 3 (6.48 g, 30 mmol) was dissolved in dichloromethane (100 mL) and water (100 mL), and AgNO₃ (1 g, 6 mmol) and potassium persulfate (20 g, 270 mmol) were added. The mixture was stirred at room temperature for 16 h, new spot was detected using plate. The reaction mixture was concentrated under reduced pressure to remove organic solvent, the residue was purified by column chromatography (petroleum ether: ethyl acetate=0-10%) to afford compound 4 (584 mg, 9.0 % yield).

### Step 3):

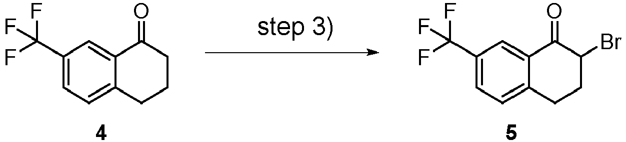

Compound 4 (584 mg, 1.98 mmol) was dissolved in dichloromethane (20 mL), and C₈H₁₈N₂O₂HBr₃ (820 mg, 1.98 mmol) was added. The mixture was stirred at room temperature for 1 h, new spot was detected using plate. The reaction mixture was dried, the residue was purified by thin layer chromatography to afford compound 5 (670 mg, 94.7% yield)

### Step 4:

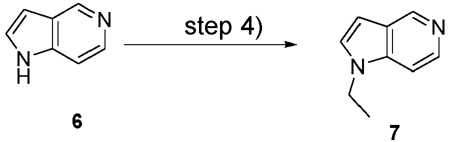

Compound 6 (236 mg, 2.0 mmol) was dissolved in N,N-dimethylformamide (10 mL), NaH (24 mg, 1.0 mmol) was added in ice bath. The mixture was replaced with N₂ three times, and the mixture was stirred for 30 min in ice bath, and iodoethane (374 mg, 2.4 mmol) was slowly added in ice bath. The mixture was stirred at room temperature for 90 min, new spot was detected using plate. The reaction mixture was diluted with ice water (20 ml), and concentrated under reduced pressure to remove organic solvent, the crude product was filtered with methanol, and the filtrate was concentrated under reduced pressure to remove organic solvent, the residue was purified by thin layer chromatography (ethyl acetate: petroleum ether=0-30%) to afford compound 7 (185 mg, 62.9 % yield).

### Step 5):

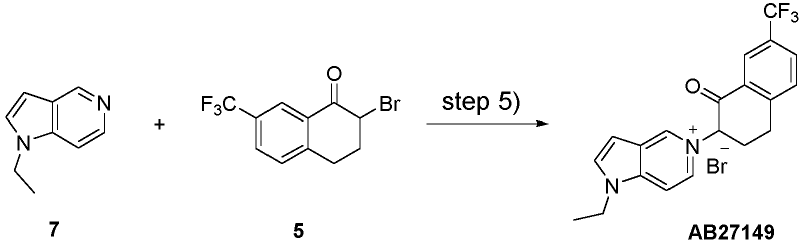

Compound 7 (50 mg, 0.34 mmol) was dissolved in acetonitrile (10 mL), and compound 5 (100 mg, 0.34 mmol) was added. The mixture was stirred at 80°C for overnight, new spot was detected using plate. The reaction mixture was concentrated under reduced pressure to remove organic solvent, the residue was purified by thin layer chromatography (methanol: dichloromethane=10%) to afford compound AB27149 (13.1 mg, 10.7 % yield).

¹H NMR (400 MHz, CD₃OD-d4) δ 9.23 (s, 1H), 8.44 (d, J = 6.6 Hz, 1H), 8.28 (s, 1H), 8.14 (d, J = 6.6 Hz, 1H), 7.94 (s, 2H), 7.70 (d, J = 7.7 Hz, 1H), 7.10 (s, 1H), 6.05 (d, J = 13.8 Hz, 1H), 4.48 (d, J = 7.0 Hz, 2H), 3.55 (d, J = 13.6 Hz, 1H), 3.43 (d, J = 16.8 Hz, 1H), 3.07 (d, J = 10.6 Hz, 1H), 2.81 (d, J = 10.1 Hz, 1H), 1.54 (dd, J = 6.9 Hz, 3H).

MS-ESI: Calculated.: [M-Br]⁺ 359.36, Found: 359.25.

### Example 86

### Synthesis of compound AB27118

### Step 1):

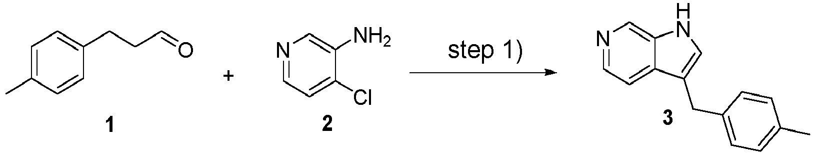

Compound 1 (300 mg, 4.05 mmol) was dissolved in N,N-dimethylacetamide (20ml), and compound 2 (259 mg, 2.02 mmol), triethylenediamine (585.8 mg, 2.02 mmol), bis(dibenzylideneacetone)palladium (92.4 mg, 0.1 mmol) and tri-tert-butylphosphine tetrafluoroborate (678 mg, 6.06 mmol) and bis(dibenzylideneacetone)palladium (92.4 mg, 0.1 mmol) were added. The mixture was stirred at 110°C for 16 h, new spot was detected using plate. The reaction mixture was extracted with water and ethyl acetate (10 mL). The combined organic phase was concentrated under reduced pressure to remove organic solvent, the residue was purified by column chromatography (methanol: dichloromethane=10%) to afford compound 3 (100 mg, 34.8% yield).

### Step 2:

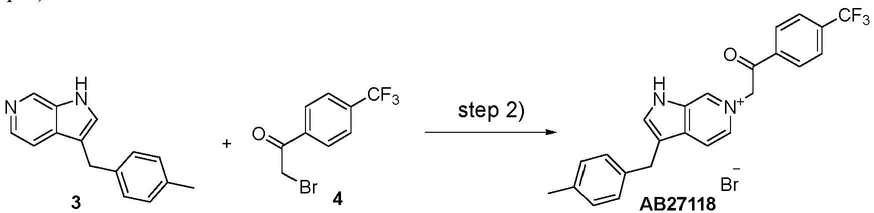

Compound 3 (50 mg, 0.225 mmol) was dissolved in acetonitrile (10 mL), and compound 4 (160 g, 0.56 mmol) was added. The mixture was replaced with N₂ three times, and stirred at 110°C for 2h, new spot was detected using plate. The reaction mixture was filtered, and concentrated under reduced pressure to remove organic solvent. The residue was purified by thin layer chromatography (methanol: dichloromethane=0-10%) to afford compound AB27118 (22.9 mg, 24.8% yield).

¹HNMR (400 MHz, DMSO-d6) δ 9.14 (s, 1H), 8.24 (s, 3H), 8.18 (s, 1H), 8.04 (s, 3H), 7.19 (s, 2H), 7.09 (s, 2H), 6.41 (s, 2H), 4.13 (s, 2H), 2.23 (s, 3H).

MS-ESI: Calculated.: [M-Br]⁺ 409.42, Found: 409.20.

### Example 87

### Synthesis of compound AB24987

### Step 1:

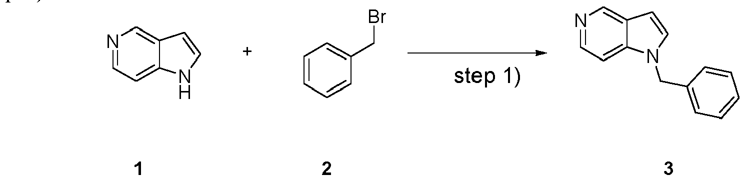

Compound 1 (100 mg, 0.85 mmol) was dissolved in dry N,N-dimethylformamide (10 mL), NaH (68 mg, 1.69 mmol) was added in ice bath. The mixture was stirred for 10 min in ice bath, compound 2 (145 mg, 0.85 mmol) was added. The mixture was replaced with N₂ three times, and was stirred at room temperature for 5 h, new spot was detected using plate. The reaction mixture was diluted with water (20 ml) and extracted with ethyl acetate (15 ml x 3). The combined organic phase was washed with saturated brine (20 ml), dried over anhydrous sodium sulfate, filtered and concentrated under reduced pressure to remove organic solvent. The residue was purified by thin layer chromatography (methanol: dichloromethane=0-10%) to afford compound 3 (90 mg, 50.90 % yield).

### Step 2):

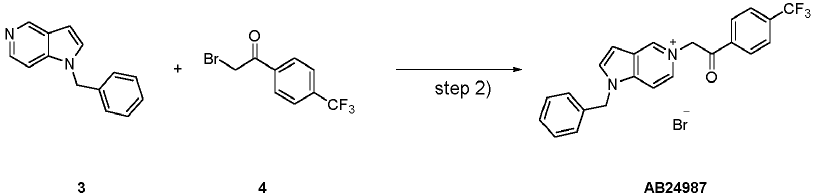

Compound 3 (100 mg, 0.48 mmol) was dissolved in acetonitrile (10 mL), and compound 4 (136 mg, 0.48 mmol) was added. The mixture was replaced with N₂ three times, and stirred at room temperature for overnight, new spot was detected using plate. The reaction mixture was concentrated under reduced pressure to remove organic solvent. The residue was purified by column chromatography (methanol: dichloromethane=0-10%) to afford compound AB24987 (50 mg, 26.37 % yield).

¹H NMR (400 MHz, DMSO-d6) δ 9.29 (s, 1H), 8.48 (s, 1H), 8.36 (d, *J=* 5.3 Hz, 1H), 8.24 (s, 2H), 8.18 (s, 1H), 8.04 (d, *J=* 5.9 Hz, 2H), 7.35 (s, 5H), 7.21 (s, 1H), 6.44 (s, 2H), 5.68 (s, 2H).

MS-ESI: Calculated.: [M-Br]⁺ 395.40, Found: 395.25.

### Example 88

### Synthesis of compound AB27148

### Step 1:

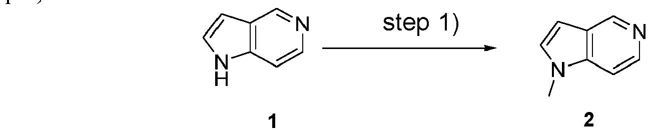

Compound 1 (500 mg, 4.32 mmol) was dissolved in dry N,N-dimethylformamide (10 mL), NaH (304.56 mg, 12.7 mmol) was added in ice bath. The mixture was replaced with N₂ three times, and stirred for 30 min in ice bath, iodomethane (723.89 mg, 5.1 mmol) was slowly injected in ice bath, then the mixture was stirred at room temperature for 90 min, new spot was detected using plate. The reaction mixture was diluted with water (20 ml) and concentrated under reduced pressure to remove organic solvent, the crude product was filtered with methanol, and the filtrate was concentrated under reduced pressure to remove organic solvent, the residue was purified by thin layer chromatography (methanol: dichloromethane=10%) to afford compound 2 (479 mg, 85.78 % yield).

### Step 2:

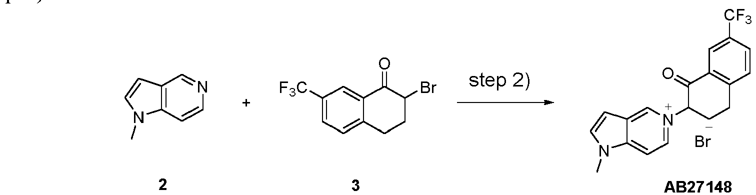

Compound 2 (100 mg, 0.76 mmol) was dissolved in acetonitrile (10 mL), and compound 3 (200mg, 0.76 mmol) was added. The mixture was stirred at 80°C for overnight, new spot was detected using plate. The reaction mixture was concentrated under reduced pressure to remove organic solvent, the residue was purified by thin layer chromatography (methanol: dichloromethane=0-10%) to afford compound AB27148 (40 mg, 18.81% yield).

¹H NMR (400 MHz, DMSO-d6)δ 9.36 (s, 1H), 8.57 (d, *J* = 6.7 Hz, 1H), 8.25 (d, *J* = 6.5 Hz, 1H), 8.14 (s, 1H), 8.07 (d, *J* = 7.7 Hz, 1H), 8.00 (s, 1H), 7.77 (d, *J* = 7.9 Hz, 1H), 7.11 (s, 1H), 6.22 (d, *J=* 10.6 Hz, 1H), 4.01 (s, 3H), 3.40 (s, 2H), 3.08 (s, 1H), 2.70 (s, 1H).

MS-ESI: Calculated.: [M-Br]⁺ 345.34, Found: 345.05.

### Example 89

### Synthesis of compound AB24852

### Step 1):

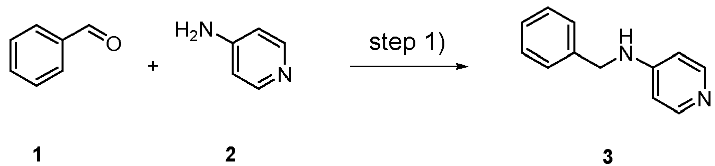

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

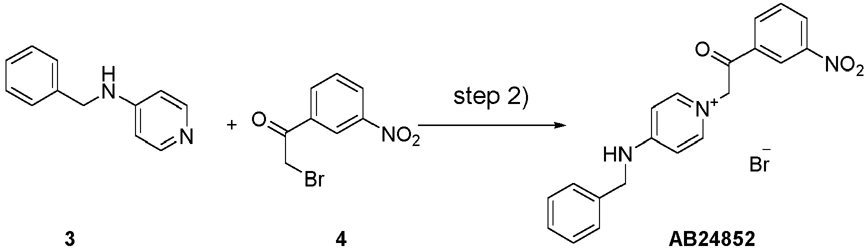

Compound 3 (368 mg, 2 mmol) and compound 4 (1.2 mg, 6 mmol) were dissolved in acetonitrile (10 mL). The mixture was stirred at 80°C for 16 h, new spot was detected using plate. The mixture was cooled to room temperature, new spot was detected using plate, filtered, and concentrated under reduced pressure to remove organic solvent, the residue was purified by thin layer chromatography (methanol: dichloromethane=0-10%) to afford compound AB24852 (107.3mg, 12.5% yield).

### Compound AB24852

¹H NMR (400 MHz, DMSO-d6) δ 9.61 (s, 1H), 8.69 (s, 1H), 8.56 (d, J = 8.2 Hz, 1H), 8.42 (d, J = 7.5 Hz, 1H), 8.19 (d, J = 6.7 Hz, 1H), 8.09 (d, J = 6.8 Hz, 1H), 7.93 (t, J = 7.8 Hz, 1H), 7.39 (s, 4H), 7.31 (s, 1H), 7.11 (d, J = 6.8 Hz, 1H), 7.04 (d, J = 6.6 Hz, 1H), 6.06 (s, 2H), 4.58 (d, J = 4.6 Hz, 2H).

MS-ESI: Calculated.: [M-Br]⁺ 348.37, Found: 348.30.

### Example 90

### Synthesis of compound AB24807A

### Step 1):

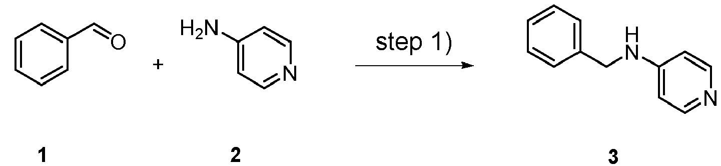

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

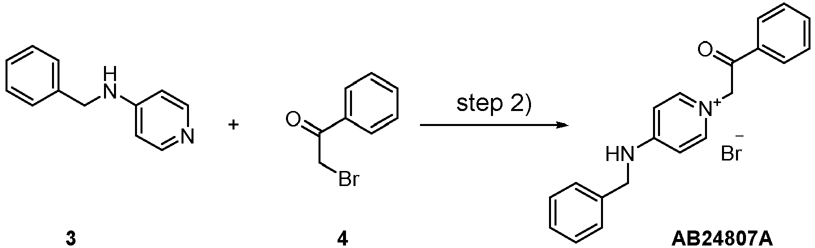

Compound 3 (368 mg, 2 mmol) and compound 4 (1.2 g, 6 mmol) were dissolved in acetonitrile (10 mL). The mixture was stirred at 80 °C for 16 h, new spot was detected using plate. The reaction mixture was cooled to room temperature, and dried, the residue was purified by preparation purification to afford compound AB24807A (22.9 mg, 3.7% yield).

### Compound AB24807A

1H NMR (400MHz, DMSO-d6) δ 9.41 (s, 1H), 8.15 (d, J = 7.2 Hz, 1H), 8.04 (d, J = 7.4 Hz, 1H), 7.97 (d, J = 7.7 Hz, 2H), 7.70 (d, J = 6.7 Hz, 1H), 7.58 (t, J = 7.5 Hz, 2H), 7.35 (d, J = 4.1 Hz, 4H), 7.28 (d, J = 3.7 Hz, 1H), 7.01 (dd, J = 16.5, 7.3 Hz, 2H), 5.92 (s, 2H), 4.54 (d, J = 5.5 Hz, 2H).

MS-ESI: Calculated.: [M-Br]⁺ 330.41, Found: 330.25.

### Example 91

### Synthesis of compound AB24803A

### Step 1):

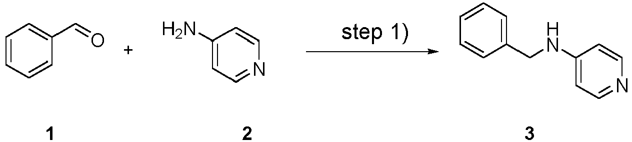

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

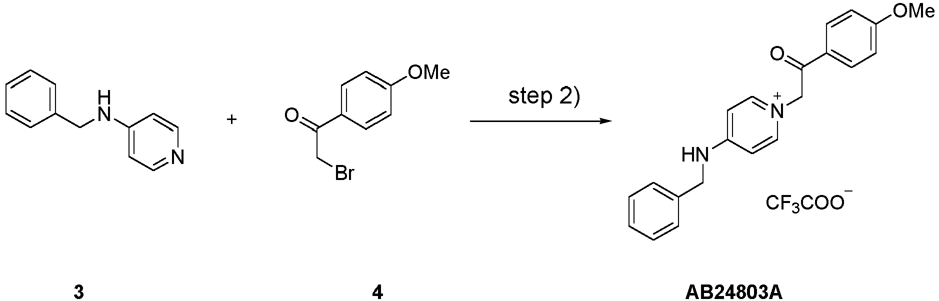

Compound 3 (500 mg, 2.72 mmol) and compound 4 (1.23 g, 5.42 mmol) were dissolved in acetonitrile (10 mL). The mixture was stirred at 80°C for 16 h, new spot was detected using plate. The reaction mixture was cooled to room temperature, and dried, the residue was purified by preparation purification to afford compound AB24803A (100 mg, 11.08 % yield).

### Compound AB24803A

¹H NMR (400 MHz,DMSO,d6) δ 9.19 (s, 0H), 8.13 (s, 0H), 8.03 (s, 0H), 7.96 (d, *J* = 8.1 Hz, 1H), 7.36 (s, 2H), 7.11 (d, J = 8.2 Hz, 1H), 6.99 (s, 1H), 5.84 (s, 1H), 4.56 (s, 1H), 3.85 (s, 3H).

MS-ESI: Calculated.: [M-CF₃COO]⁺ 333.16, Found: 330.30.

### Example 92

### Synthesis of compound AB24810A

### Step 1:

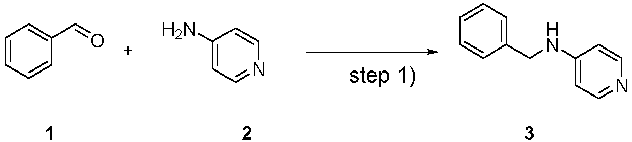

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

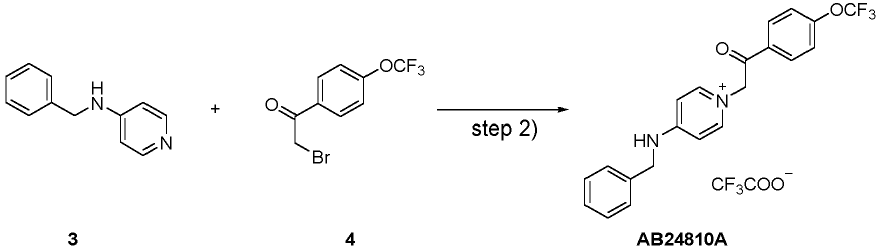

Compound 5 (500 mg, 2.72 mmol) and compound 4 (1.5 g, 5.43 mmol) were dissolved in acetonitrile (10 mL). The mixture was replaced with N₂ three times, and stirred at 80 °C for 16 h, The acetonitrile was removed by vacuum distillation, the residue was purified by preparation purification to afford compound AB24810A (120 mg, 26.8% yield).

### Compound AB24810A

¹H NMR (399 MHz, DMSO-d6) δ 9.25 (s, 1H), 8.15 (d, *J* = 6.3 Hz, 1H), 8.04 (d, *J* = 6.8 Hz, 1H), 7.96 (d, *J* = 7.4 Hz, 1H), 7.63 (s, 2H), 7.55 (s, 1H), 7.37 (d, *J* = 6.3 Hz, 4H), 7.30 (s, 1H), 7.01 (s, 2H), 5.80 (s, 2H), 4.56 (d, *J* = 5.8 Hz, 2H).

MS-ESI: Calculated.: [M- CF₃COO]⁺ 387.38, Found: 387.30.

### Example 93

### Synthesis of compound AB24805A

### Step 1:

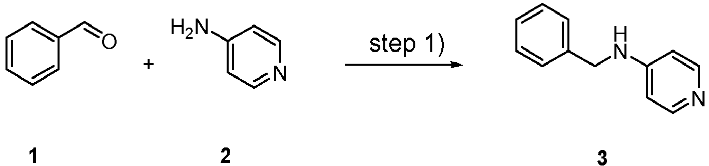

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2:

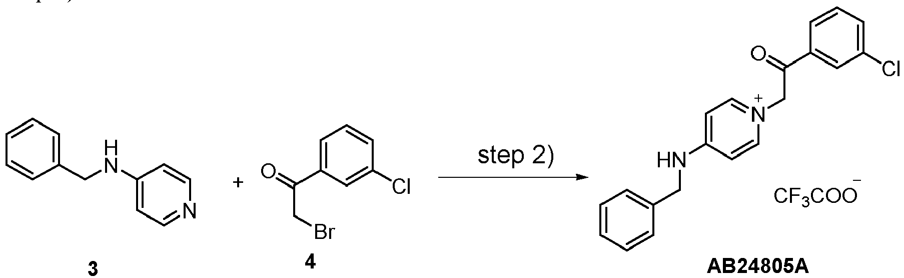

Compound 3 (552 mg, 3 mmol) was dissolved in acetonitrile (20 mL), and compound 4 (2.1 g, 9 mmol) was added. The mixture was replaced with N₂ three times, and stirred at 80°C for overnight, new spot was detected using plate. The reaction mixture was concentrated under reduced pressure to remove organic solvent. The residue was purified by preparation purification to afford compound AB24805A (24.4 mg, 2.4 % yield).

### Compound AB24805A

1H NMR (400 MHz, DMSO-d6) δ 9.24 (s, 1H), 8.13 (d, J = 7.0 Hz, 1H), 8.01 (s, 2H), 7.93 (d, J = 7.6 Hz, 1H), 7.80 (s, 1H), 7.64 (s, 1H), 7.37 (d, J = 6.6 Hz, 4H), 7.30 (s, 1H), 7.01 (s, 2H), 5.89 (s, 2H), 4.56 (d, J = 5.9 Hz, 2H).

MS-ESI: Calculated.: [M-CF₃COO]⁺ 337.82, Found: 337.30.

### Example 94

### Synthesis of compound AB24808A

### Step 1):

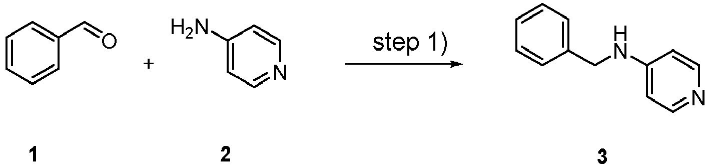

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

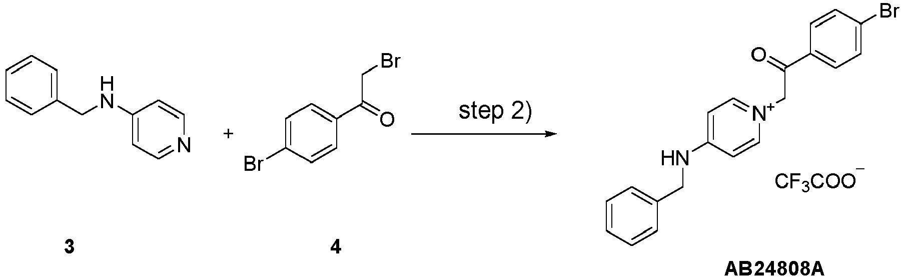

Compound 3 (500 mg, 2.72 mmol) and compound 4 (1.5 g, 5.43 mmol) were dissolved in acetonitrile (10 mL). The mixture was stirred at 80°C for 16 h, new spot was detected using plate. The mixture was cooled to room temperature, dried, and the residue was purified by preparation purification to afford compound AB24808A (120 mg, 11.6% yield).

### Compound AB24808A

¹H NMR (400MHz, DMSO-d6) δ 9.24 (s, 1H), 8.13 (d, J = 6.8 Hz, 1H), 8.01 (d, J = 7.7 Hz, 1H), 7.91 (d, J = 8.4 Hz, 2H), 7.83 (d, J = 6.9 Hz, 2H), 7.33 (d, J = 23.6 Hz, 5H), 7.00 (s, 2H), 5.87 (s, 2H), 4.56 (d, J = 5.7 Hz, 2H).

MS-ESI: Calculated.: [M- CF₃COO]⁺ 382.27, Found: 382.95.

### Example 95

### Synthesis of compound AB24845A

### Step 1:

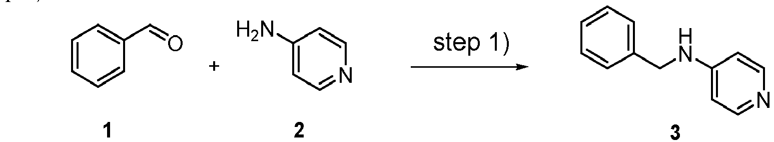

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2:

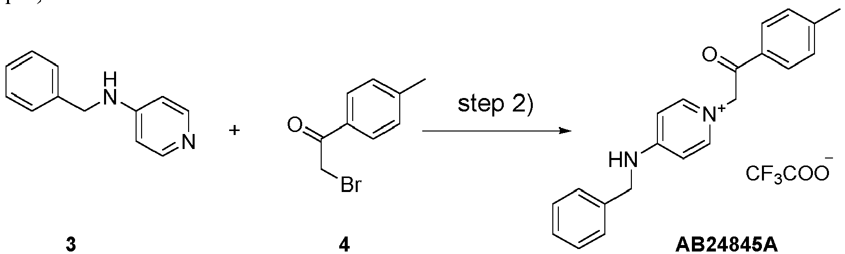

Compound 3 (184 mg, 1 mmol) was dissolved in acetonitrile (10 mL), and compound 4 (426mg, 2 mmol) was added. The mixture was replaced with N₂ three times, and stirred at 80°C for overnight, new spot was detected using plate. The reaction mixture was concentrated under reduced pressure to remove organic solvent, the residue was purified by preparation purification to afford compound AB24845A (23.2 mg, 5.3 % yield).

### Compound AB24845A

¹H NMR (400MHz, DMSO-d6) δ 9.24 (s, 1H), 8.16 (d, J = 6.8 Hz, 1H), 8.05 (d, J = 6.7 Hz, 1H), 7.90 (d, J = 8.0 Hz, 2H), 7.42 (d, J = 7.6 Hz, 2H), 7.38 (s, 4H), 7.32 (s, 1H), 7.01 (s, 2H), 5.89 (s, 2H), 4.57 (d, J = 5.6 Hz, 2H), 2.41 (s, 3H).

MS-ESI: Calculated.: [M- CF₃COO]⁺ 371.40, Found: 317.35..

### Example 96

### Synthesis of compound AB24955

### Step 1:

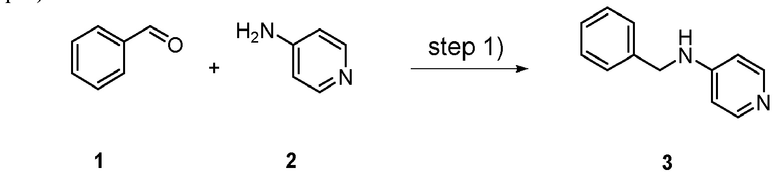

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

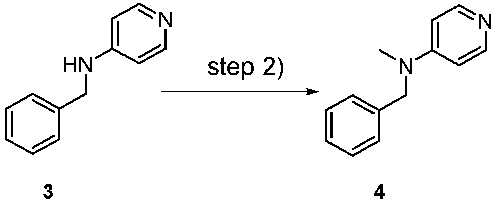

Compound 3 (368 mg, 2 mmol) was dissolved in N,N-dimethylformamide (8 mL), NaH (120mg, 5 mmol) was added in ice bath. The mixture was stirred at 0 °C for 1 h, and iodomethane (284 mg, 2 mmol) was slowly injected in ice bath, then the mixture was stirred at room temperature for overnight, new spot was detected using plate. The reaction mixture was extracted with ethyl acetate (10 mL), the organic phase was concentrated under reduced pressure, the residue was purified by colomn chromatography (methanol: dichloromethane=10%) to afford compound 4 (100 mg, 25.25 % yield).

### Step 3:

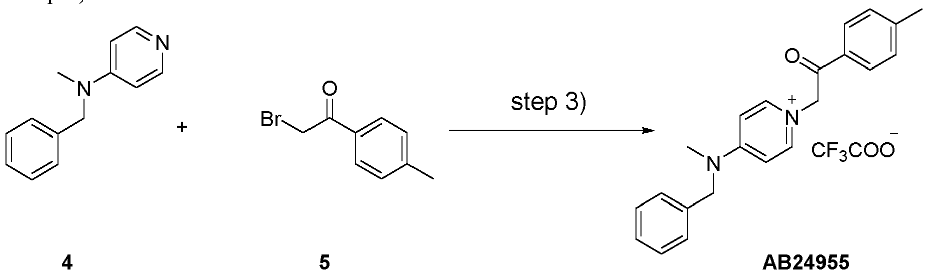

Compound 4 (100 mg, 1.07 mmol) was dissolved in acetonitrile (5 mL), and compound 5 (80 mg, 0.40 mmol) was added. The mixture was replaced with N₂ three times, and stirred at room temperature for overnight, new spot was detected using plate. The reaction mixture was dried, and the residue was purified by preparation purification to afford compound AB24955 (24.8 mg, 5.2% yield).

### Compound AB24955

¹H NMR (400 MHz,DMSO-d6) δ 8.23 (s, 1H), 8.13 (s, 1H), 7.90 (d, J = 7.3 Hz, 2H), 7.41 (d, J = 7.7 Hz, 2H), 7.36 (d, J = 6.8 Hz, 2H), 7.31 (d, J = 6.6 Hz, 1H), 7.24 (d, J = 6.7 Hz, 2H), 7.18 (d, J = 6.2 Hz, 2H), 5.91 (s, 2H), 4.89 (s, 2H), 3.28 (s, 3H), 2.40 (s, 3H).

MS-ESI: Calculated.: [M- CF₃COO]⁺ 331.43, Found: 331.30.

### Example 97

### Synthesis of compound AB24809A

### Step 1:

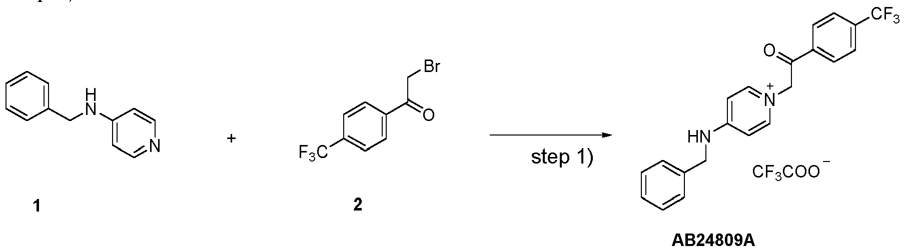

Compound 1 (500 mg, 2.72 mmol) and compound 2 (1.5g, 5.43 mmol) were dissolved in acetonitrile (10 mL) The mixture was stirred at 80 °C for 16 h, new spot was detected using plate. The reaction mixture was cooled to room temperature, and dried, the residue was purified by preparation purification to afford compound AB24809A (120 mg, 11.89% yield).

### Compound AB24809A

¹H NMR (400 MHz, DMSO-d6) δ 9.25 (s, 1H), 8.17 (d, J = 7.4 Hz, 3H), 7.99 (d, J = 7.9 Hz, 3H), 7.36 (s, 5H), 7.01 (s, 2H), 5.94 (s, 2H), 4.56 (s, 2H).

MS-ESI: Calculated.: [M- CF₃COO]⁺ 3713.38, Found: 371.30.

### Example 98

### Synthesis of compound AB24983

### Step 1):

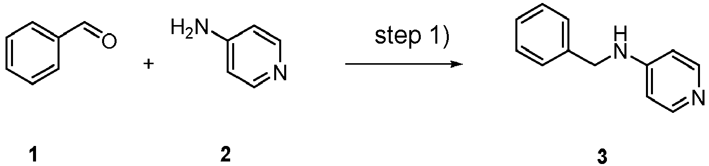

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2:

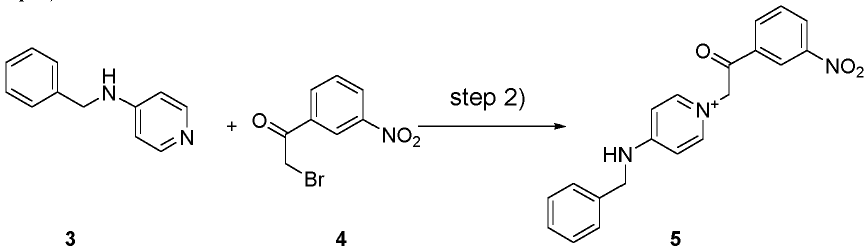

Compound 3 (368 mg, 2 mmol) and compound 4 (1.2 g, 6 mmol) were dissolved in acetonitrile (10 mL). The mixture was stirred at 80°C for 16 h, new spot was detected using plate. The reaction mixture was cooled to room temperature, new spot was detected using plate, filtered, and concentrated under reduced pressure to remove organic solvent, the residue was purified by thin layer chromatography (methanol: dichloromethane=0-10%) to afford compound 5 (107.3 mg, 12.5 % yield).

### Step 3:

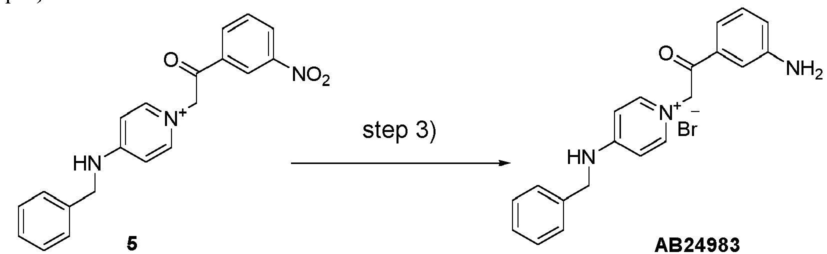

Compound 5 (347 mg, 1 mmol) was dissolved in anhydrous ethanol (4 mL) and water (1 mL), and reduced iron powder (560 mg, 10 mmol) and ammonium chloride (324 mg, 6 mmol) were added. The mixture was stirred at 80°C for 3 h, new spot was detected using plate. The reaction mixture was filtered, the filtrate was extracted with ethyl acetate (30 mL x 3). The combined organic phase was washed with saturated brine (30 mL), dried over anhydrous sodium sulfate, and concentrated under reduced pressure to remove organic solvent. The residue was purified by column chromatography (methanol: dichloromethane=0-30%) to afford compound AB24983 (10.6 mg, 2.6% yield).

### Compound AB24983

¹H NMR (400 MHz, DMSO-d6) δ 9.43 (s, 1H), 8.15 (d, J = 6.8 Hz, 1H), 8.04 (d, J = 6.4 Hz, 1H), 7.37 (s, 4H), 7.30 (s, 1H), 7.21 (d, J = 7.3 Hz, 1H), 7.12 (s, 2H), 7.04 (d, J = 6.1 Hz, 1H), 6.99 (d, J = 6.9 Hz, 1H), 6.87 (d, J = 7.2 Hz, 1H), 5.83 (s, 2H), 5.49 (s, 2H), 4.55 (s, 2H).

MS-ESI: Calculated.: [M-Br]⁺ 318.39, Found: 318.30.

### Example 99

### Synthesis of compound AB24813

### Step 1:

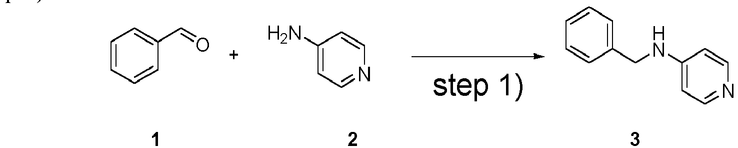

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2:

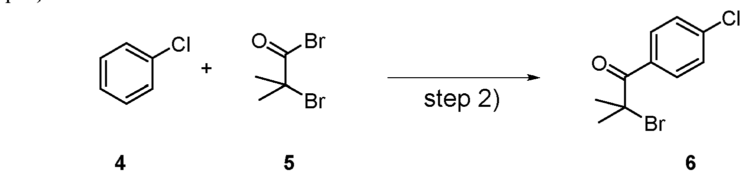

Compound 4 (1.0 g, 8.88 mmol) was dissolved in dichloromethane (30 ml), and aluminum trichloride (2.3 g, 17.76 mmol) and compound 5 (2.8 g, 13.3 mmol) were added in an ice bath. The mixture was stirred for 1 h in ice bath, new spot was detected using TLC. The reaction mixture was diluted with ice water (50 mL) and extracted with dichloromethane (30 mL x 3). The combined organic phase was washed with saturated brine (50 mL), dried over anhydrous sodium sulfate, filtered and concentrated under reduced pressure to remove organic solvent to afford compound 6 (300 mg, 12.94% yield).

### Step 3):

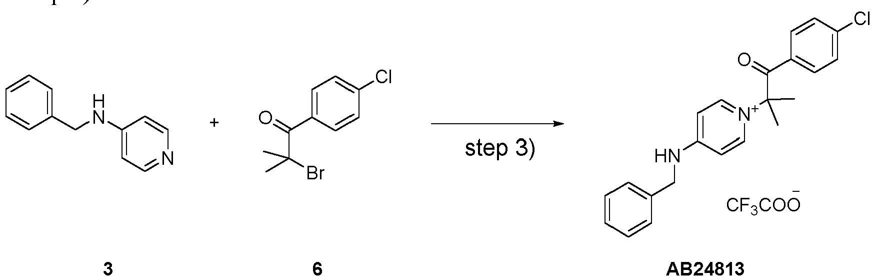

Compound 6 (400 mg, 1.53 mmol) and compound 3 (300 mg, 1.64 mmol) were dissolved in acetonitrile (10 mL). The mixture was replaced with N₂ three times, and stirred at room temperature for 5 h, new spot was detected using plate. The reaction mixture was concentrated under reduced pressure to remove organic solvent. The residue was purified by column chromatography (methanol: dichloromethane=0-10%) to afford compound AB24813 (24 mg, 3.1% yield).

### Compound AB24813

¹H NMR (400 MHz, CDCl3) δ 11.09 (s, 1H), 8.17 (s, 1H), 7.90 (s, 1H), 7.66 (s, 1H), 7.46 (s, 2H), 7.36 - 7.24 (m, 8H), 6.50 (s, 1H), 4.54 (s, 2H), 1.92 (s, 6H).

MS-ESI: Calculated.: [M- CF₃COO]⁺ 365.88, Found: 365.25.

### Example 100

### Synthesis of compound AB24814

### Step 1):

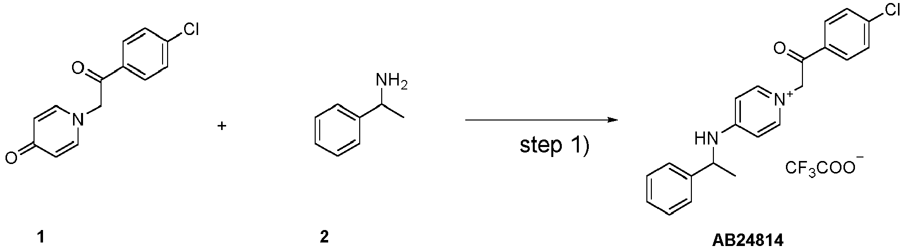

Compound 1 (100 mg, 0.403 mmol) was dissolved in phosphorus oxychloride (10 mL), the mixture was stirred at 110 °C for 3h, detected by LCMS, cooled and concentrated. Compound 2 (96 mg, 0.806 mmol) and methanol (20 mL) were added in ice bath, the mixture was reacted at room temperature, and concentrated, the residue was purified by preparation purification to afford compound AB24814 (41.2 mg, 28.1% yield).

### Compound AB24814

¹H NMR (400 MHz, DMSO-d6) δ 9.25 (d, J = 6.9 Hz, 1H), 8.05 (d, J = 7.4 Hz, 1H), 8.03 - 7.94 (m, 3H), 7.67 (d, J = 7.8 Hz, 2H), 7.36 (d, J = 7.6 Hz, 4H), 7.26 (s, 1H), 7.01 (s, 1H), 6.79 (s, 1H), 5.85 (s, 2H), 4.89 (s, 1H), 1.51 (d, J = 6.3 Hz, 3H).

MS-ESI: Calculated.: [M- 351.85, Found: 351.00.

### Example 101

### Synthesis of compound AB24817

### Step 1):

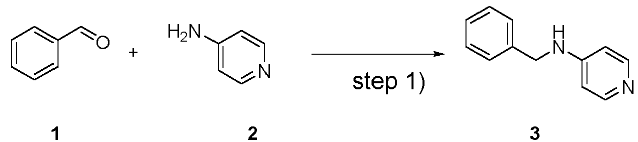

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

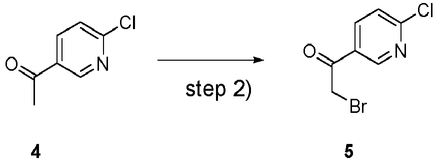

Compound 4 (500 mg, 3.21 mmol) and bis(N,N-dimethylacetamide) hydrogen dibromobromate (1.3 g, 3.21 mmol) were dissolved in methanol (20 mL). The mixture was replaced with N₂ three times, and stirred at room temperature for 16 h, new spot was detected using plate. The reaction mixture was concentrated under reduced pressure to remove organic solvent, the residue was purified by column chromatography (ethyl acetate: petroleum ether=0-5%) to afford compound 5 (510 mg, 67.28% yield).

### Step 3):

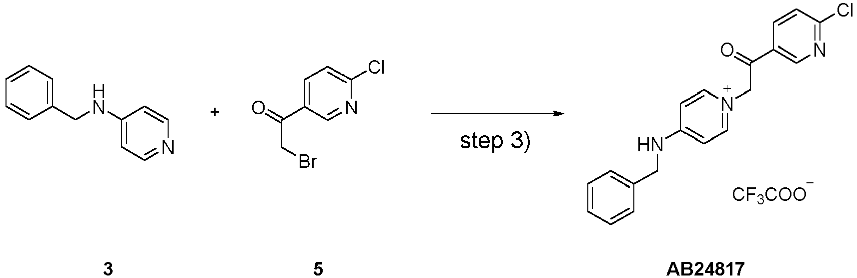

Compound 5 (350 mg, 1.5 mmol) and compound 3 (184 mg, 1.0 mmol) were dissolved in acetonitrile (10 mL). The mixture was replaced with N₂ three times, and stirred at room temperature for 5 h, new spot was detected using plate. The reaction mixture was concentrated under reduced pressure to remove organic solvent, the residue was purified by column chromatography (methanol: dichloromethane=0-10%) to afford compound AB24817 (47 mg, 14% yield).

### Compound AB24817

¹H NMR (400 MHz, DMSO-d6) δ 9.27 (s, 1H), 9.00 (s, 1H), 8.35 (d, *J=* 8.2 Hz, 1H), 8.11 (d, *J=* 6.7 Hz, 1H), 8.00 (d, *J=* 6.9 Hz, 1H), 7.78 (d, *J=* 8.2 Hz, 1H), 7.36 (s, 4H), 7.29 (s, 1H), 7.01 (s, 2H), 5.88 (s, 2H), 4.55 (d, *J=* 5.3 Hz, 2H).

MS-ESI: Calculated.: [M- CF₃COO]⁺ 338.81, Found: 338.20.

### Example 102

### Synthesis of compound AB24880

### Step 1:

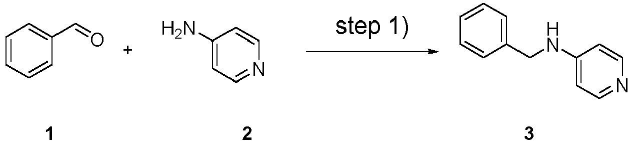

Compound 1 (5.6 g, 53.13 mmol) was dissolved in ethanol (10 mL), and compound 2 (5g, 53.13 mmol) was added. The mixture was replaced with N₂ three times, and stirred at 90°C for 2 h, new spot was detected using plate. The reaction mixture was cooled to 0 °C, sodium borohydride (4.01g, 106.2 mmol) was slowly added, and the system naturally rose to room temperature and was stirred for 1 h, new spot was detected using plate. The reaction mixture was diluted with water (200 mL) and extracted with ethyl acetate (200 mL x 3). The combined organic phase was washed with saturated brine (200 mL), dried over anhydrous sodium sulfate, filtered and concentrated under reduced pressure to remove organic solvent. The residue was purified by column chromatography (methanol: dichloromethane=10:1) to afford compound 3 (2.6g, 26.8% yield).

### Step 2):

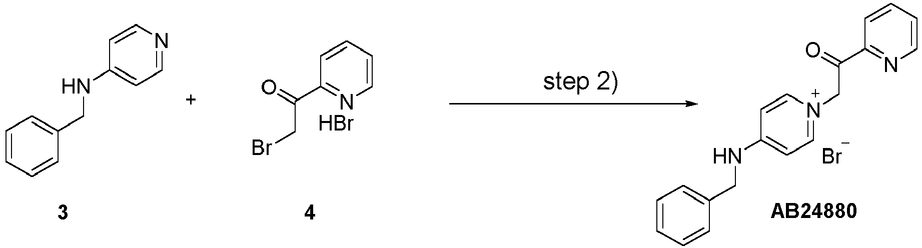

Compound 3 (300 mg, 1.63 mmol) was dissolved in acetonitrile (10 mL), and compound 4 (1.38 mg, 4.89 mmol) and sodium acetate (669 mg, 8.15 mmol) were added. The mixture was replaced with N₂ three times, and stirred at room temperature for 16 h, new spot was detected using liqiuid-MS. The reaction mixture was cooled to room temperature, and dried, the residue was purified by thin layer chromatography to afford compound AB24880 (300 mg, 60.74 % yield)

### Compound AB24880

¹H NMR (400 MHz, DMSO,d6) δ 9.46 (s, 1H), 8.78 (s, 1H), 8.17 (d, *J=* 6.7 Hz, 1H), 8.06 (t, *J* = 7.0 Hz, 2H), 7.99 (d, *J=* 7.5 Hz, 1H), 7.75 (s, 1H), 7.32 (d, *J* = 26.4 Hz, 5H), 7.09 - 6.95 (m, 2H), 5.96 (s, 2H), 4.54 (d, *J* = 5.0 Hz, 2H).

MS-ESI: Calculated.: [M-Br]⁺ 304.14, Found: 304.05.

### Example 103

### Synthesis of compound AB24822

### Step 1:

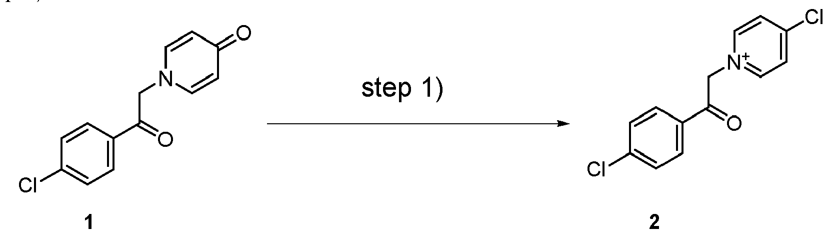

Compound 1 (247 mg, 1 mmol) was dissolved in phosphorus oxychloride (10 mL). The mixture was replaced with N₂ three times, and stirred at 110°C for 3 h, new spot was detected using plate. The reaction mixture was filtered, and concentrated under reduced pressure to afford compound 2 (200 mg, 75.18% yield).

### Step 2):

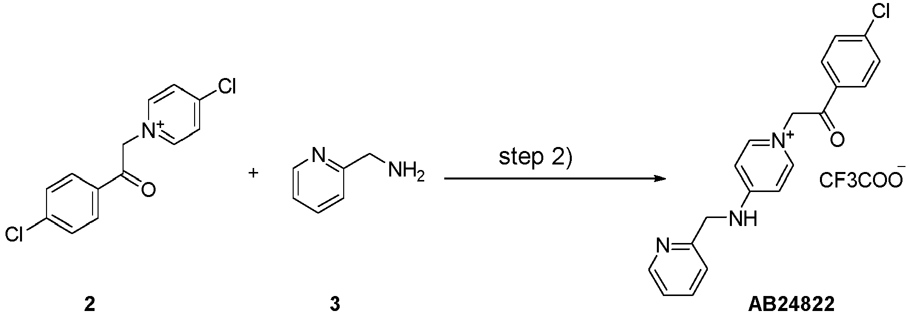

Compound 2 (200mg, 0.8 mmol) was dissolved in anhydrous methanol (10 mL), compound 3 (162.2 mg, 1.5 mmol) and triethylamine (5 mL) were added at 0 °C, the mixture was stirred for 16 h, new spot was detected using plate. The reaction mixture was filtered, and concentrated under reduced pressure to remove organic solvent. The residue was purified by thin layer chromatography (dichloromethane: methanol=8:1) to afford compound AB24822 (42.8 mg, 11.8% yield).

### Compound AB24822

¹H NMR (400 MHz, DMSO-d6) δ 9.31 (s, 1H), 8.56 (s, 1H), 8.13 (s, 1H), 7.99 (d, *J=* 8.5 Hz, 3H), 7.82 (s, 1H), 7.69 (d, *J=* 7.3 Hz, 2H), 7.41 (s, 1H), 7.33 (s, 1H), 7.03 (s, 2H), 5.89 (s, 2H), 4.67 (s, 2H).

MS-ESI: Calculated.: [M-CF₃COO]⁺ 338.81, Found: 338.15.

### Example 104

### Synthesis of compound AB24846

### Step 1:

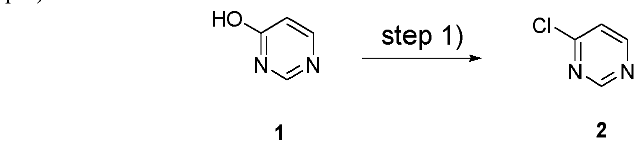

Compound 1 (8g, 83.33 mmol) was dissolved in phosphorus oxychloride (40 mL). The mixture was replaced with N₂ three times, and stirred at 90°C for overnight, new spot was detected using plate, phosphorus oxychloride was dried. Dichloromethane (50 mL) was added in the ice water bath, and ice water (50 mL) was slowly added. The mixture was extracted with dichloromethane (50 mL x 3), The combined organic phase was washed with saturated brine (100 mL), dried over anhydrous sodium sulfate, and concentrated under reduced pressure to remove organic solvent. The residue was purified by column chromatography (ethyl acetate: petroleum ether=30-80%) to afford compound 2 (5 g, 52.17% yield).

### Step 2:

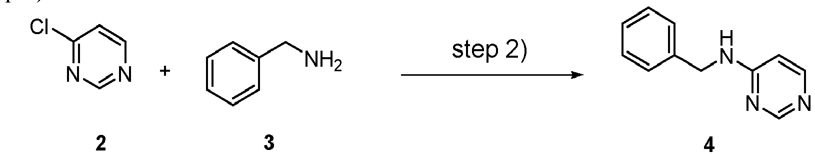

Compound 2 (2.5 g, 21.73 mmol) was dissolved in N,N-dimethylformamide (50ml), and compound 3 (7.43 g, 65.22 mmol) and potassium carbonate (9.01 g, 65.22 mmol) were added. The mixture was replaced with N₂ three times, and stirred at 80°C for overnight, new spot was detected using plate. The reaction mixture was extracted with water (50 mL) and ethyl acetate (50 mL x 3). The combined organic phase was washed with saturated brine (50 mL), dried over anhydrous sodium sulfate, and concentrated under reduced pressure to remove organic solvent. The residue was purified by column chromatography (ethyl acetate: petroleum ether=0-30%) to afford compound 4 (2.1 g, 52.24% yield).

### Step 3):

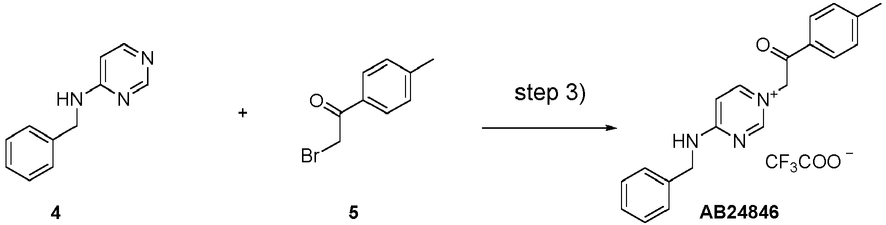

Compound 4 (200 mg, 1.08 mmol) was dissolved in acetonitrile (15 mL), and compound 5 (345mg, 1.62 mmol) was added. The mixture was replaced with N₂ three times, and stirred at 80°C for overnight, new spot was detected using plate. The reaction mixture was cooled to room temperature and dried, the residue was purified by preparation purification to afford compound AB24846 (30 mg, 8.72% yield).

### Compound AB24846

¹H NMR (400 MHz, DMSO,d6) δ 8.77 (s, 1H), 8.14 (s, 1H), 7.89 (s, 2H), 7.42 (s, 8H), 7.35 (s, 3H), 7.10 - 6.90 (m, 1H), 5.86 (s, 2H), 4.71 (s, 2H), 2.39 (s, 4H).

MS-ESI: Calculated.: [M- CF₃COO]⁺318.16, Found: 318.10.

### Example 105

### Synthesis of compound AB24838

### Step 1:

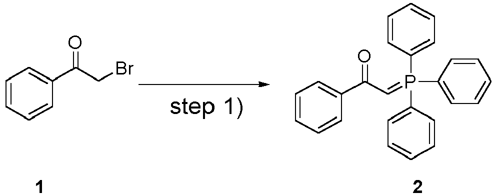

Compound 1 (200 mg, 1.01 mmol) and triphenylphosphine (400 mg, 3.54 mmol) were dissolved in tetrahydrofuran (10 mL). The mixture was replaced with N₂ three times, and stirred at 80°C for 16h, new spot was detected using plate. The reaction mixture was cooled to room temperature, filtered, and the filter cake was washed with tetrahydrofuran to afford crude compound 2 (350 mg, 91.1 % yield).

### Step 2:

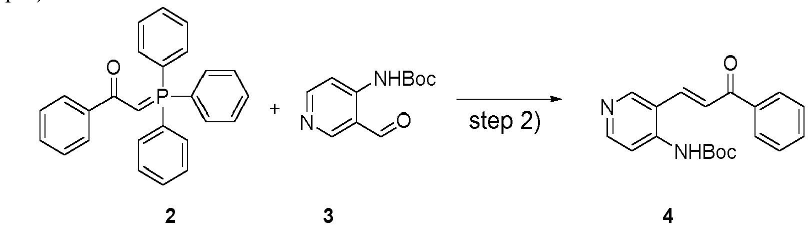

Compound 2 (200 mg, 0.52 mmol) and compound 3 (108 mg, 0.46 mmol) were dissolved in tetrahydrofuran (10 mL), and triethylamine (2 ml) was added. The mixture was replaced with N₂ three times, and stirred at 70°C for 16h, new spot was detected using plate. The reaction mixture was cooled to room temperature and concentrated under reduced pressure. The residue was purified by column chromatography (methanol: dichloromethane=0-10%) to afford compound 4 (150 mg, 89 % yield).

### Step 3:

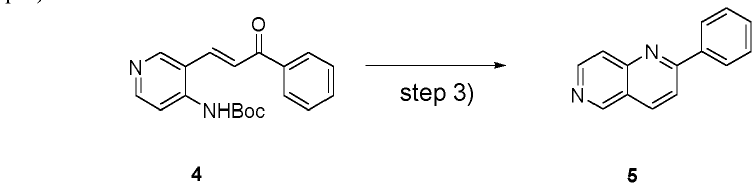

Compound 4 (200 mg, 0.61 mmol) was dissolved in ethyl acetates (5 mL), the mixture was stirred at room temperature for 3h, new spot was detected using plate. The reaction mixture was concentrated under reduced pressure to afford crude compound 5 (210 mg).

### Step 4):

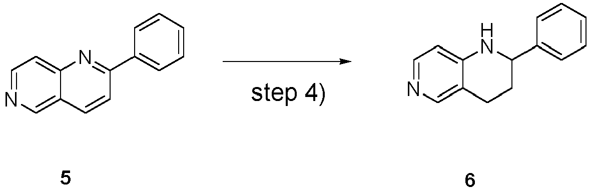

Compound 5 (200 mg, 0.97 mmol) was dissolved in ethanol (5 mL), palladium carbon (20 mg) was added. The mixture was replaced with N₂ three times, and stirred at room temperature for overnight, new spot was detected using plate. The reaction mixture was filtered, concentrated under reduced pressure. The residue was purified by column chromatography (methanol: dichloromethane=0-10%) to afford compound 6 (60 mg, 29.5 % yield).

### Step 5):

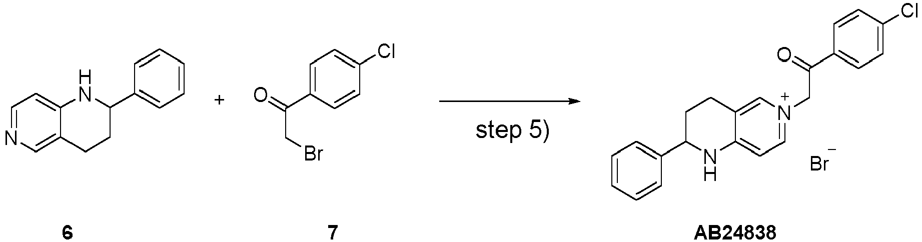

Compound 6 (110 mg, 0.52 mmol) and compound 7 (184 mg, 0.79 mmol) were dissolved in acetonitrile (10 mL), potassium carbonate (72 mg, 0.52 mmol) was added. The mixture was stirred at 70°C for 5 h, new spot was detected using plate. The reaction mixture was concentrated under reduced pressure to remove organic solvent. The residue was purified by column chromatography (methanol: dichloromethane=10:1) to afford compound AB24838 (61 mg, 32.3% yield).

### Compound AB24838

¹H NMR (399 MHz, DMSO-d6) δ 9.30 (s, 1H), 8.01 (dd, *J* = 17.6, 9.1 Hz, 2H), 7.70 (d, *J* = 7.8 Hz, 1H), 7.39 (d, *J* = 6.8 Hz, 1H), 7.33 (d, *J* = 6.2 Hz, 2H), 6.92 (d, *J* = 7.0 Hz, 1H), 5.90 (s, 2H), 4.76 (s, 1H), 2.77 (d, *J* = 10.0 Hz, 1H), 2.57 (d, *J* = 16.7 Hz, 1H), 2.09 (s, 1H), 1.87 (d, *J* = 8.4 Hz, 1H).

MS-ESI: Calculated.: [M-Br]⁺ 363.86, Found: 363.15.

### Example 106

The inhibitory effect of different compounds prepared in Examples on NCI-H82 cell was investigated.

Cell viability was detected using the Promega CellTiter-Glo kit, the kit reflected cell viability by directly measuring intracellular ATP content. Small cell lung cancer cell NCI-H82 (ATCC, No. HTB-175) was cultured in 10% fetal bovine serum-containing RPMI1640 medium (+P/S). The 50% inhibiting concentration (IC₅₀) of different compounds on NCI-H82 was determined using Promega CellTiter-Glo kit, the experiment result was shown in Table 1 below:

**Table 1 The inhibitory effect of different compounds on NCI-H82 cell**

| **Compound** | **IC₅₀(µM)** |
|---|---|
| AB24831A | 5.41 |
| AB24828A | 5.79 |
| AB24861 | 4.09 |
| AB24859 | 2.29 |
| AB24835A | 2.75 |
| AB24863 | 3.61 |
| AB24878 | 3.58 |
| AB24872 | 1.24 |
| AB24854 | 1.57 |
| AB24945 | 4.22 |
| AB24837 | 1.11 |
| AB24827A | 2.89 |
| AB24887 | 0.29 |
| AB24888 | 0.85 |
| AB24895 | 2.09 |
| AB24833A | 3.38 |
| AB24916 | 0.38 |
| AB24913 | 0.18 |
| AB24841A | 0.59 |
| AB24920 | 1.01 |
| AB24923 | 6.85 |
| AB24840A | 2.59 |
| AB24904 | 3.22 |
| AB24905 | 2.52 |
| AB24883 | 6.37 |
| AB24851 | 6.57 |
| AB24850 | 5.78 |
| AB24885 | 3.13 |
| AB24898 | 6.28 |
| AB24839B | 9.13 |
| AB24959 | 4.11 |
| AB24938 | 3.96 |
| AB24821 | 7.17 |
| AB24839 | 0.99 |
| AB27130 | 0.77 |
| AB27141 | 0.01 |
| AB24957 | 1.06 |
| AB24989 | 0.56 |
| AB24906 | 1.66 |
| AB24949 | 0.68 |
| AB24954 | 0.39 |
| AB24963 | 0.33 |
| AB24964 | 1.09 |
| AB24967 | 3.88 |
| AB24991 | 0.34 |
| AB27106 | 1.62 |
| AB27107 | 2.18 |
| AB27114 | 3.93 |
| AB27124 | 0.03 |
| AB27127 | 0.08 |
| AB24924 | 3.96 |
| AB24996 | 1.35 |
| AB27111 | 0.85 |
| AB27112 | 0.41 |
| AB27125 | 0.11 |
| AB27126 | 0.15 |
| AB27131 | 0.44 |
| AB27133 | 0.34 |
| AB27142 | 0.06 |
| AB27144 | 0.16 |
| AB27156 | 0.26 |
| AB27128 | 0.33 |
| AB27169 | 0.32 |
| AB27166 | 0.16 |
| AB27170 | 0.01 |
| AB24917 | 0.12 |
| AB24956 | 0.77 |
| AB27176 | 0.04 |
| AB27174 | 0.10 |
| AB24948 | 0.10 |
| AB27163 | 0.15 |
| AB27184 | 0.23 |
| AB27162 | 0.24 |
| AB27145 | 0.25 |
| AB27129 | 0.26 |
| AB27161 | 0.28 |
| AB27165 | 0.41 |
| AB24961 | 0.54 |
| AB27158 | 0.55 |
| AB27155 | 0.65 |
| AB27104 | 0.72 |
| AB27154 | 0.87 |
| AB24999 | 1.30 |
| AB24988 | 0.92 |
| AB27149 | 1.98 |
| AB27118 | 1.72 |
| AB24987 | 2.00 |
| AB27148 | 2.81 |
| AB24852 | 1.46 |
| AB24807A | 0.54 |
| AB24803A | 0.23 |
| AB24810A | 1.34 |
| AB24805A | 0.37 |
| AB24808A | 0.61 |
| AB24845A | 0.12 |
| AB24955 | 2.80 |
| AB24809A | 2.43 |
| AB24983 | 3.40 |
| AB24813 | 5.93 |
| AB24814 | 5.54 |
| AB24817 | 4.61 |
| AB24880 | 9.05 |
| AB24822 | 6.70 |
| AB24846 | 0.51 |
| AB24838 | 0.71 |

Table 1 showed the compound of the present invention had excellent inhibitory effect on NCI-H82 cell.

### Example 107

The inhibitory effect of compound AB24852 prepared in Example 89 on different tumor cell lines was investigated.

Cell viability was detected using the Promega CellTiter-Glo kit, the kit reflected cell viability by directly measuring intracellular ATP content. Different cancer cells was cultured in medium, the 50% inhibiting concentration (IC50) of compound AB24852 on cancer cells was determined using Promega CellTiter-Glo kit. The name, source and culture conditions of each tumor cell line were as follows:
Cell line A-375 (ATCC, No. CRL-1619) was cultured in 10% fetal bovine serum-containing DMEM medium (+P/S).

Cell line SNU-398 (ATCC, number CRL-2233) was cultured in 10% fetal bovine serum-containing RPMI1640 medium (+P/S).

Cell line NCI-H1048 (ATCC, No. CRL-5853) was cultured in 5% fetal bovine serum-containing HITES medium (+P/S).

Cell line MIA PaCa-2 (ATCC, No. CRL-1420) was cultured in 10% fetal bovine serum-containing DMEM medium (+P/S).

Cell line 22RV1 (ATCC, No. CRL-2505) was cultured in10% fetal bovine serum-containing RPMI1640 medium (+P/S).

Cell line G-401 (ATCC, No. CRL-1441) was cultured in 10% fetal bovine serum-containing McCoy's 5a medium (+P/S).

Cell line MDA-MB-453 (ATCC, No. HTB-131) was cultured in 10% fetal bovine serum-containing Leibovitz's L-15 medium (+P/S).

Cell line SU-DHL-2 (ATCC, No. CRL-2956) was cultured in 10% fetal bovine serum-containing RPMI1640 medium (+P/S).

Cell line OCI-AML-4 (DSMZ, No. ACC-729) was cultured in alpha-MEM medium containing 20% fetal bovine serum and 20% volume fraction of 5637 cell line adjusted medium (+P/S).

Cell line SW48 (ATCC, No. CCL-231) was cultured in 10% fetal bovine serum-containing Leibovitz's L-15 medium (+P/S).

The inhibitory effect of compound AB24852 on different tumor cell lines was shown in Table 2.

**Table 2 The inhibitory effect of compound AB24852 on different tumor cell lines.**

| | **Cell line** | **cancer** | **IC₅₀(µM)** |
|---|---|---|---|
| 1 | A-375 | melanoma | 1.3 |
| 2 | SNU-398 | liver cancer | 1.7 |
| 3 | NCI-H1048 | lung cancer | 1.1 |
| 4 | MIA PaCa-2 | pancreatic cancer | 0.7 |
| 5 | 22RV1 | prostatic cancer | 2.3 |
| 6 | G-401 | renal carcinoma | 1.0 |
| 7 | MDA-MB-453 | breast cancer | 0.2 |
| 8 | SU-DHL-2 | lymphoma | 0.3 |
| 9 | OCI-AML-4 | leukemia | 0.8 |
| 10 | SW48 | intestine cancer | 0.7 |

| | | | |
|---|---|---|---|
| Note: A-375 was melanoma, SNU-398 was anaplastic and lowly differentiated hepatocellular carcinoma, NCI-H 1048 was small cell lung cancer, MDA-MB-453 was triple negative breast cancer, SU-DHL-2 was B cell lymphoma, OCI-AML-4 was type M4 of acute myeloid leukemia, SW48 was Dukes' type C, grade IV of colorectal adenocarcinoma. | | | |

Table 2 showed the compound AB24852 had excellent inhibitory effect on various tumor cells.

### Example 108

The activity of mitochondria permeability transition pore in Daoy cell and NCI-H82 cell was investigated.

### 1.1 Experimental background:

The mitochondria permeability transition pore (mPTP) was a non-specific channel on the inner membrane of mitochondria, which could allow small molecules with molecular weight less than 1.5KD to pass freely, and its activity was affected by peroxides (e.g., H₂O₂), pH and calcium ion in mitochondria. Some cells (e.g., normal somatic cell cells) had active mitochondria permeability transition pore, while some cells (e.g., some tumor cells) had inactive mitochondria permeability transition pore. For cells with active mitochondria permeability transition pore, the addition of peroxide (e.g., H₂O₂) could increase the activity of mitochondria permeability transition pore, resulting in a decrease in mitochondria membrane potential. For cells with inactive or even closed mitochondria permeability transition pore, the addition of peroxide (e.g., H₂O₂) had no significant effect on the activity of mitochondria permeability transition pore, and the mitochondria membrane potential had no significant change. Based on the principle, whether the mPTP was active or inactive in specific cell could be determined by measuring the change of potential difference of the mitochondria membrane under peroxide stimulation.

### 1.2 Experimental method and result

Daoy cell was human medulloblastoma cell (ATCC, No. HTB-186) and was cultured in 10% fetal bovine serum- containing DMEM medium (+P/S). NCI-H82 cell was small cell lung cancer cell (ATCC, No. HTB-175) and was cultured in 10% fetal bovine serum- containing RPMI1640 medium (+P/S).1.5µM Cyclosporin A (CsA, CsA could effectively inhibit the activity of mPTP (mitochondria permeability transition pore) ) was added to the medium, and the cell cultured in medium without Cyclosporin A (CsA) were used as blank control. The mitochondria membrane potential difference was detected by Tetramethylrhodamine(TMRM), the high fluorescence intensity of TMRM represented the high membrane potential difference. The results was shown in Table 3.

**Table 3 Relative TMRM signal intensity (%) in different groups**

| **Cell** | **Daoy cell** | | | | **NCI-H82 cell** | | | |
|---|---|---|---|---|---|---|---|---|
| CsA | - | - | + | + | - | - | + | + |
| H₂O₂ | - | + | - | + | - | + | - | + |
| mean value | 100 | 82 | 125 | 121 | 118 | 119 | 120 | 118 |
| Standard deviation | 7 | 11 | 14 | 11 | 5 | 2 | 6 | 8 |
| Repeat times | 3 | 3 | 3 | 3 | 3 | 3 | 3 | 3 |
| P value | | <0.05 | <0.01 | | | | <0.05 | <0.05 |

| | | | | | | | | |
|---|---|---|---|---|---|---|---|---|
| Notes "+" represented existence, "-" represented none. | | | | | | | | |

The table 3 showed the membrane potential of Daoy cell was down-regulated under the action of H₂O₂, and the down-regulation could be reversed by CsA, a mitochondria permeability transition pore inhibitor, indicating that the mitochondria permeability transition pore was active in Daoy cell. The membrane potential of NCI-H82 cell was not affected by H₂O₂ and CsA, indicating that mPTP (mitochondria permeability transition pore) was inactive in NCI-H82 cell. Therefore, Table 3 showed that the mitochondria permeability transition pore was active in Daoy cell, while the mitochondria permeability transition pore was inactive in NCI-H82 cell.

### Example 109

The inhibitory effect of the relevant compound prepared in Examples on cancer cell growth was related to the activity of mPTP

### 3.1 Experimental background:

Cell viability was detected using the Promega CellTiter-Glo kit, the kit reflected cell viability by directly measuring intracellular ATP content. In the experiment, Daoy cell with active mitochondria permeability transition pore and NCI-H82 cell with inactive mitochondria permeability transition pore were used to detect the IC₅₀ value of cell viability inhibition of the relevant compound prepared in Examples on Daoy cell, Daoy cell+mPTP inhibitor (Cyclosporin A, CsA, 1.5µM, Sigma) and NCI-H82.

### 3.2 Experimental method and result

Daoy cell was cultured in 10% fetal bovine serum-containing DMEM medium (+P/S), and NCI-H82 cell was cultured in 10% fetal bovine serum-containing RPMI1640 medium (+P/S). The 50% inhibitory dose IC₅₀ of the relevant compound was measured in two cells (Daoy cell and NCI-H82 cell) under three conditions (Daoy cell without CsA, Daoy cell with CsA and NCI-H82 cell without CsA). The experimental result was shown in Table 4:

**Table 4 The inhibitory effect of the relevant compound prepared in Example on Daoy cell without CsA, Daoy cell with CsA, and NCI-H82 cell without CsA**

| | IC50 (µM) | | |
|---|---|---|---|
| | Daoy cell without CsA | Daoy cell with CsA | NCI-H82 cell without CsA |
| AB27141 | >1 | 0.028 | 0.01 |
| AB27144 | 1.02 | 0.21 | 0.16 |
| AB27156 | 1.05 | 0.24 | 0.26 |
| AB27125 | 1.46 | 0.19 | 0.11 |
| AB27126 | 1.95 | 0.28 | 0.15 |
| AB27128 | 3.03 | 0.21 | 0.33 |
| AB27131 | 4.75 | 0.71 | 0.44 |
| AB27130 | 5.50 | 0.31 | 0.77 |

The table 4 showed the relevant compounds prepared in Examples had inhibitory effect on Daoy cell and NCI-H82 cell, especially on NCI-H82 cell with inactive mPTP. In addition. When the activity of mPTP in Daoy cell was decreased by adding CsA, a mPTP inhibitor, the inhibitory effect of the relevant compound on the Daoy cell could be significantly increased, indicating that decreasing the activity of mPTP in cell could significantly enhance antitumor activity of relevant compound, the relevant compound had more significant inhibitory effect on mPTP-inactive cell.

### Example 110 Sensitivity of different compounds prepared in Examples to different tumor cell lines

The inhibitory effect of different compounds prepared in Examples on different tumor cell lines was detected using cell activity detection reagent.

### Experimental background:

Cell viability was detected using the Promega CellTiter-Glo kit, the kit reflected cell viability by directly measuring intracellular ATP content. In the experiment, the IC₅₀ value of different compounds on different tumor cell lines was detected to determine cell lines that were sensitive or insensitive to different compounds.

### Experimental method and result:

Tumor cells were cultured in 10% FBS- containing medium (+ p/s), and incubated for 3h or the following day, then the gradient diluted compound was added. After 3 days of culture, the relevant IC₅₀ (50% inhibiting concentration) was measured. The name, source and culture conditions of each tumor cell line were as follows:
Cell line NCI-H82 (ATCC, No. HTB-175) was cultured in 10% fetal bovine serum-containing RPMI1640 medium (+P/S).

Cell line G-401 (ATCC, No. CRL-1441) was cultured in 10% fetal bovine serum-containing McCoy's 5a medium (+P/S).

Cell line MDA-MB-453 (ATCC, No. HTB-131) was cultured in 10% fetal bovine serum-containing Leibovitz's L-15 medium (+P/S).

Cell line SW48 (ATCC, No. CCL-231) was cultured in 10% fetal bovine serum-containing Leibovitz's L-15 medium (+P/S).

Cell line CFPAC-1 (ATCC, No. CRL-1918) was cultured in 10% fetal bovine serum-containing IMDM medium (+P/S).

Cell line 786-O (ATCC, No. CRL-1932) was cultured in 10% fetal bovine serum-containing RPMI1640 medium (+P/S).

Cell line GB-1 (JCRB, No. IFO50489) was cultured in 10% fetal bovine serum-containing DMEM medium (+P/S).

The experiment result was shown in Table 5 below:

**Table 5 Inhibitory effect of compound prepared in Example on different cell lines (IC₅₀, µM)**

| | | NCI-H82 | MDA-MB-453 | SW48 | G-401 | GB-1 | 786-O | CFPAC-1 | SF-126 |
|---|---|---|---|---|---|---|---|---|---|
| 1 | AB24845A | 0.12 | 0.19 | 0.14 | 0.30 | 4.37 | 38.55 | 29.42 | 2.28 |
| 2 | AB24805A | 0.37 | 0.21 | 0.76 | 0.42 | 3.43 | 46.47 | 35.72 | 3.18 |
| 3 | AB24841A | 0.59 | 0.62 | 0.80 | 0.63 | 16.38 | >50 | 35.73 | 10.75 |
| 4 | AB24808A | 0.61 | 0.36 | 1.78 | 0.91 | 5.27 | 44.05 | 19.98 | 2.98 |
| 5 | AB24846 | 0.51 | 0.82 | 2.05 | 0.69 | 12.32 | 47.29 | 36.42 | 3.61 |
| 6 | AB24810A | 1.34 | 2.69 | 2.13 | 1.95 | 24.48 | >50 | 35.97 | 13.10 |
| 7 | AB24809A | 2.43 | 1.77 | 7.64 | 3.03 | >50 | >50 | >50 | 37.50 |
| 8 | AB24833A | 3.38 | 5.30 | 8.16 | 5.30 | 21.97 | 45.35 | 24.85 | 21.58 |
| 9 | AB24852 | 1.46 | 0.22 | 0.72 | 1.02 | >50 | >50 | >50 | >50 |

| | | | | | | | | | |
|---|---|---|---|---|---|---|---|---|---|
| Note: IC₅₀ refered to 50% inhibiting concentration, ie, the concentration of the inhibitor when 50% inhibitory effect was achieved. | | | | | | | | | |

The Table 5 showed the sensitivity of different cells to the different compounds, NCI-H82 (human small cell lung cancer cell), G-401 (renal carcinoma), MDA-MB-453 (triple negative breast cancer cell) and SW48 (Duke's type C, grade IV of colorectal adenocarcinoma) were sensitive to compounds with low IC₅₀ value, while 786-O (clear cell renal cell adenocarcinoma cell), CFPAC-1 (human pancreatic cancer cell), GB-1 (human glioblastoma cell) and SF126 (human brain tumor cell) were not sensitive to relevent compounds with high IC₅₀ value

### Example 111

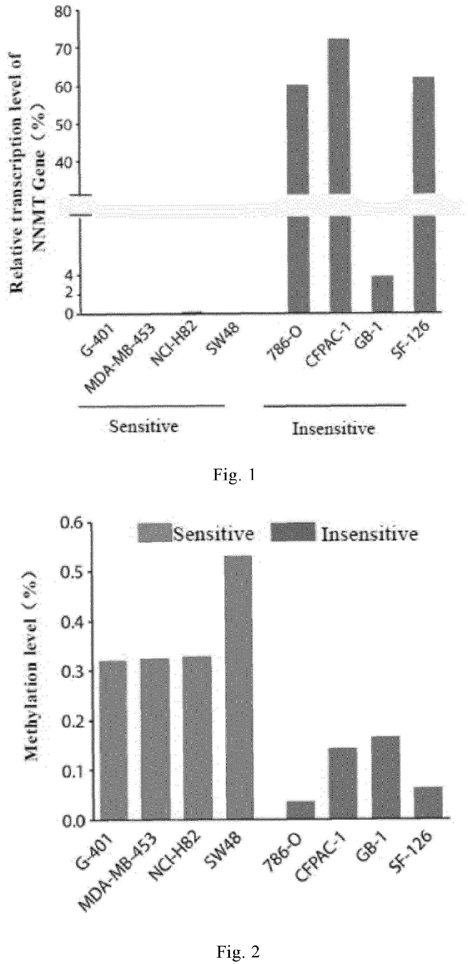

The mRNA transcription level of NNMT gene in four tumor cell lines sensitive to relevent compounds and four tumor cell lines insensitive to relevent compounds was measured using RT-qPCR gene expression analysis test, and the expression of NNMT gene in the tumor cell lines was measured respectively. The results were shown in Fig. 1.

As shown in Fig. 1, the mRNA transcription level of NNMT gene in four tumor cell lines (NCI-H82, G-401, MDA-MB-453, SW48) sensitive to compounds and four tumor cell lines (786-O, CFPAC-1, GB-1, and SF126) insensitive to compounds was measured using RT-qPCR gene expression analysis test, the results showed the expression of NNMT gene was low in sensitive cell lines (NCI-H82, G-401, MDA-MB-453 and SW48) , and the expression of NNMT gene was high in insensitive cell lines (786-O, CFPAC-1, GB-1 and SF126).

Therefore, the Fig. 1 showed that compared with tumor cell lines with high expression of NNMT gene, the inhibitory effect of compounds on tumor cell lines with low expression of NNMT gene was significantly enhanced, i.e, the expression of NNMT gene in tumor cell was negatively correlated with the sensitivity of tumor cell to relevent compounds.

### Example 112

NNMT refers to Nicotinamide N-Methyltransferase.

The nucleotide sequence of the promoter region of NNMT gene was as shown in SEQ ID NO: 1.

The nucleotide sites from 1050 bp before the transcription start site to 499 bp after the transcription start site in NNMT gene was sites 951-2500 of nucleotide sequence as shown in SEQ ID NO: 1.

The nucleotide sites from 1050 bp to 193 bp before the transcription start site in NNMT gene was sites 951-1808 of nucleotide sequence as shown in SEQ ID NO: 1.

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in four tumor cell lines sensitive to isoquinoline compounds and four tumor cell lines insensitive to relevent compounds. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.2, Fig.3, and Fig.4.

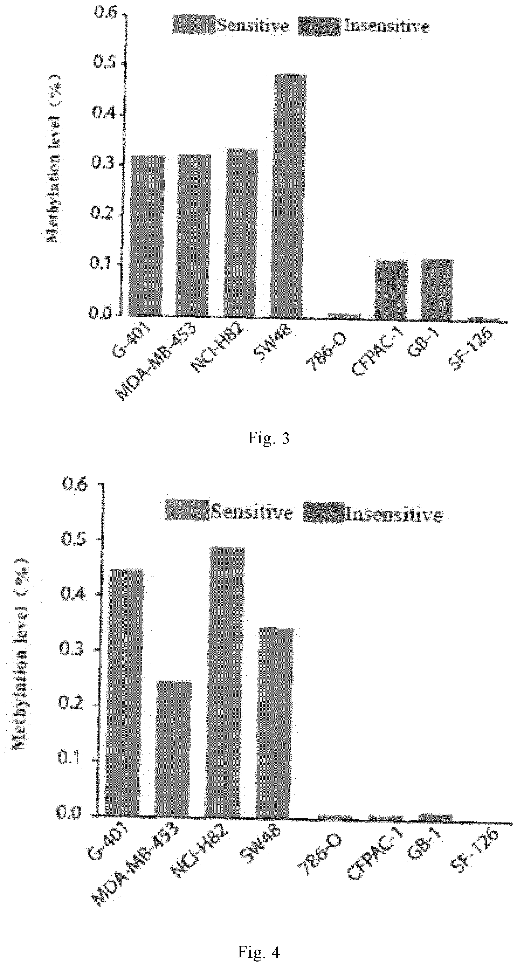

As shown in Fig.2 (the promoter region), Fig.3 (the region from 1050 bp before the transcription start site to 499 bp after the transcription start site) and Fig.4 (the region from 1050 bp to 193 bp before the transcription start site), the relevent compounds had significantly stronger inhibitory effect on tumor cells with high methylation level of DNA CpG site in the promoter region of NNMT gene or relevant region of NNMT gene, while the relevant compounds had significantly weaker inhibitory effect on tumor cells with low methylation level of DNA CpG site in the promoter region of NNMT gene or relevant region of NNMT gene, indicating the methylation level of DNA CpG site in the promoter region of NNMT gene or relevant region of NNMT gene in tumor cell was positive correlation with the sensitivity of tumor cell to compound.

### Example 113 Stability of related compounds prepared in Examples in liver microsome was investigated.

The human liver microsome (Corning ^{®}) was used in the test, and the test step were as follows:
1) preparation of buffer C:
   buffer A: 1.0 L of 0.1 M potassium dihydrogen phosphate buffer (containing 1.0 mM EDTA);
   buffer B: 1.0 L of 0.1 M dipotassium hydrogenphosphate buffer (containing 1.0 mM EDTA);
   buffer C: adding buffer A to 700 mL of buffer B and adjust pH to 7.4.
2) preparation of 10 mM stock solution:
   Dissolving the tested compound in DMSO to obtain 10mM stock solution.
3) preparation of drug solution:
   500 µ M solution: adding 10 µ L of 10 mM stock solution to 190 µ L ACN;
   1.5 µ M drug solution (dissolved in liver microsome solution):
      adding 18.75 µ L of 20 mg/mL liver microsome to 479.75 µ L buffer C, then adding 1.5 µ L of 500 µ M solution, slightly shaking and mixing.
4) preparation of 6 mM NADPH solution:
   weighing NADPH and adding appropriate amount of buffer C to prepare 6 mM NADPH solution.
5) adding 30 µ L of 1.5 µ M drug solution to 96 well plate, different time points (0 min, 5 min, 15 min, 30 min, 45 min) were set, and duplicated wells was setted.
6) preparation of sample in 0 min: adding 135 µ L of ACN (contiaining internal standard) to the well in 0 minute firstly, and then adding 15 µ L of 6 mM NADPH solution.
7) preheating the 96 well plate containing 1.5 µ M drug solution and NADPH solution in 37 °C water bath for 5 min.
8) adding a preheated 15 µ L of 6 mM NADPH solution to the wells set as 5 min, 15 min, 30 min, and 45 min, starting the reaction and counting the timing.
9) when the timer displayed 5 min, 15 min, 30 min, and 45 min, adding 135 µ L of ACN (containing internal standard) to terminate the reaction. Vortexing for 10 min, the sample was centrifuged with 5594 × g for 15 min in centrifuger (Thermo Multifuge ×3R).
10) taking 50 µ L supernatant from the centrifuged sample, transferred to 96 well sample plate containg 50 µL water, mixted, and the sample was analyzed using LC-MS/MS.

The experimental results were shown in Table 6:

**Table 6 Stability of the compounds prepared in the Examples in human liver microsome**

| | Human T_{1/2} (min) |
|---|---|
| AB24810A | 3910.2 |
| AB24809A | 398.3 |
| AB24839 | 669.2 |
| AB27124 | 106.5 |
| AB27144 | 4512.4 |
| AB27156 | 1047.7 |
| AB27125 | 854.2 |
| AB27126 | 1249.5 |
| AB27128 | 1427.1 |
| AB27131 | 408.5 |
| AB27130 | 357.6 |
| AB24964 | 216.0 |
| AB27141 | 291.0 |
| AB27142 | 746.6 |
| AB24913 | 478.3 |
| AB27112 | 206.4 |
| AB24989 | 145.9 |
| AB24840A | 139.8 |
| AB24805A | 222.5 |
| AB24807A | 198.8 |
| AB24841A | 1225.4 |
| AB24808A | 341.1 |
| AB24852 | 106.8 |

| | |
|---|---|
| Note: T_{1/2} refered to half-life. | |

The table 6 showed the compounds prepared in the examples had excellent half-life T_{1/2} in liver microsome, indicating that the compounds prepared in the examples had excellent stability in liver.

All documents mentioned in the present invention are incorporated herein by reference, as if each document is individually cited for reference. It should be understood that those skilled in the art will be able to make various changes or modifications to the present invention after reading the teachings of the present invention, which also fall within the scope of the claims appended hereto.

## Claims

1. A compound of formula I, or an optical isomer thereof, or a racemate thereof, or a solvate thereof, or a pharmaceutically acceptable salt thereof 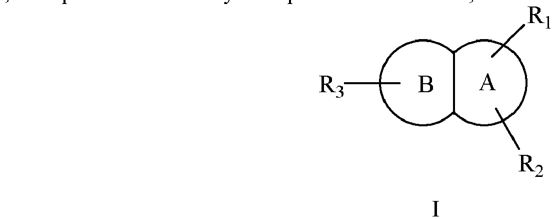 wherein,
ring A is substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring;
ring B is absent, substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring;
R₁ is 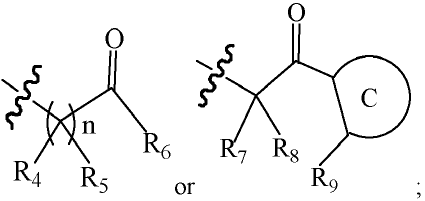
R₂ is hydrogen, 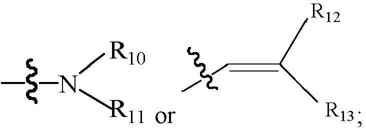
R₃ is none, hydrogen, halogen, -CN, hydroxyl, sulfhydryl, nitro, amino, F₃C-, F₃C-O-, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, substituted or unsubstituted 5-12 membered heteroaryl, substituted or unsubstituted C6-C16 aryl-substituted or unsubstituted C1-C10 alkyl-, substituted or unsubstituted 5-12 membered heteroaryl-substituted or unsubstituted C1-C10 alkyl;
R₄ and R₅ are each independently hydrogen, halogen, -CN, hydroxyl, sulfhydryl, nitro, amino, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, or substituted or unsubstituted 5-12 membered heteroaryl;
R₆ is substituted or unsubstituted C6-C16 aryl, substituted or unsubstituted 5-12 membered heteroaryl, substituted or unsubstituted 3-16 membered cycloalkyl, substituted or unsubstituted 3-16 membered heterocycloalkyl;
n is 0, 1, 2, 3, 4, 5, or 6;
ring C is absent, substituted or unsubstituted C6-C16 aromatic ring, substituted or unsubstituted C3-C16 cycloalkane ring, substituted or unsubstituted 3-16 membered heterocycloalkane ring, or substituted or unsubstituted 3-16 membered heteroaromatic ring;
R₇ is hydrogen, halogen, -CN, hydroxyl, sulfhydryl, nitro, amino, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, or substituted or unsubstituted 5-12 membered heteroaryl;
R₈ and R₉ are connected to form substituted or unsubstituted 3-16 membered cycloalkane ring, substituted or unsubstituted 3-16 membered heterocycloalkane ring;
R₁₀ and R₁₁ are each independently hydrogen, halogen, -CN, hydroxyl, sulfhydryl, nitro, amino, F₃C-O-, F₃C-, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, substituted or unsubstituted 5-12 membered heteroaryl, substituted or unsubstituted C6-C16 aryl-substituted or unsubstituted C1-C10 alkyl-, substituted or unsubstituted 5-12 membered heteroaryl-substituted or unsubstituted C1-C10 alkyl-, substituted or unsubstituted C1-C6 alkyl-C(O)-; or R₁₀ and R₁₁ are connected to form substituted or unsubstituted 3-16 membered heterocycloalkane ring, substituted or unsubstituted 3-16 membered heteroaromatic ring;
R₁₂ and R₁₃ are independently hydrogen, halogen, -CN, hydroxyl, sulfhydryl, nitro, amino, F₃C-, F₃C-O-, substituted or unsubstituted C1-C10 alkyl, substituted or unsubstituted C3-C12 cycloalkyl, substituted or unsubstituted C1-C8 alkoxyl, substituted or unsubstituted C1-C8 alkylthio, substituted or unsubstituted C6-C16 aryl, substituted or unsubstituted 5-12 membered heteroaryl, substituted or unsubstituted C6-C16 aryl-substituted or unsubstituted C1-C10 alkyl-, substituted or unsubstituted 5-12 membered heteroaryl-substituted or unsubstituted C1-C10 alkyl-;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl;
the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

2. The compound of formula I, or an optical isomer thereof, or a racemate thereof, or a solvate thereof, or a pharmaceutically acceptable salt thereof of claim 1, wherein ring A is substituted or unsubstituted C6-C12 aromatic ring, substituted or unsubstituted C3-C12 cycloalkane ring, substituted or unsubstituted 3-12 membered heterocycloalkane ring, or substituted or unsubstituted 3-12 membered heteroaromatic ring; preferably, ring A is substituted or unsubstituted C6-C10 aromatic ring, substituted or unsubstituted C3-C10 cycloalkane ring, substituted or unsubstituted 3-10 membered heterocycloalkane ring, or substituted or unsubstituted 3-10 membered heteroaromatic ring; more preferably, ring A is substituted or unsubstituted C6-C8 aromatic ring, substituted or unsubstituted C3-C8 cycloalkane ring, substituted or unsubstituted 3-8 membered heterocycloalkane ring, or substituted or unsubstituted 5-8 membered heteroaromatic ring; more preferably, ring A is absent, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring; more preferably, ring A is substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring; more preferably, ring A is substituted or unsubstituted pyridine ring, substituted or unsubstituted pyrimidine ring, substituted or unsubstituted benzene ring, substituted or unsubstituted naphthalene ring, substituted or unsubstituted thiazole ring, substituted or unsubstituted imidazole ring, substituted or unsubstituted pyrrole ring;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

3. The compound of formula I, or an optical isomer thereof, or a racemate thereof, or a solvate thereof, or a pharmaceutically acceptable salt thereof of claim 1, wherein ring B is absent, substituted or unsubstituted C6-C12 aromatic ring, substituted or unsubstituted C3-C12 cycloalkane ring, substituted or unsubstituted 3-12 membered heterocycloalkane ring, or substituted or unsubstituted 3-12 membered heteroaromatic ring; preferably, ring B is absent, substituted or unsubstituted C6-C8 aromatic ring, substituted or unsubstituted C5-C8 cycloalkane ring, substituted or unsubstituted 5-8 membered heterocycloalkane ring, or substituted or unsubstituted 5-8 membered heteroaromatic ring; more preferably, ring B is absent, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring; more preferably, ring B is absent, substituted or unsubstituted tetrahydropyridine ring, substituted or unsubstituted pyrrole ring, substituted or unsubstituted dihydropyrrole ring, substituted or unsubstituted imidazole ring, or substituted or unsubstituted pyrazole ring; more preferably, ring B is absent, tetrahydropyridine ring, piperidine ring, pyrrole ring, dihydropyrrole ring, tetrahydropyrrole ring, imidazole ring, pyrazole ring, pyridine ring, 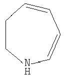 ring, 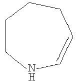 ring;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

4. The compound of formula I, or an optical isomer thereof, or a racemate thereof, or a solvate thereof, or a pharmaceutically acceptable salt thereof of claim 1, wherein R₈ and R₉ are connected to form substituted or unsubstituted 3-12 membered cycloalkane ring, substituted or unsubstituted 3-12 membered heterocycloalkane ring; more preferably, R₈ and R₉ are connected to form substituted or unsubstituted 3-10 membered cycloalkane ring, substituted or unsubstituted 3-10 membered heterocycloalkane ring; more preferably, R₈ and R₉ are connected to form substituted or unsubstituted 3-8 membered cycloalkane ring, substituted or unsubstituted 3-8 membered heterocycloalkane ring; more preferably, R₈ and R₉ are connected to form substituted or unsubstituted 5-8 membered cycloalkane ring, substituted or unsubstituted 5-8 membered heterocycloalkane ring; more preferably, R₈ and R₉ are connected to form substituted or unsubstituted 5-6 membered cycloalkane ring, substituted or unsubstituted 5-6 membered heterocycloalkane ring; more preferably, R₈ and R₉ are connected to form substituted or unsubstituted 5 membered cycloalkane ring or substituted or unsubstituted 6 membered cycloalkane ring; more preferably, R₈ and R₉ are connected to form substituted or unsubstituted cyclopentene ring or cyclohexene ring;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

5. The compound of formula I, or an optical isomer thereof, or a racemate thereof, or a solvate thereof, or a pharmaceutically acceptable salt thereof of claim 1, wherein when the ring B is absent, the compound of formula I has the following structure of formula I-1: 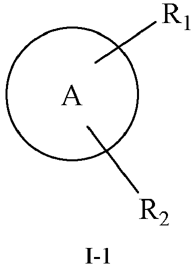 wherein, A, R₁ and R₂ are as defined in claim 1.

6. The compound of formula I, or an optical isomer thereof, or a racemate thereof, or a solvate thereof, or a pharmaceutically acceptable salt thereof of claim 1, wherein the compound of formula I has the following structure of formula I-2-46: 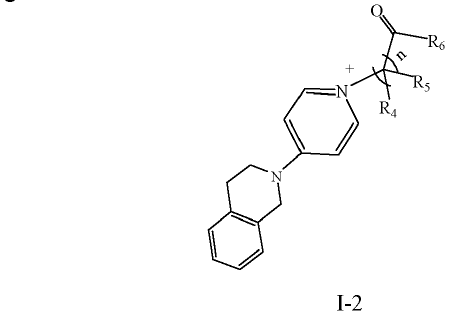
wherein in formula 1-2, R₄, R₅, R₆ and n are as defined in claim 1; 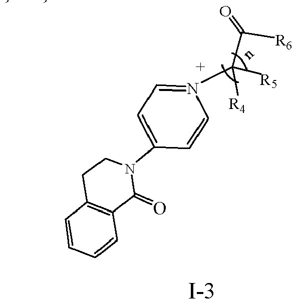
wherein, in formula I-3, R₄, R₅, R₆ and n are as defined in claim 1; 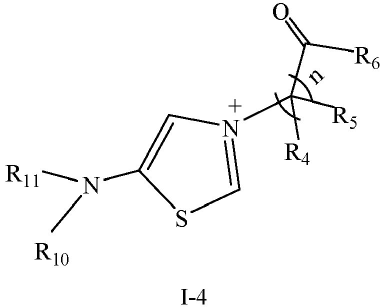
wherein, in formula 1-4, R₄, R₅, R₆, R₁₀, R₁₁ and n are as defined in claim 1 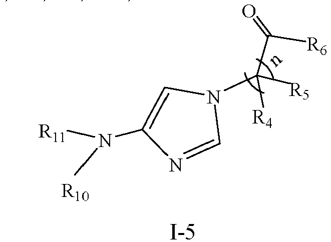
wherein, in formula 1-5, R₄, R₅, R₆, R₁₀, R₁₁ and n are as defined in claim 1; 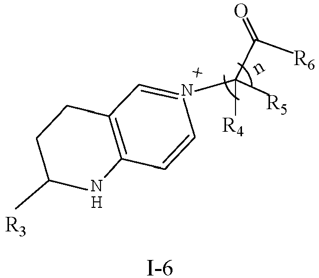
wherein, in formula I-6, R₃, R₄, R₅, R₆ and n are as defined in claim 1; 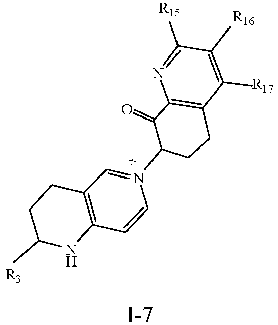
wherein, in formula I-7, R₃ is as defined in claim 1;
R₁₅, R₁₆ and R₁₇ are each independently hydrogen, halogen (e.g., Cl, F), methyl, nitro, phenyl, -OCF₃, -CF₃; 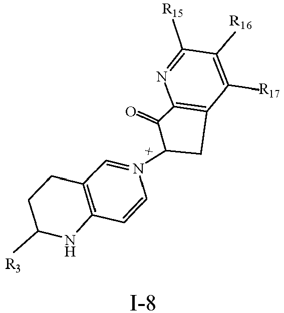
wherein, in formula I-8, R₃ is as defined in claim 1;
R₁₅, R₁₆ and R₁₇ are each independently hydrogen, halogen (e.g., Cl ), methyl, nitro, phenyl; 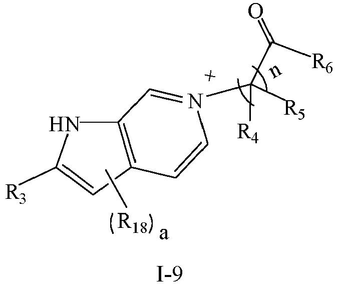
wherein, R₃, R₄, R₅, R₆ and n are as defined above;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 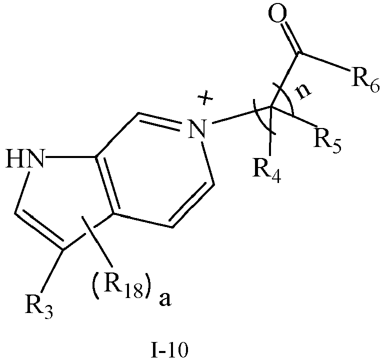
wherein, in formula I-10, R₃, R₄, R₅, R₆ and n are as defined in claim 1;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroarvh 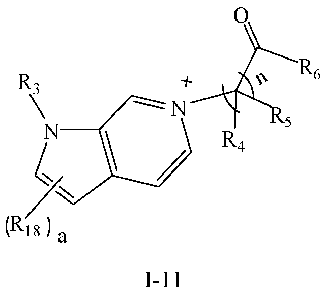
wherein, in formula I-11, R₃, R₄, R₅, R₆ and n are as defined in claim 1;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 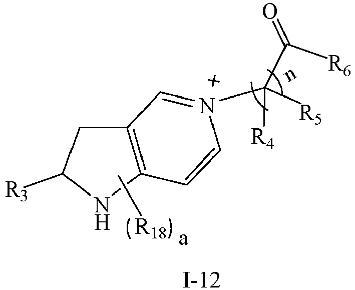
wherein, in formula I-12, R₃, R₄, R₅, R₆ and n are as defined in claim 1;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 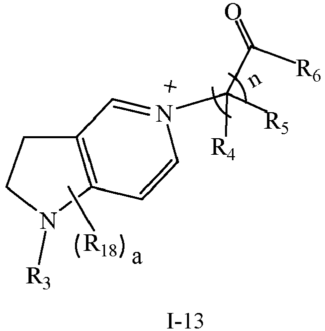
wherein, in formula I-13, R₃, R₄, R₅, R₆ and n are as defined in claim 1;
a is 0, 1, 2, 3 or 4;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 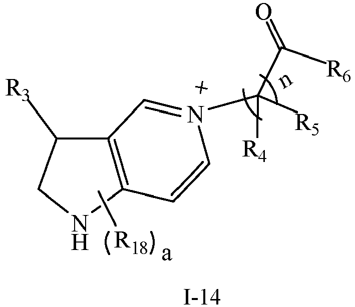
wherein, in formula I-14, R₃, R₄, R₅, R₆ and n are as defined in claim 1;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 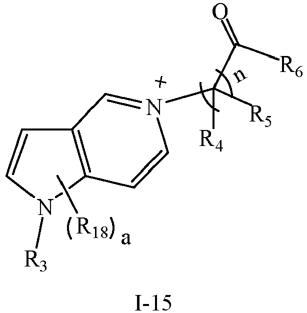
wherein, in formula 1-15, R₃, R₄, R₅, R₆ and n are as defined in claim 1;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 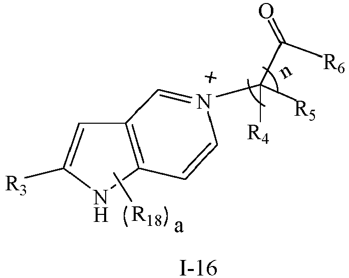
wherein, in formula I-16, R₃, R₄, R₅, R₆ and n are as defined in claim 1;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 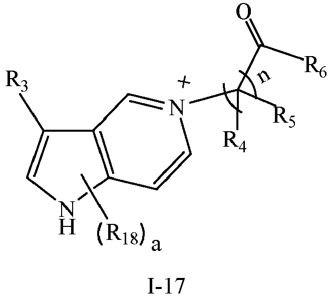
wherein, in formula I-17, R₃, R₄, R₅, R₆ and n are as defined in claim 1;
a is 0, 1 or 2;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 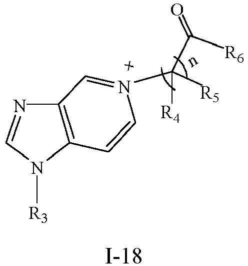
wherein, in formula 1-18, R₃, R₄, R₅, R₆ and n are as defined in claim 1; 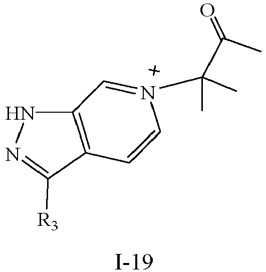
wherein, in formula 1-19, R₃ is as defined in claim 1 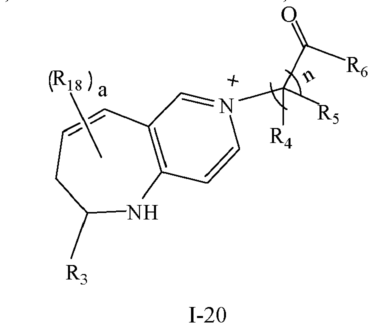
wherein, in formula I-20, R₃, R₄, R₅, R₆ and n are as defined in claim 1;
a is 0, 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 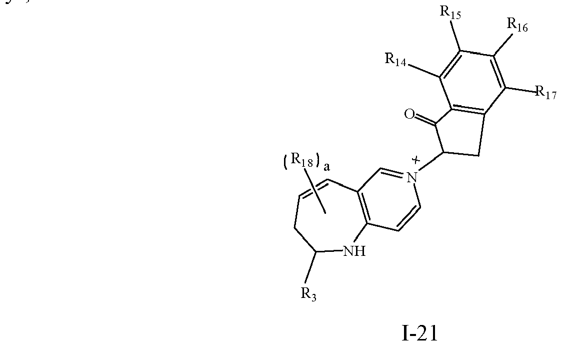
wherein, in formula I-21, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 0, 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 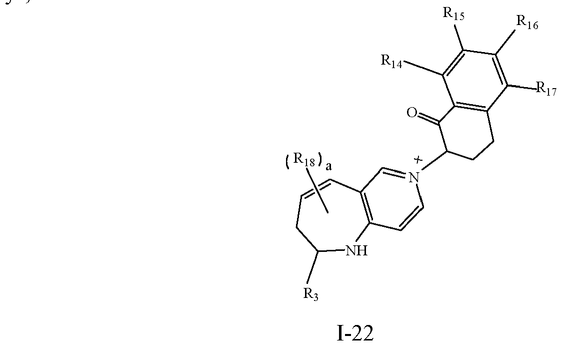
wherein, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 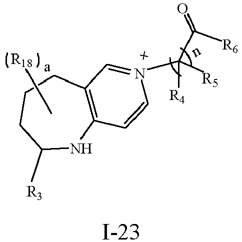
wherein, in formula I-23, R₃, R₄, R₅, R₆ and n are as defined in claim 1;
a is 1, 2, 3, 4, or 5;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 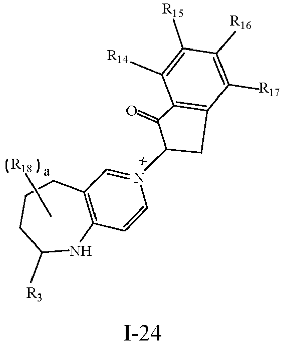
wherein, in formula I-24, R₃ is as defined in claim 1;
a is 0, 1, 2, 3, 4, or 5;
R₁₄, R₁₅, R₁₆ and R₁₇ are each independently hydrogen, halogen (e.g., Cl, F), methyl, nitro, phenyl, -OCF₃, -CF₃;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 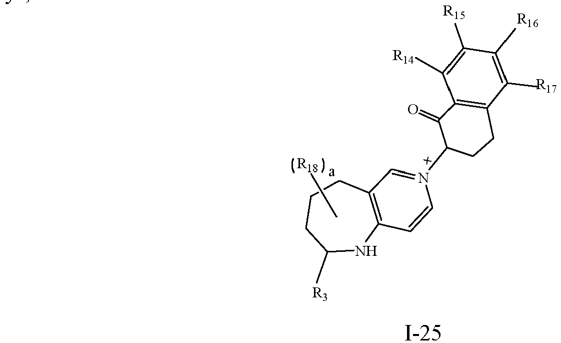
wherein, in formula I-25, R₃ is as defined in claim 1;
a is 0, 1, 2, 3, 4, or 5;
R₁₄, R₁₅, R₁₆ and R₁₇ are each independently hydrogen, halogen (e.g., Cl, F), methyl, nitro, phenyl, -OCF₃, -CF₃;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 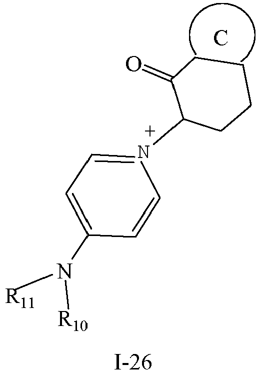
wherein, in formula I-26, R₁₀, R₁₁ and ring C are as defined in claim 1; 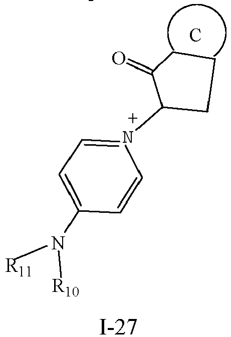
wherein, in formula 1-27, R₁₀, R₁₁ and ring C are as defined in claim 1; 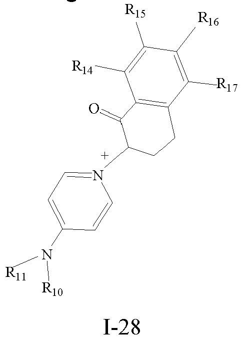
wherein, in formula I-28, R₁₀ and R₁₁ are as defined in claim 1;
R₁₄, R₁₅, R₁₆ and R₁₇ are each independently hydrogen, halogen (e.g., Cl, F), methyl, nitro, phenyl, -OCF₃, -CF₃; 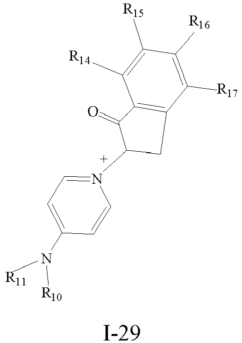
wherein, in formula I-29, R₁₀ and R₁₁ are as defined in claim 1;
R₁₄, R₁₅, R₁₆ and R₁₇ are each independently hydrogen, halogen (e.g., Cl, F), methyl, nitro, phenyl, -OCF₃, -CF₃; 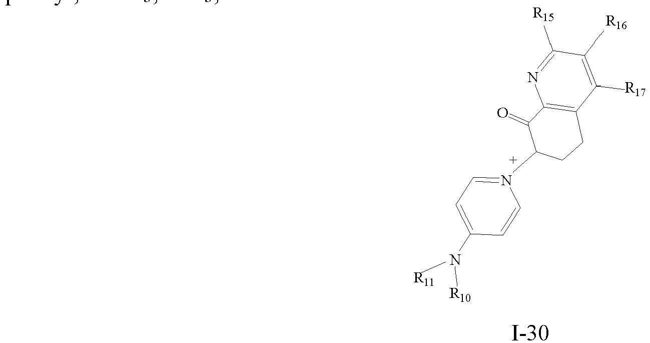
wherein, in formula I-30, R₁₀ and R₁₁ are as defined in claim 1;
R₁₅, R₁₆ and R₁₇ are each independently hydrogen, halogen (e.g., Cl, F), methyl, nitro, phenyl, -OCF₃, -CF₃; 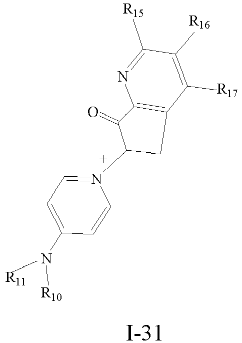
wherein, in formula 1-31, R₁₀ and R₁₁ are as defined in claim 1;
R₁₅, R₁₆ and R₁₇ are each independently hydrogen, halogen (e.g., Cl, F), methyl, nitro, phenyl, -OCF₃, -CF₃; 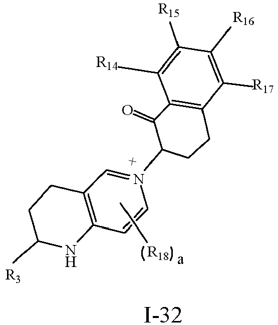
wherein, in formula I-32, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 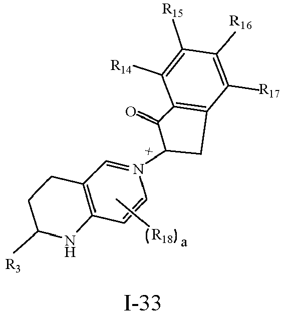
wherein, in formula I-33, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 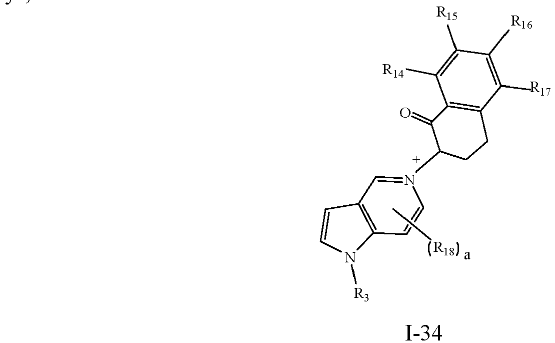
wherein, in formula I-34, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 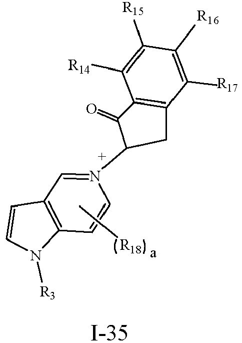
wherein, in formula I-35, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 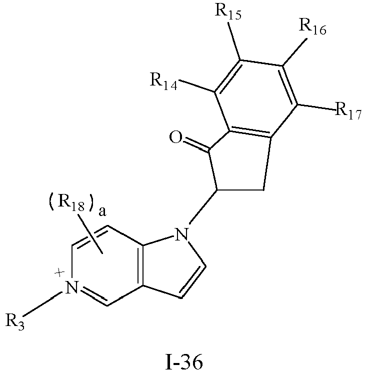
wherein, in formula I-36, R₃ is as defined in claim 1;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 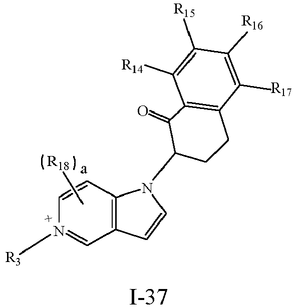
wherein, in formula I-37, R₃ is as defined above;
R₁₄, R₁₅, R₁₆ and R₁₇ are each independently hydrogen, halogen (e.g., Cl, F), methyl, nitro, phenyl, -OCF₃, -CF₃;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 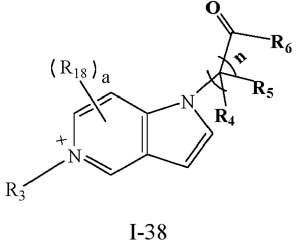
wherein, in formula I-38, R₃, R₄, R₅, R₆ and n are as defined in claim 1;
a is 0, 1, 2 or 3;
each R₁₈ is independently hydrogen, halogen, substituted or unsubstituted C1-C4 alkyl, substituted or unsubstituted C6-C12 aryl, substituted or unsubstituted C3-C8 cycloalkyl, substituted or unsubstituted 3-8 membered heterocycloalkyl, or substituted or unsubstituted 5-8 membered heteroaryl; 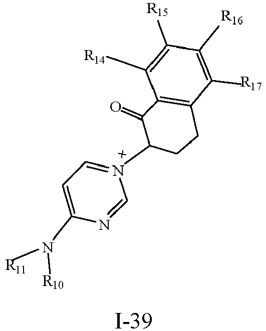
wherein, in formula I-39, R₁₀ and R₁₁ are as defined in claim 1;
R₁₄, R₁₅, R₁₆ and R₁₇ are each independently hydrogen, halogen (e.g., Cl, F), methyl, nitro, phenyl, -OCF₃, -CF₃; 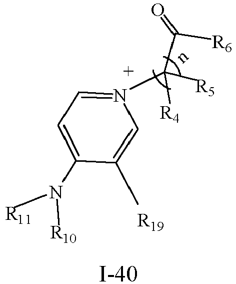
wherein, in formula I-40, R₄, R₅, R₆, R₁₀, R₁₁ and n are as defined in claim 1;
R₁₉ is hydrogen, substituted or unsubstituted C1-C6 alkyl; 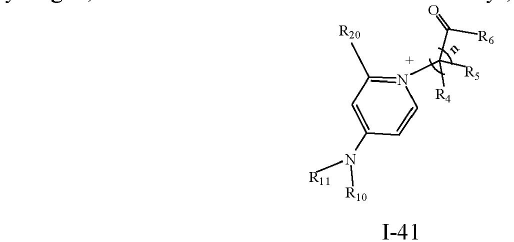
wherein, in formula I-41, R₄, R₅, R₆, R₁₀, R₁₁ and n are as defined in claim 1;
R₂₀ is hydrogen, halogen; 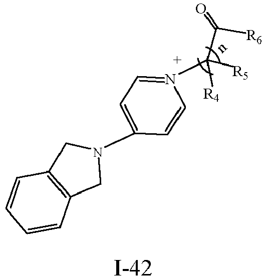
wherein, in formula 1-42, R₄, R₅, R₆ and n are as defined in claim 1 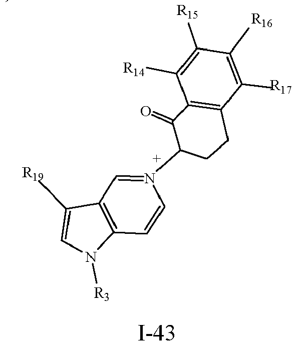
wherein, in formula I-43, R₃ is as defined in claim 1;
R₁₄, R₁₅, R₁₆ and R₁₇ are each independently hydrogen, -CF₃, halogen (e.g., Cl, F), methyl, nitro, phenyl, -OCF₃;
R₁₉ is hydrogen, substituted or unsubstituted C1-C6 alkyl; 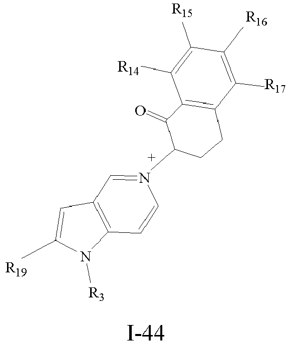
wherein, in formula I-44, R₃ is as defined in claim 1;
R₁₄, R₁₅, R₁₆ and R₁₇ are each independently hydrogen, -CF₃, halogen (e.g., Cl, F), methyl, nitro, phenyl, -OCF₃;
R₁₉ is hydrogen, substituted or unsubstituted C1-C6 alkyl; 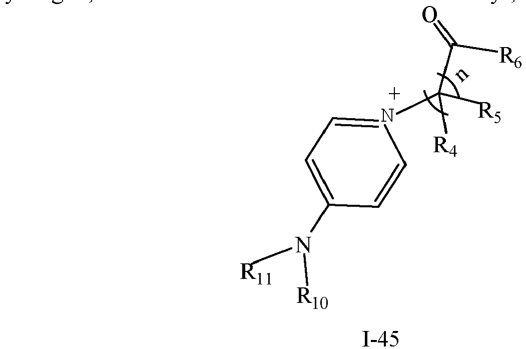
wherein, in formula I-45, R₄, R₅, R₆, R₁₀, R₁₁ and n are as defined in claim 1; 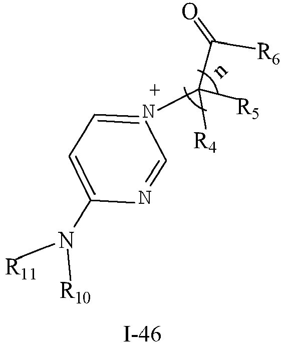
wherein, in formula I-46, R₄, R₅, R₆, R₁₀, R₁₁ and n are as defined in claim 1;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

7. The compound of formula I, or an optical isomer thereof, or a racemate thereof, or a solvate thereof, or a pharmaceutically acceptable salt thereof of claim 1, wherein R₃ is hydrogen, halogen, - CN, hydroxyl, sulfhydryl, nitro, amino, F₃C-O-, F₃C-, substituted or unsubstituted C1-C8 alkyl, substituted or unsubstituted C3-C10 cycloalkyl, substituted or unsubstituted C1-C8 alkoxyl, substituted or unsubstituted C1-C8 alkylthio, substituted or unsubstituted C6-C12 aryl, substituted or unsubstituted 5-10 membered heteroaryl, substituted or unsubstituted C6-C12 aryl-substituted or unsubstituted C1-C8 alkyl-, substituted or unsubstituted 5-10 membered heteroaryl-substituted or unsubstituted C1-C8 alkyl-; preferably, R₃ is hydrogen, halogen, -CN, hydroxyl, sulfhydryl, nitro, amino, F₃C-O-, F₃C-, substituted or unsubstituted C1-C6 alkyl, substituted or unsubstituted C3-C8 cycloalkyl, substituted or unsubstituted C1-C6 alkoxyl, substituted or unsubstituted C1-C6 alkylthio, substituted or unsubstituted C6-C10 aryl, substituted or unsubstituted 5-10 membered heteroaryl, substituted or unsubstituted C6-C10 aryl-substituted or unsubstituted C1-C4 alkyl-, substituted or unsubstituted 5-10 membered heteroaryl-substituted or unsubstituted C1-C4 alkyl-; more preferably, R₃ is hydrogen, halogen, -CN, hydroxyl, sulfhydryl, nitro, amino, F₃C-O-, F₃C-, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted C6-C8 aryl, substituted or unsubstituted 5-8 membered heteroaryl, substituted or unsubstituted C6-C8 aryl-substituted or unsubstituted C1-C2 alkyl-, substituted or unsubstituted 5-8 membered heteroaryl-substituted or unsubstituted C1-C2 alkyl-; more preferably, R₃ is hydrogen, substituted or unsubstituted C1-C6 alkyl, substituted or unsubstituted C3-C6 cycloalkyl, substituted or unsubstituted phenyl, substituted or unsubstituted phenyl-methyl-, substituted or unsubstituted pyridyl, substituted or unsubstituted pyridyl-methyl-, substituted or unsubstituted phenyl-ethyl-, substituted or unsubstituted pyridinyl-ethyl-; more preferably, R₃ is hydrogen, substituted or unsubstituted C1-C4 alkyl, substituted or unsubstituted C3-C6 cycloalkyl, substituted or unsubstituted phenyl, substituted or unsubstituted phenyl-methyl-, substituted or unsubstituted pyridyl, substituted or unsubstituted pyridyl-methyl-, substituted or unsubstituted phenyl-ethyl-, substituted or unsubstituted pyridinyl-ethyl-; more preferably, R₃ is hydrogen, substituted or unsubstituted methyl, substituted or unsubstituted ethyl, substituted or unsubstituted propyl, substituted or unsubstituted cyclopropyl, substituted or unsubstituted phenyl, substituted or unsubstituted phenyl-methyl-, substituted or unsubstituted pyridyl, substituted or unsubstituted pyridyl-methyl-, substituted or unsubstituted phenyl-ethyl-, substituted or unsubstituted pyridinyl-ethyl-;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

8. The compound of formula I, or an optical isomer thereof, or a racemate thereof, or a solvate thereof, or a pharmaceutically acceptable salt thereof of claim 1, wherein ring C is substituted or unsubstituted benzene ring, substituted or unsubstituted pyridine ring;
each "substituted" means that one or more (preferably 1, 2, 3, 4, 5, 6, 7, or 8) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, F₃C-O-, F₃C-, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

9. The compound of formula I, or an optical isomer thereof, or a racemate thereof, or a solvate thereof, or a pharmaceutically acceptable salt thereof of claim 1, wherein the compound of formula I is selected from the following group:

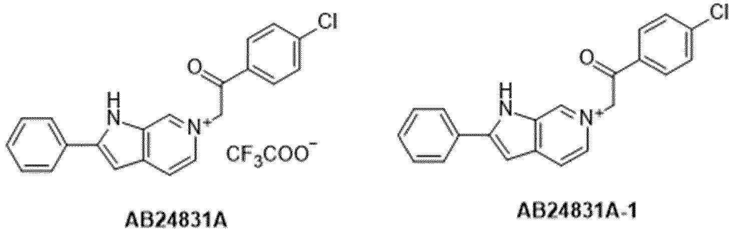

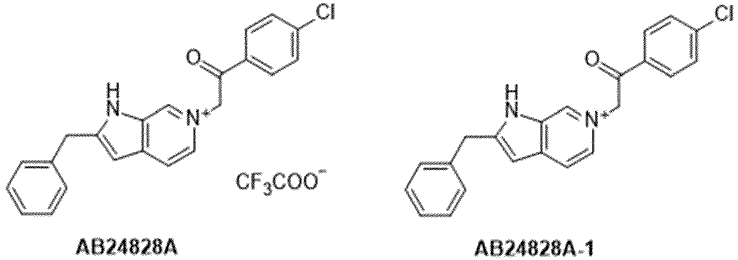

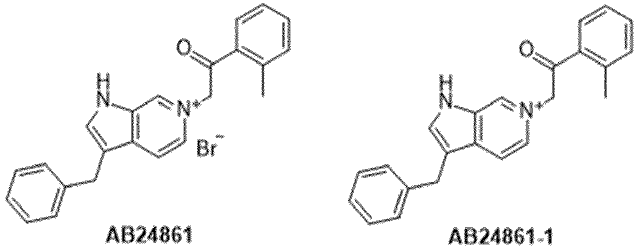

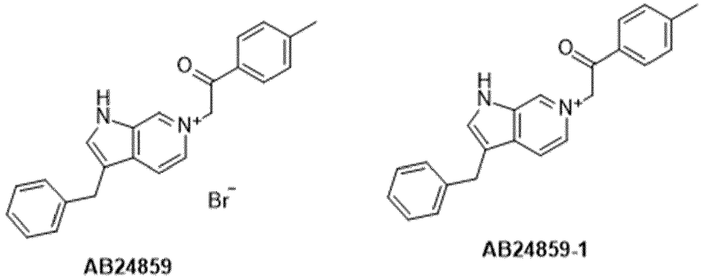

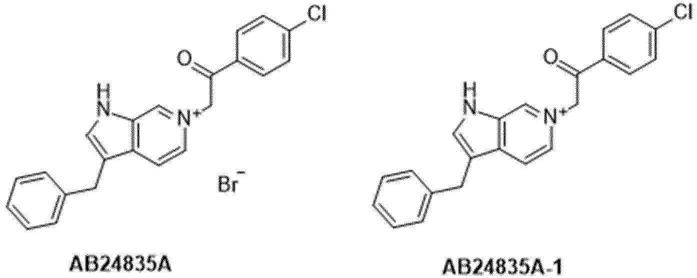

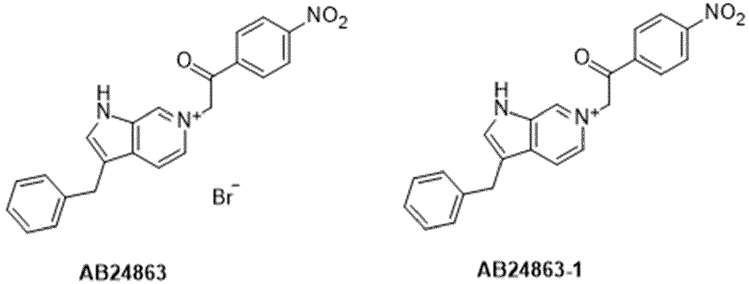

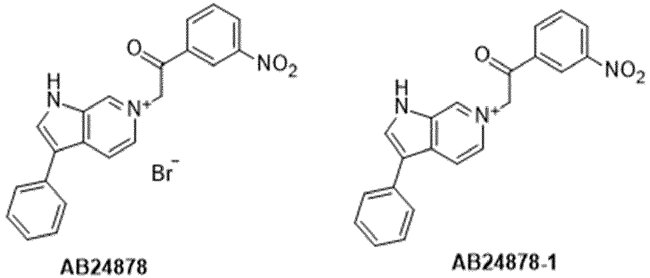

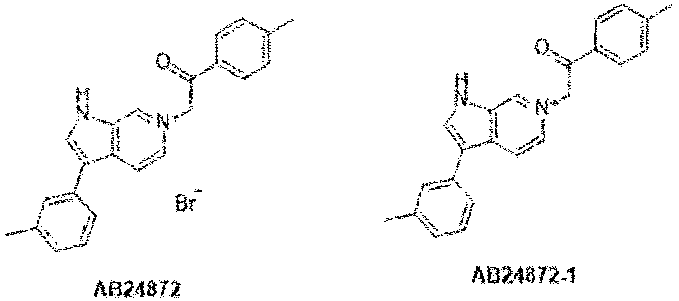

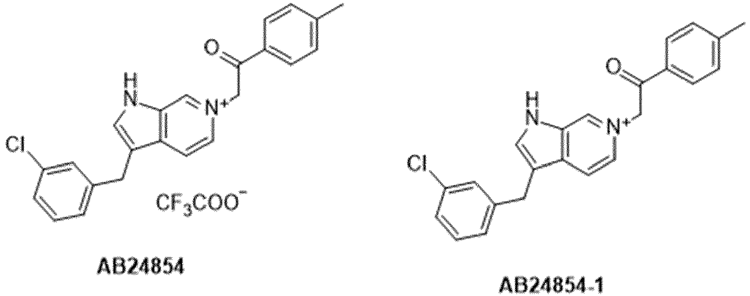

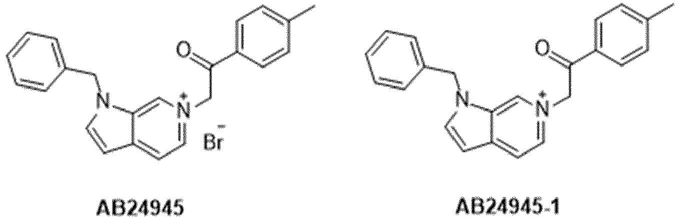

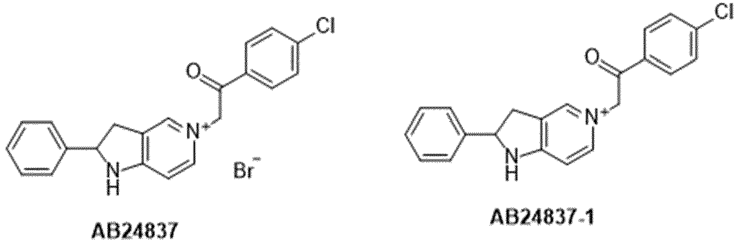

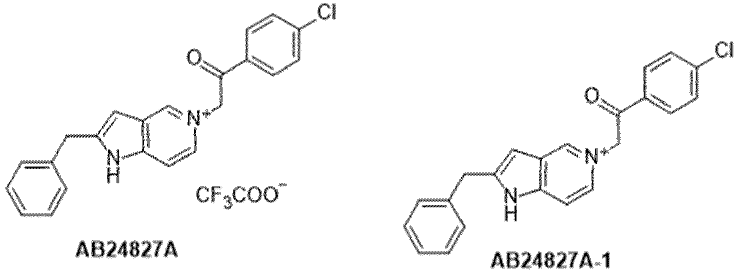

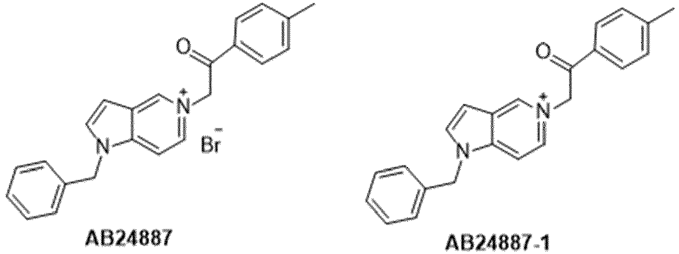

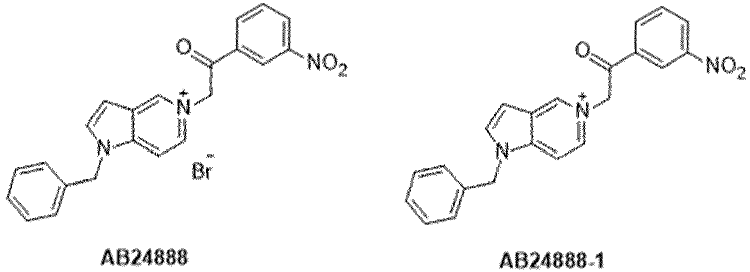

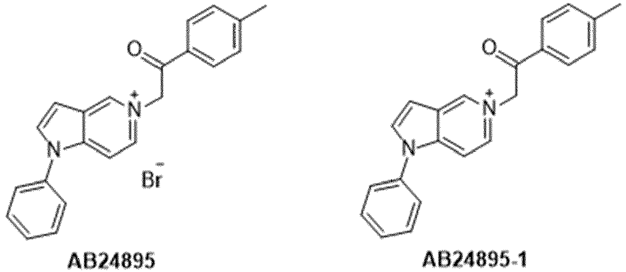

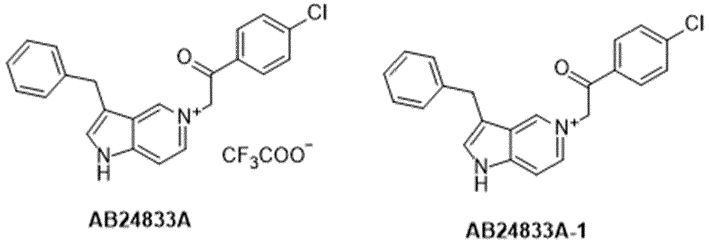

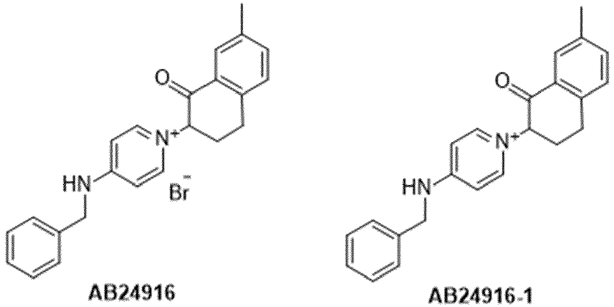

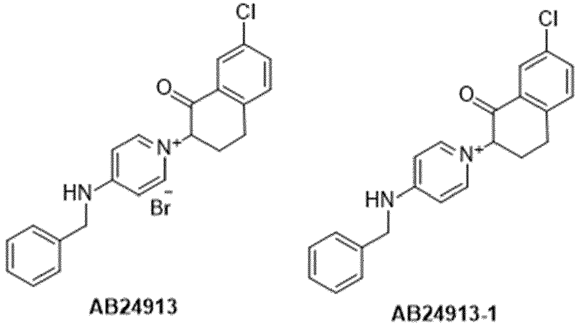

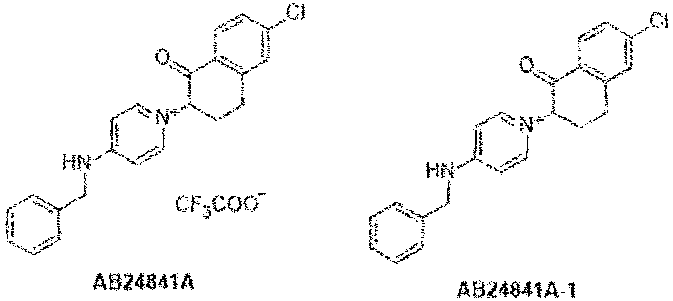

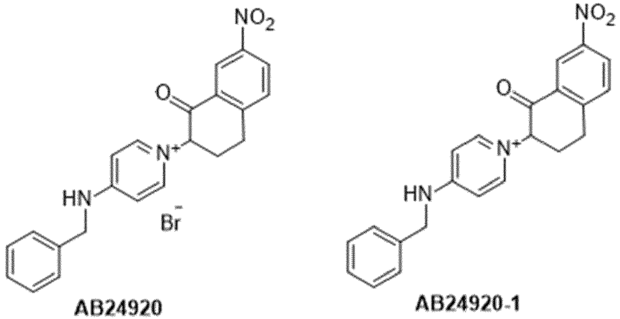

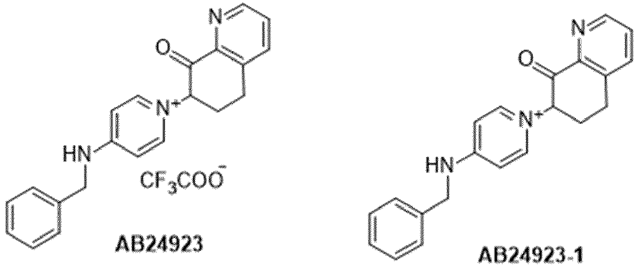

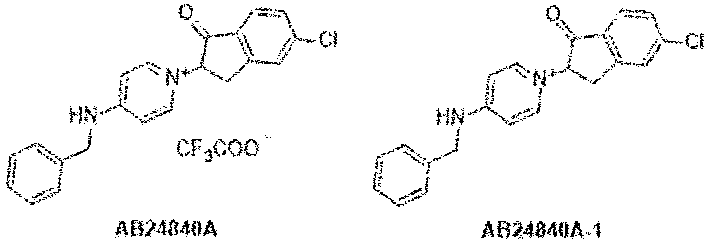

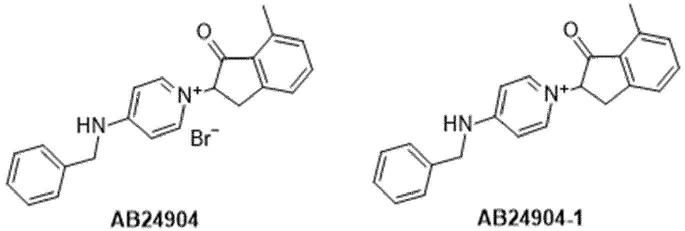

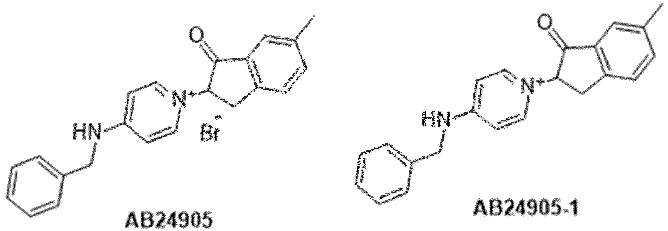

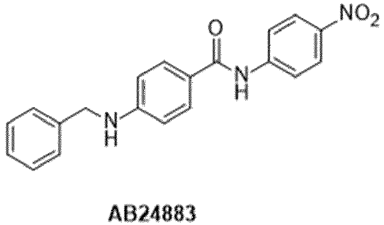

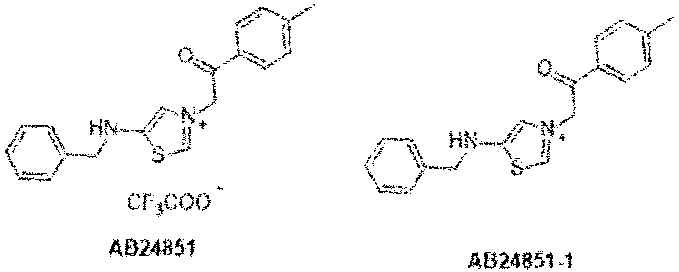

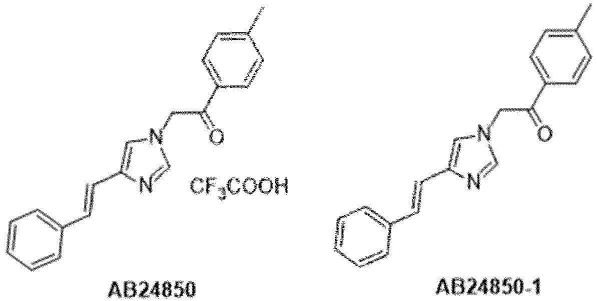

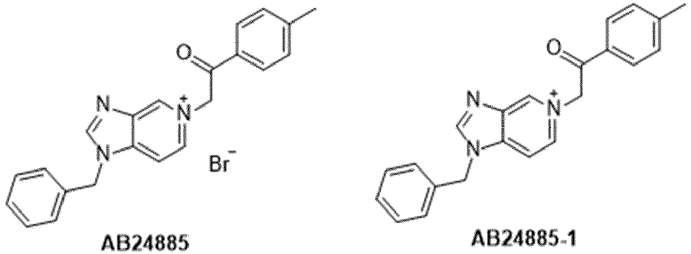

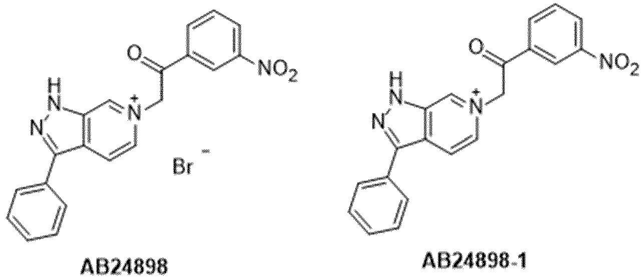

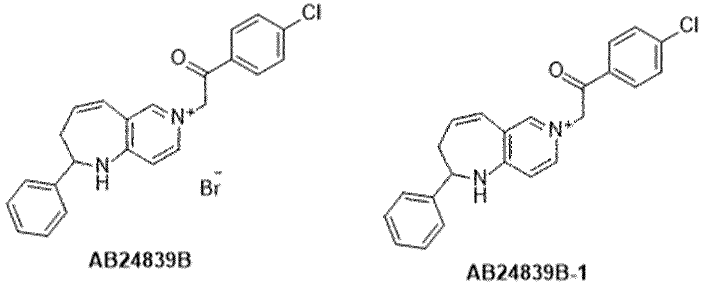

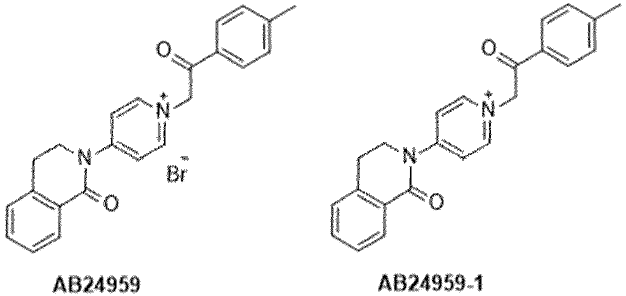

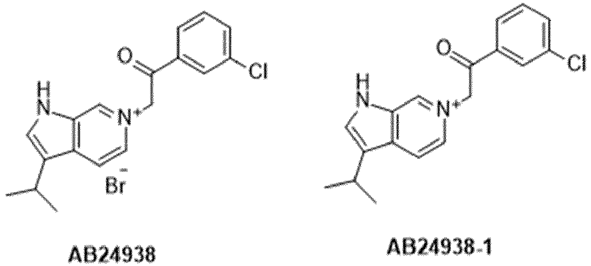

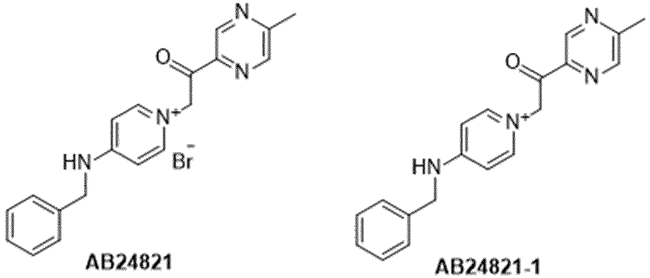

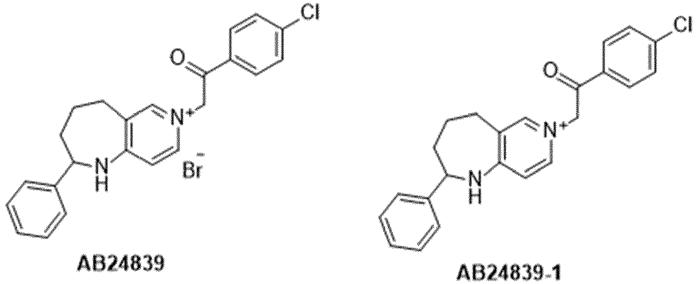

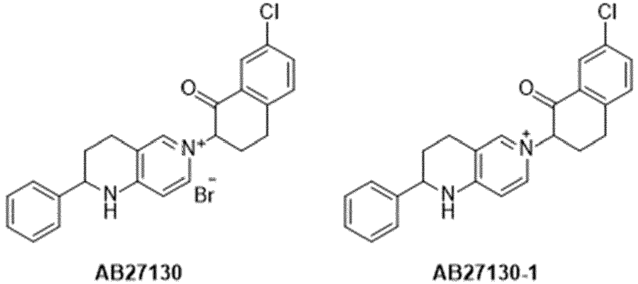

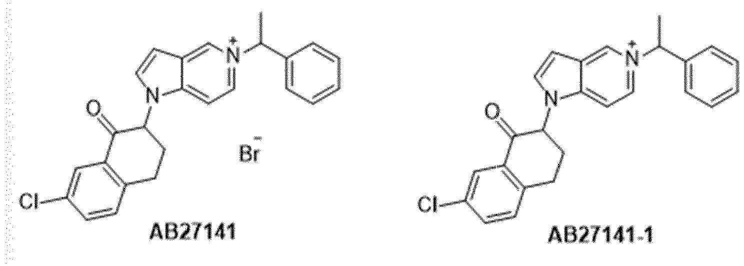

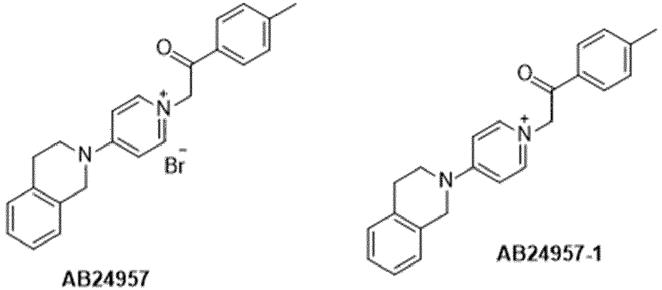

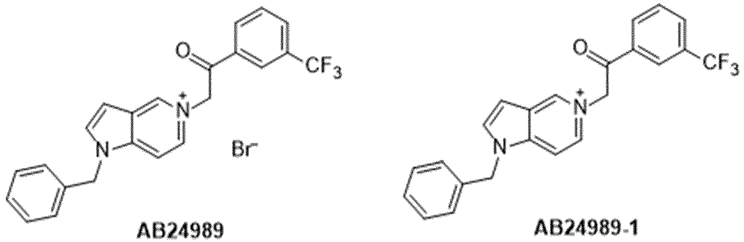

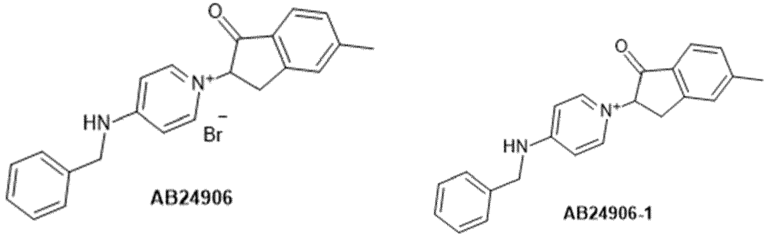

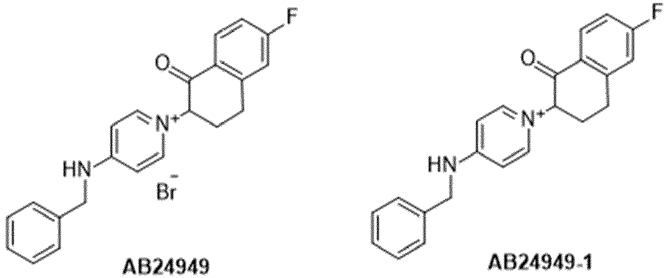

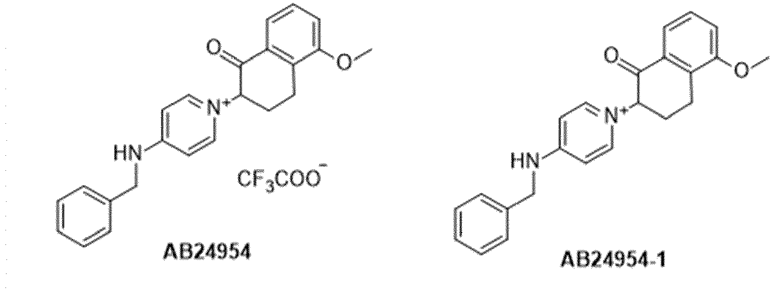

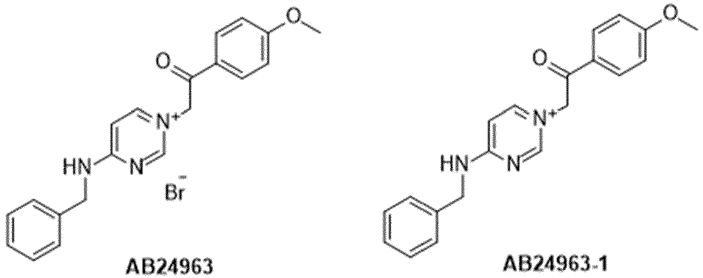

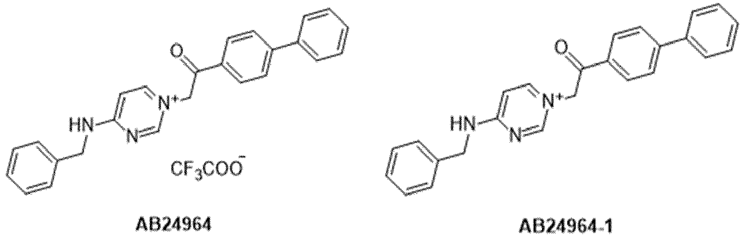

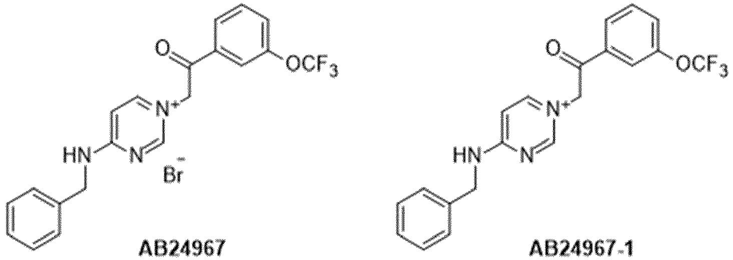

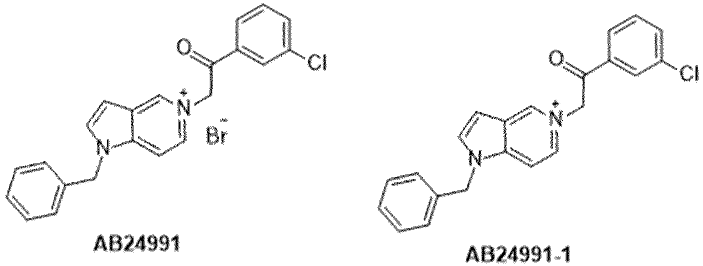

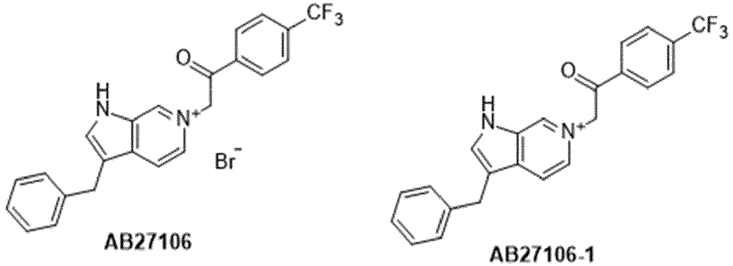

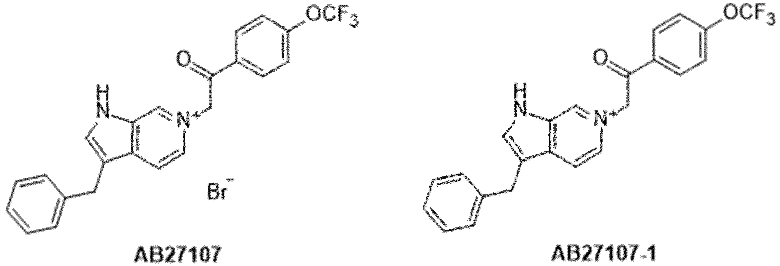

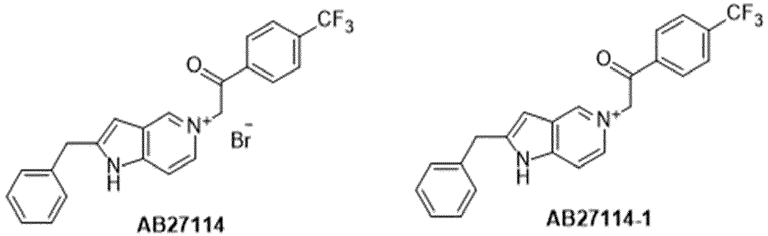

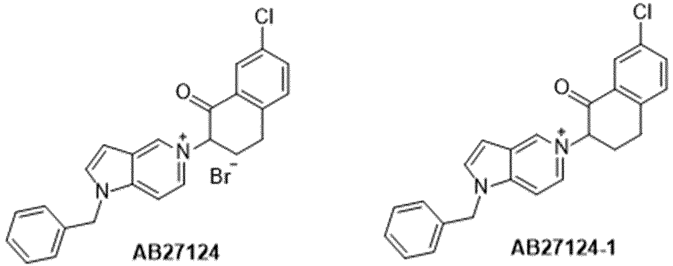

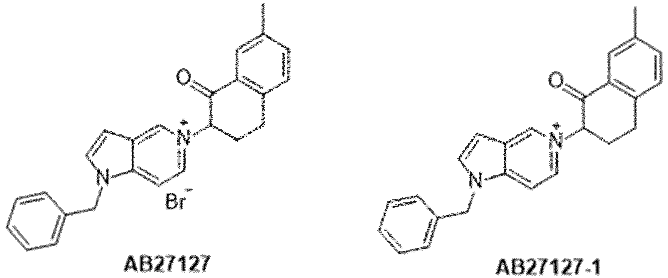

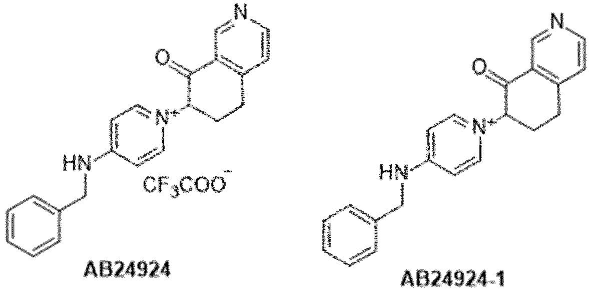

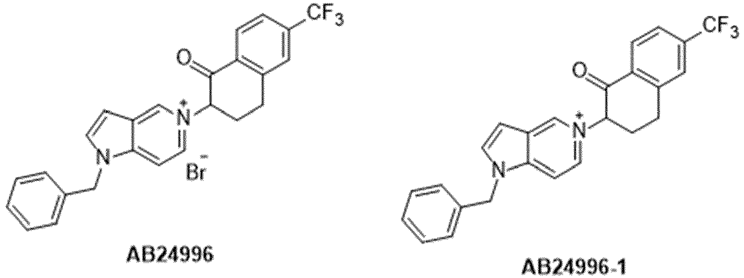

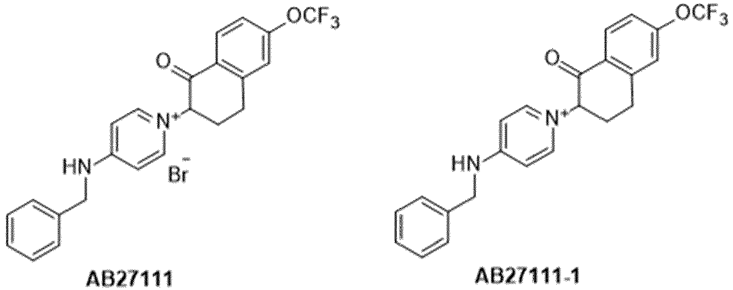

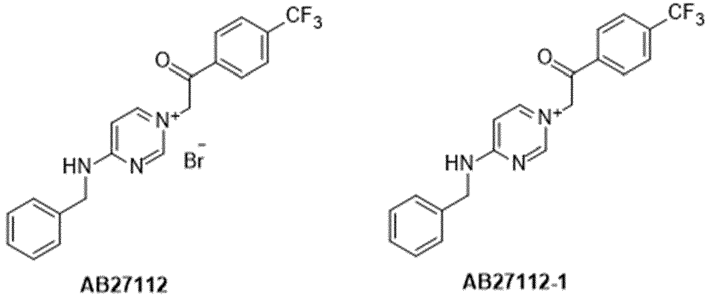

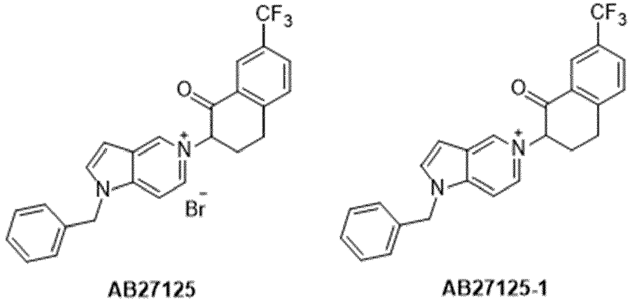

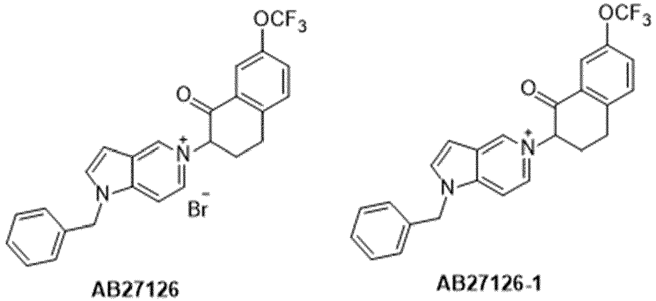

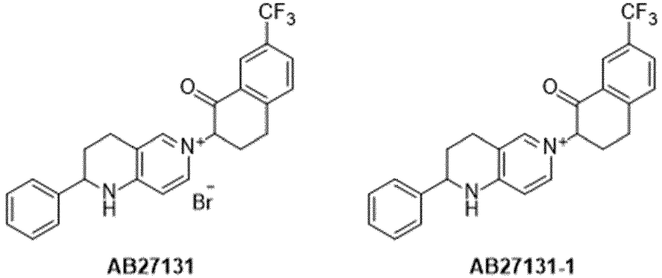

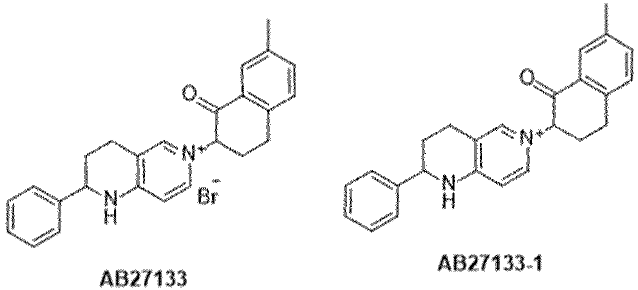

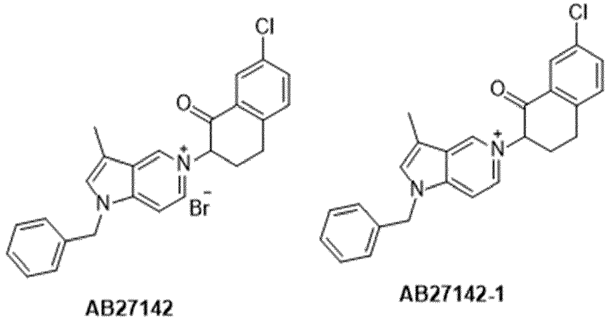

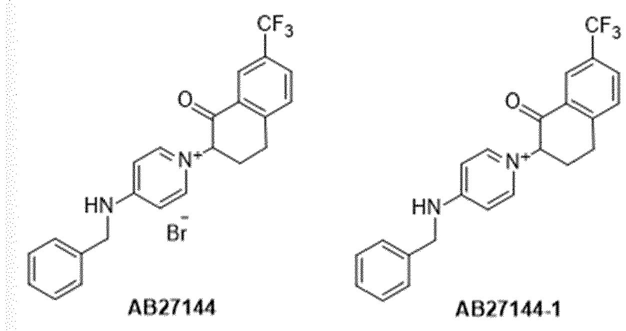

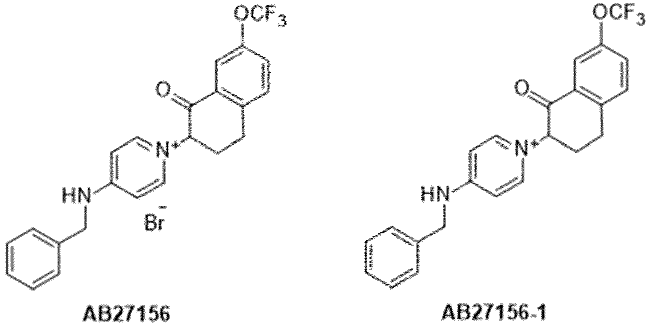

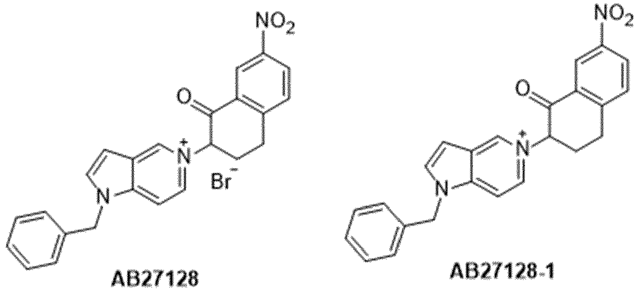

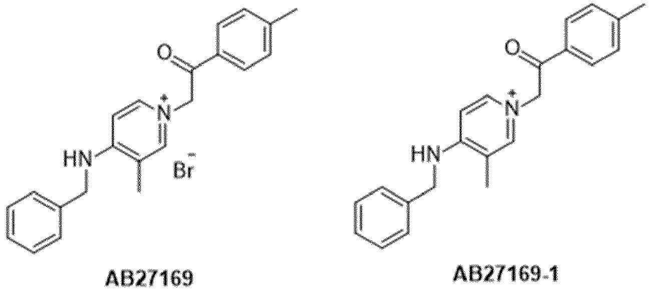

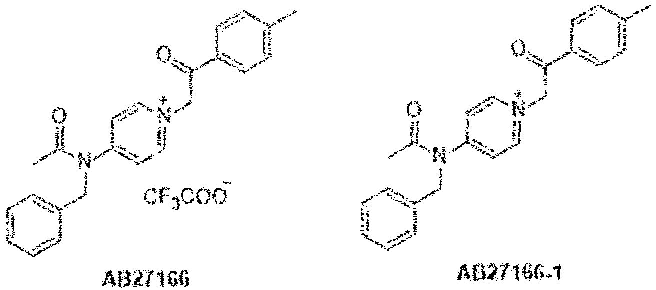

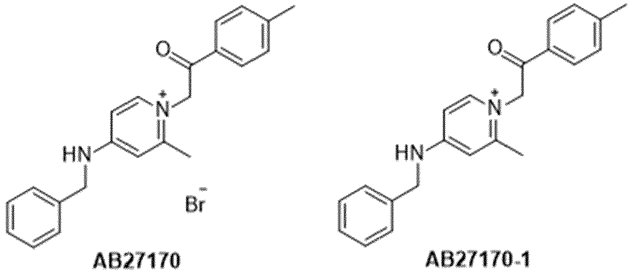

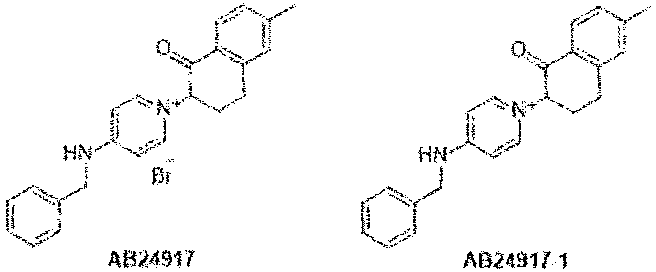

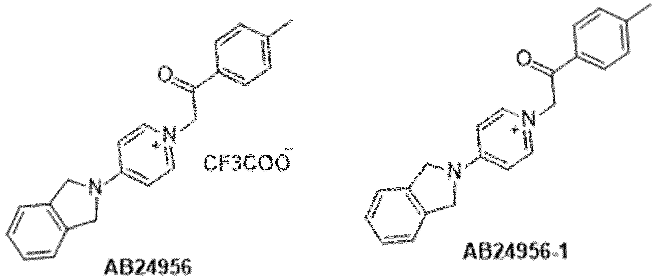

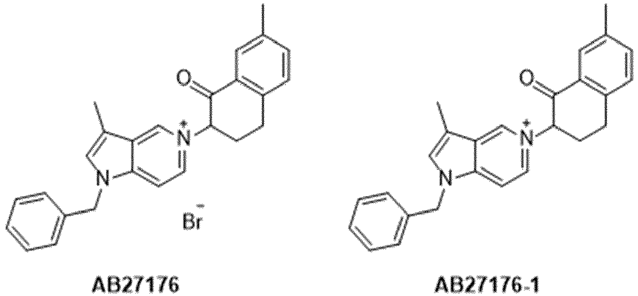

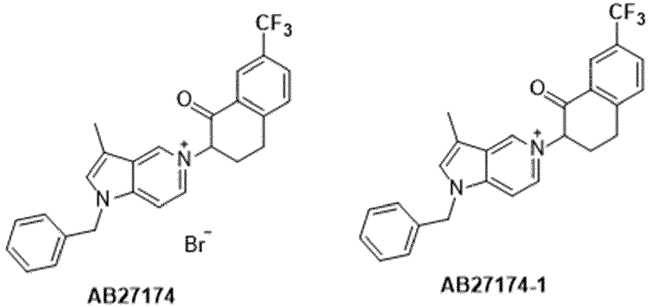

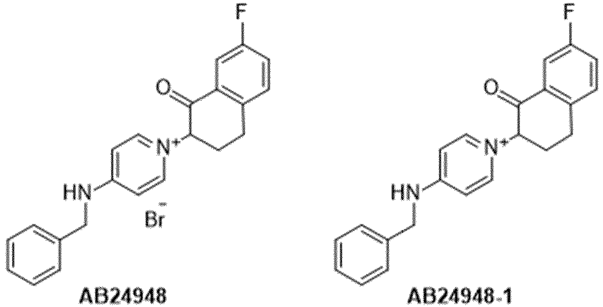

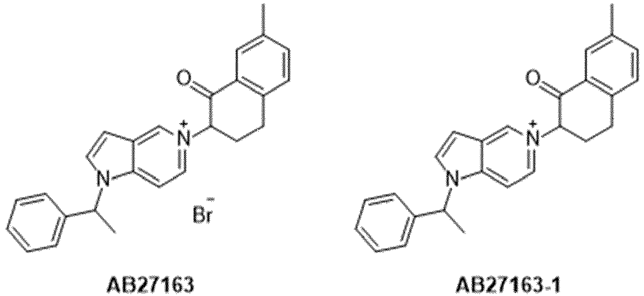

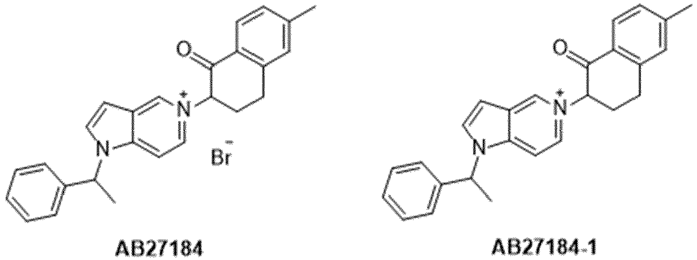

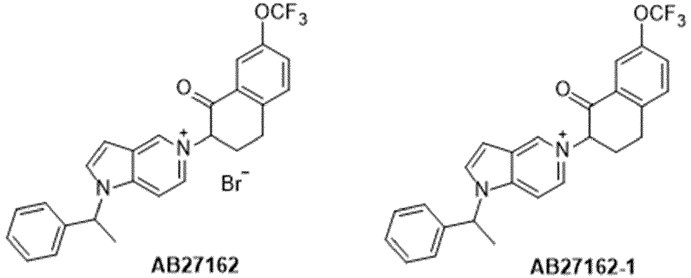

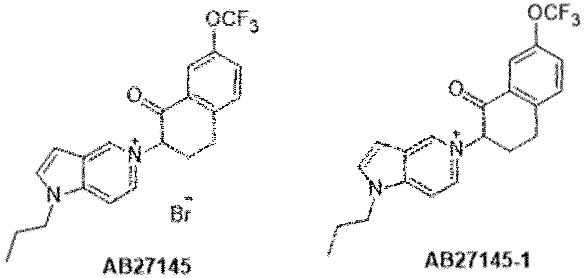

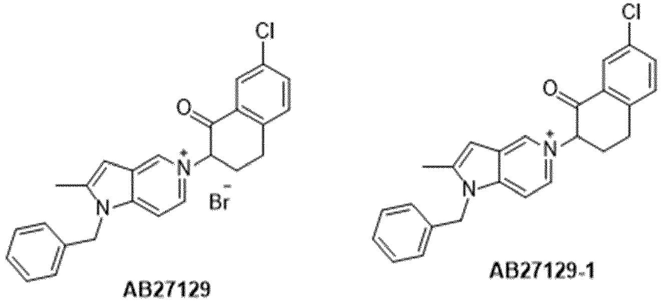

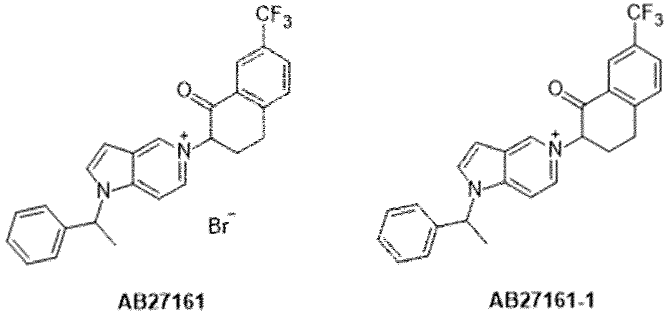

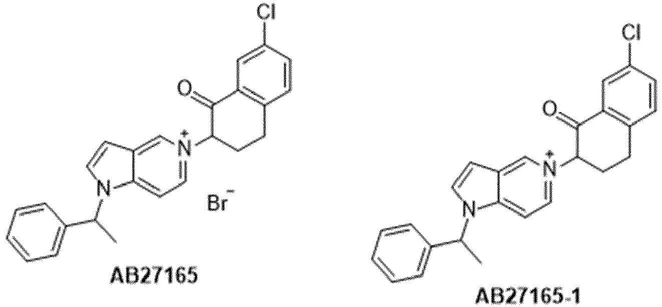

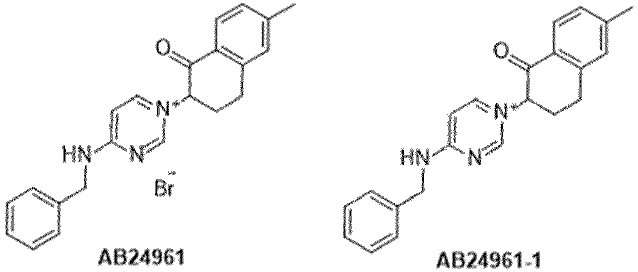

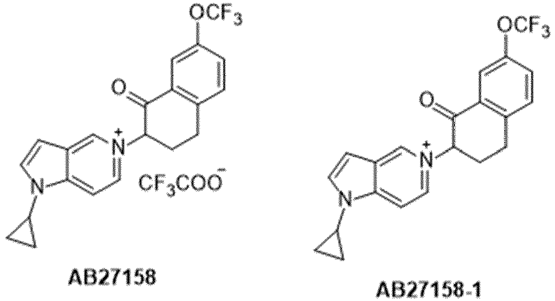

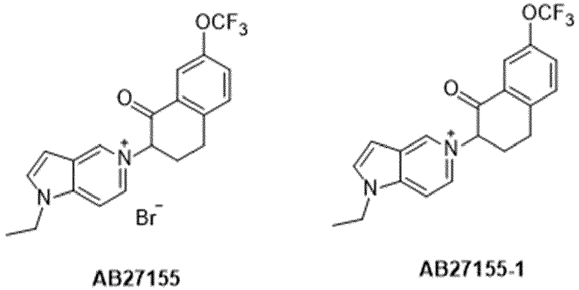

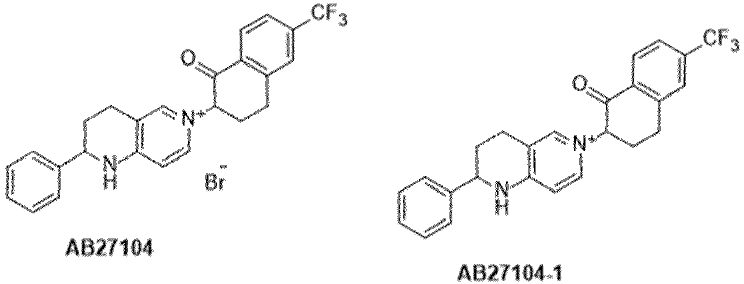

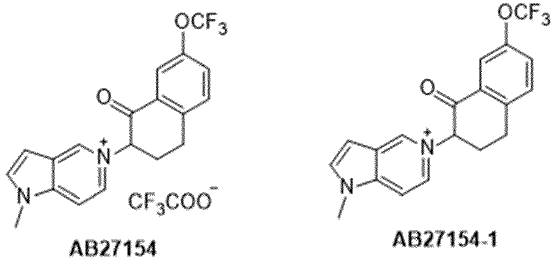

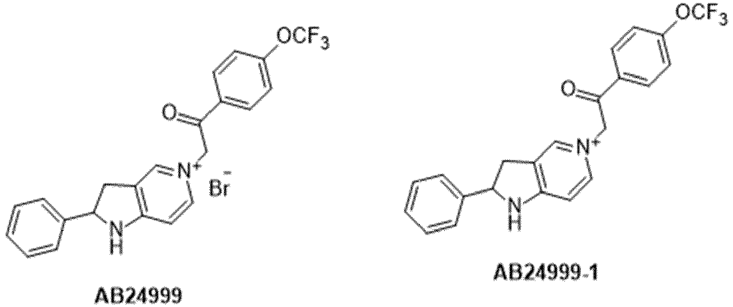

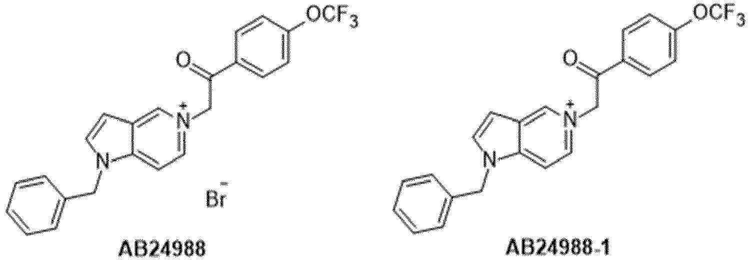

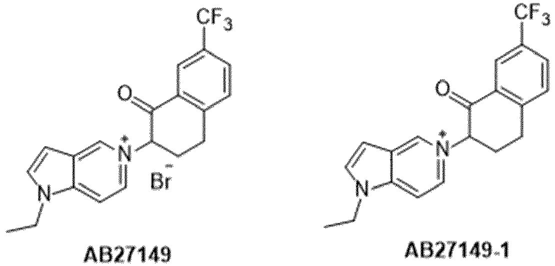

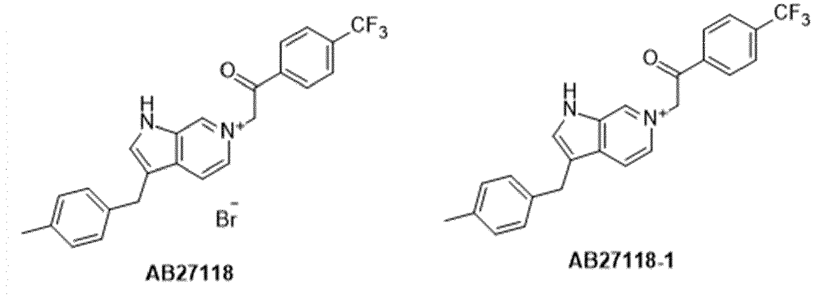

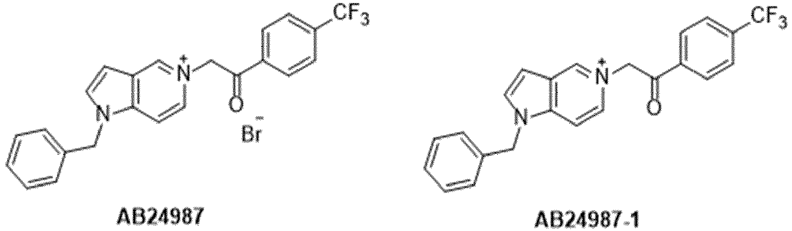

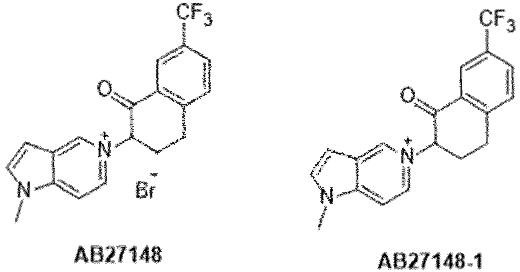

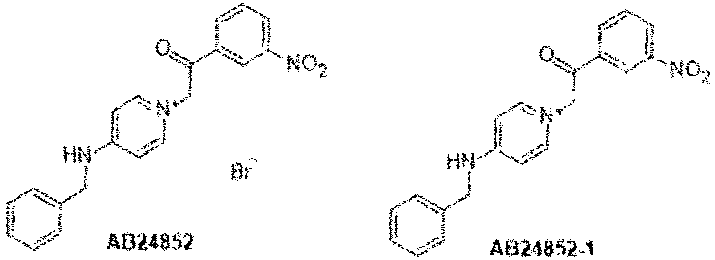

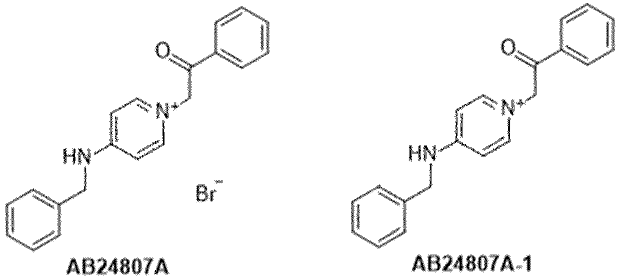

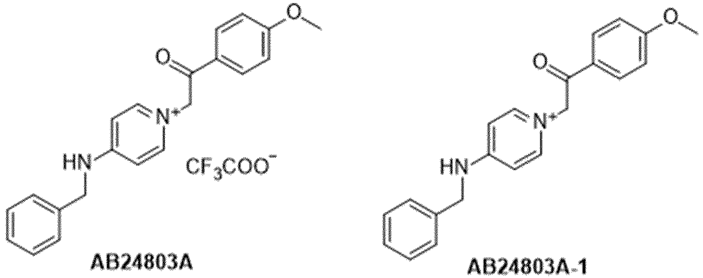

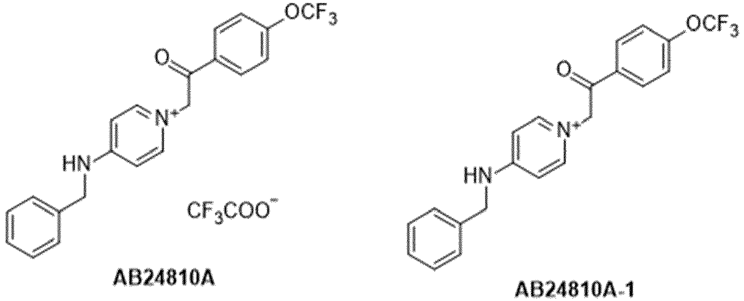

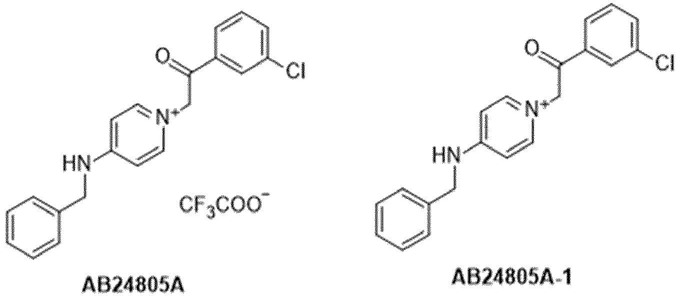

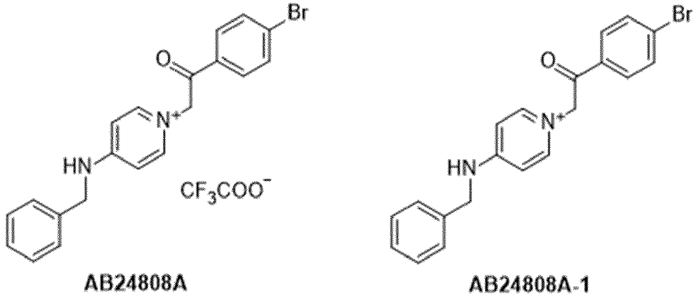

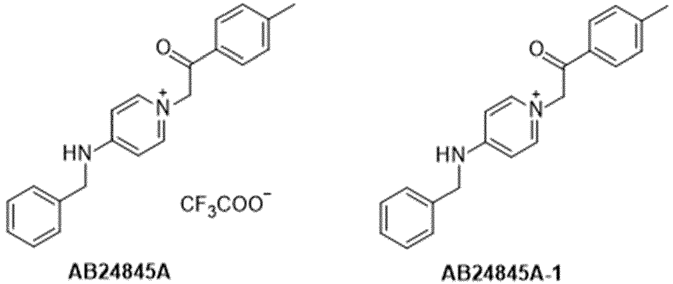

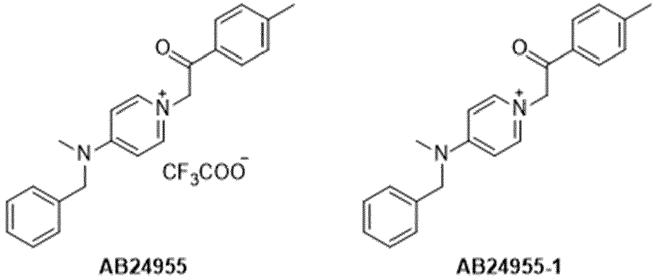

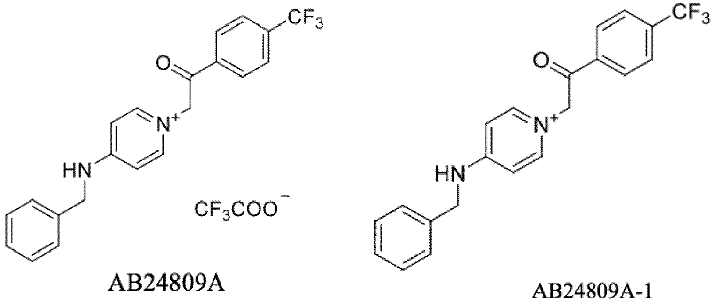

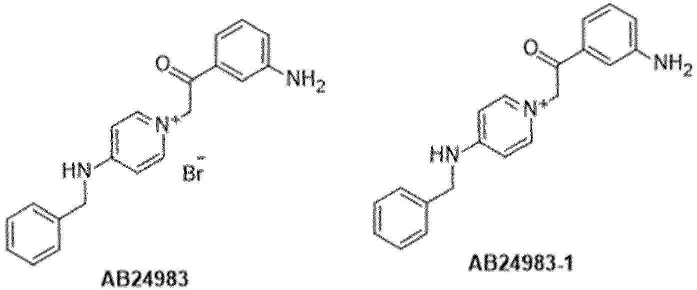

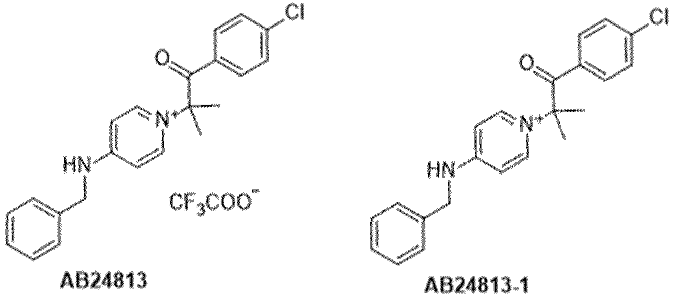

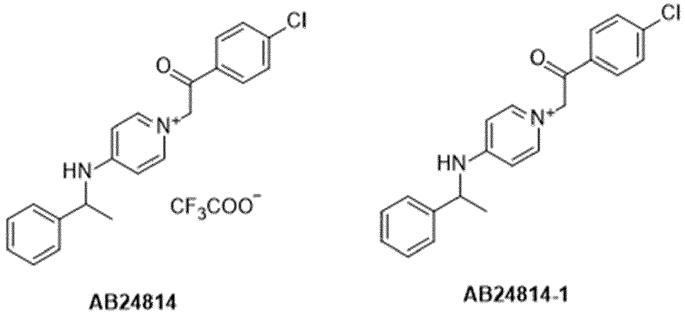

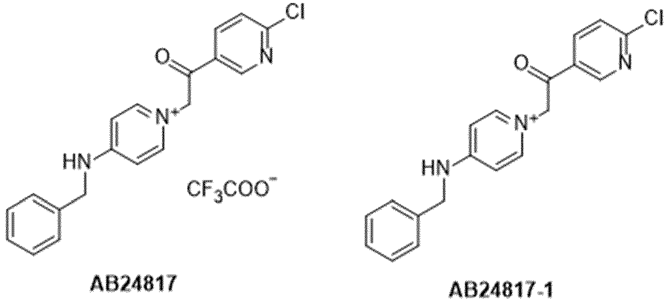

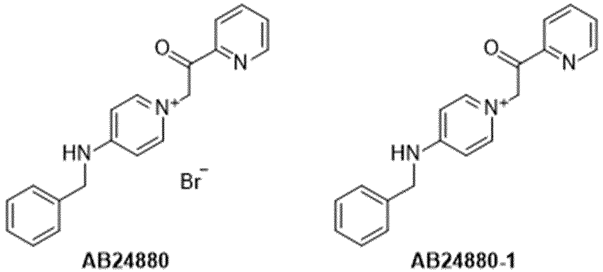

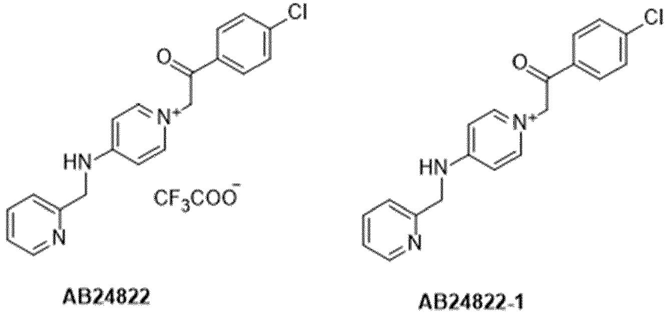

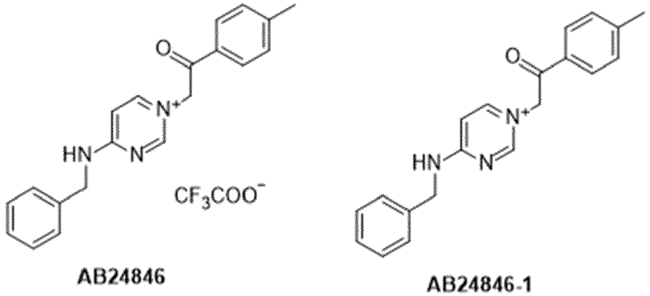

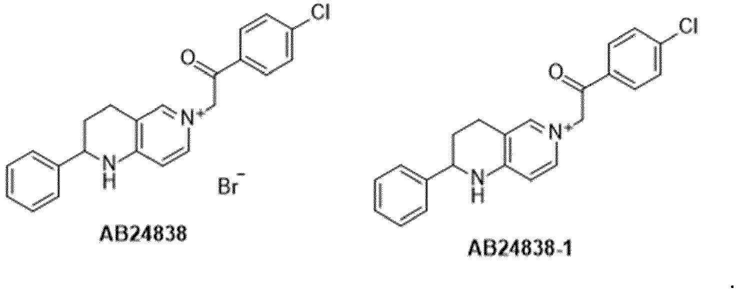

10. A use of the compound of formula I, or an optical isomer thereof, or a racemate thereof, or a solvate thereof, or a pharmaceutically acceptable salt thereof of any one of claims 1-9 in the preparation of a composition or a preparation for preventing and/or treating cancer.

11. The use of claim 10, wherein the cancer is selected from the group consisting of lung cancer, pancreatic cancer, breast cancer, lymphoma, prostate cancer, brain cancer, leukemia, liver cancer, melanoma, intestine cancer, renal carcinoma, colon cancer, and combinations thereof.

12. The use of claim 10, wherein the cancer is cancer with up-regulation of mitochondrial oxidative phosphorylation pathway in cancer cell;
the cancer is cancer with low activity of mitochondria permeability transition pore in cancer cell;
the cancer comprises tumor with low or no expression of NNMT gene; and/or
the cancer comprises tumor with high methylation level of DNA CpG site of NNMT gene.

13. The use of claim 12, wherein the up-regulation of mitochondrial oxidative phosphorylation pathway means that the ratio (E1/E0) of the level or expression E1 of mitochondrial oxidative phosphorylation pathway in a cell (e.g., cancer cell) to the level or expression E0 of mitochondrial oxidative phosphorylation pathway in a normal cell (the same type of cell) is >1.0, preferably ≥1.2, preferably ≥1.5, more preferably ≥2, more preferably ≥3, more preferably ≥5;
the low activity of mitochondria permeability transition pore means that the ratio (A1/A0) of the activity level or expression level A1 of mitochondria permeability transition pore in a cell (e.g., cancer cell) to the activity level or expression level A0 of mitochondria permeability transition pore in a normal cell (the same type of cell) is <1.0, preferably ≤0.8, preferably ≤0.7, more preferably ≤0.6, more preferably ≤0.5, more preferably ≤0.4, more preferably ≤0.3, more preferably ≤0.2, more preferably ≤0.1, most preferably ≤0.05;
the methylation level of DNA CpG site of NNMT gene comprises the methylation level of DNA CpG site in promoter region of NNMT gene;
the methylation level of DNA CpG site of NNMT gene refers to the methylation level of DNA CpG sites from 1050 bp before the transcription start site to 499 bp after the transcription start site in NNMT gene; and/or
the methylation level of DNA CpG site of NNMT gene refers to the methylation level of the DNA CpG sites from 1050 bp to 193 bp before the transcription start site in NNMT gene.

14. A marker for determining whether the compound of formula I, or an optical isomer thereof, or a racemate thereof, or a solvate thereof, or a pharmaceutically acceptable salt of claim 1 is suitable for use in the prevention and/or treatment of patient tumor, the marker comprises mitochondrial oxidative phosphorylation pathway, mitochondria permeability transition pore, NNMT gene, methylation level of nucleotide site of NNMT gene, and/or methylation level of DNA CpG site of NNMT gene.

15. A use of the detection kit in the preparation of concomitant diagnose kit for determining whether the compound of formula I, or an optical isomer thereof, or a racemate thereof, or a solvate thereof, or a pharmaceutically acceptable salt of claim 1 is suitable for use in the prevention and/or treatment of patient tumor;
the detection kit comprises:
(i) a detection reagent for detecting the level of mitochondrial oxidative phosphorylation pathway, level of mitochondria permeability transition pore, the expression of NNMT gene, and/or methylation level of DNA CpG site of NNMT gene.

16. A medicine kit, wherein the medicine kit comprises:
(i) a detection reagent for detecting the level of mitochondrial oxidative phosphorylation pathway, level of mitochondria permeability transition pore, expression level of NNMT gene, and/or methylation level of DNA CpG site of NNMT gene; and
(ii) the compound of formula I, or an optical isomer thereof, or a racemate thereof, or a solvate thereof, or a pharmaceutically acceptable salt of claim 1.

17. A method for preventing and/or treating tumor, which comprises administering the compound of formula I, or an optical isomer thereof, or a racemate thereof, or a solvate thereof, or a pharmaceutically acceptable salt thereof of any one of claims 1-9 to a subject in need.
